# FASCICULE DE BREVET EUROPEEN

(11) **EP 3 271 368 B1**
(45) Date de publication et mention de la délivrance du brevet: **31.10.2018**
(21) Numéro de dépôt: 17706525.7
(22) Date de dépôt: 13.01.2017
(51) Int. Cl.: A61K 31/66, A61K 45/06, A61K 9/00, C07F 9/6568, A61K 31/675, C07F 9/6584

(54) **NOUVEAUX DERIVES DE PHOSPHINANES ET AZAPHOSPHINANES, LEUR PROCEDE DE PREPARATION ET LES COMPOSITIONS PHARMACEUTIQUES QUI LES CONTIENNENT**
NEUARTIGE PHOSPHINAN- UND AZAPHOSPHINANDERIVATIVE, VERFAHREN ZUR HERSTELLUNG DAVON UND PHARMAZEUTISCHE ZUSAMMENSETZUNGEN DAMIT
NOVEL PHOSPHINANE AND AZAPHOSPHINANE DERIVATIVES, METHOD FOR PREPARING SAME AND PHARMACEUTICAL COMPOSITIONS CONTAINING SAME

(30) Priorité: 14.01.2016 FR 1670004
(43) Date de publication de la demande: 24.01.2018
(73) Titulaire: Les Laboratoires Servier, 92284 Suresnes (FR)
(72) Inventeur: GLOANEC, Philippe, 78160 Marly le Roi (FR); SCHAFFNER, Arnaud-Pierre, 78160 Marly le Roi (FR); SANSILVESTRI-MOREL, Patricia, 92160 Antony (FR); RUPIN, Alain, 37510 Savonnieres (FR); MENNECIER, Philippe, 78700 Conflans Sainte Honorine (FR); VALLEZ, Marie-Odile, 93100 Montreuil (FR)
(86) Numéro de dépôt international: PCT/FR2017/050075
(87) Numéro de publication internationale: WO 2017/121969

(56) Documents cités:
- US-A1- 2005 234 021

## Description

La présente invention a pour objet de nouveaux dérivés de phosphinanes et azaphosphinanes, leur procédé de préparation et les compositions pharmaceutiques qui les contiennent.

Les composés de l'invention sont des inhibiteurs du TAFIa (activated thrombin-activatable fibrinolysis inhibitor).

Le TAFI (aussi nommé procarboxypeptidase B plasmatique, procarboxypeptidase R ou procarboxypeptidase U) est une glycoprotéine plasmatique de 60 kDa produite par le foie qui circule sous la forme d'un zymogène. Pendant la coagulation et la fibrinolyse sanguine, la thrombine et la plasmine clivent le prosegment du TAFI au niveau de la liaison Arg92-Ala93 pour le transformer en une enzyme active, le TAFIa, dont la demi-vie est de 8 à 15 minutes à 37°C. Le clivage du prosegment par la thrombine est accéléré par la thrombomoduline, un cofacteur présent dans le plasma et à la surface des cellules vasculaires endothéliales (Bouma BN and Meijers JC, Thrombin-activatable fibrinolysis inhibitor, 2003, Journal of Thrombosis and Haemostasis, 1 : 1566-1574). Le TAFIa régule négativement la fibrinolyse en clivant les résidus lysine C-terminaux des fibres de fibrine qui apparaissent lors de la dégradation partielle de la fibrine par les premières traces de plasmine. Ces résidus lysine C-terminaux sur la fibrine partiellement dégradée se comportent comme des ligands du plasminogène plasmatique circulant et de l'activateur tissulaire du plasminogène (tPA) généré par les cellules endothéliales lors de l'ischémie thrombotique. Ils permettent ainsi de localiser la transformation du plasminogène en plasmine par le tPA sans interférence ni avec l'inhibiteur circulant de la plasmine l'α2-antiplasmine ni avec l'inhibiteur circulant de l'activateur tissulaire du plasminogène (PAI-1). Le clivage des sites lysine C-terminaux par le TAFIa diminue donc la vitesse de génération de la plasmine. La fibrinolyse endogène est alors inhibée et la lyse des thromboses artérielles et veineuses fibrineuses ainsi que la thrombolyse thérapeutique entreprise chez des patients en phase aiguë ischémique post-thrombotique sont également diminuées. Des inhibiteurs de TAFIa ont donc le potentiel d'augmenter la fibrinolyse endogène et thérapeutique et de se comporter comme des agents antithrombotiques et profibrinolytiques sans risque hémorragique majeur puisqu'ils n'interfèrent ni avec l'activation plaquettaire ni avec la coagulation lors de l'hémostase sanguine.

La propriété d'inhibition du TAFIa permet donc d'envisager l'utilisation des composés de l'invention dans le traitement et la prévention des événements thrombotiques chez les patients à risque.
Leur utilisation sera intéressante dans le traitement, la prévention et la prévention secondaire des complications vasculaires, plus particulièrement cardiovasculaires, pulmonaires et cérébrovasculaires liées aux maladies athérothrombotiques, à l'athérosclérose, au diabète, à l'hyperlipidémie, à l'hypertension, aux maladies veineuses chroniques, au syndrome métabolique lié à l'obésité ou au cancer.
Les composés selon l'invention sont particulièrement utiles pour le traitement, la prévention et la prévention secondaire de l'infarctus du myocarde, de l'angine de poitrine, des accidents vasculaires cérébraux, des anévrismes aortiques, de l'artérite des membres inférieurs, des maladies fibrotiques, des thromboses veineuses et de l'embolie pulmonaire.

Les facteurs de risques vasculaires et les maladies vasculaires tels que l'hypertension, l'obésité, le diabète, les maladies cardiaques, les maladies cérébrovasculaires et l'hyperlipidémie et donc l'athérosclérose jouent un rôle dans la genèse des démences telles que la maladie d'Alzheimer et la démence vasculaire (Qiu C., De Ronchi D. Et Fratiglioni L., The epidemiology of the dementias : an update, 2007, Current Opinion in Psychiatry, 20 : 380-385). Les composés de l'invention seront donc également utiles pour le traitement et/ou la prévention des démences telles que la maladie d'Alzheimer et la démence vasculaire.

Le TAFIa diminue le potentiel fibrinolytique endogène. En tant qu'inhibiteurs de TAFIa, les composés de la présente invention sont donc utiles pour accompagner le traitement aigu par les fibrinolytiques injectables, tels que le tPA recombinant (par exemple altéplase, tenecteplase, reteplase, desmoteplase), l'uPA recombinant ou la streptokinase, qui sont utilisés en situation d'urgence (par exemple infarctus du myocarde, accident vasculaire cérébral).
Les composés de la présente invention renforcent l'activité de ces fibrinolytiques injectables et conduisent donc à leur utilisation avec moins de risques hémorragiques et neurotoxiques (diminution de leur dose et donc réduction de leurs effets secondaires).
US 2005/0234021 A1 décrit l'utilisation de composés aminophosphonates et aminobisphosphonates dans le traitement de diverses maladies, dont les maladies cardiovasculaires.

La présente invention concerne plus spécialement les composés de formule (I) : 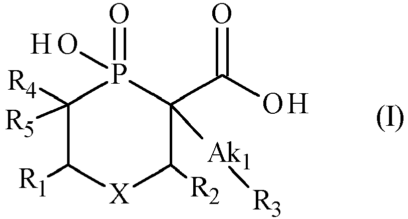 dans laquelle :
Ak₁ représente une chaîne alkyle en C₁-C₆,
X représente -(CH₂)ₘ-, -CH(R)-, -N(R)-, -CH₂-N(R)-, -N(R)-CH₂- ou -CH₂-N(R)-CH₂-,
   m représente 0 ou un entier de 1 à 4,
   R représente un atome d'hydrogène ou un groupe choisi parmi alkyle C₁-C₆, -Ak₂-Ar₁, -Ak₂-Ar₁-Ar₂ et -Ak₂-Ar₁-O-Ar₂, -Ak₂-cycloalkyle ou -Ak₂-OH,
      Ak₂ représente une chaîne alkyle linéaire ou ramifiée en C₁-C₆,
      Ar₁ et Ar₂, identiques ou différents, représentent chacun un groupement aryle ou hétéroaryle,
R₁ et R₂ représentent chacun un atome d'hydrogène lorsque X représente -(CH₂)ₘ-, -CH(R)-,-N(R)-, -CH₂-N(R)- ou -N(R)-CH₂-,
ou bien forment ensemble une liaison lorsque X représente -CH₂-N(R)-CH₂-,
R₃ représente NH₂, Cy-NH₂, Cy-Ak₃-NH₂ ou pipéridin-4-yle,
   Cy représente un groupement choisi parmi cycloalkyle, aryle et hétéroaryle,
   Ak₃ représente une chaîne alkyle en C₁-C₃,
R₄ et R₅, identiques ou différents, représentent chacun un atome d'hydrogène ou un atome de fluor,
leurs isomères optiques, ainsi que leurs sels d'addition à un acide pharmaceutiquement acceptable.

Par groupement aryle, on entend phényle, naphtyle ou biphényle éventuellement substitué par un ou plusieurs groupements, identiques ou différents, choisis parmi halogène, hydroxy, amino, alkyle (C₁-C₆) linéaire ou ramifié éventuellement substitué par un ou plusieurs atomes d'halogène, méthylsulfonyle, méthylthio, carboxy, alkoxy (C₁-C₆) linéaire ou ramifié éventuellement substitué par un ou plusieurs atomes d'halogène, aminoalkyle (C₁-C₆) linéaire ou ramifié, le groupement amino du groupement aminoalkyle étant éventuellement substitué par un ou deux groupements alkyle (C₁-C₆) linéaire ou ramifié.

Par groupement hétéroaryle, on entend un groupement monocyclique aromatique ou un groupement bicyclique aromatique ou partiellement aromatique, de 5 à 12 chaînons, contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, hydroxy, amino, oxo, alkyle (C₁-C₆) linéaire ou ramifié éventuellement substitué par un ou plusieurs atomes d'halogène, alkoxy (C₁-C₆) linéaire ou ramifié éventuellement substitué par un ou plusieurs atomes d'halogène, aminoalkyle (C₁-C₆) linéaire ou ramifié, le groupement amino du groupement aminoalkyle étant éventuellement substitué par un ou deux groupements alkyle (C₁-C₆) linéaire ou ramifié.

Parmi les groupements hétéroaryle, on peut citer à titre non limitatif les groupements pyridyle, thiényle, furyle, imidazolyle, pyrimidinyle, pyrazolyle, pyrrolyle, oxazolyle, isoxazolyle, thiazolyle, isothiazolyle, pyrazinyle, pyridazinyle benzofuryle, benzothiényle, benzimidazolyle, imidazopyridinyle, isoquinoléinyle, dihydrobenzo furyle, dihydrobenzoxazolyle, dihydroindolyle, dihydroindazolyle, benzodioxolyle.

Par groupement cycloalkyle, on entend un groupement hydrocarboné monocyclique, saturé, de 5 à 7 chaînons, étant entendu que le cycle peut être éventuellement substitué par un ou plusieurs groupements, identiques ou différents, choisis parmi halogène, alkyle (C₁-C₆) linéaire ou ramifié. Parmi les groupements cycloalkyle, on peut citer à titre non limitatif les groupements cyclopentyle, cyclohexyle, cycloheptyle.

Par isomères optiques, on entend les diastéréoisomères et les énantiomères.
Les composés de formule (I) possèdent au moins un centre asymétrique : 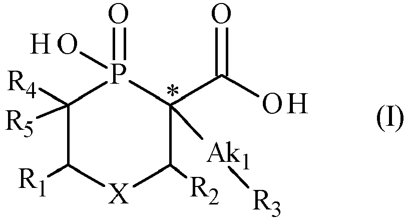 Lorsque la configuration d'un composé de formule (I) présentant un seul centre asymétrique n'est pas précisée, celui-ci est obtenu sous la forme d'un mélange des deux énantiomères. Lorsque la configuration d'un composé de formule (I) présentant deux centres asymétriques n'est pas précisée, celui-ci est obtenu sous la forme d'un mélange de diastéréoisomères.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, benzènesulfonique, *p*-toluènesulfonique, camphorique.

Un aspect de la présente invention concerne les composés de formule (I) pour lesquels Ak₁ représente -(CH₂)₄-.

Un autre aspect de la présente invention concerne les composés de formule (I) pour lesquels X représente -N(R)-.

Un autre aspect de la présente invention concerne les composés de formule (I) pour lesquels X représente -N(R)-, -CH₂-N(R)-, -N(R)-CH₂- ou -CH₂-N(R)-CH₂-, et R représente un groupe choisi parmi -Ak₂-Ar₁, -Ak₂-Ar₁-Ar₂ et -Ak₂-Ar₁-O-Ar₂.

Un autre aspect de la présente invention concerne les composés de formule (I) pour lesquels X représente -N(R)-, -CH₂-N(R)-, -N(R)-CH₂- ou -CH₂-N(R)-CH₂-, R représente un groupe choisi parmi -Ak₂-Ar₁, -Ak₂-Ar₁-Ar₂ et -Ak₂-Ar₁-O-Ar₂, et Ak₂ représente -CH₂-.

Un autre aspect de la présente invention concerne les composés de formule (I) pour lesquels R₁ et R₂ représentent chacun un atome d'hydrogène.

Un autre aspect de la présente invention concerne les composés de formule (I) pour lesquels R₃ représente NH₂.

Un autre aspect de la présente invention concerne les composés de formule (I) pour lesquels R₄ et R₅ représentent chacun un atome d'hydrogène.

Un autre aspect de la présente invention concerne les composés de formule (I) pour lesquels R₁, R₂, R₄ et R₅ représentent chacun un atome d'hydrogène, R₃ représente NH₂, X représente -N(R)-, -CH₂-N(R)-, -N(R)-CH₂- ou -CH₂-N(R)-CH₂-, et R représente un groupe choisi parmi -Ak₂-Ar₁, -Ak₂-Ar₁-Ar₂ et -Ak₂-Ar₁-O-Ar₂.

Un autre aspect de la présente invention concerne les composés de formule (la), cas particulier des composés de formule (I) : 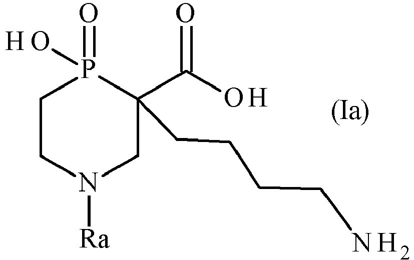 dans laquelle Ra représente un groupe choisi parmi -CH₂-Ar₁ et -CH₂-Ar₁-Ar₂, où Ar₁ et Ar₂ sont tels que définis dans la formule (I).

La présente invention a également pour objet le procédé de préparation des composés de formule (I) à partir du composé de formule (II) : 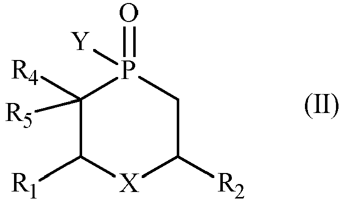 où X, R₁, R₂, R₄ et R₅ sont tels que défini dans la formule (I), Y représente un groupement alkoxy C₁-C₄ linéaire ou ramifié ou dialkylamino dans lequel les groupements alkyle sont C₁-C₄, linéaires ou ramifiés,
que l'on met en réaction avec CO(OG)₂, où G représente un groupement protecteur de la fonction acide tel qu'alkyle ou benzyle, préférentiellement tert-butyle ou benzyle,
en présence d'une base, pour conduire au composé de formule (III) : 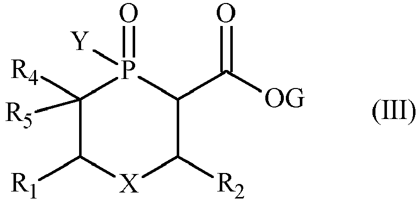 où X, Y, R₁, R₂ et G sont tels que définis précédemment,
que l'on met en réaction, en présence d'une base, avec le composé Br-Ak₁-R'₃, où Ak₁ est tel que défini dans la formule (I), et R'₃ représente N(Boc)₂, Cy-N(Boc)₂, Cy-Ak₃-N(Boc)₂ ou N-Boc-pipéridin-4-yle,
pour conduire au composé de formule (IV) : 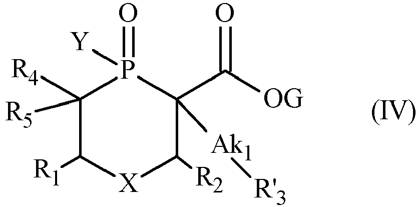 où X, Y, R₁, R₂, G, Ak₁ et R'₃ sont tels que définis précédemment,
dont on déprotège les fonctions amino, carboxy et phosphinique, pour conduire au composé de formule (I) ou à l'un de ses sels d'addition à un acide pharmaceutiquement acceptable.

Le composé de formule (IV) comporte au moins deux centres asymétriques : 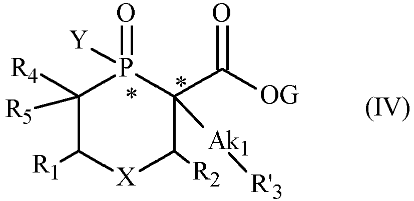 Les diastéroioisomères du composé de formule (IV) peuvent être séparés à l'aide d'une colonne chirale, permettant l'obtention des composés optiquement purs de formule (I) par le procédé décrit ci-dessus.

La nomenclature (3a*R**, 4*S***,* 6a*S**), utilisée pour les dérivés d'octahydrophospholo[3,4-c]pyrroles, indique une configuration relative. Elle signifie que le composé est sous la forme d'un mélange des composés de configurations absolues (3a*R*, 4S, 6a*S*) et (3a*S*, 4*R*, 6a*R*).

La présente invention a également pour objet le procédé de préparation des composés de formule (la), cas particulier des composés de formule (I),
à partir du composé de formule (IVa), cas particulier des composés de formule (IV) : 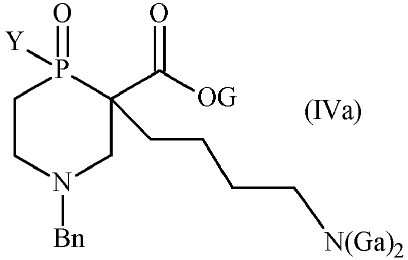 où Y et G sont tels que définis dans la formule (II), et Ga représente un groupement protecteur de la fonction amino tel que Boc,
que l'on débenzyle, pour conduire au composé de formule (V) : 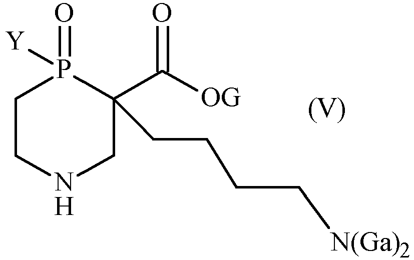 où Y, G et Ga sont tels que définis précédemment,
que l'on soumet à une réaction d'amination réductrice avec l'aldéhyde de formule R₆-CHO, où R₆ représente -Ar₁ ou -Ar₁-Ar₂, où Ar₁ et Ar₂ sont tels que définis dans la formule (I), pour conduire au composé de formule (VI) : 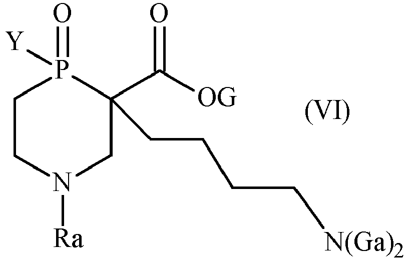 où Y, G, Ga et Ra sont tels que définis précédemment,
dont on déprotège les fonctions amino, carboxy et phosphinique, pour conduire au composé de formule (la) ou à l'un de ses sels d'addition à un acide pharmaceutiquement acceptable.

Le composé de formule (IVa) comporte deux centres asymétriques : 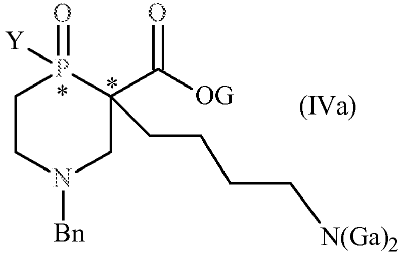 Les diastéroioisomères du composé de formule (IVa) peuvent être aisément séparés à l'aide d'une colonne chirale, permettant l'obtention des composés optiquement purs de formule (la) par le procédé décrit ci-dessus.

Les composés de l'invention sont des inhibiteurs de TAFIa.

A ce titre, ils sont utiles dans la prévention ou le traitement des événements thrombotiques chez les patients à risque. Leur utilisation sera intéressante dans le traitement et la prévention des complications vasculaires, plus particulièrement cardiovasculaires, pulmonaires et cérébrovasculaires liées aux maladies athérothrombotiques, à l'athérosclérose, au diabète, à l'hyperlipidémie, à l'hypertension, aux maladies veineuses chroniques, au syndrome métabolique lié à l'obésité ou au cancer.
Les composés selon l'invention sont particulièrement utiles pour le traitement, la prévention et la prévention secondaire de l'infarctus du myocarde, de l'angine de poitrine, des accidents vasculaires cérébraux quelle que soit leur origine (notamment athérothrombotique, cardioembolique ou causée par une fibrillation atriale), des anévrismes aortiques ou de l'artérite des membres inférieurs, des thromboses veineuses (notamment chez le patient cancéreux cathétérisé) et de l'embolie pulmonaire.

Les facteurs de risques vasculaires et les maladies vasculaires tels que l'hypertension, l'obésité, le diabète, les maladies cardiaques, les maladies cérébrovasculaires et l'hyperlipidémie et donc l'athérosclérose jouent un rôle dans la génèse des démences telles que la maladie d'Alzheimer et la démence vasculaire (Qiu C., De Ronchi D. Et Fratiglioni L., The epidemiology of the dementias : an update, 2007, Current Opinion in Psychiatry, 20 : 380-385). Les composés de l'invention seront donc également utiles pour le traitement et/ou la prévention des démences telles que la maladie d'Alzheimer et la démence vasculaire.

Le TAFIa diminue le potentiel fibrinolytique endogène. En tant qu'inhibiteurs de TAFIa, les composés de la présente invention sont donc utiles pour accompagner le traitement aigu par les fibrinolytiques injectables, tels que le tPA recombinant (par exemple altéplase, tenecteplase, reteplase, desmoteplase), l'uPA recombinant ou la streptokinase, qui sont utilisés en situation d'urgence (par exemple infarctus du myocarde, accident vasculaire cérébral).
Les composés de la présente invention renforcent l'activité de ces fibrinolytiques injectables et conduisent donc à leur utilisation avec moins de risques hémorragiques et neurotoxiques (diminution de leur dose et donc réduction de leurs effets secondaires).

La présente invention a également pour objet les compositions pharmaceutiques contenant un composé de formule (I), en combinaison avec un ou plusieurs excipients ou véhicules inertes, non toxiques, pharmaceutiquement acceptables.

La posologie utile varie selon l'âge et le poids du patient, la voie d'administration, la nature et la sévérité de l'affection, et la prise de traitements éventuels associés et s'échelonne de 0,5 mg à 1000 mg en une ou plusieurs prises par jour.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales.
Selon un aspect de la présente invention, la composition pharmaceutique est une préparation injectable pour administration intraveineuse.
Selon un autre aspect de la présente invention, la composition pharmaceutique est un comprimé pour administration orale.
Outre le composé de formule (I), les comprimés selon l'invention contiennent un ou plusieurs excipients ou véhicules tels que des diluants, des lubrifiants, des liants, des agents de désintégration, des absorbants, des colorants, des édulcorants.
A titre d'exemple d'excipients ou véhicules, on peut citer :
◆ *pour les diluants* : le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.
Le pourcentage de principe actif de formule (I) dans le comprimé est préférentiellement compris entre 5% et 50% en poids.

Le composé de formule (I) selon la présente invention peut être administré en association avec un fibrinolytique, plus particulièrement un fibrinolytique injectable tel que le tPA recombinant (par exemple altéplase, tenectéplase, reteplase ou desmoteplase), l'uPA recombinant ou la streptokinase, ou avec un anticoagulant tel que, par exemple, la warfarine, le dabigatran etexilate, le rivaroxaban.
L'administration en association peut être sous la forme d'une co-administration simultanée ou successive de deux compositions pharmaceutiques séparées contenant chacune l'un des principes actifs (association libre), ou sous la forme de l'administration d'une association fixe des deux principes actifs au sein de la même composition pharmaceutique.

Le composé de formule (I) peut être administré sous la forme d'une préparation injectable, en association libre avec une préparation injectable d'altéplase. Le composé de formule (I) peut être administré sous la forme d'une préparation injectable, en association libre avec une préparation injectable de tenectéplase.
Les exemples suivants illustrent la présente invention. Les structures des composés décrits dans les exemples ont été déterminées selon les techniques spectrophotométriques usuelles (infrarouge, résonance magnétique nucléaire, spectrométrie de masse).

### ABREVIATIONS

AcOEt : acétate d'éthyle
AIBN : azobisisobutyronitrile
DCM : dichlorométhane
DEA : diéthylamine
DIBAlH : hydrure de diisobutylaluminium
DMAP : diméthylaminopyridine
DMF : diméthylformamide
DMSO ou dmso : diméthylsulfoxyde
DO : densité optique
EDTA : acide éthylènediaminetétraacétique
eq : équivalent molaire
HMPA : hexaméthylphosphoramide
HPLC : chromatographie en phase liquide à haute performance
IR : infra-rouge
LDA : diisopropylamidure de lithium
LiHMDS : hexaméthyldisilazane de lithium ou bis(triméthylsilyl)amidure de lithium
MTBE : méthyl *tert*-butyl éther
PR : pouvoir rotatoire
RMN : Résonance Magnétique Nucléaire
TAFIa : activated thrombin-activatable fibrinolysis inhibitor
TEA : triéthylamine
TFA : acide trifluoro acétique
THF : tétrahydrofurane
TMS : triméthylsilyle
tPA : activateur tissulaire du plasminogène (**t**issue **p**lasminogen **a**ctivator)
uPa : activateur du plasminogène de type urokinase (**u**rokinase **p**lasminogen **a**ctivator) ou urokinase
Xantphos : 4,5-bis(diphénylphosphino)-9,9-diméthylxanthène

### Mode opératoire de synthèse des chaînes latérales - Procédures I, J, K (intermédiaires 204 à 212)

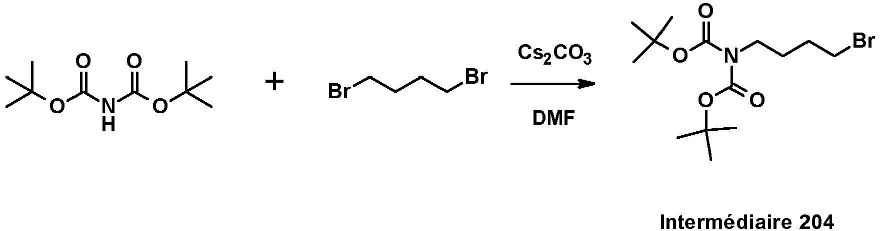

### Procédure I :

A une solution d'iminodicarboxylate de di-*tert*-butyle (20 g, 92 mmol) dans 1L de DMF sous une atmosphère d'Argon et à température ambiante est ajouté par portion du carbonate de césium (60 g, 184 mmol, 2 eq). Une agitation vigoureuse est maintenue pendant 1h avant l'ajout de 1,4-dibromobutane (99.2 g, 459 mmol, 5 eq). Après 24h à température ambiante, le mélange réactionnel est filtré sur Célite et concentré sous pression réduite. Le résidu obtenu est purifié par flash chromatographie sur gel de silice en utilisant comme éluant un gradient heptane/AcOEt (100% à 90:10). L'intermédiaire 204 (26.1 g, 74.1 mmol) est obtenu sous forme d'une huile incolore avec un rendement de 81 %.

### Intermédiaire 204

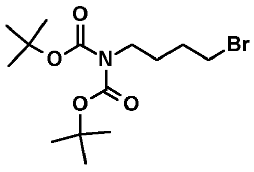

RMN ¹H: (CDCl₃, 400 MHz) δ 3.60 (t, 2 H), 3.42 (t, 2 H), 1.87 (quint., 2 H), 1.73 (quint., 2 H), 1.51 (s, 18 H).
IR: 1790-1744-1693 cm⁻¹ (C=O), 1125 cm⁻¹ (C-O).

### Intermédiaire 205

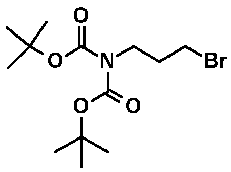

L'intermédiaire 205 est obtenu à partir du 1,3-dibromopropane selon la procédure I précédemment décrite.

Produit isolé sous forme d'une huile incolore (24.7 g, 73.0 mmol) avec un rendement de 79%.
RMN ¹H: (CDCl₃, 400 MHz) δ 3.6 (m, 2 H), 3.5 (t, 2 H), 2.05 (quint., 2 H), 1.51 (s, 18 H).
IR: 1791-1744-1693 cm⁻¹ (C=O), 1125 cm⁻¹ (C-O).

### Intermédiaire 206

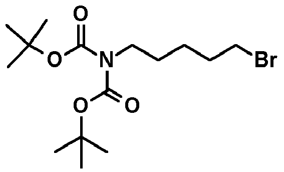

L'intermédiaire 206 est obtenu à partir du 1,5-dibromopentane selon la procédure I précédemment décrite.

Produit obtenu sous forme d'une huile incolore (27.9 g, 76.2 mmol) avec un rendement de 83%.
RMN ¹H: (CDCl₃, 400 MHz) δ 3.60 (t, 2 H), 3.40 (t, 2 H), 1.90 (m, 2 H), 1.60 (m, 2 H), 1.45 (m, 2 H), 1.50 (s, 18 H) ppm.
IR: 1740, 1693 (C=O), 1787 cm⁻¹ (C=O faible).

### Intermédiaire 207

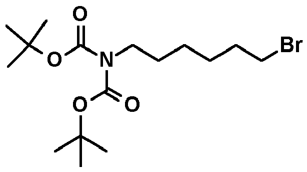

L'intermédiaire 207 est obtenu à partir du 1,6-dibromohexane selon la procédure I précédemment décrite.
RMN ¹H: (CDCl₃, 400 MHz) δ 3.58 (t, 2 H), 3.40 (t, 2 H), 1.89 (m, 2 H), 1.65-1.25 (m, 6 H), 1.45 (m, 6H), 1.51 (s, 18 H) ppm.

### Procédure J

### Intermédiaire 208

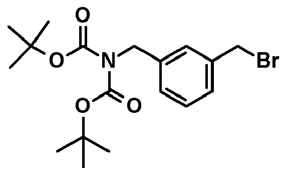

A une suspension de NaH à 60 % (0.682 g, 17 mmol, 1.5 eq) dans un mélange de THF (25 mL) et de DMF (3 mL) sous argon et à température ambiante, sont ajoutés portion par portion du *tert*-butyliminodicarboxylate (2.47 g, 11.37 mmol, 1 eq). Le milieu réactionnel est agité à température ambiante pendant 15 minutes puis tiédit à 45°C pendant 45 minutes. Cette suspension est alors additionnée à une solution de 1,3-dibromométhylbenzène (3 g, 11.37 mmol, 1 eq) dans du THF (100 mL). L'agitation se poursuit à température ambiante pendant 16h. Le mélange est hydrolysé goutte à goutte avec une solution de NH₄Cl à 10% (100 mL). La phase organique est extraite à l'AcOEt (2 x 50 mL), lavée avec une solution saturée en NaCl (50 mL) et séchée sur MgSO₄. Le solvant est évaporé sous pression réduite et le brut est purifié par flash chromatographie sur gel de silice en utilisant comme éluant un gradient Heptane/DCM (50:50 à 100%). L'intermédiaire 208 (2.07 g, 5.17 mmol) est obtenu sous forme d'une huile incolore avec un rendement de 45 %.
RMN ¹H: (DMSO-d₆, 400 MHz) δ 7.33 (d, 2 H), 7.3 (sl, 1 H), δ 3.15 (t, 1 H), 4.70 /4.68 (2s, 4 H), 1.40 (s, 18 H).
IR: 1790-1747-1699 cm⁻¹ (C=O), 1224-1143-1110 cm⁻¹ (C-O-C), 854-781-699 cm⁻¹ (CHAr).
MS : m/z 422 [M+Na].

### Intermédiaire 209

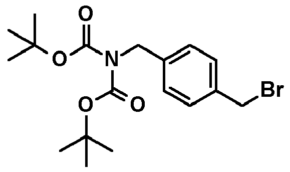

L'intermédiaire 209 est obtenu à partir du 1,4-dibromométhylbenzène selon la procédure J précédemment décrite.

Obtention d'un solide blanc (2.02 g, 5.05 mmol) avec un rendement de 44 %.
RMN ¹H: (DMSO-d₆, 400 MHz) δ 7.40 (d, 2 H), 7.20 (d, 2 H), 4.69 (2s, 4 H), 1.39 (s, 18 H). IR : 1767-1693 cm⁻¹ (C=O).
MS : m/z 343 [M-C₄H₈].

### Procédure K

### Intermédiaire 210

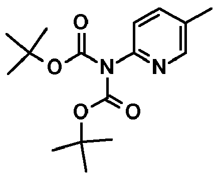

A une solution de 2-amino-5-méthylpyridine (25 g, 230 mmol) dans du DCM (450 mL) à 0°C sous atmosphère d'Argon est additionnée goutte à goutte de la diisopropyléthylamine (59.34 g, 462 mmol, 2 eq). Une solution de (Boc)₂O (125.5 g, 575 mmol, 2.5 eq) est alors ajoutée goutte à goutte suivie par de la *N,N*-diméthylaminopyridine (28.1 g, 230 mmol, 1 eq). Le mélange réactionnel est agité 15h à température ambiante. La phase aqueuse est extraite à l'AcOEt (2 x 400 mL). Les phases organiques sont rassemblées, lavées avec une solution de NH₄Cl à 10% (400 mL), avec une solution saturée en NaCl (400 mL), avec une solution de NaHCO₃ à 10% (400 mL) et enfin avec une solution saturée en NaCl (400 mL). La phase organique est séchée sur MgSO₄ et le solvant est évaporé sous pression réduite. Le résidu obtenu est purifié par flash chromatographie sur gel de silice en utilisant comme éluant un gradient heptane/AcOEt (90:10 à 75:25). L'intermédiaire 210 (32.78 g, 106.3 mmol) est obtenu sous forme d'une huile incolore avec un rendement de 46 %.
RMN ¹H: (400 MHz, CDCl3) δ ppm 8.3 (s, 1 H), 7.75 (dd, 1 H), 7.1 (d, 1 H), 2.3 (s, 3 H), 1.45 (s, 18 H)
IR : 1742-1707 cm⁻¹(C=O).

### Intermédiaire 211

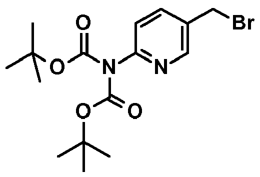

A une solution de l'intermédiaire 210 (3.08 g, 10 mmol) et de *N*-bromosuccinimide (1.87 g, 10.5 mmol, 1.05 eq) dans du CCl₄ (50 mL) est additionné de l'AIBN (0.164 g, 1 mmol). Le milieu réactionnel est chauffé à reflux pendant 20h. Une fois à température ambiante, les insolubles sont filtrés et le filtrat est concentré sous pression réduite. Le résidu est purifié par flash chromatographie sur gel de silice en utilisant comme éluant un gradient DCM/AcOEt (de 99:1 à 95:5). L'intermédiaire 211 (2.15 g, 5.56 mmol) est obtenu sous forme d'un solide blanc avec un rendement de 56 %.
RMN ¹H : (400 MHz, CDCl3) δ ppm 8.55 (d, 1 H), 7.75 (dd, 1 H), 7.3 (d, 1 H), 4.45 (s, 2 H), 1.45 (s, 18 H)
IR : 1753-1743-1710 cm⁻¹(C=O).

### Intermédiaire 212

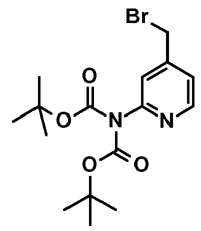

L'intermédiaire 212 est obtenu à partir de la 2-amino-4-méthylpyridine selon la procédure K précédemment décrite.
RMN ¹H: (400 MHz, CDCl3) δ ppm 8.48 (d, 1 H), 7.3 (d, 1 H), 7.22 (d, 1 H), 4.4 (s, 2 H), 1.45 (s, 18 H)
IR : 1788-1755-1724 cm⁻¹(C=O).

### Procédure A - synthèse des exemples 1 à 19.

### Stade A1

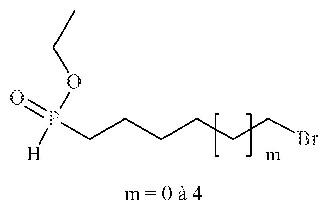

A une solution d'acide hypophosphoreux (7.42 g, 112 mmol, 1.0 eq) dans l'acétonitrile (160 mL) sous argon et à température ambiante est additionné le tétraéthylorthosilicate (25 mL, 112 mmol, 1.0 eq). Le mélange réactionnel est chauffé à reflux pendant 2h30 puis refroidi à température ambiante. Le bromoalcène CH₂=CH-(CH₂)ₘ₊₂-Br (0.5 eq), Pd₂dba₃ (0.769 g, 0.84 mmol) et le Xantphos (0.356 g, 0.62 mmol) sont alors additionnés et le mélange réactionnel est chauffé à reflux pendant 18h. La solution est alors filtrée sur papier filtre à température ambiante, et concentrée sous pression réduite. Le brut est purifié par flash chromatographie sur colonne de silice en utilisant un gradient heptane/AcOEt comme éluant. Le composé du stade A1 est obtenu sous forme d'huile.

### Stade A2

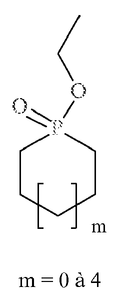

A une solution dégazée avec de l'argon pendant 30 min du composé du stade A1 (233 mmol, 1 eq) dans du THF (870 mL) à -78°C est additionnée goutte à goutte une solution de LiHMDS à 1,06M/THF (220 mL, 1.0 eq). Après addition, le mélange réactionnel est agité à température ambiante pendant 2h30. La réaction est alors quenchée à 0°C par addition d'une solution aqueuse saturée de NaCl (870 mL). Après extraction à l'acétate d'éthyle (3 x 800 mL), les phases organiques sont rassemblées, lavées avec une solution saturée de NaCl, séchées sur MgSO₄ avant d'être concentrées sous pression réduite. Le brut est alors purifié par flash chromatographie sur colonne de silice en utilisant comme éluant un mélange DCM/EtOH 95:5. Le composé du stade A2 est obtenu sous forme d'huile.

### Stade A3

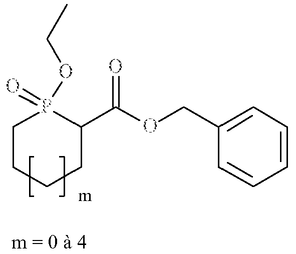

A une solution du composé du stade A2 (8.5 mmol, 1 eq) dans du THF (20 mL) à -70°C et sous flux d'argon, est ajouté du LDA (6.4 mL, 12.8 mmol, 1.5 eq). L'agitation est maintenue pendant 20 min. Une solution de dibenzylcarbonate (2.88 g, 11.9 mmol, 1.4 éq) dans le THF (11 mL) est alors ajoutée goutte à goutte. Le mélange est agité 45 min, puis une seconde addition de LDA (6.4 mL, 12.8 mmol, 1.5 eq) est réalisée. La solution est agitée pendant 2h à -70°C. Une solution de NH₄Cl à 10% (60 mL) est alors additionnée goutte à goutte en maintenant la température du mélange réactionnel à -70°C. De l'AcOEt (20 mL) est alors ajouté et le milieu réactionnel est remonté progressivement jusqu'à température ambiante. Le mélange réactionnel est alors extrait avec de l'AcOEt (2 x 80 mL). Les phases organiques sont rassemblées et séchées sur MgSO₄, filtrées et concentrées sous pression réduite. Le brut est purifié par flash chromatographie sur colonne de silice en utilisant comme éluant un gradient DCM/EtOH. Le composé du stade A3 est obtenu sous forme d'une huile.

### Stade A4

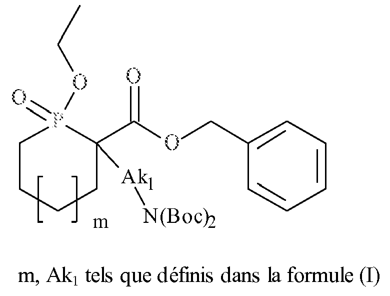

A une suspension de NaH à 60% (0.448 g, 11.14 mmol) dans 5 mL de DMSO à 10 °C et sous Argon, sont additionnées successivement une solution de l'intermédiaire 204 à 212 (7.65 mmol, 1.1 eq.) dans 8 ml de DMSO puis, goutte à goutte, une solution du composé du stade A3 (6.96 mmol, 1 eq) dans 5 mL de DMSO. A la fin de l'addition, le mélange réactionnel est remonté à température ambiante et agité pendant 3h. Le traitement est réalisé à 0°C en ajoutant du NH₄Cl 10% (100 mL) puis l'AcOEt (100 mL). Après décantation, la phase aqueuse est ré-extraite par de l'AcOEt (2 x 80 mL). Les phases organiques sont rassemblées, lavées par une solution de NaCl saturée (2 x 80 mL), séchées sur MgSO₄, filtrées et concentrées sous pression réduite. Le brut est purifié par flash chromatographie sur colonne de silice en utilisant comme éluant un gradient DCM/EtOH. Le composé du stade A4, un mélange de diastéréoisomères, est obtenu sous forme d'une huile incolore.

### Stade A5

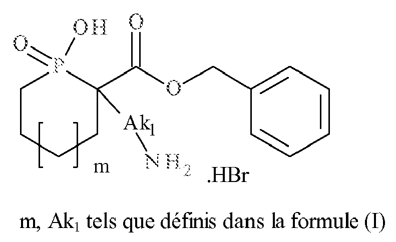

A une solution du composé du stade A4 (0.683 mmol, 1.0 eq) dans du DCM (30 mL) à 0°C et sous Argon, est ajouté goutte à goutte le bromotriméthylsilane (0.721 mL, 5.47 mmol, 8 eq). Le mélange est agité 20h à température ambiante. Une évaporation sous pression réduite est réalisée puis le mélange est évaporé à sec à la pompe à palette pendant 30 min. Le mélange est repris dans le MeOH (30 mL), agité pendant 30 min, puis évaporé sous pression réduite et à sec à la pompe à palette pendant 30 min. Le mélange est à nouveau repris dans le MeOH, agité, et évaporé. Cette opération est réalisée une troisième fois. Le composé du stade A5 est obtenu sous forme de sel de bromhydrate et est engagé directement dans la réaction suivante d'hydrogénation.

### Stade A6 - Exemples 1 à 19

A une solution du composé du stade A5 dans du MeOH (15 mL), est ajouté le Pd/C (10mol%) à 10%. Le mélange est agité 18h sous atmosphère H₂ à température ambiante. Le catalyseur est filtré sur fritté Whatman. Le filtrat est évaporé à sec. 12 eq de TFA sont ajoutés au brut. Le produit est purifié par flash chromatographie sur colonne RP18 phase inverse en utilisant un gradient H₂O/MeCN/TFA comme éluant. Après lyophilisation, le produit attendu (exemples 1 à 19), sel de TFA, est obtenu sous forme d'un solide blanc hygroscopique.

### EXEMPLE 1 : Acide 2-(3-aminopropyl)-1-hydroxy-1-oxo-1-phospholane-2-carboxylique, trifluroacétate

L'exemple 1 est obtenu à partir de l'intermédiaire 205 selon la procédure A précédemment décrite.
RMN ¹H: (300 MHz, dmso-d6) δ ppm 12-11 (sl, 1H), 8.3-7.3 (sl, 3 H), 2.8 (q, 2 H), 2.25 (m, 1 H), 1.9 (m, 1 H), 1.8-1.4 (m, 8 H)
ESI/FIA/HR et MS/MS : ESI +/- : infusion : [M+H]+ = 222.1
Analyse Elémentaire : C=35.39(35.83);H=5.44(5.11);N=4.60(4.18)

### EXEMPLE 2 : Acide 2-(4-aminobutyl)-1-hydroxy-1-oxo-1-phospholane-2-carboxylique, trifluroacétate

L'exemple 2 est obtenu à partir de l'intermédiaire 204 selon la procédure A précédemment décrite.
RMN ¹H: (400 MHz, dmso-d6) δ ppm 12.5 (sl, 1 H), 7.75 (sl, 3 H), 2.8 (q, 2 H), 2.21-1.6 (m, 2H), 1.9-1.35 (m, 2H), 1.8-1.5 (m, 6H), 1.6-1.2 (m, 2H)
ESI/FIA/HR et MS/MS : [M+H]+ = 236.1041 (236.1051)
Analyse Elémentaire : C=38.94(37.83);H=5.81(5.48);N=4.50(4.01)

### EXEMPLE 3 : Acide 2-[(6-aminopyridin-3-yl)méthyl]-1-hydroxy-1-oxo-1-phospholane-2-carboxylique, trifluroacétate

L'exemple 3 est obtenu à partir de l'intermédiaire 211 selon la procédure A précédemment décrite.
RMN ¹H : (400 MHz, dmso-d6) δ ppm 13 (sl, 2 H), 7.9 (sl, 3 H), 7.7 (m, 2 H), 6.85 (d, 1 H), 3.2 (m, 1 H), 2.7 (m, 1 H), 2.05 (m, 1 H), 1.85-1.5 (m, 5 H)
ESI/FIA/HR et MS/MS : [M+H]+ = 271.0842 (271.0847)
Analyse Elémentaire : C=41.20(40.64);H=4.52(4.20);N=8.06(7.29)

### EXEMPLE 4 : Acide 2-(5-aminopentyl)-1-hydroxy-1-oxo-1-phospholane-2-carboxylique, trifluroacétate

L'exemple 4 est obtenu à partir de l'intermédiaire 206 selon la procédure A précédemment décrite.
RMN ¹H: (300 MHz, dmso-d6) δ ppm 12.5 (sl, 1 H), 2.78 (m, 2 H), 1.8 à 1.4 (m, 4 H), 7.68 (sl, 3 H), 2.22/1.65 (m, 2 H), 1.98/1.3 (m, 2 H), 1.52 (t, 2 H), 1.4 à 1 (m, 4 H)
ESI/FIA/HR et MS/MS : [M+H]+ = 250.1209 (250.1208)
Analyse Elémentaire : C=39.69(39.68);H=5.91(5.83);N=4.37(3.86)

### EXEMPLE 5 : Acide 2-(3-aminopropyl)-1-hydroxy-1-oxo-1-phosphinane-2-carboxylique, trifluroacétate

L'exemple 5 est obtenu à partir de l'intermédiaire 205 selon la procédure A précédemment décrite.
RMN ¹H: (400 MHz, dmso-d6) δ ppm 13.0 (sl, 1 H), 7.73 (sl, 3 H), 2.77 (m, 2 H), 1.93-1.66-1.44 (3*(m, 2+10 H)
ESI/FIA/HR et MS/MS: [M+H]+ = 236.1051 (236.1051)
Analyse Elémentaire : C=38.07(37.83);H=5.85(5.48);N=4.11(4.01)

### EXEMPLE 6 : Acide 2-(5-aminopentyl)-1-hydroxy-1-oxo-1-phosphinane-2-carboxylique, trifluroacétate

L'exemple 6 est obtenu à partir de l'intermédiaire 206 selon la procédure A précédemment décrite.
RMN ¹H: (300 MHz, dmso-d6) δ ppm 7.65 (sl, 3 H), 2.75 (m, 2 H), 1.9 (m, 2 H), 1.0-1.85 (m, 14 H)
ESI/FIA/HR et MS/MS: ESI +/- : infusion : [M+H]+ = 264.1
Analyse Elémentaire : C=40.84(41.39);H=5.66(6.14);N=3.76(3.71)

### EXEMPLE 7 : Acide 2-(4-aminobutyl)-1-hydroxy-1-oxo-1-phosphinane-2-carboxylique, trifluroacétate

L'exemple 7 est obtenu à partir de l'intermédiaire 204 selon la procédure A précédemment décrite.
RMN ¹H: (300 MHz, dmso-d6) δ ppm 7.68 (sl, 3 H), 2.78 (m, 2 H), 2.02 à 1.16 (m, 14 H) ESI/FIA/HR et MS/MS: ESI +/- : infusion : [M+H]+ = 250.1
Analyse Elémentaire : C=39.61(39.68);H=5.71(5.83);N=3.86(3.86)

### EXEMPLE 8 : Acide 2-(4-aminobutyl)-1-hydroxy-1-oxo-1-phosphépane-2-carboxylique, trifluroacétate

L'exemple 8 est obtenu à partir de l'intermédiaire 204 selon la procédure A précédemment décrite.
RMN ¹H: (400 MHz, dmso-d6) δ ppm 13 à 11.5 (sl, 1 H), 7.75 (sl, 3 H), 2.8 (m, 2 H), 2.1 à 1.85 (m, 2 H), 1.85 à 1.2 (m, 14 H)
ESI/FIA/HR et MS/MS: ESI +/- : infusion : [M+H]+ = 264.1

### EXEMPLE 9 : Acide 2-[[4-(aminométhyl)phényl]méthyl]-1-hydroxy-1-oxo-1-phospholane-2-carboxylique, trifluroacétate

L'exemple 9 est obtenu à partir de l'intermédiaire 209 selon la procédure A précédemment décrite.
RMN ¹H: (400 MHz, dmso-d6) δ ppm 13 à 12 (sl, 1 H), 7.39 (d, 2 H), 2.1 à 1.5 (m, 6 H), 7.22 (d, 2 H), 8.3 (sl, 3 H), 3.98 (m, 2 H), 3.39/2.75 (2dd, 2 H)
ESI/FIA/HR et MS/MS: [M+H]+ = 284.1048 (284.1051)
Analyse Elémentaire : C=48.29(48.84);H=5.45(5.99);N=4.64(4.38)

### EXEMPLE 10 : Acide 2-(5-aminopentyl)-1-hydroxy-1-oxo-1-phosphépane-2-carboxylique, trifluroacétate

L'exemple 10 est obtenu à partir de l'intermédiaire 206 selon la procédure A précédemment décrite.
RMN ¹H: (400 MHz, dmso-d6) δ ppm 12 (sl, 1 H), 7.68 (sl, 3 H), 2.76 (m, 2 H), 2.12 à 1.9 (m, 2 H), 1.87 à 1.11 (m, 16 H)
ESI/FIA/HR et MS/MS: [M+H]+ = 278.1518 (278.1521)
Analyse Elémentaire : C=41.99(42.97);H=6.38(6.44);N=3.53(3.58)

### EXEMPLE 11 : Acide 1-hydroxy-1-oxo-2-(2-pipéridin-4-yléthyl)-1-phosphinane-2-carboxylique, trifluroacétate

L'exemple 11 est obtenu à partir de la *N*-Boc-4-bromoéthylpipéridine selon la procédure A précédemment décrite.
RMN ¹H: (400 MHz, dmso-d6) δ ppm 12 (sl, 1 H), 8.49-8.2 (2*(m, 2 H), 3.24 (dl, 2 H), 2.82 (m, 2 H), 1.94-1.65-1.45-1.2 (4*(m, 12 H), 1.79 (tl, 2 H), 1.39 (m, 1 H), 1.2 (m, 2 H)
ESI/FIA/HR et MS/MS: [M+H]+ = 290.1526 (290.1521)
Analyse Elémentaire : C=44.65(44.67);H=5.93(6.25);N=3.41(3.47)

### EXEMPLE 12 : Acide 2-[[3-(aminométhyl)phényl]méthyl]-1-hydroxy-1-oxo-1-phosphinane-2-carboxylique, trifluroacétate

L'exemple 12 est obtenu à partir de l'intermédiaire 208 selon la procédure A précédemment décrite.
RMN ¹H: (400 MHz, dmso-d6) δ ppm 8.19 (sl, 3 H), 7.3 (m, 2 H), 7.22 (s, 1 H), 7.14 (d, 1 H), 3.99 (m, 2 H), 3.32/2.97 (2dd, 2 H), 1.9 à 1.4 (m, 8 H)
ESI/FIA/HR et MS/MS: [M+H]+ = 298.12 (298.120821)
Analyse Elémentaire : C=47.00(46.72);H=4.82(5.15);N=3.39(3.41)

### EXEMPLE 13 : Acide 2-(6-aminohexyl)-1-hydroxy-1-oxo-1-phosphinane-2-carboxylique, trifluoroacétate

L'exemple 13 est obtenu à partir de l'intermédiaire 207 selon la procédure A précédemment décrite.
RMN ¹H: (400 MHz, dmso-d6) δ ppm 7.65 (sl, 3 H), 2.77 (m, 2 H), 1.93 (m, 2 H), 1.8 à 1.14 (m, 16 H)
ESI/FIA/HR et MS/MS: [M+H]+ = 278.1522 (278.15212)
Analyse Elémentaire : C=42.69(42.97);H=6.26(6.44);N=3.79(3.58)

### EXEMPLE 14 : Acide 2-(4-aminobutyl)-1-hydroxy-1-oxo-1-phosphocane-2-carboxylique, trifluoroacétate

L'exemple 14 est obtenu à partir de l'intermédiaire 204 selon la procédure A précédemment décrite.
RMN ¹H: (400 MHz, dmso-d6) δ ppm 7.72 (sl, 3 H), 2.8 (m, 2 H), 2.11 à 1.32 (m, 18 H)
ESI/FIA/HR et MS/MS: [M+H]+ = 278.1522 (278.1521)
Analyse Elémentaire : C=43.34(42.97);H=6.35(6.44);N=2.91(3.58)

### EXEMPLE 15 : Acide 2-[(2-aminopyridin-4-yl)méthyl]-1-hydroxy-1-oxo-1-phosphinane-2-carboxylique, trifluoroacétate

L'exemple 15 est obtenu à partir de l'intermédiaire 212 selon la procédure A précédemment décrite.
RMN ¹H: (400 MHz, dmso-d6) δ ppm 13.5 à 12 (sl, 1 H), 7.8 (d, 1 H), 7.87 (sl, 3 H), 6.75 (s, 1 H), 6.6 (d, 1 H), 3.25/3 (m, 2 H), 2 à 1.35 (m, 8 H)
ESI/FIA/HR et MS/MS: [M+H]+ = 285.1012 (285.1004)

### EXEMPLE 16 : Acide 2-[2-(trans-4-aminocyclohexyl)éthyl]-1-hydroxy-1-oxo-1-phosphinane-2-carboxylique, trifluoroacétate

L'exemple 16 est obtenu à partir de [*trans*-4-(2-bromoéthyl)cyclohexyl]carbamate de *tert-*butyle, selon la procédure A précédemment décrite.
RMN ¹H: (300/400/500 MHz, dmso-d6) δ ppm 7.9 (sl, 3H), 2.9 (m, 1 H), 2.01 (m, 2 H), 1.91-1.74 (2m, 4 H), 1.64 (m, 2 H), 1.53 (m, 2 H), 1.39 (m, 2 H), 1.29-0.95 (m, 4 H), 1.11 (m, 4 H), 1.09 (m, 1 H)
RMN ¹³C : (300/400/500 MHz, dmso-d6) δ ppm 49.8, 36.8, 31.6, 30.8, 30.5, 30.5, 30.5, 23.6, 22.9
ESI/FIA/HR et MS/MS : [M+H]+ = 304.1648 (304.1677)

### EXEMPLE 17 : Acide 2-[2-(cis-4-aminocyclohexyl)éthyl]-1-hydroxy-1-oxo-1-phosphinane-2-carboxylique, trifluoroacétate

L'exemple 17 est obtenu à partir de [cis-4-(2-bromoéthyl)cyclohexyl]carbamate de *tert-*butyle, selon la procédure A précédemment décrite.
RMN ¹H: (400/500 MHz, dmso-d6) δ ppm 7.9 (sl, 3H), 3.15 (m, 1 H), 1.96-1.62 (2m, 2 H), 1.94-1.73 (2m, 2 H), 1.71-1.65 (2m, 2 H), 1.68-1.65 (2m, 2 H), 1.56-1.39 (m, 4 H), 1.49-1.36 (2m, 2 H), 1.38 (m, 1 H), 1.23-1.16 (2m, 2 H)
RMN ¹³C : (400/500 MHz MHz, dmso-d6) δ ppm 173.3, 51, 47.9, 34, 31, 28.7, 27.9, 26.5, 26.5, 23.4, 21.6
RMN ¹³C : (400/500 MHz, dmso-d6) δ ppm 173.3, 51, 47.9, 34, 31, 28.7, 27.9, 26.5, 26.5, 23.4, 21.6
ESI/FIA/HR et MS/MS : [M+H]+ = 304.1671 (304.1677)
Analyse Elémentaire : C=45.47(46.05);H=6.68(6.52);N=3.58(3.36)

### EXEMPLE 18 : Acide 2-(5-aminopentyl)-1-hydroxy-1-oxo-1-phosphocane-2-carboxylique, trifluoroacétate

L'exemple 18 est obtenu à partir de l'intermédiaire 206 selon la procédure A précédemment décrite.
RMN ¹H: (400 MHz, dmso-d6) δ ppm 12.0-11.0 (sl, 1H), 7.7 (m, 3 H), 2.8 (m, 2 H), 2.05 (m, 2 H), 1.9-1.3 (m, 18 H)
ESI/FIA/HR et MS/MS : [M+H]+ = 292.1656 (292.1677)
Analyse Elémentaire : C=44.17(44.45);H=6.69(6.71);N=3.42(3.46)

### EXEMPLE 19 : Acide 2-(5-aminopentyl)-1-hydroxy-1-oxo-1-phosphonane-2-carboxylique, trifluoroacétate

L'exemple 19 est obtenu à partir de l'intermédiaire 206 selon la procédure A précédemment décrite.
RMN ¹H: (400 MHz, dmso-d6) δ ppm 12.4-11 (sl, 1 H), 7.7 (sl, 3 H), 2.75 (t, 2 H), 2.3 (m, 1 H), 2.05 (m, 1 H), 1.9-1.2 (m, 20 H)
ESI/FIA/HR et MS/MS : [M+H]+ = 306.1834 (306.1834)
Analyse Elémentaire : C=45.54(45.82);H=6.98(6.97);N=3.26(3.34)

### Procédure B - Synthèse des phosphinanes

### Stade B1 (Intermédiaire 6)

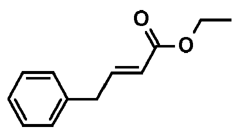

A une solution de phénylacetaldehyde (28.74 g, 239 mmol) dans du DCM (380 mL) et à température ambiante est additionné par portions l'éthyle (triphénylphosphorylidene)acétate (100 g, 287 mmol, 1.2 eq). Le milieu réactionnel est agité 20 h à température ambiante puis le mélange est concentré sous vide. Le résidu est repris dans l'heptane (400 mL), agité 1h puis l'insoluble est filtré. L'heptane est évaporé sous vide et le résidu obtenu est purifié par flash chromatographie sur gel de silice en utilisant comme éluant un mélange Heptane/DCM (60:40). L'intermédiaire 6 (27.25 g, 143 mmol) est obtenu sous forme d'une huile incolore avec un rendement de 60%.
RMN ¹H: (400 MHz, CDCl3) δ ppm 7.3 (t, 2 H), 7.25 (t, 1 H), 7.2 (d, 2 H), 7.1 (dt, 1 H), 5.8 (d, 1 H), 4.2 (quad, 2 H), 3.5 (d, 2 H), 1.3 (t, 3 H)
IR (cm⁻¹) : 1716, 1653

### Stade B2 (Intermédiaire 7)

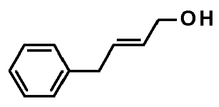

A une solution de l'intermédiaire 6 (28.35 g, 149 mmol) dans du DCM (375 mL) à température ambiante et sous atmosphère d'Argon est additionnée une solution de DIBA1H 1M dans le THF (355 mL, 355 mmol). Après 45 minutes à 0°C, le milieu réactionnel est agité 16h à température ambiante. Le milieu réactionnel est ensuite refroidi à 0°C et traité avec HCl 3N (300 mL). Le mélange est extrait avec du DCM (350 mL), la phase organique est lavée avec H₂O (2 x 100 mL), séchée sur Na₂SO₄ puis évaporée sous vide. Le résidu obtenu est purifié par flash chromatographie sur gel de silice en utilisant comme éluant un mélange est Heptane/AcOEt (75:25). L'intermédiaire 7 (16.05 g, 108 mmol) est obtenu sous forme d'une huile incolore avec un rendement de 72%.
RMN ¹H: (400 MHz, CDCl3) δ ppm 7.1-7.4 (m, 5 H), 5.65-5.9 (2m, 2 H), 4.1 (d, 2 H), 3.35 (d, 2 H), 1.5 (m, 1 H)
IR (cm⁻¹) : 3600-3200, 1716, 1650

### Stade B3 (Intermédiaire 8)

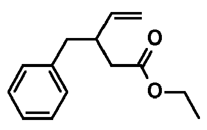

Le mélange de l'intermédiaire 7 (3.7 g, 25 mmol), avec le triéthylorthoacétate (16.2 g, 100 mmol, 4 eq) et de l'acide propionique (5 gouttes) est agité 1h20 dans un microondes (250W) à 140°C. Le mélange est repris avec Et₂O (300 mL) et H₂O (100 mL). La phase organique est ensuite lavée avec H₂O (100 mL), séchée sur MgSO₄ puis concentrée sous vide. Le résidu obtenu est purifié par flash chromatographie sur gel de silice en utilisant comme éluant un mélange est heptane/AcOEt (80:20). L'intermédiaire 8 (4.53 g, 20.7 mmol) est obtenu sous forme d'une huile avec un rendement de 83%.
RMN ¹H: (400 MHz, CDCl3) δ ppm 7.15-7.3 (m, 5 H), 5.7 (m, 1 H), 5 (m, 2 H), 4.1 (q, 2 H), 2.85 (m, 1 H), 2.7 (m, 2 H), 2.25-2.4 (m, 2 H), 1.25 (t, 3 H)
IR (cm⁻¹) : 1732, 699-747

### Stade B4 (Intermédiaire 9)

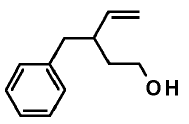

A une solution de l'intermédiaire 8 (17.58 g, 80.5 mmol) dans le THF (275 mL) à 0°C et sous Argon, est additionné par portions LiAlH₄ (6.12 g, 161 mmol, 2 eq). Le milieu réactionnel est agité à température ambiante pendant 16h. L'excès de LiAlH₄ est hydrolysé par l'addition de H₂O (4.2 mL) puis avec une solution de NaOH 20% (3.4 mL) et H₂O (15.4 mL). Le précipité est filtré et le filtrat est évaporé sous vide. Le résidu obtenu est purifié par flash chromatographie sur gel de silice en utilisant comme éluant un mélange est heptane/AcOEt (70:30). L'intermédiaire 9 (8.09 g, 45.9 mmol) est obtenu sous forme d'une huile incolore avec un rendement de 57%.

### Stade B5 (Intermédiaire 10) :

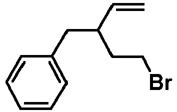

A une solution de l'intermédiaire 9 (8.09 g, 45.9 mmol) et de CBr₄ (30.4 g, 91.8 mmol) à température ambiante dans du Et₂O (325 mL) est ajouté par portions du triphenylphosphine (24.07 g, 91.8 mmol, 2 eq). Le mélange est agité pendant 16h. Le milieu réactionnel est filtré et le filtrat est évaporé sous vide. Le résidu est repris dans de l'heptane (250 mL), agité 30 minutes puis filtré. Le filtrat est évaporé sous vide. Le résidu obtenu est purifié par flash chromatographie sur gel de silice en utilisant l'heptane comme éluant. L'intermédiaire 10 (8.64 g, 36 mmol) est obtenu sous forme d'une huile incolore avec un rendement de 78%.
RMN ¹H: (400 MHz, CDCl₃) δ ppm 7.28 (t, 2 H), 7.18 (t, 1 H), 7.13 (d, 2 H), 5.53 (ddd, 1 H), 5.02 (d, 1 H), 5 (d, 1 H), 3.42 (m, 1 H), 3.3 (m, 1 H), 2.65 (d, 2 H), 2.53 (m, 1 H), 1.95 (m, 1 H), 1.8 (m, 1 H)
IR (cm⁻¹) : 916, 739-698

### Stade B6 (Intermédiaire 11)

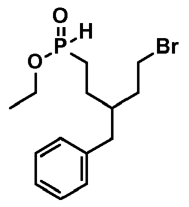

A une solution d'acide hypophosphoreux (1.1 g, 16.7 mmol) dans l'acétonitrile (25 mL) sous Argon et à température ambiante est additionné le tétraéthylorthosilicate (3.48 g, 16.7 mmol, 1 eq). Le mélange réactionnel est chauffé à reflux pendant 2h30 puis refroidi à température ambiante. Le milieu réactionnel est dégazé à l'argon, puis sont ajoutés successivement l'intermédiaire 10 (2 g, 8.36 mmol) en solution dans MeCN (5 mL), le Xantphos (0.048 g, 0.0836 mmol) puis du Pd₂dba₃ (0.038 g, 0.0418 mmol). Le mélange est chauffé au reflux pendant 16h. Après évaporation sous vide, le résidu obtenu est purifié par flash chromatographie sur gel de silice en utilisant l'AcOEt comme éluant. L'intermédiaire 11 (1.61 g, 4.85 mmol) est obtenu sous forme d'une huile avec un rendement de 58%.
RMN ¹H: (400 MHz, dmso-d6) δ ppm centré à 6.95 (d, 1 H), 7.3 (t, 2 H), 7.2 (m, 3 H), 4 (m, 2 H), 3.55 (m, 2 H), 2.59 (d, 2 H), 1.9 (m, 1 H), 1.78 (m, 4 H), 1.45 (m, 2 H), 1.21 (t, 3 H)
IR (cm⁻¹) : 2343

### Stade B7 (Intermédiaire 12)

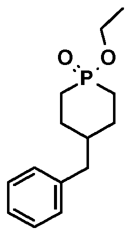

A une solution de l'intermédiaire 11 (1.6 g, 4.8 mmol) dans 50 mL de THF et sous atmosphère d'Argon, est additionnée goutte à goutte à -78°C une solution de LiHMDS à 1.06 M dans le THF (4.53 mL, 4.8 mmol, 1 eq). Le milieu réactionnel est agité 30 minutes à -78°C puis 4h30 à température ambiante avant d'être traité avec une solution saturée en NaCl (50 mL). Après addition d'AcOEt (150 mL), la phase organique est séchée sur MgSO₄ puis évaporée sous vide. Le résidu obtenu est purifié par flash chromatographie sur gel de silice en utilisant un mélange DCM/EtOH (95:5) comme éluant. L'intermédiaire 12 (0.889 g, 3.52 mmol) est obtenu sous forme d'une huile incolore avec un rendement de 73%.
RMN ¹H: (400 MHz, CDCl3) δ ppm 7.3 (t, 2 H), 7.2 (t, 1 H), 7.12 (d, 2 H), 4.08 (m, 2 H), 2.6/2.51 (2d, 2 H), 2.1-1.8 (m, 4 H), 1.8-1.55 (m, 3 H), 1.5-1.3 (m+t, 5 H)
IR (cm⁻¹) : 3433

### Stade B8 (Intermédiaire 13)

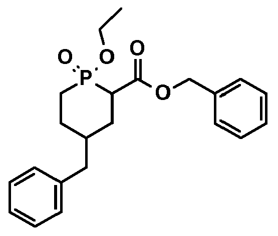

A une solution de l'intermédiaire 12 (0.875 g, 3.47 mmol) dans du THF (10 mL) à -78°C et sous Argon, est additionnée une solution de LDA 2M dans le THF (2.6 mL, 5.2 mmol, 1.5 eq). Après 40 minutes, une solution de dibenzylcarbonate (1.18 g, 4.85 mmol) dans du THF (5 mL) est ajouté goutte à goutte. Le milieu réactionnel est agité 1h puis 1.5 eq de LDA 2M dans le THF (2.6 mL, 5.2 mmol) sont rajoutés. Après 4h à -78°C, le milieu réactionnel est hydrolysé à froid avec une solution aqueuse de NH₄Cl 10% (9 mL). De l'AcOEt (18 mL) et une solution aqueuse de NH₄Cl 10% (18 mL) sont alors ajoutés. Après retour à température ambiante, le milieu réactionnel est extrait avec de l'AcOEt (2 x 50 mL). La phase organique est séchée sur MgSO₄ puis concentrée sous vide. Le résidu obtenu est purifié par flash chromatographie sur gel de silice en utilisant un mélange DCM/EtOH (97.5:2.5) comme éluant. L'intermédiaire 13(0.996 g, 2.58 mmol) est obtenu sous forme d'une huile avec un rendement de 74%.
RMN ¹H: (400 MHz, CDCl3) δ ppm 7.4-7 (m, 10 H), 5.18 (s, 2 H), 4.15-3.9 (m, 2 H), 3.2-2.8 (1 dd, 1 H), 2.58 (d, 2 H), 2.3-1.4 (m, 7 H), 1.25 (t, 3 H)
IR (cm⁻¹): 1726

### Stade B9 (Intermédiaire 14)

A une suspension d'hydrure de sodium (0.150 g, 3.75 mmol, 1.6 eq) dans du DMSO (5 mL) sont successivement ajoutées goutte à goutte une solution d'intermédiaire 206 (1.11 g, 3.03 mmol, 1.3 eq) dans du DMSO (5 mL) et une solution de l'intermédiaire 13 (0.9 g, 2.33 mmol) dans du DMSO (4 mL). Après 4h d'agitation à température ambiante, le milieu réactionnel est additionné à 0°C sur un mélange (1:1) d'une solution de NH₄Cl à 10% (100 mL) et d'AcOEt (100 mL). La phase aqueuse est ré-extraite avec de l'AcOEt (2 x 50 mL). Les phases organiques sont rassemblées, séchées sur MgSO₄ puis concentrées sous vide. L'intermédiaire 14 ainsi obtenu est directement utilisé sans autre purification.

### Stade B10 (Intermédiaires 15 et 16)

A une solution d'intermédiaire 14 (1.56 g, 2.33 mmol) dans du DCM (125 mL) à température ambiante et sous argon, est ajouté du TMSBr (3.68 mL, 28 mmol, 12 eq). Le milieu réactionnel est agité 16h à température ambiante avant d'être concentré sous vide. L'huile obtenue est reprise dans du MeOH (60 mL), agitée pendant 30 min et concentrée sous vide. La même opération est répétée 2 fois. Le résidu obtenu est purifié par chromatographie en phase inverse en utilisant comme éluant un gradient H₂O/MeCN/TFA. Les intermédiaires 15 (0.423 g, 0.95 mmol) puis 16 (0.155 g, 0.35 mmol) (dans l'ordre d'élution) sont obtenus sous forme de solides blancs après lyophilisation avec des rendements respectifs de 41% et de 15 %. La configuration absolue des intermédiaires 15 et 16 n'a pas été vérifiée.

### Intermédiaire 15 : 2-(5-Aminopentyl)-4-benzyl-1-hydroxy-1-oxo-1-phosphinane-2-carboxylate de benzyle, trifluoroacétate - dia 1 racémique

RMN ¹H: (400 MHz, dmso-d6) δ ppm 12 (m, 1 H), 7.75 (m, 3 H), 7.35-7.05 (m, 10 H), 5.18/5 (2d, 2 H), 2.6 (m, 3 H), 2.4 (dd, 1 H), 2.1 (m, 1 H), 1.9-1.5 (m, 8 H), 1.4 (m, 2 H), 1.15 (m, 2 H), 0.8/0.52 (2m, 2 H)
RMN ¹⁹F : (400 MHz, dmso-d6) δ ppm -74
IR (cm⁻¹) : 3300-2500, 1716, 1678

### Intermédiaire 16 : 2-(5-Aminopentyl)-4-benzyl-1-hydroxy-1-oxo-1-phosphinane-2-carboxylate de benzyle, trifluoroacétate - dia 2 racémique

RMN ¹H (400 MHz, dmso-d6) δ ppm 7.4 (m, 5 H), 7.25 (t, 2 H), 7.18 (t, 1 H), 7 (d, 2 H), centré à 5.11 (AB, 2 H), 7.65 (m, 3 H), 2.71 (m, 2 H), 2.4 (d, 2 H), 1.95-1.1 (m, 15 H)
RMN ¹⁹F : (400 MHz, dmso-d6) δ ppm -74
IR (cm⁻¹) : 3300-2500, 1774, 1716, 1676

### EXEMPLE 20 : Acide 2-(5-aminopentyl)-4-benzyl-1-hydroxy-1-oxo-1-phosphinane-2-carboxylique, dia 1 racémique

L'intermédiaire 15 (0.413g, 0.74 mmol) en solution dans 60 mL d'un mélange (3:1) H₂O/MeOH est agité à température ambiante sous atmosphère de H₂, en présence de Pd 10%/C (41 mg) pendant 4h. Le milieu réactionnel est filtré puis concentré sous vide. Le résidu obtenu est purifié par chromatographie en phase inverse en utilisant comme éluant un gradient H₂O/MeCN. L'exemple 20 (0.209 g, 0.591 mmol) est obtenu sous forme d'un solide blanc après lyophilisation avec un rendement de 80 %.
RMN ¹H: (500 MHz, D2O) δ ppm 7.3 (t, 2 H), 7.24 (d, 2 H), 7.19 (t, 1 H), 2.59/2.37 (m, 2 H), 2.41 (t, 2 H), 1.91/1.55 (m, 2 H), 1.77/1.6 (m, 2 H), 1.77/1.35 (m, 2 H), 1.64/1.38 (m, 2 H), 1.63 (m, 1 H), 1.24 (m, 2 H), 1.16/1.1 (m, 2 H), 0.7 (m, 2 H)
RMN ¹³C : (500 MHz, D2O) δ ppm 129.2, 128.2, 125.7, 42.5, 40, 35.5, 35.4, 31.3, 31.1, 30.2, 26, 25.9, 22.8
IR (cm⁻¹) : 3300-2100, 1691, 1631, 1605
Analyse Elémentaire : C=60.65(61.18);H=7.58(7.99);N=3.91(3.96)
ESI/FIA/HR et MS/MS : [M+H]+ = 354.1831 (354.1834)

### EXEMPLE 21 : Acide 2-(5-aminopentyl)-4-benzyl-1-hydroxy-1-oxo-1-phosphinane-2-carboxylique, dia 2 racémique

L'intermédiaire 16 (0.148 g, 0.265 mmol) en solution dans 20 mL d'un mélange (3:1) H₂O/MeOH est agité à température ambiante sous atmosphère de H₂, en présence de Pd 10%/C (15 mg) pendant 2h30. Le milieu réactionnel est filtré puis concentré sous vide. Le résidu obtenu est purifié par chromatographie en phase inverse en utilisant comme éluant un gradient H₂O/MeCN. L'exemple 21 (0.053 g, 0.15 mmol) est obtenu sous forme d'un solide blanc après lyophilisation avec un rendement de 56 %.
RMN ¹H: (500 MHz, D2O) δ ppm 7.29 (t, 2 H), 7.2 (d, 2 H), 7.2 (t, 1 H), 2.57 (t, 2 H), 2.45 (m, 2 H), 1.84 (m, 1 H), 1.81/1.33 (m)+(m, 1+1 H), 1.78/1.29 (m)+(m, 1+1 H), 1.69/1.31 (m)+(m, 1+1 H), 1.68/1.27 (m)+(m, 1+1 H), 1.41/1.19 (m)+(m, 1+1 H), 1.38 (m, 2 H), 1.2 (m, 2 H)
RMN ¹³C : (500 MHz, D₂O) δ ppm 129.3, 128.2, 125.7, 43, 42.4, 40, 35.9, 34.6, 30.4, 29.4, 27, 26.7, 25
IR (cm⁻¹) : 3250-1800, 1694+1661, 1618
Analyse Elémentaire : C=61.18(61.18);H=7.90(7.99);N=3.96(3.96)
ESI/FIA/HR et MS/MS : [M+H]+ = 354.1837 (354.1834)

De la même façon, les exemples 22 et 23 sont obtenus selon la procédure B précédemment décrite en remplaçant l'intermédiaire 206 par l'intermédiaire 204.

### EXEMPLE 22 : Acide 2-(4-aminobutyl)-4-benzyl-1-hydroxy-1-oxo-1-phosphinane-2-carboxylique, dia 1 racémique

RMN ¹H: (400 MHz, dmso-d6) δ ppm 7.7 (sl, 3 H), 7.3 (dd, 2 H), 7.2 (dd+t, 3 H), 2.7 (m, 2 H), 2.55-2.4 (m, 2 H), 1.95 (m, 1 H), 1.8-13 (m, 10 H), 1.1-0.85 (m, 2 H)
ESI/FIA/HR et MS/MS : [M+H]+ = 340.1677 (340.1677)
Analyse Elémentaire : C=51.00(50.33);H=5.90(6.00);N=3.22(3.09)

### EXEMPLE 23 : Acide 2-(4-aminobutyl)-4-benzyl-1-hydroxy-1-oxo-1-phosphinane-2-carboxylique, dia 2 racémique

RMN ¹H: (300 MHz, dmso-d6) δ ppm 15.8 (m, 2 H), 7.95 (m, 2 H), 7.25 (t, 2 H), 7.18 (t, 1 H), 7.1 (d, 2 H), 2.75 (m, 2 H), 2.6-2.35 (m, 2 H), 2.15 (m, 1 H), 1.75-1 (m, 12 H)
ESI/FIA/HR et MS/MS : [M+H]+ = 340.1683 (340.1677)
Analyse Elémentaire : C=60.20(60.17);H=7.34(7.72);N=3.96(4.13)

### Synthèse des azaphosphinanes

### Intermédiaire 17

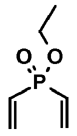

L'intermédiaire 17 a été synthétisé en utilisant une procédure décrite dans la littérature par V. Gouverneur et al., J. Org. Chem. 2005, 70, 10803.

A une solution de dichlorophosphinate d'éthyle (10 mL, 84.26 mmol) dans 100 mL de THF à -78°C et sous Argon, est additionnée une solution à 1M dans le THF de bromure de vinyle magnésium (170 mL, 170 mmol, 2 eq). L'agitation est maintenue pendant 1h à -78°C. EtOH (30 mL) est alors additionné goutte à goutte et la réaction est remontée à température ambiante. Le milieu réactionnel est alors concentré sous pression réduite et le résidu obtenu est purifié par flash chromatographie sur gel de silice en utilisant un mélange DCM/EtOH (96:4) comme éluant. L'intermédiaire 17 (6.37 g, 43.6 mmol) est obtenu sous forme d'une huile incolore avec un rendement de 52 %.
RMN ¹H: (400 MHz, DMSO - d₆) δ 6.1-6.4 (m, 6H), δ 3.90 (q, 2H), δ 1.25 (t, 3H).
IR (cm⁻¹) : 3500 (OH(H₂O)), 1609 (C=C), 1210 (P=O), 1032 et 935 cm⁻¹ (P-O).
GC : tᵣ 5.83 min.

### Intermédiaire 18

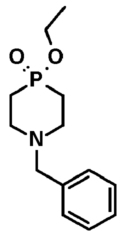

A une solution de l'intermédiaire 17 (6.37 g, 43.6 mmol) dans un autoclave et sous Argon, est additionnée en une fois de la *N*-benzylamine (4.79 mL, 43.6 mmol, 1 eq.) Le mélange réactionnel est chauffé à 100°C, agité pendant 16h et concentré sous pression réduite. Le résidu est repris avec de l'AcOEt (100 mL), la phase organique est lavée avec une solution de NaCl saturée (3 x 100 mL) et séchée au MgSO₄. Le solvant est évaporé et le résidu obtenu est purifié par flash chromatographie sur gel de silice en utilisant un mélange DCM/EtOH (95:5) comme éluant. L'intermédiaire 18 (8.68 g, 34.3 mmol) est obtenu sous forme d'une huile jaunâtre avec un rendement de 79 %.
RMN ¹H: (CDCl₃, 400 MHz) δ 7.31 (m, 5H), 4.08 (m, 2H), 3.60 (s, 2H), 2.98 (m, 2H), 2.64 (m, 2H), 1.97 (m, 2H), 1.85 (m, 2H), 1.35 (t, 3H).
GC : tᵣ 12.29 min.

### Intermédiaire 19

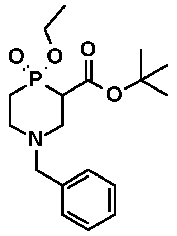

A une solution d'intermédiaire 18 (6.38 g, 25.19 mmol) dans du THF (30 mL) à -70°C et sous Argon, est additionnée une solution de LDA (75.6 mL, 37.8 mmol, 1.5 eq) à 2M dans le THF. Après 15 min à -70°C, une solution de Boc₂O (7.68 g, 35.3 mmol, 1.4 eq) dans 30 mL de THF est alors additionnée goutte à goutte. L'agitation est maintenue pendant 90 min et 1.5 eq de LDA (75.6 mL, 37.8 mmol) sont alors additionnés goutte à goutte. A la fin de l'addition, le milieu réactionnel est maintenu à -70°C pendant 90 min. Une solution saturée de NH₄Cl (30 mL) ainsi que de l'AcOEt (60 mL) sont ajoutés et le milieu réactionnel est remonté lentement à température ambiante. Le produit est alors extrait avec de l'AcOEt (2 x 150 mL). Les phases organiques sont rassemblées, lavées avec une solution saturée en NaCl (2 x 150 mL), séchées sur MgSO₄ et concentrées sous pression réduites. Le résidu obtenu est purifié par flash chromatographie sur gel de silice en utilisant un gradient de DCM/THF (95:5 à 40:60) comme éluant. L'intermédiaire 19 (6.73 g, 19.04 mmol) est obtenu sous forme d'une huile jaunâtre avec un rendement de 76 %.
RMN ¹H: (CDCl₃, 400 MHz) δ 7.35 à 7.2 (m, 5H), δ 4.3 à 4.05 (m, 2H), δ 3.60 (dd, 2H), δ 3.3 à 2.5 (m, 5H), δ 2.1 à 1.8 (m, 2H), δ 1.50 (s, 9H), δ 1.35 (t, 3H).
IR (cm⁻¹) : 3500 (OH), 1721 (C=O), 1150 (P=O), 1032 et 935 (P-O).
MS : m/z 355 [M+1].

### Procédure C : Mode opératoire d'alkylation de l'intermédiaire 19

A une solution d'intermédiaire 204 à 212 (5 mmol, 1 eq) dans du DMSO (10 mL) sous Argon, est ajouté par portions NaH à 60% (8 mmol, 1.6 eq) à 10 °C. L'intermédiaire 19 (5 mmol) en solution dans du DMSO (5 mL) est alors additionné sur la suspension et le mélange est agité pendant 4h à température ambiante. Le milieu réactionnel est alors hydrolysé avec une solution aqueuse de NH₄Cl (50 mL) et extrait avec AcOEt (2 x 100 mL). La phase organique est lavée avec H₂O (2 x 100 mL), séchée sur MgSO₄ et concentrée sous vide. Le résidu obtenu est purifié par flash chromatographie sur gel de silice en utilisant comme éluant un gradient DCM/AcOEt (90:10 à 50:50). L'intermédiaire 20a-f est obtenu sous la forme d'un mélange de 4 diastéréoisomères.

### Procédure D : Déprotection des fonctions amino et phosphinique

A une solution de l'intermédiaire 20a-f (5 mmol) dans DCM (40 mL) sous Argon et à température ambiante, est additionné goutte à goutte du TMSBr (7.92 mL, 60 mmol, 12 eq). Le mélange est agité pendant 16h à température ambiante puis concentré sous vide. Le résidu est repris dans du MeOH (40 mL) et agité 20 min à température ambiante avant d'être évaporé à sec. L'évaporat est mis en solution dans du DCM (20 mL) et l'acide trifluoroacétique (44.6 mL, 60 mmol, 12 eq) est ajouté. Le milieu réactionnel est agité 10h à température ambiante puis concentré sous vide. Le résidu obtenu est purifié par chromatographie en phase inverse en utilisant comme éluant un gradient H₂O/MeCN. Le produit final (exemples 24 à 30) (zwitterion ou sel de TFA), mélange de 2 énantiomères, est obtenu sous forme d'un solide blanc après lyophilisation.

### Intermédiaire 20a : 3-{3-[Bis(tert-butoxycarbonyl)amino]propyl}-1-benzyl-4-éthoxy-4-oxo-1,4-azaphosphinane-3-carboxylate de tert-butyle

L'intermédiaire 20a est obtenu à partir des intermédiaires 19 et 205 selon la procédure C précédemment décrite.
RMN ¹H: (400 MHz, dmso-d6) δ ppm 7.35-7.2 (m, 5 H), 4 (m, 2 H), 3.8 (m, 2 H), centré à 3.52 (AB, 2 H), 3-2.25 (m, 4 H), 2-1.8 (m, 4 H), 1.45/1.35 (2s, 27 H), 1.2 (t, 3 H), 1.2 (m, 2 H)

### EXEMPLE 24 : Acide 3-(3-aminopropyl)-1-benzyl-4-hydroxy-4-oxo-1,4-azaphosphinane-3-carboxylique

L'exemple 24 est obtenu à partir de l'intermédiaire 20a selon la procédure D précédemment décrite.
RMN ¹H: (400 MHz, D2O) δ ppm 7.5 (m, 5 H), centré à 4.35 (AB, 2 H), 3.75/3.35 (2m, 2 H), 3.5/3.15 (2dd, 2 H), 2.92 (t, 2 H), 2.3/1.8 (2m, 2 H), 1.95 (m, 1 H), 1.6-1.4 (m, 3 H)
ESI/FIA/HR et MS/MS : [M+H]+ = 327.1478 (327.1473)
Analyse Elémentaire : C=55.38(55.21);H=6.85(7.10);N=8.41(8.58)

### Intermédiaire 20b : 3-{4[Bis(tert-butoxycarbonyl)amino]butyl}-1-benzyl-4-éthoxy-4-oxo-1,4-azaphosphinane-3-carboxylate de tert-butyle

L'intermédiaire 20b est obtenu à partir des intermédiaires 19 et 204 selon la procédure C précédemment décrite.
RMN ¹H: (DMSO-d₆, 400 MHz) δ 7.30 (m, 5 H), 3.97 (m, 2 H), 3.63-3.43 (dd, 2 H), 3.36 (m, 2 H), 2.93-2.33 (m, 2 H), 2.79 à 2.47 (m, 2 H), 1.93 (m, 2 H), 1.93 (m, 2 H), 1.43 (m, 2 H), 1.42 (s, 18 H), 1.36 (s, 9 H), 1.21 (t, 3 H), 0.83 (m, 2 H).
IR (cm⁻¹) : 1744, 1711 cm⁻¹ (C=O), 1276 cm⁻¹ (P=O).

### EXEMPLE 25 : Acide 3-(4-aminobutyl)-1-benzyl-4-hydroxy-4-oxo-1,4-azaphosphinane-3-carboxylique

L'exemple 25 est obtenu selon la procédure D précédemment décrite à partir de l'intermédiaire 20b.
RMN ¹H: (400 MHz, dmso-d6) δ ppm 8.2 (sl, 3 H), 7.3 (m, 5 H), 3.7/3.5 (2*(d, 1+1 H), 3.05/2.4 (2*(m, 1+1 H), 2.75/2.55 (2*(m, 1+1 H), 2.7 (m, 2 H), 1.8 (m, 1 H), 1.6-1.1 (m, 7 H) ESI/FIA/HR et MS/MS : [M+H]+ = 341.1628 (341.1630)
Analyse Elémentaire : C=55.99(56.46);H=7.14(7.40);N=8.13(8.23)

### Intermédiaire 20c : 3-{5-Bis(tert-butoxycarbonyl)amino]pentyl}-1-benzyl-4-éthoxy-4-oxo-1,4-azaphosphinane-3-carboxylate de tert-butyle

L'intermédiaire 20c est obtenu à partir des intermédiaires 19 et 206 selon la procédure C précédemment décrite.
RMN ¹H : (400 MHz, dmso-d6) δ ppm 7.32-7.2 (m, 5 H), 3.99 (m, 2 H), 3.8 (m, 2 H), centré à 3.5 (AB, 2 H), 3-2.3 (m, 4 H), 2-1.8 (m, 4 H), 1.45/1.35 (2s, 27 H), 1.35/1.15 (2m, 4 H), 1.2 (t, 3 H), 0.75 (m, 2 H)

### EXEMPLE 26 : Acide 3-(5-aminopentyl)-1-benzyl-4-hydroxy-4-oxo-1,4-azaphosphinane-3-carboxylique

L'exemple 26 est obtenu à partir de l'intermédiaire 20c selon la procédure D précédemment décrite.
RMN ¹H: (400 MHz, D2O) δ ppm 7.5 (m, 5 H), centré à 4.32 (AB, 2 H), 3.7/3.35 (2m, 2 H), 3.5/3.1 (2dd, 2 H), 2.9 (t, 2 H), 2.2/1.78 (2m, 2 H), 1.95/1.45 (2m, 2 H), 1.6 (m, 2 H), 1.3 (m, 2 H), 1.1 (m, 2 H)
ESI/FIA/HR et MS/MS : [M+H]+ = 355.1792 (355.1786)
Analyse Elémentaire : C=58.04(57.62);H=7.37(7.68);N=7.95(7.90)

### Intermédiaire 20d : 1-Benzyl-3-({6-[bis-tert-butoxycarbonyl)amino]pyridin-3-yl}méthyl)-4-éthoxy-4-oxo-1,4-azaphosphinane-3-carboxylate de tert-butyle

L'intermédiaire 20d est obtenu à partir des intermédiaires 19 et 211 selon la procédure C précédemment décrite.
RMN ¹H : (400 MHz, dmso-d6) δ ppm 8.18 (s, 1 H), 7.4-7.25 (m, 6 H), 7.18 (d, 1 H), 4 (m, 2 H), 3.8 (m, 2 H), 3.58/3.5 (2d, 2 H), 3-2.6 (m, 4 H), 2.25/2.1 (2m, 2 H), 1.4 (s, 27 H), 1.2 (t, 3 H)

### EXEMPLE 27 : Acide 3-[(6-aminopyridin-3-yl)méthyl]-1-benzyl-4-hydroxy-4-oxo-1,4-azaphosphinane-3-carboxylique

L'exemple 27 est obtenu à partir de l'intermédiaire 20d selon la procédure D précédemment décrite.
RMN ¹H : (400 MHz, D2O) δ ppm 7.51 (dd, 1 H), 7.42 (d, 1 H), 7.4-7.25 (m, 5 H), 6.75 (d, 1 H), centré à 4.25 (AB, 2 H), 3.78 (m, 1 H), 3.4 (dd+m, 2 H), 2.95 (dd, 1 H), 2.7 (dd, 1 H), 2.6 (dd, 1 H), 2.28/1.85 (2m, 2 H)
ESI/FIA/HR et MS/MS : [M+H]+ = 376.1418 (376.1426)
Analyse Elémentaire : C=57.18(57.60);H=5.82(5.91);N=11.25(11.19)

### EXEMPLE 28 : Acide 3-[(2-aminopyridin-4-yl)méthyl]-1-benzyl-4-hydroxy-4-oxo-1,4-azaphosphinane-3-carboxylique

L'exemple 28 est obtenu à partir des intermédiaires 19 et 212 selon les procédures C et D précédemment décrite sans purification intermédiaire.
RMN ¹H: (400 MHz, D2O) δ ppm 7.6 (dl, 1 H), 7.4 (m, 5 H), 6.55 (m, 2 H), 4.6/4 (dd, 2 H), 3.9-3.4 (m, 2 H), 3.6/2.75 (dd, 2 H), 3/2.7 (dd, 2 H), 2.3/1.9 (m, 2 H)
ESI/FIA/HR et MS/MS : [M+H]+ = 376.1428 (376.1426)
Analyse Elémentaire : C=56.70(57.60);H=5.45(5.91);N=10.87(11.19)

### Intermédiaire 20e : 1-Benzyl-3-(3-{[bis-(tert-butoxycarbonyl)amino]méthyl}-benzyl)-4-éthoxy-4-oxo-1,4-azaphosphinane-3-carboxylate de tert-butyle

L'intermédiaire 20e est obtenu à partir des intermédiaires 19 et 208 selon la procédure C précédemment décrite.
RMN ¹H: (400 MHz, dmso-d6) δ ppm 7.4-7.3 (m, 5 H), 7.15 (t, 1 H), 7.1 (sl, 1 H), 7.05/6.95 (d, 2 H), 4.6 (s, 2 H), 4 (m, 2 H), 3.7/3.45 (dd, 2 H), 3.35 (m, 2 H), 2.9/2.15 (m, 2 H), 2.7/2.55 (dd, 2 H), 2.05 (m, 2 H), 1.4 (s, 27 H), 1.2 (t, 3 H)

### EXEMPLE 29 : Acide 3-[[3-(aminométhyl)phényl]méthyl]-1-benzyl-4-hydroxy-4-oxo-1,4-azaphosphinane-3-carboxylique

L'exemple 29 est obtenu à partir de l'intermédiaire 20e selon la procédure D précédemment décrite.
RMN ¹H: (400 MHz, D2O) δ ppm 7.3 (m, 5 H), 7.3-7.05 (m, 4 H), 4.4/3.95 (dd, 2 H), 4 (dd, 2 H), 3.8-3.3 (m, 2 H), 3.55/2.75 (dd, 2 H), 3/2.7 (dd, 2 H), 2.2/1.8 (m, 2 H)
ESI/FIA/HR et MS/MS : [M+H]+ = 389.1623 (389.1630)
Analyse Elémentaire : C=61.37(61.85);H=6.30(6.49);N=7.05(7.21)

### Intermédiaire 20f : 1-Benzyl-3-(4-{[bis(tert-butoxycarbonyl)amino]méthyl)}benzyl)-4-éthoxy-4-oxo-1,4-azaphosphinane-3-carboxylate de tert-butyle

L'intermédiaire 20f est obtenu à partir des intermédiaires 19 et 209 selon la procédure C précédemment décrite.
RMN ¹H: (400 MHz, dmso-d6) δ ppm 7.35 (m, 5 H), 6.99 (d, 2 H), 6.91 (d, 2 H), 4.6 (s, 2 H), 3.98 (m, 2 H), 3.5 (s, 2 H), 3.27 (m, 2 H), 2.95/2.22 (2*m, 2 H), 2.75/2.49 (2*m, 2 H), 2.05 (m, 2 H), 1.35 (2*s, 27 H), 1.18 (t, 3 H)
RMN ¹³C : (400 MHz, dmso-d6) δ ppm 152, 138, 137, 135, 130, 128, 126, 82, 62, 60, 55.5, 50.5, 48.5, 34, 28, 24.5, 16

### EXEMPLE 30 : Acide 3-[[4-(aminométhyl)phényl]méthyl]-1-benzyl-4-hydroxy-4-oxo-1,4-azaphosphinane-3-carboxylique

L'exemple 30 est obtenu à partir de l'intermédiaire 20f selon la procédure D précédemment décrite.
RMN ¹H: (400 MHz, D2O) δ ppm 7.2 (m, 5 H), 7.1/7 (dd, 4 H), 4.3/3.9 (dd, 2 H), 4 (m, 2 H), 3.7-3.25 (m, 2 H), 3.45/2.65 (dd, 2 H), 2.9/2.65 (dd, 2 H), 2.15/1.75 (m, 2 H)
ESI/FIA/HR et MS/MS : [M+H]+ = 389.1623 (389.1630)
Analyse Elémentaire : C=61.41(61.85);H=6.36(6.49);N=7.40(7.21)

### Intermédiaire 20b : 3-{4[Bis(tert-butoxycarbonyl)amino]butyl}-1-benzyl-4-éthoxy-4-oxo-1,4-azaphosphinane-3-carboxylate de tert-butyle

Dans un ballon tricol de 1L équipé d'une agitation mécanique sont introduits successivement sous une atmosphère d'argon du DMSO (30 mL) et NaH à 60% (8.48 g, 212 mmol, 1.5 eq). Le ballon est maintenu à température ambiante à l'aide d'un bain d'eau. Une solution de l'intermédiaire 204 (54.6 g, 155 mmol, 1.1 eq) dans du DMSO (25 mL) est alors ajoutée goutte à goutte sur une période de 5 minutes. Une solution de l'intermédiaire 19 (50 g, 141.5 mmol) dans du DMSO (120 mL) est alors additionnée goutte à goutte en maintenant la température en dessous de 20°C. Après la fin de l'addition, 100 mL de THF anhydre sont alors ajoutés pour pouvoir maintenir une agitation. Après 3h, le mélange réactionnel est refroidit à l'aide d'un bain d'eau glacée et hydrolysé par ajout de 500 mL d'une solution saturée de NH₄Cl. Le mélange est ensuite extrait avec de l'AcOEt (3 x 300 mL). Les phases organiques sont alors rassemblées, lavées avec une solution saturée de NaCl (2 x 300 mL), et séchées sur MgSO₄ avant d'être concentrées sous pression réduite. Le résidu jaunâtre (92.5 g) ainsi obtenu, est alors purifié par chromatographie sur gel de silice en utilisant un mélange CH₂Cl₂/AcOEt/MeOH comme éluant. Le produit attendu (69.3 g, 110.9 mmol), mélange de 4 diastéréoisomères, est obtenu sous forme d'un solide blanc avec un rendement de 78%.

### Procédure E : Mode opératoire débenzylation par hydrogénolyse

### Intermédiaire 21 : 3-{4-[Bis(tert-butoxycarbonyl)amino]butyl}-4-éthoxy-4-oxo-1,4-azaphosphinane-3-carboxylate de tert-butyle

Dans un ballon de 2L à température ambiante et sous flux argon sont introduits successivement l'intermédiaire 20b (73.6 g, 117.8 mmol), de l'éthanol (1 L), du Pd/C (7.36 g, 10% massique) et de l'HCl à 37% (7.85 mL, 0.8 eq). L'argon est ensuite remplacé par une atmosphère d'hydrogène. La réaction est suivie par LC/MS. Après 4h, la réaction est totale et le catalyseur est filtré sur fibre de verre. Le filtrat est évaporé à sec afin d'obtenir une huile jaune qui est reprise par de l'AcOEt (400 mL) et par une solution de NaHCO₃ à 10% (400 mL). Après décantation, la phase aqueuse est extraite avec de l'AcOEt (3 x 100 mL). Les phases organiques sont réunies puis lavées avec une solution saturée en NaCl (400 mL), séchées sur MgSO₄ et concentrées pour conduire à l'intermédiaire 21 attendu sous forme d'un solide blanc (57.6 g, 107.7 mmol) avec un rendement de 91%.
RMN ¹H: (DMSO-d₆, 400 MHz) δ 4.05 à 3.85 (m, 2 H), 3.45 (m, 2 H), 3.1 à 2.6 (m, 4 H), 2.3 -1.7 (m, 4H), 1.45 (s, 18 H), 1.40 (s, 9 H), 1.20 (t, 3 H), 1.5-0.9 (m, 4 H).
IR (cm⁻¹) : 3100-3500 cm⁻¹(OH), 3314 cm⁻¹ (NH), 1712-1693 cm⁻¹ (C=O).

### Procédure L : Mode opératoire de synthèse des aldéhydes Ar₂-Ar₁-CHO par un couplage de Suzuki

*Les aldéhydes non-commerciaux ont été préparés suivant la procédure décrite ci-dessous* :

Dans un ballon de 1 L sous argon à température ambiante sont introduits successivement de l'éthanol (500 mL), l'acide boronique Ar₂-B(OH)₂ (92.7 mmol, 1.2 eq) et le bromoarylaldéhyde ou bromohétéroarylaldéhyde Br-Ar₁-CHO (77.3 mmol). La solution est dégazée à l'Argon pendant 15 min. Pd(PPh₃)₄ (1.78 g, 1.55 mmol) et Na₂CO₃ (92.7 mL d'une solution 2M dans H₂O, 185 mmol, 2.4 eq) sont alors introduits en une portion. Après l'addition, le mélange réactionnel est chauffé au reflux pendant 5h. Le milieu est alors évaporé à sec. Le résidu est repris avec du DCM (1 L) et H₂O (200 mL). Après décantation, la phase aqueuse est extraite avec du DCM (200 mL). Les phases organiques sont réunies puis lavées avec une solution saturée en NaCl (400 mL), séchées sur MgSO₄ et concentrées sous pression réduite. Le résidu est alors purifié par flash chromatographie sur gel de silice. Le produit attendu est obtenu avec des rendements de 59 à 94%.

### Intermédiaire 213

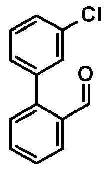

RMN ¹H: (400 MHz, dmso-d6) δ ppm 9.89 (s, 1 H), 7.94 (dd, 1 H), 7.77 (td, 1 H), 7.63 (tl, 1 H), 7.54 (dl, 1 H), 7.54/7.4 (2m, 4 H)

### Intermédiaire 214

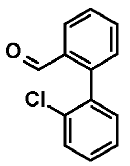

RMN ¹H: (400/500 MHz, dmso-d6) δ ppm 9.72 (s, 1 H), 7.96 (d, 1 H), 7.79 (t, 1 H), 7.65 (t, 1 H), 7.6 (t, 1 H), 7.48 (m, 3 H), 7.4 (d, 1 H)

### Intermédiaire 215

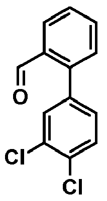

RMN ¹H: (400/500 MHz, dmso-d6) δ ppm 9.91 (s, 1 H), 7.95 (d, 1 H), 7.78 (m, 1 H), 7.78 (m, 1 H), 7.74 (d, 1 H), 7.64 (t, 1 H), 7.53 (d, 1 H), 7.44 (dd, 1 H)
DEI (70eV) : [M]+. = 250

### Intermédiaire 216

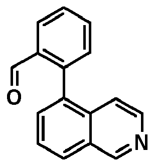

RMN ¹H: (400 MHz, dmso-d6) δ ppm 9.59 (s, 1 H), 9.43 (s, 1 H), 8.46 (d, 1 H), 8.26 (d, 1 H), 8.06 (d, 1 H), 7.85 (t, 1 H), 7.81 (t, 1 H), 7.77 (d, 1 H), 7.74 (t, 1 H), 7.51 (d, 1 H), 7.24 (d, 1 H)

### Intermédiaire 217

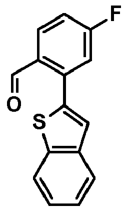

RMN ¹H: (400 MHz, CDCl3) δ ppm 9.2 (s, 1 H), 8.1 (dd, 1 H), 7.85 (m, 2 H), 7.4 (m, 2 H), 7.3 (dd+s, 2 H), 7.2 (td, 1 H)
RMN ¹⁹F: (400 MHz, CDCl3) δ ppm -101.1
GC-EI (70eV) : [M]+. = 256

### Intermédiaire 218

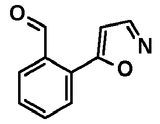

RMN ¹H: (400 MHz, dmso-d6) δ ppm 9.25 (s, 1 H), 8.8 (d, 1 H), 8 (m, 1 H), 7.85 (m, 1 H), 7.85/7.75 (2m, 2 H), 7.05 (d, 1 H)

### Intermédiaire 219

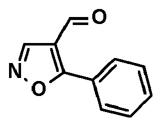

RMN ¹H: (400 MHz, dmso-d6) δ ppm 10.05 (s, 1 H), 9.2 (s, 1 H), 8.05 (m, 2 H), 7.7 (m, 3 H)

### Intermédiaire 220

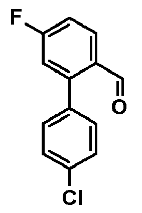

RMN ¹H: (400 MHz, dmso-d6) δ ppm 9.81 (s, 1 H), 8.02 (dd, 1 H), 7.58/7.52 (2d, 4 H), 7.46 (td, 1 H), 7.4 (dd, 1 H)
GC-EI (70eV) : [M]+. = 234

### Intermédiaire 221

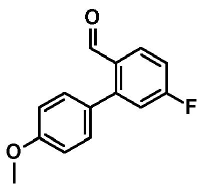

RMN ¹H: (400 MHz, dmso-d6) δ ppm 9.82 (s, 1 H), 7.97 (dd, 1 H), 7.42 (d, 2 H), 7.38 (td, 1 H), 7.35 (dd, 1 H), 7.09 (d, 2 H), 3.83 (s, 3 H)

### Intermédiaire 222

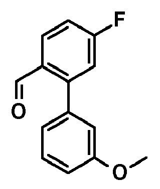

RMN ¹H: (400 MHz, dmso-d6) δ ppm 9.82 (s, 1 H), 7.99 (dd, 1 H), 7.42 (m, 3 H), 7.08/7.01 (2dl, 2 H), 7.06 (sl, 1 H), 3.82 (s, 3 H)

### Intermédiaire 223

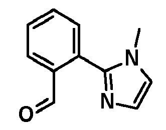

RMN ¹H: (400 MHz, dmso-d6) δ ppm 9.9 (s, 1 H), 7.92 (dd, 1 H), 7.78 (td, 1 H), 7.69 (dd, 1 H), 7.65 (dd, 1 H), 7.38 (d, 1 H), 7.07 (d, 1 H), 3.68 (s, 3 H)

### Intermédiaire 224

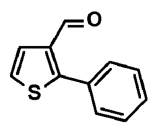

RMN ¹H: (400 MHz, dmso-d6) δ ppm 9.8 (sl, 1 H), 7.68 (dd, 1 H), 7.62 (m, 2 H), 7.54 (m, 3 H), 7.5 (d, 1 H)
GC-EI (70eV) : [M]+. = 188

### Intermédiaire 225

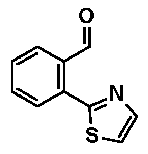

RMN ¹H: (400 MHz, dmso-d6) δ ppm 10.35 (s, 1 H), 8.07 (d, 1 H), 7.99 (d, 1 H), 7.88 (m, 2 H), 7.79/7.67 (2*t, 2 H)

### Intermédiaire 226

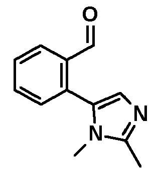

RMN ¹H : (400 MHz, dmso-d6) δ ppm 9.9 (s, 1 H), 7.95 (d, 1 H), 7.8 (m, 1 H), 7.65 (m, 1 H), 7.5 (d, 1 H), 6.85 (s, 1 H), 3.4 (s, 3 H), 2.4 (s, 3 H)

### Intermédiaire 227

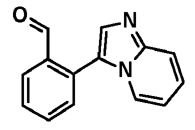

RMN ¹H: (400 MHz, dmso-d6) δ ppm 9.85 (s, 1 H), 8.15 (m, 1 H), 8.06 (m, 1 H), 7.85 (m, 1 H), 7.74 (s, 1 H), 7.7 (m, 1 H), 7.7 (m, 1 H), 7.7 (m, 1 H), 7.35 (m, 1 H), 6.95 (m, 1 H)

### Intermédiaire 228

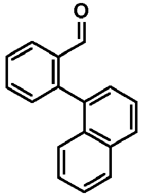

RMN ¹H: (400/500 MHz, dmso-d6) δ ppm 9.53 (s, 1 H), 8.08 (d, 1 H), 8.08 (d, 1 H), 8.02 (dd, 1 H), 7.83 (td, 1 H), 7.71 (tt, 1 H), 7.63 (dd, 1 H), 7.57 (td, 1 H), 7.51 (d, 1 H), 7.49 (d, 1 H), 7.49 (td, 1 H), 7.38 (dt, 1 H)
RMN ¹³C : (400/500 MHz, dmso-d6) δ ppm 192, 143.5, 135.5, 135, 134, 133, 132, 128.5, 128.5, 128, 128, 127, 127, 126.5, 125.5, 125

### Intermédiaire 229

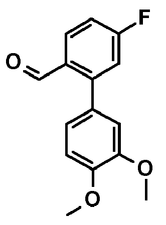

RMN ¹H: (400 MHz, CDCl3) δ ppm 9.9 (s, 1 H), 8.05 (dd, 1 H), 7.15 (m, 2 H), 7 (d, 1 H), 6.9 (m, 2 H), 3.95 (2 s, 6 H)
DEI (70eV) : [M]+. = 260

### Intermédiaire 230

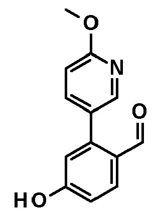

RMN ¹H: (400 MHz, dmso-d6) δ ppm 10.8 (sl, 1 H), 9.7 (s, 1 H), 8.2 (d, 1 H), 7.83 (d, 1 H), 7.79 (dd, 1 H), 6.95 (dd, 1 H), 6.91 (d, 1 H), 6.77 (d, 1 H), 3.9 (s, 3 H)

### Intermédiaire 231

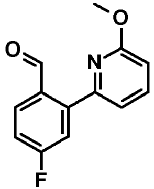

RMN ¹H: (400 MHz, CDCl3) δ ppm 10.2 (s, 1 H), 8 (dd, 1 H), 7.75 (t, 1 H), 7.4 (dd, 1 H), 7.2 (m, 2 H), 6.8 (d, 1 H), 3.95 (s, 3 H)
GC-EI (70eV) : [M]+. = 231

### Intermédiaire 232

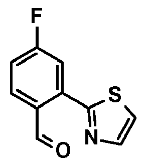

RMN ¹H : (400 MHz, CDCl3) δ ppm 10.5 (m, 1 H), 8.1 (dd, 1 H), 8 (m, 1 H), 7.5 (d, 1 H), 7.4 (dd, 1 H), 7.25 (td, 1 H)
GC-EI (70eV) : [M]+. = 207

### Intermédiaire 233

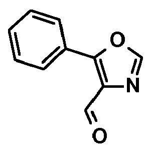

RMN ¹H: (400 MHz, dmso-d6) δ ppm 10 (s, 1 H), 8.65 (s, 1 H), 8.06 (m, 2 H), 7.6 (m, 3 H)

### Intermédiaire 234

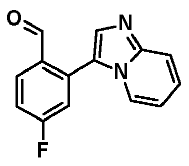

RMN ¹H : (400 MHz, CDCl3) δ ppm 9.8 (s, 1 H), 8.2 (dd, 1 H), 8 (d, 1 H), 7.8 (d, 1 H), 7.75 (s, 1 H), 7.3 (m, 3 H), 6.95 (t, 1 H)
GC-EI (70eV) : [M]+. = 240

### Intermédiaire 235

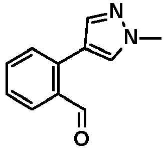

RMN ¹H: (400 MHz, dmso-d6) δ ppm 10.15 (d, 1 H), 8.05 (s, 1 H), 7.86 (dd, 1 H), 7.7 (d, 1 H), 7.68 (td, 1 H), 7.53 (dd, 1 H), 7.46 (td, 1 H), 3.91 (s, 3 H)

### Intermédiaire 236

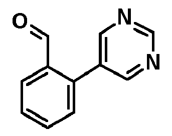

RMN ¹H : (400 MHz, dmso-d6) δ ppm 10 (s, 1 H), 9.25 (s, 1 H), 8.91 (s, 2 H), 8.05 (dd, 1 H), 7.83 (td, 1 H), 7.73 (td, 1 H), 7.58 (dd, 1 H)

### Intermédiaire 237

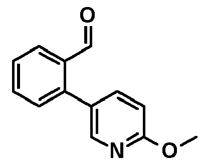

RMN ¹H: (300 MHz, dmso-d6) δ ppm 9.9 (s, 1 H), 8.25 (s, 1 H), 7.95 (d, 1 H), 7.85 (dd, 1 H), 7.75/7.6 (2*m, 2 H), 7.55 (d, 1 H), 6.95 (d, 1 H), 3.9 (s, 3 H)

### Intermédiaire 238

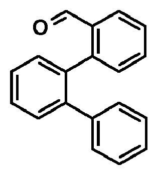

RMN ¹H: (400/500 MHz, dmso-d6) δ ppm 9.64 (s, 1 H), 7.68 (d, 1 H), 7.63 (t, 1 H), 7.57 (t, 1 H), 7.51 (t, 1 H), 7.47 (d, 1 H), 7.45 (t, 1 H), 7.42 (d, 1 H), 7.34 (d, 1 H), 7.17/7.03 (2*m, 5 H)

### Intermédiaire 239

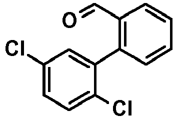

RMN ¹H: (400 MHz, dmso-d6) δ ppm 9.8 (s, 1 H), 8 (d, 1 H), 7.8 (t, 1 H), 7.69 (t, 1 H), 7.6 (d, 1 H), 7.55 (m, 2 H), 7.4 (d, 1 H)
GC-EI (70eV) : [M]+. = 250

### Intermédiaire 240

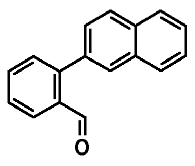

RMN ¹H: (400/500 MHz, dmso-d6) δ ppm 9.95 (s, 1 H), 8.07 (d, 1 H), 8.02 (m, 2 H), 7.99 (d, 1 H), 7.98 (dd, 1 H), 7.8 (td, 1 H), 7.64 (d, 1 H), 7.62 (td, 1 H), 7.62 (m, 1 H), 7.6 (m, 2 H)
RMN ¹³C: (400/500 MHz, dmso-d6) δ ppm 192, 134, 131.5, 129, 128, 128, 128, 128, 127.5, 127

### Intermédiaire 241

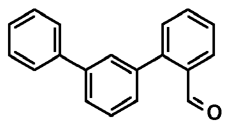

RMN ¹H: (400/500 MHz, dmso-d6) δ ppm 9.98 (s, 1 H), 7.95 (dd, 1 H), 7.78 (m, 1 H), 7.78 (m, 1 H), 7.75 (d, 2 H), 7.72 (t, 1 H), 7.63 (d, 1 H), 7.62 (t, 1 H), 7.61 (t, 1 H), 7.49 (t, 2 H), 7.44 (dt, 1 H), 7.4 (t, 1 H)
RMN ¹³C : (400/500 MHz, dmso-d6) δ ppm 192, 145.5, 140, 134, 133.5, 131, 129, 129, 128, 128, 128, 127.5, 127, 127, 127

### Intermédiaire 242

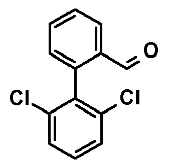

RMN ¹H: (400 MHz, dmso-d6) δ ppm 9.77 (s, 1 H), 8.05 (dd, 1 H), 7.82 (td, 1 H), 7.7 (td, 1 H), 7.6 (d, 2 H), 7.5 (t, 1 H), 7.35 (dd, 1 H)

### Intermédiaire 243

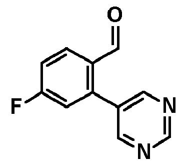

RMN ¹H: (400 MHz, dmso-d6) δ ppm 9.9 (s, 1 H), 9.25 (s, 1 H), 8.95 (s, 2 H), 8.15 (dd, 1 H), 7.55 (m, 2 H)
RMN ¹⁹F: (400 MHz, dmso-d6) δ ppm -102.8

### Intermédiaire 244

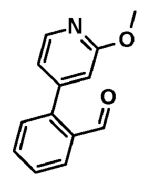

RMN ¹H : (400 MHz, CDCl3) δ ppm 10 (s, 1 H), 8.25 (dd, 1 H), 8.05 (dd, 1 H), 7.7 (td, 1 H), 7.55 (td, 1 H), 7.4 (dd, 1 H), 6.9 (dd, 1 H), 6.75 (dd, 1 H), 4 (s, 3 H)

### Intermédiaire 245

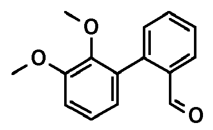

RMN ¹H: (400 MHz, dmso-d6) δ ppm 9.7 (s, 1 H), 7.9 (dd, 1 H), 7.75 (td, 1 H), 7.58 (t, 1 H), 7.43 (d, 1 H), 7.25-7.15 (m, 2 H), 6.91 (dd, 1 H), 3.87 (s, 3 H), 3.38 (s, 3 H)

### Intermédiaire 246

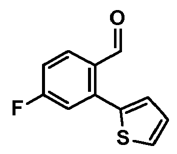

RMN ¹H: (300/400/500 MHz, dmso-d6) δ ppm 10.03 (s, 1 H), 7.98 (dd, 1 H), 7.83 (dd, 1 H), 7.46 (dd, 1 H), 7.43 (td, 1 H), 7.36 (dd, 1 H), 7.25 (dd, 1 H)
RMN ¹³C : (300/400/500 MHz, dmso-d6) δ ppm 190.2, 165, 140.3, 137.3, 131.2, 130.7, 130.6, 129, 128.4, 117.8, 115.9
RMN ¹⁹F : (300/400/500 MHz, dmso-d6) δ ppm -103

### Intermédiaire 247

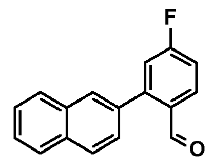

RMN ¹H: (300/400/500 MHz, dmso-d6) δ ppm 9.9 (s, 1 H), 8.06 (s, 1 H), 8.05 (d, 1 H), 8.04-8.02 (m, 3 H), 7.64 (dd, 1 H), 7.61 (td, 2 H), 7.51-7.47 (m, 2 H)
RMN ¹³C : (300/400/500 MHz, dmso-d6) δ ppm 190.4, 165, 148.3, 131.2, 130.6, 129.5-128.2, 128.2, 127.7, 127.1, 117.9/115.5
RMN ¹⁹F : (300/400/500 MHz, dmso-d6) δ ppm -103.2

### Intermédiaire 248

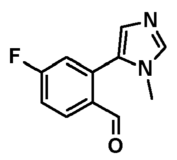

RMN ¹H : (400 MHz, CDCl3) δ ppm 9.88 (s, 1 H), 8.1 (dd, 1 H), 7.74 (s, 1 H), 7.29 (td, 1 H), 7.14 (s, 1 H), 7.1 (dd, 1 H), 3.56 (s, 3 H)

### Intermédiaire 249

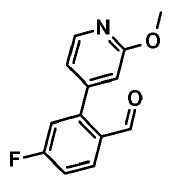

RMN ¹H: (400 MHz, dmso-d6) δ ppm 9.84 (s, 1 H), 8.27 (d, 1 H), 8.04 (dd, 1 H), 7.5 (td, 1 H), 7.43 (dd, 1 H), 7.1 (dd, 1 H), 6.96 (s, 1 H), 3.91 (s, 3 H)
RMN ¹⁹F : (400 MHz, CDCl3) δ ppm -101.2

### Intermédiaire 250

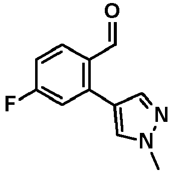

RMN ¹H: (400 MHz, dmso-d6) δ ppm 10.1 (s, 1 H), 8.12 (d, 1 H), 7.93 (d, 1 H), 7.78 (dd, 1 H), 7.39 (dd, 1 H), 7.3 (td, 1 H), 3.91 (s, 3 H)

### Intermédiaire 251

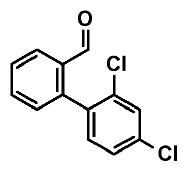

RMN ¹H: (400 MHz, dmso-d6) δ ppm 9.76 (s, 1 H), 7.98 (dd, 1 H), 7.79 (td, 1 H), 7.77 (d, 1 H), 7.68 (td, 1 H), 7.56 (dd, 1 H), 7.47 (d, 1 H), 7.39 (dd, 1 H)

### Intermédiaire 252

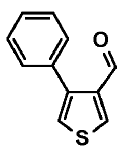

RMN ¹H: (400 MHz, dmso-d6) δ ppm 9.84 (s, 1 H), 8.59 (d, 1 H), 7.69 (d, 1 H), 7.5-7.3 (m, 5 H)

### Intermédiaire 253

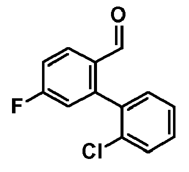

RMN ¹H: (400 MHz, dmso-d6) δ ppm 9.64 (s, 1 H), 8.04 (dd, 1 H), 7.62 (d, 1 H), 7.5 (m, 1 H), 7.5 (m, 3 H), 7.32 (dd, 1 H)

### Intermédiaire 254

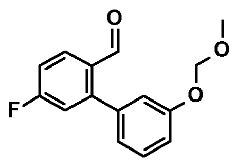

RMN ¹H : (400 MHz, dmso-d6) δ ppm 9.8 (s, 1 H), 8 (dd, 1 H), 7.45 (m, 3 H), 7.15 (m, 2 H), 7.1 (dd, 1 H), 5.25 (s, 2 H), 3.4 (s, 3 H)

### Intermédiaire 255

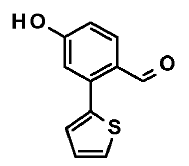

RNLN ¹H : (400 MHz, dmso-d6) δ ppm 10.75 (s, 1 H), 9.95 (s, 1 H), 7.85 (d, 1 H), 7.75 (d, 1 H), 7.25 (d, 1 H), 7.2 (d, 1 H), 6.95 (dd, 1 H), 6.9 (s, 1 H)
GC-EI (70eV) : [M]+. = 204

### Intermédiaire 256

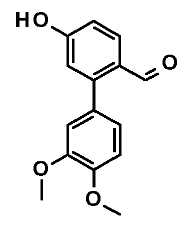

RMN ¹H : (400 MHz, dmso-d6) δ ppm 10.6 (s, 1 H), 9.7 (s, 1 H), 7.8 (d, 1 H), 7.05 (d, 1 H), 7 (s, 1 H), 6.9 (m, 2 H), 6.8 (s, 1 H), 3.8 (2s, 6 H)

### Intermédiaire 257

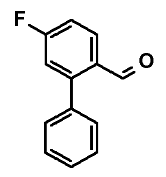

RMN ¹H : (300 MHz, CDCl3) δ ppm 9.9 (s, 1 H), 8.09 (dd, 1 H), 7.5/7.4 (massif, 5 H), 7.21 (dd, 1 H), 7.15 (dd, 1 H)
RMN ¹⁹F : (300 MHz, CDCl3) δ ppm -103.7

### Intermédiaire 258

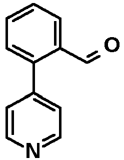

RMN¹H : (400 MHz, CDCl3) δ ppm 10 (s, 1 H), 8.73 (d, 2 H), 8.08 (dd, 1 H), 7.71 (dt, 1 H), 7.61 (dt, 1 H), 7.42 (dd, 1 H), 7.35 (d, 2 H)

### Intermédiaire 259

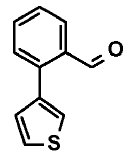

RMN ¹H : (400 MHz, CDCl3) δ ppm 10.1 (d, 1 H), 8 (dd, 1 H), 7.62 (dt, 1 H), 7.48 (2*m, 2 H), 7.46 (dd, 1 H), 7.3 (dd, 1 H), 7.2 (dd, 1 H)

### Intermédiaire 260

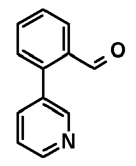

RMN ¹H : (400 MHz, CDCl3) δ ppm 9.99 (s, 1 H), 8.71 (dd, 1 H), 8.67 (d, 1 H), 8.07 (dl, 1 H), 7.75 (dt, 1 H), 7.7 (tl, 1 H), 7.59 (tl, 1 H), 7.44 (dd, 1 H), 7.43 (dl, 1 H)

### Intermédiaire 261

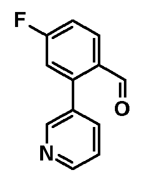

RMN ¹H : (300 MHz, CDCl3) δ ppm 9.9 (d, 1 H), 8.74 (dd, 1 H), 8.69 (dd, 1 H), 8.12 (dd, 1 H), 7.74 (ddd, 1 H), 7.47 (ddd, 1 H), 7.28 (m, 1 H), 7.15 (dd, 1 H)
RMN ¹⁹F : (300 MHz, CDCl3) δ ppm -102.6

### Intermédiaire 262

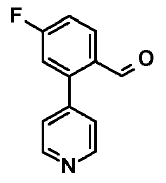

RMN ¹H : (400 MHz, CDCl3) δ ppm 9.89 (s, 1 H), 8.75 (d, 2 H), 8.11 (dd, 1 H), 7.33 (d, 2 H), 7.27 (td, 1 H), 7.12 (dd, 1 H)

### Intermédiaire 263

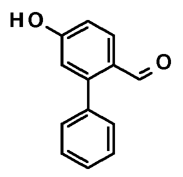

RMN ¹H : (400 MHz, CDCl3) δ ppm 9.82 (s, 1 H), 8.01 (d, 1 H), 7.45 (m, 3 H), 7.38 (m, 2 H), 6.94 (dd, 1 H), 6.85 (df, 1 H), 5.72 (sl, 1 H)

### Intermédiaire 264

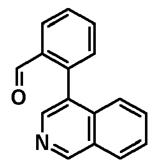

RMN¹H : (400 MHz, CDCl3) δ ppm 9.68 (s, 1 H), 9.35 (s, 1 H), 8.5 (s, 1 H), 8.15 (dd, 1 H), 8.1 (m, 1 H), 7.75 (dt, 1 H), 7.67 (2*m, 2 H), 7.65 (dt, 1 H), 7.51 (m, 1 H), 7.47 (dd, 1 H) GC-EI (70eV) : [M]+. = 233.1

### Intermédiaire 265

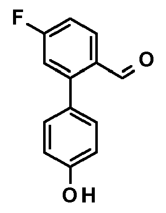

RMN ¹H : (300 MHz, CDCl3) δ ppm 9.9 (sl, 1 H), 8.03 (dd, 1 H), 7.24 (d, 2 H), 7.12 (td, 1 H), 7.1 (dd, 1 H), 6.95 (d, 2 H), 5.3 (s, 1 H)
RMN ¹⁹F : (300 MHz, CDCl3) δ ppm -102.4

### Intermédiaire 266

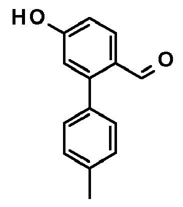

RMN ¹H : (400 MHz, dmso-d6) δ ppm 10.65 (s, 1 H), 9.65 (s, 1 H), 7.8 (d, 1 H), 7.3 (s, 4 H), 6.9 (dd, 1 H), 6.75 (s, 1 H), 2.35 (d, 3 H)

### Intermédiaire 267

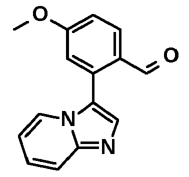

RMN ¹H : (400 MHz, dmso-d6) δ ppm 9.8 (s, 1 H), 8.2 (d, 1 H), 8.05 (d, 1 H), 7.75 (s, 1 H), 7.7 (d, 1 H), 7.35 (t, 1 H), 7.25 (dd, 1 H), 7.2 (s, 1 H), 6.95 (t, 1 H), 3.9 (s, 3 H)

### Intermédiaire 268

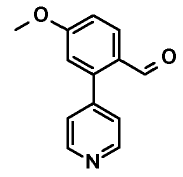

RMN ¹H : (400 MHz, dmso-d6) δ ppm 9.75 (s, 1 H), 8.69 (d, 2 H), 7.97 (d, 1 H), 7.7 (dd, 1 H), 7.5 (d, 2 H), 7 (d, 1 H), 3.9 (s, 3 H)

### Intermédiaire 269

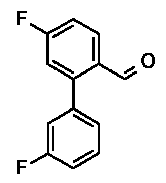

RMN ¹H : (300/400 MHz, dmso-d6) δ ppm 9.8 (s, 1 H), 8 (dd, 1 H), 7.55 (m, 1 H), 7.5 (m, 1 H), 7.45 (d, 1 H), 7.45 (d, 1 H), 7.35 (m, 1 H), 7.3 (dd, 1 H)
RMN ¹⁹F : (300/400 MHz, dmso-d6) δ ppm -103.1, -111.7

### Intermédiaire 270

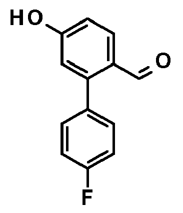

RMN ¹H : (400 MHz, dmso-d6) δ ppm 10.75 (sl, 1 H), 9.65 (s, 1 H), 7.81 (d, 1 H), 7.45 (dd, 2 H), 7.3 (dd, 2 H), 6.93 (dd, 1 H), 6.75 (d, 1 H)

### Intermédiaire 271

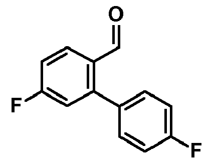

RMN ¹H : (400 MHz, CDCl3) δ ppm 9.88 (s, 1 H), 8.06 (dd, 1 H), 7.36 (dd, 2 H), 7.19 (m, 1 H), 7.19 (dd, 2 H), 7.11 (dd, 1 H)

### Intermédiaire 272

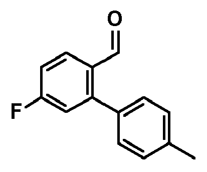

RMN ¹H : (400 MHz, CDCl3) δ ppm 9.9 (s, 1 H), 8.05 (dd, 1 H), 7.28 (dd, 4 H), 7.16 (td, 1 H), 7.12 (dd, 1 H), 2.44 (s, 3 H)

### Intermédiaire 273

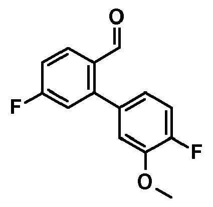

RMN ¹H : (400 MHz, dmso-d6) δ ppm 9.82 (s, 1 H), 8 (dd, 1 H), 7.48-7.3 (m, 2 H), 7.48-7.3 (m, 2 H), 7 (ddd, 1 H), 3.9 (s, 3 H)
RMN ¹⁹F : (400 MHz, dmso-d6) δ ppm -104.4, -135.7

### Intermédiaire 274

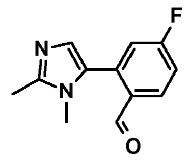

RMN ¹H : (400 MHz, dmso-d6) δ ppm 9.8 (s, 1 H), 8.05 (dd, 1 H), 7.45 (m, 1 H), 7.4 (d, 1 H), 6.9 (s, 1 H), 3.45 (s, 3 H), 2.35 (s, 3 H)
RMN ¹⁹F : (400 MHz, dmso-d6) δ ppm -102.9

### Intermédiaire 275

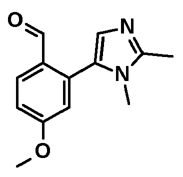

RMN ¹H : (400 MHz, dmso-d6) δ ppm 9.7 (s, 1 H), 7.95 (d, 1 H), 7.15 (dd, 1 H), 7 (s, 1 H), 6.85 (s, 1 H), 3.9 (s, 3 H), 3.4 (s, 3 H), 2.35 (s, 3 H)

### Intermédiaire 276

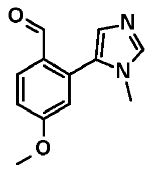

RMN ¹H : (300/400 MHz, dmso-d6) δ ppm 9.7 (s, 1 H), 7.93 (d, 1 H), 7.8 (s, 1 H), 7.18 (dd, 1 H), 7.03 (d, 1 H), 7 (s, 1 H), 3.9 (s, 3 H), 3.54 (s, 3 H)

### Intermédiaire 277

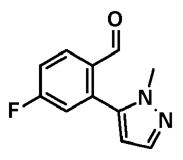

RMN ¹H : (400 MHz, CDCl3) δ ppm 9.85 (s, 1 H), 8.15 (dd, 1 H), 7.6 (s, 1 H), 7.3 (m, 1 H), 7.15 (d, 1 H), 6.35 (s, 1 H), 3.75 (s, 3 H)
RMN ¹⁹F : (400 MHz, CDCl3) δ ppm -101.2

### Intermédiaire 278

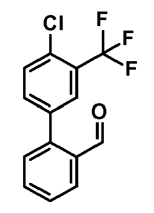

RMN ¹H : (400 MHz, dmso-d6) δ ppm 9.9 (s, 1 H), 7.98 (dd, 1 H), 7.9 (d, 1 H), 7.84 (d, 1 H), 7.78 (td, 1 H), 7.77 (dd, 1 H), 7.67 (tl, 1 H), 7.55 (dd, 1 H)
DEI (70eV) : [M]+. = 284

### Intermédiaire 279

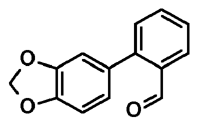

RMN¹H : (400 MHz, dmso-d6) δ ppm 9.9 (s, 1 H), 7.9 (d, 1 H), 7.75 (m, 1 H), 7.55 (m, 1 H), 7.5 (d, 1 H), 7.1 (s, 1 H), 7.05 (d, 1 H), 6.85 (dd, 1 H), 6.1 (s, 2 H)

### Intermédiaire 280

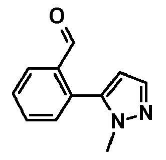

RMN ¹H : (400 MHz, dmso-d6) δ ppm 9.8 (s, 1 H), 7.99 (d, 1 H), 7.8 (t, 1 H), 7.69 (t, 1 H), 7.58 (d, 1 H), 7.55 (d, 1 H), 6.41 (d, 1 H), 3.69 (s, 3 H)
RMN ¹³C : (400 MHz, dmso-d6) δ ppm 191, 138, 138, 136, 134, 133, 132, 130, 128, 109, 37

### Intermédiaire 281

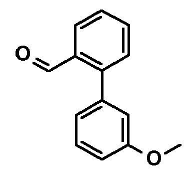

RMN ¹H : (400 MHz, dmso-d6) δ ppm 9.9 (s, 1 H), 7.91 (d, 1 H), 7.75 (t, 1 H), 7.59 (t, 1 H), 7.54 (d, 1 H), 7.42 (t, 1 H), 7.05 (dd, 1 H), 7.01 (t, 1 H), 6.98 (dd, 1 H), 3.82 (s, 3 H)

### Intermédiaire 282

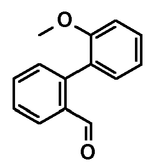

RMN ¹H : (400 MHz, dmso-d6) δ ppm 9.66 (s, 1 H), 7.86 (d, 1 H), 7.73 (t, 1 H), 7.54 (t, 1 H), 7.46 (t, 1 H), 7.38 (d, 1 H), 7.31 (d, 1 H), 7.14 (d, 1 H), 7.11 (t, 1 H), 3.69 (s, 3 H)

### Intermédiaire 283

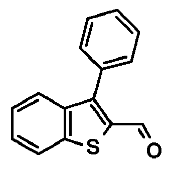

RMN ¹H : (400 MHz, dmso-d6) δ ppm 9.86 (s, 1 H), 8.17 (d, 1 H), 7.78 (d, 1 H), 7.7-7.55 (m, 1 H), 7.7-7.55 (m, 5 H), 7.52 (t, 1 H)

### Intermédiaire 284

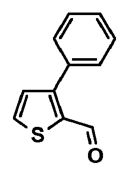

RMN ¹H : (400 MHz, dmso-d6) δ ppm 9.82 (d, 1 H), 8.17 (dd, 1 H), 7.62 (d, 2 H), 7.52 (m, 3 H), 7.43 (d, 1 H)

### Intermédiaire 285

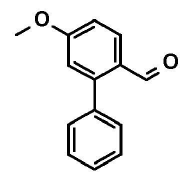

RMN ¹H : (400 MHz, CDCl3) δ ppm 9.85 (s, 1 H), 8.03 (d, 1 H), 7.5-7.37 (m, 5 H), 7 (dd, 1 H), 6.89 (df, 1 H), 3.9 (s, 3 H)

### Intermédiaire 286

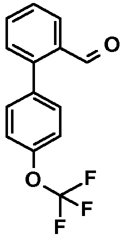

RMN ¹H : (400 MHz, dmso-d6) δ ppm 9.89 (s, 1 H), 7.95 (d, 1 H), 7.77 (t, 1 H), 7.62 (m, 3 H), 7.53 (d, 1 H), 7.49 (d, 2 H)
RMN ¹⁹F : (400 MHz, dmso-d6) δ ppm -55.59

### Intermédiaire 287

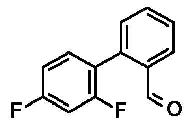

RMN ¹H : (400 MHz, CDCl3) δ ppm 9.9 (d, 1 H), 8.04 (dd, 1 H), 7.68 (dt, 1 H), 7.56 (t, 1 H), 7.39 (d, 1H), 7.31 (dt, 1 H), 7.01 (dt, 1 H), 6.95 (dt, 1 H)
RMN ¹⁹F : (400 MHz, CDCl3) δ ppm -109/-110

### Intermédiaire 288

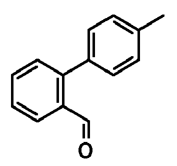

RMN ¹H : (400 MHz, CDCl3) δ ppm 10 (d, 1 H), 8.01 (dd, 1 H), 7.63 (dt, 1 H), 7.48 (t, 1 H), 7.44 (d, 1 H), 7.29 (s, 4 H), 2.43 (s, 3 H)

### Intermédiaire 289

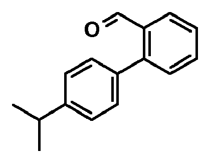

RMN ¹H : (400 MHz, CDCl3) δ ppm 10 (s, 1 H), 8.02 (d, 1 H), 7.62 (t, 1 H), 7.45 (d, 1 H), 7.32 (2*d, 4 H), 4.48 (t, 1 H), 2.99 (hept., 1 H), 1.31 (d, 6 H)

### Intermédiaire 290

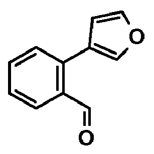

RMN ¹H : (400 MHz, CDCl3) δ ppm 10.27 (d, 1 H), 7.99 (dd, 1 H), 7.61 (dt, 1 H), 7.55 (2*m, 2 H), 7.46 (2*m, 2 H), 6.59 (dd, 1 H)

### Intermédiaire 291

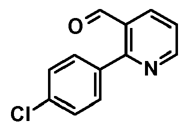

RMN ¹H : (300 MHz, dmso-d6) δ ppm 9.99 (s, 1 H), 8.86 (d, 1 H), 8.82 (df, 1 H), 7.79 (dd, 1 H), 7.6 (2*d, 4 H)

### Intermédiaire 292

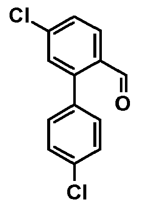

RMN ¹H : (400 MHz, dmso-d6) δ ppm 9.85 (s, 1 H), 7.95 (d, 1 H), 7.65 (dd, 1 H), 7.6 (s, 1 H), 7.55 (d, 2 H), 7.5 (d, 2 H)

### Intermédiaire 293

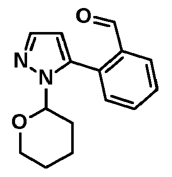

RMN ¹H : (400 MHz, dmso-d6) δ ppm 9.7 (s, 1 H), 8 (dd, 1 H), 7.8 (td, 1 H), 7.7 (td, 1 H), 7.65 (d, 1 H), 7.55 (dd, 1 H), 6.5 (d, 1 H), 5 (dd, 1 H), 3.8/3.25 (2m, 2 H), 2.25/1.8 (2m, 2 H), 1.9/1.5 (2m, 2 H), 1.4 (m, 2 H)

### Procédure F : Mode opératoire générique d'amination réductrice à partir de l'intermédiaire 21 (synthèse des intermédiaires 22 à 127).

Dans un ballon tricol de 500 mL à température ambiante et sous flux argon sont introduits successivement l'intermédiaire 21 (14 g, 26.2 mmol) du DCM anhydre (280 mL), l'aldéhyde (intermédiaires 213 à 293) (39.3 mmol, 1.5 eq) ainsi que du MgSO₄ (14 g). Après 1h d'agitation, NaBH(OAc)₃ (8.32 g, 39.3 mmol, 1.5 eq) est ajouté par portion et le mélange réactionnel est maintenu à température ambiante pendant 16h. La réaction est monitorée par LC/MS. Les insolubles sont filtrés sur microfibre et rincés avec du DCM (100 mL). Le filtrat est alors lavé à l'eau (1 x 200 mL) puis avec une solution saturée en NaCl (2 x 200 mL). La phase organique est séchée sur MgSO₄ et concentrée sous pression réduite. L'huile obtenue est alors purifiée par flash chromatographie sur gel de silice (330g) pour conduire aux intermédiaires 22 à 127.

### EXEMPLE 31 : Acide 3-(4-aminobutyl)-4-hydroxy-4-oxo-1,4-azaphosphinane-3-carboxylique

L'exemple 31 est obtenu à partir de l'intermédiaire 21 selon la procédure D précédemment décrite.
RMN ¹H : (400 MHz, D2O) δ ppm 3.5-3.65 (2m, 2 H), 3.2 (m, 1 H), 3.05 (dd, 1 H), 2.95 (m, 2 H), 2.15 (m, 1 H), 1.95 (m, 1 H), 1.75 (m, 1 H), 1.65 (m, 2 H), 1.5 (m, 1 H), 1.4 (m, 1 H), 1.25 (m, 1 H)
ESI/FIA/HR et MS/MS : [M+H]+ = 251.1154 (251.1160)
Analyse Elémentaire : C=42.65(43.20);H=7.23(7.65);N=11.24(11.20)

### Intermédiaire 22 : 3-{4-[Bis(tert-butoxycarbonyl)amino]butyl}-1-[(3-fluoro-4-hydroxyphényl)méthyl]-4-éthoxy-4-oxo-1,4-azaphosphinane-3-carboxylate de tert-butyle

L'intermédiaire 22 est obtenu à partir de l'intermédiaire 21 et du 3-fluoro-4-hydroxybenzaldéhyde selon la procédure F précédemment décrite.
RMN ¹H : (400 MHz, dmso-d6) δ ppm 7.0 (d, 1 H), 6.88 (d, 2 H), 3.95 (m, 2 H), 3.5/3.33 (2*d, 2 H), 3.4 (m, 2 H), 2.85/2.3 (2*m, 2 H), 2.8/2.45 (dd, 2 H), 2-1.6 (m, 4 H), 1.42/1.35 (2*s, 27 H), 1.4 (m, 2 H), 1.2 (t, 3 H), 0.9 (m, 2 H)

### EXEMPLE 32 : Acide 3-(4-aminobutyl)-1-[(3-fluoro-4-hydroxyphényl)méthyl]-4-hydroxy-4-oxo-1,4-azaphosphinane-3-carboxylique

L'exemple 32 est obtenu à partir de l'intermédiaire 22 selon la procédure D précédemment décrite.
RMN ¹H : (300/400 MHz, D2O) δ ppm 7.24 (dd, 1 H), 7.12 (dd, 1 H), 7.03 (t, 1 H), 4.32/4.12 (2*d, 2 H), 3.68/3.3 (2*m, 2 H), 3.45/3.07 (2*m, 2 H), 2.92 (m, 2 H), 2.22/1.78 (2*m, 2 H), 1.92/1.58 (2*m, 2 H), 1.58/1.46 (2*m, 2 H), 1.23/1.1 (2*m, 2 H)
RMN ¹⁹F : (300/400 MHz, D2O) δ ppm -135.8
ESI/FIA/HR et MS/MS : [M+H]+ = 375.1455 (375.1480)
Analyse Elémentaire : C=51.71(51.34);H=6.44(6.46);N=7.64(7.48)

### Intermédiaire 23 : 3-{4-[Bis(tert-butoxycarbonyl)amino]butyl}-1-[(2,4-difluorophényl)méthyl]-4-éthoxy-4-oxo-1,4-azaphosphinane-3-carboxylate de tert-butyle

L'intermédiaire 23 est obtenu à partir de l'intermédiaire 21 et du 2,4-difluorobenzaldéhyde selon la procédure F précédemment décrite.
RMN ¹H : (400 MHz, dmso-d6) δ ppm 7.44 (m, 1 H), 7.19 (m, 1 H), 7.07 (m, 1 H), 4 (m, 2 H), 3.57 (m, 2 H), 3-2.6/2.35 (m, 2 H), 3-2.6/2.52 (m, 2 H), 2.78 (m, 2 H), 2-1.6 (m, 2 H), 2-1.6 (m, 2 H), 1.39 (m, 27 H), 1.39 (m, 2 H), 1.2 (m, 3 H), 0.82 (m, 2 H)

### EXEMPLE 33 : Acide 3-(4-aminobutyl)-1-[(2,4-difluorophényl)méthyl]-4-hydroxy-4-oxo-1,4-azaphosphinane-3-carboxylique

L'exemple 33 est obtenu à partir de l'intermédiaire 23 selon la procédure D précédemment décrite.
RMN ¹H : (400 MHz, D2O) δ ppm 7.51 (m, 1 H), 7.07 (m, 2 H), 4.37 (sl, 2 H), 3.69/3.33 (m, 2 H), 3.49/3.19 (m, 2 H), 2.95 (m, 2 H), 2.21/1.77 (m, 2 H), 1.95/1.49 (m, 2 H), 1.61 (m, 2 H), 1.28/1.14 (m, 2 H)
ESI/FIA/HR et MS/MS : [M+H]+ = 377.1419 (377.1441)
Analyse Elémentaire : C=50.66(51.06);H=5.79(6.16);N=7.37(7.44)

### Intermédiaire 24 : 3-{4-[Bis(tert-butoxycarbonyl)amino]butyl}-1-[(3,5-difluoro-4-hydroxyphényl)méthyl]-4-éthoxy-4-oxo-1,4-azaphosphinane-3-carboxylate de tert-butyle

L'intermédiaire 24 est obtenu à partir de l'intermédiaire 21 et du 3,5-difluoro-4hydroxy-benzaldéhyde selon la procédure F précédemment décrite.
RMN ¹H : (300 MHz, dmso-d6) δ ppm 10.1 (sl, 1 H), 6.63 (d, 2 H), 4.1-3.8 (m, 2 H), 3.58-3.3 (m, 2 H), 3.58-3.3 (m, 2 H), 3-2.6/2.3 (2*m, 2 H), 3-2.6/2.49 (2*m, 2 H), 2-1.5 (m, 2 H), 2-1.5 (m, 2 H), 1.4 (m, 2 H), 1.4/1.37 (2*s, 27 H), 1.2 (t, 3 H), 1.1-0.8 (m, 2 H)

### EXEMPLE 34 : Acide 3-(4-aminobutyl)-1-[(3,5-difluoro-4-hydroxyphényl)méthyl]-4-hydroxy-4-oxo-1,4-azaphosphinane-3-carboxylique

L'exemple 34 est obtenu à partir de l'intermédiaire 24 selon la procédure D précédemment décrite.
RMN ¹H : (300/400 MHz, D2O) δ ppm 7.1 (m, 2 H), 4.33/4.12 (2*d, 2 H), 3.69/3.31 (2*dd, 2 H), 3.45/3.09 (2*dd, 2 H), 2.94 (m, 2 H), 2.24/1.78 (2*m, 2 H), 1.93/1.6 (2*m, 2 H), 1.6/1.47 (2*m, 2 H), 1.25/1.13 (2*m, 2 H)
RMN ¹⁹F : (300/400 MHz, D2O) δ ppm -132
ESI/FIA/HR et MS/MS : [M+H]+ = 393.1388 (393.1390)
Analyse Elémentaire : C=48.74(48.98);H=5.90(5.91);N=7.10(7.14)

### Intermédiaire 25 : 3-{4-[Bis(tert-butoxycarbonyl)amino]butyl}-1-[[4-(difluorométhyl)phényl]méthyl]-4-éthoxy-4-oxo-1,4-azaphosphinane-3-carboxylate de tert-butyle

L'intermédiaire 25 est obtenu à partir de l'intermédiaire 21 et du 4-(difluorométhyl)benzaldéhyde selon la procédure F précédemment décrite.
RMN ¹H : (400 MHz, dmso-d6) δ ppm 7.55-7.4 (dd, 4 H), 7 (t, 1 H), 4.1-3.85 (m, 2 H), 3.7-3.5 (dd, 2 H), 3.35 (m, 2 H), 3-2.25 (m, 4 H), 2-1.8 (m, 4 H), 1.4 (m, 2 H), 1.4 (s, 18 H), 1.35 (s, 9 H), 1.2 (t, 3 H), 0.8 (m, 2 H).

### EXEMPLE 35 : Acide 3-(4-aminobutyl)-1-[[4-(difluorométhyl)phényl]méthyl]-4-hydroxy-4-oxo-1,4-azaphosphinane-3-carboxylique

L'exemple 35 est obtenu à partir de l'intermédiaire 25 selon la procédure D précédemment décrite.
RMN ¹H : (400 MHz, D2O) δ ppm 7.7-7.6 (d, 4 H), 6.85 (t, 1 H), 4.5/4.3 (m, 2 H), 3.8-3.65 (m, 1 H), 3.55-3.35 (m, 2 H), 3.15 (m, 1 H), 2.95 (m, 2 H), 2.25 (m, 1 H), 2-1.75 (m, 2 H), 1.65-1.5 (m, 3 H), 1.3-1.1 (m, 2 H)
RMN ³¹P : (400 MHz, D2O) δ ppm 26
RMN ¹⁹F : (400 MHz, D2O) δ ppm -110
ESI/FIA/HR et MS/MS : [M+H]+ = 391.1583 (391.1598)
Analyse Elémentaire : C=51.85(52.31);H=6.46(6.45);N=7.04(7.18)

### Intermédiaire 26 : 3-{4-[Bis(tert-butoxycarbonyl)amino]butyl}-4-éthoxy-l-[[4-hydroxy-3-(trifluorométhyl)phényl]méthyl]-4-oxo-1,4-azaphosphinane-3-carboxylate de tert-butyle

L'intermédiaire 26 est obtenu à partir de l'intermédiaire 21 et du 4-hydroxy-3-(trifluorométhyl)benzaldéhyde selon la procédure F précédemment décrite.
RMN ¹H : (400 MHz, dmso-d6) δ ppm 10.45 (massif, 1 H), 7.4 (sl, 1 H), 7.34 (dl, 1 H), 6.96 (d, 1 H), 4.1-3.85 (m, 2 H), 3.65-3.3 (m, 2 H), 3.65-3.3 (m, 2 H), 3-2.2 (m, 4 H), 2-1.6 (m, 4 H), 1.38 (m, 29 H), 1.2 (t, 3 H), 0.88 (m, 2 H)

### EXEMPLE 36 : Acide 3-(4-aminobutyl)-4-hydroxy-1-[[4-hydroxy-3-(trifluorométhyl)phényl]méthyl]-4-oxo-1,4-azaphosphinane-3-carboxylique

L'exemple 36 est obtenu à partir de l'intermédiaire 26 selon la procédure D précédemment décrite.
RMN ¹H : (400 MHz, D2O) δ ppm 7.68 (df, 1 H), 7.55 (dd, 1 H), 7.09 (d, 1 H), 4.38/4.2 (2*d, 2 H), 3.7/3.3 (2*dd, 2 H), 3.45/3.09 (2*dd, 2 H), 2.92 (m, 2 H), 2.23/1.77 (2*m, 2 H), 1.93/1.6 (2*m, 2 H), 1.6/1.47 (2*m, 2 H), 1.25/1.11 (2*m, 2 H)
ESI/FIA/HR et MS/MS : [M+H]+ = 425.1453 (425.1453)
Analyse Elémentaire : C=48.19(48.12);H=5.16(5.70);N=6.72(6.60)

### Intermédiaire 27 : 3-{4-[Bis(tert-butoxycarbonyl)amino]butyl}-1-[(3-chloro-5-fluoro-4-hydroxyphényl)méthyl]-4-éthoxy-4-oxo-1,4-azaphosphinane-3-carboxylate de tert-butyle

L'intermédiaire 27 est obtenu à partir de l'intermédiaire 21 et du 3-chloro-5-fluoro-4-hydroxybenzaldéhyde selon la procédure F précédemment décrite.
RMN ¹H : (400 MHz, dmso-d6) δ ppm 10.26 (sl, 1 H), 7.12 (sl, 1 H), 7.07 (dl, 1 H), 4.06/3.98 (2quad, 2 H), 3.47 (AB, 2 H), 3.42 (m, 2 H), 3-2.26 (m, 4 H), 1.94 (m, 4 H), 1.41/1.37 (2s, 27 H), 1.4 (m, 2 H), 1.25/1.21 (2t, 3 H), 0.92 (m, 2 H)
RMN ¹⁹F : (400 MHz, dmso-d6) δ ppm -131.7
RMN ³¹P : (400 MHz, dmso-d6) δ ppm -43
ESI/FIA/HR et MS/MS : [M+H]+ = 693.308 (693.3083)

### EXEMPLE 37 : Acide 3-(4-aminobutyl)-1-[(3-chloro-5-fluoro-4-hydroxyphényl)méthyl]-4- hydroxy-4-oxo-1,4-azaphosphinane-3-carboxylique

L'exemple 37 est obtenu à partir de l'intermédiaire 27 selon la procédure D précédemment décrite.
RMN ¹H : (400 MHz, D2O) δ ppm 7.3 (m, 1 H), 7.21 (m, 1 H), 4.31/4.12 (2*d, 2 H), 3.69/3.3 (2*dd, 2 H), 3.45/3.09 (2*dd, 2 H), 2.93 (m, 2 H), 2.24/1.77 (2*m, 2 H), 1.93/1.6 (2*m, 2 H), 1.6/1.47 (2*m, 2 H), 1.27/1.13 (2*m, 2 H)
RMN ¹⁹F : (400 MHz, D2O) δ ppm -131.6
ESI/FIA/HR et MS/MS : [M+H]+ = 409.1091 (409.1095)
Analyse Elémentaire : C=47.25(47.01);H=5.75(5.67);N=6.92(6.85)

### Intermédiaire 28 : 3-{4-[Bis(tert-butoxycarbonyl)amino]butyl}-1-(2,3-dihydro-1-benzofuran-5-ylméthyl)-4-éthoxy-4-oxo-1,4-azaphosphinane-3-carboxylate de tert-butyle

L'intermédiaire 28 est obtenu à partir de l'intermédiaire 21 et du 2,3-dihydrobenzofuran-5-carbaldéhyde selon la procédure F précédemment décrite.
RMN ¹H : (400 MHz, dmso-d6) δ ppm 7.13 (d, 1 H), 6.97 (dd, 1 H), 6.68 (d, 1 H), 4.5 (t, 2 H), 4 (m, 2 H), 3.55-3.3 (m, 4 H), 3.15 (m, 2 H), 3-2.2 (m, 4 H), 1.91 (m, 4 H), 1.39 (m, 29 H), 1.2 (t, 3 H), 0.87 (m, 2 H)

### EXEMPLE 38 : Acide 3-(4-aminobutyl)-1-(2,3-dihydro-1-benzofuran-5-ylméthyl)-4-hydroxy-4-oxo-1,4-azaphosphinane-3-carboxylique

L'exemple 38 est obtenu à partir de l'intermédiaire 28 selon la procédure D précédemment décrite.
RMN ¹H : (400 MHz, D2O) δ ppm 7.34 (d, 1 H), 7.2 (dd, 1 H), 6.85 (d, 1 H), 4.59 (t, 2 H), 4.14/3.68 (dd, 2 H), 3.68/3.28 (m, 2 H), 3.47/3.06 (m, 2 H), 3.22 (t, 2 H), 2.94 (m, 2 H), 2.22/1.76 (m, 2 H), 1.93/1.47 (m, 2 H), 1.6 (m, 2 H), 1.26/1.12 (m, 2 H)
ESI/FIA/HR et MS/MS : [M+H]+ = 383.1725 (383.1735)
Analyse Elémentaire : C=55.92(56.54);H=6.71(7.12);N=7.17(7.33)

### Intermédiaire 29 : 3-{4-[Bis(tert-butoxycarbonyl)amino]butyl}-4-éthoxy-4-oxo-1-[(2-oxo-3H-1,3-benzoxazol-6-yl)méthyl] -1,4-azaphosphinane-3-carboxylate de tert-butyle

L'intermédiaire 29 est obtenu à partir de l'intermédiaire 21 et du 3H-1,3-benzoxazole-carbaldéhyde selon la procédure F précédemment décrite.
RMN ¹H : (400 MHz, dmso-d6) δ ppm 12.55 (s, 1 H), 7.15 (s, 1 H), 7.05 (dd, 1 H), 7 (d, 1 H), 4 (m, 2 H), 3.65 (d, 1 H), 3.4 (d, 1 H), 3.35 (m, 2 H), 3.05-2.3 (m, 4 H), 2.05-1.8 (m, 4 H), 1.4 (m, 2 H), 1.4 (3s, 27 H), 1.25 (t, 3 H), 0.9-0.7 (m, 2 H)
ESI/FIA/HR et MS/MS : [M+H]+ = 682.3461 (682.3468)

### EXEMPLE 39 : Acide 3-(4-aminobutyl)-4-hydroxy-4-oxo-1-[(2-oxo-3H-1,3-benzoxazol-6-yl)méthyl]-1,4-azaphosphinane-3-carboxylique

L'exemple 39 est obtenu à partir de l'intermédiaire 29 selon la procédure D précédemment décrite.
RMN ¹H : (400 MHz, D2O) δ ppm 7.4 (s, 1 H), 7.3 (dd, 1 H), 7.25 (d, 1 H), 4.45/4.25 (2dd, 2 H), 3.8-3.6 (m, 1 H), 3.45 (m, 1 H), 3.35 (m, 1 H), 3.1 (m, 1 H), 2.95 (m, 2 H), 2.25 (m, 2 H), 1.95 (m, 1 H), 1.65-1.4 (m, 3 H), 1.25/1.1 (2*m, 2 H)
ESI/FIA/HR et MS/MS : [M+H]+ = 398.1455 (398.1480)
Analyse Elémentaire : C=51.17(51.39);H=5.85(6.09);N=10.49(10.57)

### Intermédiaire 30 : 3-{4-[Bis(tert-butoxycarbonyl)amino]butyl}-1-(1H-benzimidazol-5-ylméthyl)-4-éthoxy-4-oxo-1,4-azaphosphinane-3-carboxylate de tert-butyle

L'intermédiaire 30 est obtenu à partir de l'intermédiaire 21 et du 1H-benzimidazole-5-carbaldéhyde selon la procédure F précédemment décrite.
RMN ¹H : (400 MHz, dmso-d6) δ ppm 12.4 (sl, 1 H), 8.16 (s, 1 H), 7.7-7.3 (m, 2 H), 7.14 (d, 1 H), 3.97 (m, 2 H), 3.72/3.54 (2*d, 2 H), 3.45-3.2 (m, 2 H), 2.97/2.33 (2*m, 2 H), 2.85/2.48 (2*m, 2 H), 2.02-1.85 (m, 2 H), 2.02-1.85 (m, 2 H), 1.39/1.34 (2*s, 27 H), 1.38 (m, 2 H), 1.2 (t, 3 H), 0.81 (m, 2 H)

### EXEMPLE 40 : Acide 3-(4-aminobutyl)-1-(1H-benzimidazol-5-ylméthyl)-4-hydroxy-4-oxo-1,4-azaphosphinane-3-carboxylique

L'exemple 40 est obtenu à partir de l'intermédiaire 30 selon la procédure D précédemment décrite.
RMN ¹H : (400 MHz, D2O) δ ppm 8.26 (s, 1 H), 7.77 (df, 1 H), 7.72 (d, 1 H), 7.38 (dd, 1 H), 4.53/4.34 (2*d, 2 H), 3.73/3.36 (2*m, 2 H), 3.47/3.12 (2*m, 2 H), 2.88 (m, 2 H), 2.23/1.78 (2*m, 2 H), 1.91/1.55 (2*m, 2 H), 1.55/1.45 (2*m, 2 H), 1.18/1.04 (2*m, 2 H)
ESI/FIA/HR et MS/MS : [M+H]+ = 381.1692 (381.1691)
Analyse Elémentaire : C=54.46(53.68);H=6.00(6.62);N=14.67(14.73)

### Intermédiaire 31 : 3-{4-[Bis(tert-butoxycarbonyl)amino]butyl}-1-[(2-fluoro-4-hydroxyphényl)méthyl]-4-éthoxy-4-oxo-1,4-azaphosphinane-3-carboxylate de tert-butyle

L'intermédiaire 31 est obtenu à partir de l'intermédiaire 21 et du 2-fluoro-4-methoxy-benzaldéhyde selon la procédure F précédemment décrite.
RMN ¹H : (400 MHz, dmso-d6) δ ppm 7.25 (t, 1 H), 6.78 (2*m, 2 H), 4.1-3.9 (m, 2 H), 3.76 (s, 3 H), 3.57/3.46 (2*d, 2 H), 3.36 (m, 2 H), 2.93/2.32 (2*m, 2 H), 2.81/2.47 (2*dd, 2 H), 2-1.8 (m, 2 H), 2-1.8 (m, 2 H), 1.42/1.36 (2*s, 27 H), 1.4 (m, 2 H), 1.2 (t, 3 H), 1-0.75 (m, 2 H)

### EXEMPLE 41 : Acide 3-(4-aminobutyl)-1-[(2-fluoro-4-hydroxyphényl)méthyl]-4-hydroxy-4-oxo-1,4-azaphosphinane-3-carboxylique

L'exemple 41 est obtenu à partir de l'intermédiaire 31 selon la procédure D précédemment décrite.
RMN¹H : (400 MHz, D2O) δ ppm 7.21 (t, 1 H), 6.63 (m, 1 H), 6.63 (m, 1 H), 4.19 (dd, 2 H), 3.58/3.19 (m, 2 H), 3.38/3.04 (dd, 2 H), 2.84 (m, 2 H), 2.1/1.65 (m, 2 H), 1.84/1.5 (m, 2 H), 1.5/1.38 (m, 2 H), 1.17/1.04 (m, 2 H)
ESI/FIA/HR et MS/MS : [M+H]+ = 375.1486 (375.1485)
Analyse Elémentaire : C=51.05(51.34);H=5.28(6.46);N=7.76(7.48)

### Intermédiaire 32 : 3-{4-[Bis(tert-butoxycarbonyl)amino]butyl}-4-éthoxy-4-oxo-1-[(6-oxo-1H-pyridin-3-yl)méthyl]-1,4-azaphosphinane-3-carboxylate de tert-butyle

L'intermédiaire 32 est obtenu à partir de l'intermédiaire 21 et du 6-hydroxynicotinaldéhyde selon la procédure F précédemment décrite.
RMN ¹H : (400 MHz, dmso-d6) δ ppm 11.45 (s, 1 H), 7.35 (dd, 1 H), 7.21 (df, 1 H), 6.31 (d, 1 H), 4.1-3.9 (m, 2 H), 3.4 (m, 2 H), 3.32/3.19 (2*d, 2 H), 2.9/2.28 (2*m, 2 H), 2.8/2.44 (2*dd, 2 H), 2-1.7 (m, 2 H), 2-1.7 (m, 2 H), 1.42/1.38 (2*s, 27 H), 1.4 (m, 2 H), 1.21 (t, 3 H), 1.1-0.85 (m, 2 H)

### EXEMPLE 42 : Acide 3-(4-aminobutyl)-4-hydroxy-4-oxo-1-[(6-oxo-1H-pyridin-3-yl)méthyl]-1,4-azaphosphinane-3-carboxylique

L'exemple 42 est obtenu à partir de l'intermédiaire 32 selon la procédure D précédemment décrite.
RMN ¹H : (300 MHz, D2O) δ ppm 7.74 (dd, 1 H), 7.7 (d, 1 H), 6.66 (d, 1 H), 4.17 (AB, 2 H), 3.69/3.31 (2m, 2 H), 3.45/3.09 (2m, 2 H), 2.94 (m, 2 H), 2.24/1.79 (2m, 2 H), 1.94/1.49 (2m, 2 H), 1.61 (quint., 2 H), 1.29/1.14 (2m, 2 H)
RNLN ³¹ P : (300 MHz, D2O) δ ppm 25.8
ESI/FIA/HR et MS/MS : [M+H]+ = 358.1537 (358.1531)
Analyse Elémentaire : C=50.77(50.42);H=6.41(6.77);N=11.96(11.76)

### Intermédiaire 33 : 3-{4-[Bis(tert-butoxycarbonyl)amino]butyl}-1-(1-benzofuran-5-ylméthyl)-4-éthoxy-4-oxo-1,4-azaphosphinane-3-carboxylate de tert-butyle

L'intermédiaire 33 est obtenu à partir de l'intermédiaire 21 et du 5-formylbenzofurane selon la procédure F précédemment décrite.
RMN ¹H : (400 MHz, dmso-d6) δ ppm 7.98 (df, 1 H), 7.55 (df, 1 H), 7.53 (d, 1 H), 7.25 (dd, 1 H), 6.91 (df, 1 H), 4.1-3.9 (m, 2 H), 3.72/3.51 (2*d, 2 H), 3.4-3.2 (m, 2 H), 2.98/2.35 (2*dd, 2 H), 2.81/2.48 (2*dd, 2 H), 2-1.65 (m, 2 H), 2-1.65 (m, 2 H), 1.4/1.34 (2*s, 27 H), 1.39 (m, 2 H), 1.21 (t, 3 H), 1-0.7 (m, 2 H)

### EXEMPLE 43 : Acide 3-(4-aminobutyl)-1-(1-benzofuran-5-ylméthyl)-4-hydroxy-4-oxo-1,4-azaphosphinane-3-carboxylique

L'exemple 43 est obtenu à partir de l'intermédiaire 33 selon la procédure D précédemment décrite.
RMN ¹H : (400 MHz, D2O) δ ppm 7.79 (df, 1 H), 7.76 (df, 1 H), 7.61 (d, 1 H), 7.39 (dd, 1 H), 7.31/4.5 (2*d, 2 H), 6.91 (df, 1 H), 3.72/3.33 (2*dd, 2 H), 3.49/3.11 (2*dd, 2 H), 2.9 (m, 2 H), 2.23/1.77 (2*m, 2 H), 1.91/1.57 (2*m, 2 H), 1.57/1.45 (2*m, 2 H), 1.2/1.15 (2*m, 2 H)
ESI/FIA/HR et MS/MS : [M+H]+ = 381.1577 (381.1579)
Analyse Elémentaire : C=56.67(56.84);H=6.52(6.62);N=7.42(7.36)

### Intermédiaire 34 : 3-{4-[Bis(tert-butoxycarbonyl)amino]butyl}-4-éthoxy-1-[(4-hydroxy-2-méthylphényl)méthyl]-4-oxo-1,4-azaphosphinane-3-carboxylate de tert-butyle

L'intermédiaire 34 est obtenu à partir de l'intermédiaire 21 et du 4-méthoxy-2-méthylbenzaldéhyde selon la procédure F précédemment décrite.
RMN ¹H : (400 MHz, dmso-d6) δ ppm 7.05 (d, 1 H), 6.75 (df, 1 H), 6.69 (dd, 1 H), 4.1-3.9 (m, 2 H), 3.72 (s, 3 H), 3.5/3.3 (2*d, 2 H), 3.3 (m, 2 H), 2.9/2.41 (2*m, 2 H), 2.8/2.29 (2*m, 2 H), 2.3 (2*s, 3 H), 2.02-1.79 (massif, 2 H), 2.02-1.79 (massif, 2 H), 1.42/1.36 (2*s, 27 H), 1.32 (m, 2 H), 1.21 (t, 3 H), 0.68 (m, 2 H)

### EXEMPLE 44 : Acide 3-(4-aminobutyl)-4-hydroxy-1-[(4-hydroxy-2-méthylphényl)méthyl]-4-oxo-1,4-azaphosphinane-3-carboxylique

L'exemple 44 est obtenu à partir de l'intermédiaire 34 selon la procédure D précédemment décrite.
RMN ¹H : (400 MHz, D2O) δ ppm 7.25 (d, 1 H), 6.81 (df, 1 H), 6.77 (dd, 1 H), 4.29/4.22 (2*d, 2 H), 3.69/3.32 (2*dd, 2 H), 3.49/3.18 (2*dd, 2 H), 2.94 (m, 2 H), 2.32 (s, 3 H), 2.19/1.75 (2*m, 2 H), 1.94/1.6 (2*m, 2 H), 1.6/1.5 (2*m, 2 H), 1.27/1.12 (2*m, 2 H) RMN ³¹P : (400 MHz, D2O) δ ppm 26
ESI/FIA/HR et MS/MS : [M+H]+ = 371.1739 (371.1735)
Analyse Elémentaire : C=54.81(55.13);H=6.88(7.35);N=7.52(7.56)

### Intermédiaire 35 : 3-{4-[Bis(tert-butoxycarbonyl)amino]butyl}-1-[(2-chloro-4-fluorophényl)méthyl]-4-éthoxy-4-oxo-1,4-azaphosphinane-3-carboxylate de tert-butyle

L'intermédiaire 35 est obtenu à partir de l'intermédiaire 21 et du 2-chloro-4-fluorobenzaldéhyde selon la procédure F précédemment décrite.
RMN ¹H : (400 MHz, dmso-d6) δ ppm 7.5 (dd, 1 H), 7.35 (dd, 1 H), 7.2 (dd, 1 H), 4.1-3.9 (quad., 2 H), 3.65-3.55 (d, 2 H), 3.4-3.3 (m, 2 H), 2.9-2.35 (m, 4 H), 1.9 (m, 4 H), 1.55-1.35 (m, 2 H), 1.4-1.35 (s, 27 H), 1.2 (t, 3 H), 0.85-0.75 (m, 2 H)
RMN ³¹P : (400 MHz, dmso-d6) δ ppm 45
RMN ¹⁹F : (400 MHz, dmso-d6) δ ppm -112
ESI/FIA/HR et MS/MS : [M+H]+ = 677.3146 (677.3133)

### EXEMPLE 45 : Acide 3-(4-aminobutyl)-1-[(2-chloro-4-fluorophényl)méthyl]-4-hydroxy-4-oxo-1,4-azaphosphinane-3-carboxylique

L'exemple 45 est obtenu à partir de l'intermédiaire 35 selon la procédure D précédemment décrite.
RMN ¹H : (400 MHz, D2O) δ ppm 7.56 (dd, 1 H), 7.39 (dd, 1 H), 7.18 (td, 1 H), 4.43 (si, 2 H), 3.7/3.4 (m, 2 H), 3.5/3.26 (m, 2 H), 2.95 (m, 2 H), 2.22/1.78 (m, 2 H), 1.95/1.62 (m, 2 H), 1.62/1.5 (m, 2 H), 1.28/1.12 (m, 2 H)
ESI/FIA/HR et MS/MS : [M+H]+ = 393.1149 (393.1146)
Analyse Elémentaire : C=49.40(48.93);H=5.31(5.90);N=7.17(7.13)

### Intermédiaire 36 : 3-{4-[Bis(tert-butoxycarbonyl)amino]butyl}-1-[[2-(4-fluorophényl)phényl]méthyl]-4-éthoxy-4-oxo-1,4-azaphosphinane-3-carboxylate de tert-butyle

L'intermédiaire 36 est obtenu à partir de l'intermédiaire 21 et du 2-(4-fluorophényl)benzaldéhyde selon la procédure F précédemment décrite.
RMN ¹H : (400 MHz, dmso-d6) δ ppm 7.5-7.2 (m, 4 H), 7.5-7.2 (m, 4 H), 4.08-3.73 (m, 2 H), 3.52/3.3 (2*d, 2 H), 3.3 (m, 2 H), 2.8-2.62/2.33 (2*m, 2 H), 2.8-2.62/2.2 (2*m, 2 H), 1.98-1.63 (m, 2 H), 1.98-1.63 (m, 2 H), 1.4/1.32 (2*s, 27 H), 1.38 (m, 2 H), 1.21/1.18 (2*t, 3 H), 0.9-0.6 (m, 2 H)

### EXEMPLE 46 : Acide 3-(4-aminobutyl)-1-[[2-(4-fluorophényl)phényl]méthyl]-4-hydroxy-4-oxo-1,4-azaphosphinane-3-carboxylique

L'exemple 46 est obtenu à partir de l'intermédiaire 36 selon la procédure D précédemment décrite.
RMN ¹H : (400 MHz, D2O) δ ppm 7.6 (d, 1 H), 7.52 (m, 2 H), 7.4 (d, 1 H), 7.35 (dd, 2 H), 7.24 (dd, 2 H), 4.41/4.29 (dd, 2 H), 3.39/3.1 (2*m, 2 H), 3.19/2.88 (2*m, 2 H), 2.93 (m, 2 H), 2.09/1.65 (2*m, 2 H), 1.85/1.59 (2*m, 2 H), 1.59/1.35 (2*m, 2 H), 1.2-1 (m, 2 H)
ESI/FIA/HR et MS/MS : [M+H]+ = 435.1850 (435.1848)
Analyse Elémentaire : C=60.73(60.82);H=5.96(6.50);N=6.73(6.45)

### Intermédiaire 37 : 3-{4-[Bis(tert-butoxycarbonyl)amino]butyl}-1-[[2-(2-fluorophényl)phényl]méthyl]-4-éthoxy-4-oxo-1,4-azaphosphinane-3-carboxylate de tert-butyle

L'intermédiaire 37 est obtenu à partir de l'intermédiaire 21 et du 2-(2-fluorophényl)benzaldéhyde selon la procédure F précédemment décrite.
RMN ¹H : (400 MHz, dmso-d6) δ ppm 7.52 (d, 1 H), 7.45/7.42 (t, 2 H), 7.45 (m, 1 H), 7.35 (m, 3 H), 7.2 (d, 1 H), 3.91 (m, 2 H), 3.5-3.2 (m, 2 H), 3.5-3.2 (m, 2 H), 2.67/2.29 (m, 2 H), 2.67/2.13 (m, 2 H), 1.95-1.6 (m, 2 H), 1.95-1.6 (m, 2 H), 1.42 (s, 18 H), 1.38 (m, 2 H), 1.34 (s, 9 H), 1.17 (t, 3 H), 0.68 (m, 2 H)

### EXEMPLE 47 : Acide 3-(4-aminobutyl)-1-[[2-(2-fluorophényl)phényl]méthyl]-4-hydroxy-4-oxo-1,4-azaphosphinane-3-carboxylique

L'exemple 47 est obtenu à partir de l'intermédiaire 37 selon la procédure D précédemment décrite.
RMN ¹H : (400 MHz, D2O) δ ppm 7.66 (m, 1 H), 7.57 (m, 1 H), 7.57 (m, 1 H), 7.51 (m, 1 H), 7.42 (m, 1 H), 7.34 (m, 1 H), 7.34 (m, 1 H), 7.28 (t, 1 H), 4.6-4 (massif, 2 H), 3.7-2.7 (massif, 2 H), 3.7-2.7 (massif, 2 H), 2.93 (m, 2 H), 2.14/1.69 (m, 2 H), 1.87/1.37 (m, 2 H), 1.59 (m, 2 H), 1.15/1.06 (m, 2 H)
ESI/FIA/HR et MS/MS : [M+H]+ = 435.1855 (435.1848)
Analyse Elémentaire : C=60.87(60.82);H=6.12(6.50);N=6.42(6.45)

### Intermédiaire 38 : 3-{4-[Bis(tert-butoxycarbonyl)amino]butyl}-1-[[2-(2,5-dichlorophényl)phényl]méthyl]-4-éthoxy-4-oxo-1,4-azaphosphinane-3-carboxylate de tert-butyle

L'intermédiaire 38 est obtenu à partir de l'intermédiaire 21 et du 2-(2,5-dichlorophényl)benzaldéhyde selon la procédure F précédemment décrite.
RMN ¹H : (400 MHz, dmso-d6) δ ppm 7.65-7.1 (m, 7 H), 4.05-3.85 (m, 2 H), 3.5-3.2 (m, 4 H), 2.8-2.3 (m, 4 H), 2.2-1.65 (m, 4 H), 1.5-1.3 (m, 2 H), 1.45 (s, 18 H), 1.35 (s, 9 H), 1.2 (t, 3 H), 0.8-0.5 (m, 2 H)

### EXEMPLE 48 : Acide 3-(4-aminobutyl)-1-[[2-(2,5-dichlorophényl)phényl]méthyl]-4-hydroxy-4-oxo-1,4-azaphosphinane-3-carboxylique

L'exemple 48 est obtenu à partir de l'intermédiaire 38 selon la procédure D précédemment décrite.
RMN ¹H : (300 MHz, D2O) δ ppm 7.7-7.3 (m, 7 H), 4.45/4.21/4.02 (m, 2 H), 3.75/3.1 (2*m, 2 H), 3.75/3.1 (2*m, 2 H), 2.98 (m, 2 H), 2.2/1.65 (2*m, 2 H), 1.95/1.65 (2*m, 2 H), 1.65/1.15 (2*m, 2 H), 1.45/1.15 (2*m, 2 H)
ESI/FIA/HR et MS/MS : [M+H]+ = 485.1184 (485.1163)
Analyse Elémentaire : C=54.33(54.44);H=4.99(5.61);N=5.86(5.77)

### Intermédiaire 39 : 3-{4-[Bis(tert-butoxycarbonyl)amino]butyl}-4-éthoxy-4-oxo-1-[[2-(3-phénylphényl)phényl]méthyl]-1,4-azaphosphinane-3-carboxylate de tert-butyle

L'intermédiaire 39 est obtenu à partir des intermédiaires 21 et 241 selon la procédure F précédemment décrite.
RMN ¹H : (400 MHz, dmso-d6) δ ppm 7.7 (d, 2 H), 7.65 (d, 1 H), 7.6 (s, 1 H), 7.55 (m, 2 H), 7.45 (m, 2 H), 7.4-7.3 (m, 5 H), 3.9 (m, 2 H), 3.6/3.4 (2*d, 2 H), 3.4-3.2 (m, 2 H), 2.85-2.65 (m, 2 H), 2.35 (m, 1 H), 2.2 (m, 1 H), 1.9 (m, 1 H), 1.85-1.6 (m, 3 H), 1.45-1.3 (m, 2 H), 1.4/1.3 (2*s, 27 H), 1.15 (t, 3 H), 0.65 (m, 2 H)

### EXEMPLE 49 : Acide 3-(4-aminobutyl)-4-hydroxy-4-oxo-1-[[2-(3-phénylphényl)phényl]méthyl]-1,4-azaphosphinane-3-carboxylique

L'exemple 49 est obtenu à partir de l'intermédiaire 39 selon la procédure D précédemment décrite.
RMN ¹H : (300 MHz, D2O) δ ppm 7.6-7.1 (m, 13 H), 4.25 (d, 1 H), 4.1 (d, 1 H), 3.4-3.2 (m, 1 H), 3.15-2.9 (m, 2 H), 2.75-2.55 (m, 3 H), 2.05 (m, 1 H), 1.75 (m, 1 H), 1.55 (m, 1 H), 1.4 (m, 2 H), 1.25 (m, 1 H), 0.9 (m, 2 H)
ESI/FIA/HR et MS/MS : [M+H]+ = 493.2256 (493.2256)
Analyse Elémentaire : C=68.08(68.28);H=6.02(6.75);N=5.71(5.69)

### Intermédiaire 40 : 3-{4-[Bis(tert-butoxycarbonyl)amino]butyll-4-éthoxy-1-[(2-naphthalen-2-ylphényl)méthyl]-4-oxo-1,4-azaphosphinane-3-carboxylate de tert-butyle

L'intermédiaire 40 est obtenu à partir des intermédiaires 21 et 240 selon la procédure F précédemment décrite.
RMN ¹H : (400 MHz, dmso-d6) δ ppm 7.95 (d, 1 H), 7.95 (m, 2 H), 7.9 (s, 1 H), 7.55 (m, 4 H), 7.4 (m, 2 H), 7.3 (d, 1 H), 3.9 (m, 2 H), 3.65/3.45 (2*d, 2 H), 3.45-3.2 (m, 2 H), 3.3-2.6 (m, 2 H), 2.35 (dd, 1 H), 2.2 (m, 1 H), 1.9 (m, 1 H), 1.85-1.65 (m, 3 H), 1.8 (m, 2 H), 1.4/1.3 (2*s, 27 H), 1.15 (t, 3 H), 0.7 (m, 2 H)

### EXEMPLE 50 : Acide 3-(4-aminobutyl)-4-hydroxy-1-[(2-naphthalen-2-ylphényl)méthyl]-4-oxo-1,4-azaphosphinane-3-carboxylique

L'exemple 50 est obtenu à partir de l'intermédiaire 40 selon la procédure D précédemment décrite.
RMN ¹H : (300 MHz, D2O) δ ppm 7.9 (d, 1 H), 7.9/7.85 (2*m, 2 H), 7.7 (s, 1 H), 7.6-7.4 (m, 5 H), 7.3 (m, 2 H), 4.35/4.2 (2dd, 2 H), 3.4-3.2 (m, 1 H), 3.15-2.9 (m, 2 H), 2.8 (m, 2 H), 2.7 (dd, 1 H), 2 (m, 1 H), 1.75 (m, 1 H), 1.65-1.35 (m, 3 H), 1.25 (m, 1 H), 0.85 (m, 2 H)
ESI/FIA/HR et MS/MS : [M+H]+ = 467.2090 (467.2099)
Analyse Elémentaire : C=67.58(66.94);H=6.40(6.70);N=5.73(6.00)

### Intermédiaire 41 : 3-{4-[Bis(tert-butoxycarbonyl)amino]butyl}-1-[[2-(2,6-dichlorophényl)phényl]méthyl]-4-éthoxy-4-oxo-1,4-azaphosphinane-3-carboxylate de tert-butyle

L'intermédiaire 41 est obtenu à partir des intermédiaires 21 et 242 selon la procédure F précédemment décrite.
RMN ¹H : (300 MHz, dmso-d6) δ ppm 7.65-7.5 (m, 3 H), 7.5-7.3 (m, 3 H), 7.1 (d, 1 H), 4 (m, 2 H), 3.45 (m, 2 H), 3.25 (2*d, 2 H), 2.85-2.1 (m, 4 H), 2-1.75 (m, 4 H), 1.6-1.15 (m, 2 H), 1.45/1.4 (2*s, 27 H), 1.25 (t, 3 H), 0.95 (m, 2 H)

### EXEMPLE 51 : Acide 3-(4-aminobutyl)-1-[[2-(2,6-dichlorophényl)phényl]méthyl]-4-hydroxy-4-oxo-1,4-azaphosphinane-3-carboxylique

L'exemple 51 est obtenu à partir de l'intermédiaire 41 selon la procédure D précédemment décrite.
RMN ¹H : (300 MHz, D2O) δ ppm 7.6 (d, 1 H), 7.5/7.2 (m, 3 H), 7.5-7.3 (m, 2 H), 7.12 (d, 1 H), 3.38/3.21 (2*d, 2 H), 2.82/2.38 (2*dd, 2 H), 2.65/2.22 (2*m, 2 H), 2.45 (t, 2 H), 1.85/1.71 (2*m, 2 H), 1.7/1.35 (2*m, 2 H), 1.32/0.8 (2*m, 2 H), 1.32/0.8 (2*m, 2 H)
ESI/FIA/HR et MS/MS : [M+H]+ = 485.1169 (485.1163)
Analyse Elémentaire : C=54.71(54.44);H=5.14(5.61);N=5.81(5.77)

### Intermédiaire 42 : 3-{4-[Bis(tert-butoxycarbonyl)amino]butyl}-4-éthoxy-1-méthyl-4-oxo-1,4-azaphosphinane-3-carboxylate de tert-butyle

L'intermédiaire 42 est obtenu à partir de l'intermédiaire 21 et du formaldéhyde selon la procédure F précédemment décrite.
RMN ¹H : (400 MHz, dmso-d6) δ ppm 3.98 (m, 2 H), 3.48 (m, 2 H), 2.75 (m, 2 H), 2.49/2.2 (2m, 2 H), 2.01 (s, 3 H), 1.92 (m, 4 H), 1.5 (m, 2 H), 1.45/1.4 (2s, 27 H), 1.25/1 (2m, 2 H), 1.2 (t, 3 H)

### EXEMPLE 52 : Acide 3-(4-aminobutyl)-4-hydroxy-1-méthyl-4-oxo-1,4-azaphosphinane-3-carboxylique

L'exemple 52 est obtenu à partir de l'intermédiaire 42 selon la procédure D précédemment décrite.
RMN ¹H : (400 MHz, D2O) δ ppm 3.7-3.5 (m, 2 H), 3.3 (t, 1 H), 3.2 (dd, 1 H), 3 (t, 2 H), 2.9 (s, 3 H), 2.3 (m, 1 H), 2 (m, 1 H), 1.75 (m, 1 H), 1.7 (m, 2 H), 1.5 (m, 1 H), 1.4/1.25 (2m, 2 H)
ESI/FIA/HR et MS/MS : [M+H]+ = 265.1300 (265.1317)
Analyse Elémentaire : C=45.59(45.45);H=7.97(8.01);N=10.61(10.60)

### Intermédiaire 43 : 3-{4-[Bis-(tert-butoxycarbonyl)amino]butyl}-4-éthoxy-4-oxo-1-(2-phényléthyl)-1,4-azaphosphinane-3-carboxylate de tert-butyle

L'intermédiaire 43 est obtenu à partir de l'intermédiaire 21 et du phénylacétaldéhyde selon la procédure F précédemment décrite.
RMN ¹H : (400 MHz, dmso-d6) δ ppm 7.3-7.15 (m, 5 H), 3.98 (m, 2 H), 3.45 (t, 2 H), 3.05-2.3 (m, 8 H), 2-1.8 (m, 4 H), 1.4 (m+2s, 29 H), 1.2 (t, 3 H), 1.18/0.9 (2m, 2 H)

### EXEMPLE 53 : Acide 3-(4-aminobutyl)-4-hydroxy-4-oxo-1-(2-phényléthyl)-1,4-azaphosphinane-3-carboxylique

L'exemple 53 est obtenu à partir de l'intermédiaire 43 selon la procédure D précédemment décrite.
RMN ¹H : (400 MHz, D2O) δ ppm 7.4-7.25 (m, 5 H), 3.7/3.3 (2m, 2 H), 3.52 (m, 1 H), 3.45 (t, 2 H), 3.1 (m, 3 H), 2.95 (m, 2 H), 2.2/1.75 (2m, 2 H), 1.95 (m, 1 H), 1.62 (m, 2 H), 1.5-1.1 (m, 3 H)
ESI/FIA/HR et MS/MS : [M+H]+ = 355.1777 (355.1786)
Analyse Elémentaire : C=57.63(57.62);H=7.36(7.68);N=7.90(7.90)

### Intermédiaire 44 : 3-{4-[Bis(tert-butoxycarbonyl)amino]butyl}-4-éthoxy-4-oxo-1-pentyl-1,4-azaphosphinane-3-carboxylate de tert-butyle

L'intermédiaire 44 est obtenu à partir de l'intermédiaire 21 et du 1-pentanal selon la procédure F précédemment décrite.
RMN ¹H : (400 MHz, dmso-d6) δ ppm 3.98 (m, 2 H), 3.8 (m, 2 H), 3.49 (t, 2 H), 2.85 (m, 2 H), 2.49/2.25 (2m, 2 H), 1.9 (m, 4 H), 1.45/1.4 (2s, 27 H), 1.45 (m, 4 H), 1.25/1 (2m, 6 H), 1.2 (t, 3 H), 0.85 (t, 3 H)

### EXEMPLE 54 : Acide 3-(4-aminobutyl)-4-hydroxy-4-oxo-1-pentyl-1,4-azaphosphinane-3-carboxylique

L'exemple 54 est obtenu à partir de l'intermédiaire 44 selon la procédure D précédemment décrite.
RMN ¹H : (400 MHz, D2O) δ ppm 3.7-3.5 (m, 2 H), 3.3 (m, 1 H), 3.15/3 (2m, 5 H), 2.27 (m, 1 H), 2 (m, 1 H), 1.85-1.6 (m, 5 H), 1.5/1.41 (2m, 2 H), 1.3 (m, 5 H), 0.85 (t, 3 H)
ESI/FIA/HR et MS/MS : [M+H]+ = 321.1923 (321.1943)
Analyse Elémentaire : C=52.77(52.49);H=8.93(9.12);N=9.00(8.74)

### Intermédiaire 45 : 3-{4-[Bis(tert-butoxycarbonyl)amino-butyl}-4-éthoxy-1-(naphthalen-1-ylméthyl)-4-oxo-1,4-azaphosphinane-3-carboxylate de tert-butyle

L'intermédiaire 45 est obtenu à partir de l'intermédiaire 21 et du 1-naphtaldéhyde selon la procédure F précédemment décrite.
RMN ¹H : (400 MHz, dmso-d6) δ ppm 8.29 (d, 1 H), 7.9/7.85 (2d, 2 H), 7.6-7.4 (m, 4 H), 3.95 (AB, 2 H), 4 (m, 2 H), 3.6 (m, 2 H), 3.2-2.8 (m, 4 H), 2.4/2 (2m, 2 H), 1.7 (m, 2 H), 1.4/1.3 (2s, 27 H), 1.22 (t, 3 H), 1.05/0.75 (2m, 2 H), 0.5/0.2 (2m, 2 H)

### EXEMPLE 55 : Acide 3-(4-aminobutyl)-4-hydroxy-1-(naphthalen-1-ylméthyl)-4-oxo-1,4-azaphosphinane-3-carboxylique

L'exemple 55 est obtenu à partir de l'intermédiaire 45 selon la procédure D précédemment décrite.
RMN ¹H : (400 MHz, D2O) δ ppm 7.9 (m, 3 H), 7.6-7.3 (m, 4 H), 4.55 (s, 2 H), 3.55/3.28 (2m, 2 H), 3.4/3.15 (2dd, 2 H), 2.75 (m, 2 H), 2/1.6 (2m, 2 H), 1.8 (m, 1 H), 1.5-1.25 (m, 3 H), 1/0.9 (2m, 2 H)
ESI/FIA/HR et MS/MS : [M+H]+ = 391.1778 (391.1786)
Analyse Elémentaire : C=61.34(61.53);H=6.58(6.97);N=7.01(7.18)

### Intermédiaire 46 : 3-{4-[Bis(tert-butoxycarbonyl)amino]butyll-1-(cyclohexylméthyl)-4-éthoxy-4-oxo-1,4-azaphosphinane-3-carboxylate de tert-butyle

L'intermédiaire 46 est obtenu à partir de l'intermédiaire 21 et du cyclohexanal selon la procédure F précédemment décrite.
RMN ¹H : (400 MHz, dmso-d6) δ ppm 4 (m, 2 H), 3.5 (t, 2 H), 2.8 (m, 2 H), 2.48/2.25 (2m, 2 H), 2.15 (dd, 2 H), 1.95 (m, 2 H), 1.68 (m, 1 H), 1.65/1.4/0.8 (3m, 10 H), 1.45 (m, 3 H), 1.45/1.4 (2s, 27 H), 1.2 (m, 2 H), 1.2 (t, 3 H), 1 (m, 1 H)

### EXEMPLE 56 : Acide 3-(4-aminobutyl)-1-(cyclohexylméthyl)-4-hydroxy-4-oxo-1,4-azaphosphinane-3-carboxylique

L'exemple 56 est obtenu à partir de l'intermédiaire 46 selon la procédure D précédemment décrite.
RMN ¹H : (400 MHz, D2O) δ ppm 3.7/3.25 (2m, 2 H), 3.55/3.15 (2dd, 2 H), 3.1-2.9 (t+2dd, 4 H), 2.25 (m, 1 H), 2 (m, 1 H), 1.85-1.55 (m, 9 H), 1.52/1.41 (2m, 2 H), 1.3/1.1 (m, 4 H), 1 (m, 2 H)
ESI/FIA/HR et MS/MS : [M+H]+ = 347.2095 (347.2099)
Analyse Elémentaire : C=55.29(55.48);H=9.08(9.02);N=7.95(8.09)

### Intermédiaire 47 : 3-{4-Bis[tert-butoxycarbonyl)amino]butyl}-4-éthoxy-1-(naphthalen-2-ylméthyl)-4-oxo-1,4-azaphosphinane-3-carboxylate de tert-butyle

L'intermédiaire 47 est obtenu à partir de l'intermédiaire 21 et du 2-naphtaldéhyde selon la procédure F précédemment décrite.
RMN ¹H : (400 MHz, dmso-d6) δ ppm 7.9 (m, 3 H), 7.8 (s, 1 H), 7.5 (m, 3 H), 3.98 (m, 2 H), 3.8 (d, 1 H), 3.6 (m, 3 H), 3.35/3.2 (2m, 2 H), 3.1-2.75 (2m, 2 H), 2.4 (m, 1 H), 2.05-1.8 (m, 3 H), 1.4-0.7 (m, 4 H), 1.38 (3s, 27 H), 1.22 (t, 3 H)

### EXEMPLE 57 : Acide 3-(4-aminobutyl)-4-hydroxy-1-(naphthalen-2-ylméthyl)-4-oxo-1,4-azaphosphinane-3-carboxylique

L'exemple 57 est obtenu à partir de l'intermédiaire 47 selon la procédure D précédemment décrite.
RMN ¹H : (400 MHz, D2O) δ ppm 7.95-7.9 (m, 4 H), 7.6-7.5 (m, 3 H), 4.5;4.3 (d, 2*1H H), 3.65 (m, 1 H), 3.45-3.3 (m, 2 H), 3.15 (m, 1 H), 2.8 (m, 2 H), 2.25 (m, 1 H), 1.9-1.7 (m, 2 H), 1.55-1.4 (m, 3 H), 1.15-0.95 (m, 2 H)
ESI/FIA/HR et MS/MS : [M+H]+ = 391.1778 (391.1786)
Analyse Elémentaire : C=61.31(61.53);H=6.56(6.97);N=7.15(7.18)

### Intermédiaire 48 : 3-{4-[Bis(tert-butoxycarbonyl)amino]butyl}-1-[(4-chlorophényl)méthyl]-4-éthoxy-4-oxo-1,4-azaphosphinane-3-carboxylate de tert-butyle

L'intermédiaire 48 est obtenu à partir de l'intermédiaire 21 et du 4-chlorobenzaldéhyde selon la procédure F précédemment décrite.
RMN ¹H : (400 MHz, dmso-d6) δ ppm 7.4 (d, 2 H), 7.31 (d, 2 H), 3.98 (m, 2 H), 3.65-3.3 (m, 4 H), 3-2.3 (m, 4 H), 2-1.8 (m, 4 H), 1.4 (s+m, 29 H), 1.22 (2t, 3 H), 0.8 (m, 2 H)

### EXEMPLE 58 : Acide 3-(4-aminobutyl)-1-[(4-chlorophényl)méthyl]-4-hydroxy-4-oxo-1,4-azaphosphinane-3-carboxylique

L'exemple 58 est obtenu à partir de l'intermédiaire 48 selon la procédure D précédemment décrite.
RMN ¹H : (400 MHz, D2O) δ ppm 7.5/7.45 (2d, 4 H), 4.41/4.21 (2d, 2 H), 3.7/3.33 (2m, 2 H), 3.45/3.1 (2dd, 2 H), 2.95 (m, 2 H), 2.25/1.78 (2m, 2 H), 1.95/1.5 (2m, 2 H), 1.6 (m, 2 H), 1.25/1.1 (2m, 2 H)
ESI/FIA/HR et MS/MS : [M+H]+ = 375.1242 (375.1240)
Analyse Elémentaire : C=50.89(51.27);H=6.01(6.45);N=7.44(7.47)

### Intermédiaire 49 : 3-{4-[Bis(tert-butoxycarbonyl)amino]butyl}-4-éthoxy-1-[(4-fluorophényl)méthyl]-4-oxo-1,4-azaphosphinane-3-carboxylate de tert-butyle

L'intermédiaire 49 est obtenu à partir de l'intermédiaire 21 et du 4-fluorobenzaldéhyde selon la procédure F précédemment décrite.
RMN ¹H : (400 MHz, dmso-d6) δ ppm 7.35 (m, 2 H), 7.15 (t, 2 H), 4 (m, 2 H), 3.65-3.3 (m, 4 H), 3-2.3 (m, 4 H), 2-1.8 (m, 4 H), 1.4 (s+m, 29 H), 1.2 (2t, 3 H), 0.9/0.8 (2m, 2 H)

### EXEMPLE 59 : Acide 3-(4-aminobutyl)-1-[(4-fluorophényl)méthyl]-4-hydroxy-4-oxo-1,4-azaphosphinane-3-carboxylique

L'exemple 59 est obtenu à partir de l'intermédiaire 49 selon la procédure D précédemment décrite.
RMN ¹H : (400 MHz, D2O) δ ppm 7.5 (dd, 2 H), 7.2 (t, 2 H), 4.41/4.21 (2d, 2 H), 3.7/3.31 (2m, 2 H), 3.45/3.1 (2dd, 2 H), 2.95 (m, 2 H), 2.25/1.78 (2m, 2 H), 1.95/1.5 (2m, 2 H), 1.6 (m, 2 H), 1.25/1.1 (2m, 2 H)
ESI/FIA/HR et MS/MS : [M+H]+ = 359.1532 (359.1535)
Analyse Elémentaire : C=53.65(53.63);H=6.20(6.75);N=7.83(7.82)

### Intermédiaire 50 : 3-{4-[Bis(tert-butoxycarbonyl)amino]butyl}-4-éthoxy-1-(furan-2-ylméthyl)-4-oxo-1,4-azaphosphinane-3-carboxylate de tert-butyle

L'intermédiaire 50 est obtenu à partir de l'intermédiaire 21 et du 2-furaldéhyde selon la procédure F précédemment décrite.
RMN ¹H : (400 MHz, CDCl3) δ ppm 7.41 (s, 1 H), 6.32 (s, 1 H), 6.2 (s, 1 H), 4.09 (s, 2 H), 3.62/3.52 (AB, 2 H), 3.5 (m, 2 H), 3/2.65 (m, 2 H), 2.96/2.52 (m, 2 H), 2.7-1.8 (m, 4 H), 1.5/1.46 (m, 30 H), 1.3 (t, 3 H), 1.01 (m, 1 H)
RNLN ³¹P : (400 MHz, CDCl3) δ ppm 46.14

### EXEMPLE 60 : Acide 3-(4-aminobutyl)-1-(furan-2-ylméthyl)-4-hydroxy-4-oxo-1,4-azaphosphinane-3-carboxylique

L'exemple 60 est obtenu à partir de l'intermédiaire 50 selon la procédure D précédemment décrite.
RMN ¹H : (400 MHz, D2O) δ ppm 7.6 (si, 1 H), 6.7 (tf, 1 H), 6.5 (tf, 1 H), 4.45/4.32 (2d, 2 H), 3.7/3.3 (2*m, 2 H), 3.52/3.12 (2*m, 2 H), 2.98 (m, 2 H), 2.25/1.8 (2*m, 2 H), 1.95/1.5 (2*m, 2 H), 1.62 (m, 2 H), 1.32/1.2 (2*m, 2 H)
ESI/FIA/HR et MS/MS : [M+H]+ = 331.1422 (331.1422)
Analyse Elémentaire : C=50.48(50.91);H=6.48(7.02);N=8.37(8.48)

### Intermédiaire 51 : 3-{4-[Bis(tert-butoxycarbonyl)amino]butyl}-4-éthoxy-4-oxo-1-[[4-(trifluorométhyl)phényl]méthyl]-1,4-azaphosphinane-3-carboxylate de tert-butyle

L'intermédiaire 51 est obtenu à partir de l'intermédiaire 21 et du 4-trifluorométhylbenzaldéhyde selon la procédure F précédemment décrite.
RMN ¹H : (400 MHz, dmso-d6) δ ppm 7.71 (d, 2 H), 7.52 (d, 2 H), 4.03 (m, 2 H), 3.72/3.51 (2*d, 2 H), 3.35 (m, 2 H), 3-2.3 (m, 4 H), 1.98 (m, 4 H), 1.4 (s+m, 29 H), 1.2 (t, 3 H), 0.8 (m, 2 H)

### EXEMPLE 61 : Acide 3-(4-aminobutyl)-4-hydroxy-4-oxo-1-[[4-(trifluorométhyl)phényl]méthyl]-1,4-azaphosphinane-3-carboxylique

L'exemple 61 est obtenu à partir de l'intermédiaire 51 selon la procédure D précédemment décrite.
RMN ¹H : (400 MHz, D2O) δ ppm 7.8 (d, 2 H), 7.65 (d, 2 H), 4.5/4.3 (AB, 2 H), 3.7/3.45 (m, 2 H), 3.39/3.11 (m, 2 H), 2.9 (m, 2 H), 2.28/1.8 (m, 2 H), 1.95/1.49 (m, 2 H), 1.6 (m, 2 H), 1.2/1.1 (m, 2 H)
RMN ¹⁹F : (400 MHz, D2O) δ ppm -62.5
RMN ³¹P : (400 MHz, D2O) δ ppm 24
ESI/FIA/HR et MS/MS : [M+H]+ = 409.1510 (409.1504)
Analyse Elémentaire : C=49.86(50.00);H=5.32(5.92);N=6.83(6.86)

### Intermédiaire 52 : 3-{4-[Bis(tert-butoxycarbonyl)amino]butyl}-4-éthoxy-1-[(4-méthoxyphényl)méthyl]-4-oxo-1,4-azaphosphinane-3-carboxylate de tert-butyle

L'intermédiaire 52 est obtenu à partir de l'intermédiaire 21 et du 4-méthoxybenzaldéhyde selon la procédure F précédemment décrite.
RMN ¹H : (400 MHz, dmso-d6) δ ppm 7.2 (d, 2 H), 6.9 (d, 2 H), 3.98 (m, 2 H), 3.72 (s, 3 H), 3.6/3.32 (2*d, 2 H), 3.4 (m, 2 H), 3-2.25 (m, 4 H), 1.9 (m, 4 H), 1.4 (s+m, 29 H), 1.2 (t, 3 H), 0.8 (m, 2 H)

### EXEMPLE 62 : Acide 3-(4-aminobutyl)-4-hydroxy-1-[(4-méthoxyphényl)méthyl]-4-oxo-1,4-azaphosphinane-3-carboxylique

L'exemple 62 est obtenu à partir de l'intermédiaire 52 selon la procédure D précédemment décrite.
RMN ¹H : (400 MHz, D2O) δ ppm 7.41 (d, 2 H), 7.02 (d, 2 H), centré à 4.25 (AB, 2 H), 3.8 (s, 3 H), 3.7/3.3 (2m, 2 H), 3.45/3.08 (2dd, 2 H), 2.95 (m, 2 H), 2.25/1.75 (2m, 2 H), 1.95/1.45 (2m, 2 H), 1.6 (m, 2 H), 1.25/1.1 (2m, 2 H)
ESI/FIA/HR et MS/MS : [M+H]+ = 371.1712 (371.1730)
Analyse Elémentaire : C=54.93(55.13);H=7.41(7.35);N=7.56(7.56)

### Intermédiaire 53 : 3-{4-[Bis(tert-butoxycarbonyl)amino]butyl}-4-éthoxy-4-oxo-1-(thiophen-3-ylméthyl)-1,4-azaphosphinane-3-carboxylate de tert-butyle

L'intermédiaire 53 est obtenu à partir de l'intermédiaire 21 et du 3-thiophenaldéhyde selon la procédure F précédemment décrite.
RMN ¹H : (400 MHz, dmso-d6) δ ppm 7.49 (m, 1 H), 7.31 (m, 1 H), 7.02 (m, 1 H), 3.95 (m, 2 H), 3.6/3.49 (AB, 2 H), 3.4 (m, 2 H), 3/2.8 (m, 2 H), 2.8/2.45 (m, 2 H), 1.9 (m, 4 H), 1.4 (m, 29 H), 1.2 (t, 3 H), 0.95/0.85 (m, 2 H)
RMN ³¹P : (400 MHz, dmso-d6) δ ppm 47/45

### EXEMPLE 63 : Acide 3-(4-aminobutyl)-4-hydroxy-4-oxo-1-(thiophen-3-ylméthyl)-1,4-azaphosphinane-3-carboxylique

L'exemple 63 est obtenu à partir de l'intermédiaire 53 selon la procédure D précédemment décrite.
RMN ¹H : (400 MHz, D2O) δ ppm 7.61 (d, 1 H), 7.54 (dd, 1 H), 7.2 (d, 1 H), 4.42/4.28 (2*d, 2 H), 3.71/3.32 (m, 2 H), 3.48/3.06 (m, 2 H), 2.94 (m, 2 H), 2.25/1.77 (m, 2 H), 1.93/1.46 (m, 2 H), 1.6 (m, 2 H), 1.26/1.13 (m, 2 H)
ESI/FIA/HR et MS/MS : [M+H]+ = 347.1182 (347.1189)
Analyse Elémentaire : C=48.14(48.55);H=6.55(6.69);N=7.87(8.09);S=8.65(9.26)

### Intermédiaire 54 : 3-{4-[Bis(tert-butoxycarbonyl)amino]butyl}-4-éthoxy-1-[(4-hydroxy-3-méthoxyphényl)méthyl]-4-oxo-1,4-azaphosphinane-3-carboxylate de tert-butyle

L'intermédiaire 54 est obtenu à partir de l'intermédiaire 21 et du 4-hydroxy-3-methoxybenzaldéhyde selon la procédure F précédemment décrite.
RMN ¹H : (400 MHz, dmso-d6) δ ppm 8.8 (s, 1 H), 6.8 (df, 1 H), 6.7 (d, 1 H), 6.62 (dd, 1 H), 3.95 (m, 2 H), 3.71 (s, 3 H), 3.49/3.31 (2d, 2 H), 3.4 (m, 2 H), 3-2.2 (m, 4 H), 2-1.8 (m, 4 H), 1.4 (2s+m, 29 H), 1.2 (2t, 3 H), 1/0.85 (2m, 2 H)

### EXEMPLE 64 : Acide 3-(4-aminobutyl)-4-hydroxy-1-[(4-hydroxy-3-méthoxyphényl)méthyl]-4-oxo-1,4-azaphosphinane-3-carboxylique

L'exemple 64 est obtenu à partir de l'intermédiaire 54 selon la procédure D précédemment décrite.
RMN ¹H : (300 MHz, D2O) δ ppm 7.09 (m, 1 H), 6.94 (m, 2 H), 4.3/4.13 (2*d, 2 H), 3.7/3.3 (2*m, 2 H), 3.65 (s, 3 H), 3.45/3.07 (2*m, 2 H), 2.92 (m, 2 H), 2.22/1.77 (2*m, 2 H), 1.92/1.6 (2*m, 2 H), 1.6/1.48 (2*m, 2 H), 1.23/1.11 (2*m, 2 H)
ESI/FIA/HR et MS/MS : [M+H]+ = 387.1665 (387.1685)
Analyse Elémentaire : C=53.10(52.85);H=7.01(7.04);N=7.30(7.25)

### Intermédiaire 55 : 3-{4-[Bis(tert-butoxycarbonyl)amino]butyl}-1-[(2-carboxyphényl)méthyl]-4-éthoxy-4-oxo-1,4-azaphosphinane-3-carboxylate de tert-butyle

L'intermédiaire 55 est obtenu à partir de l'intermédiaire 21 et du 2-formylbenzoate de méthyle selon la procédure F précédemment décrite.
RMN ¹H : (400 MHz, dmso-d6) δ ppm 7.65 (d, 1 H), 7.5 (t, 1 H), 7.38 (m, 2 H), 4.05/3.4 (2d, 2 H), 3.95 (m, 2 H), 3.8 (2s, 3 H), 3.3/3.2 (2m, 2 H), 3-2.35 (m, 4 H), 2 (m, 1 H), 1.8-1.6 (m, 3 H), 1.4/1.3 (2s+m, 29 H), 1.2 (2t, 3 H), 0.5/0.35 (2m, 2 H)

### EXEMPLE 65 : Acide 3-(4-aminobutyl)-1-[(2-carboxyphényl)méthyl]-4-hydroxy-4-oxo-1,4-azaphosphinane-3-carboxylique

L'exemple 55 est obtenu à partir de l'intermédiaire 65 selon la procédure D précédemment décrite.
ESI/FIA/HR et MS/MS : [M+H]+ = 385.1504 (385.1523)
Analyse Elémentaire : C=53.42(53.12);H=6.25(6.56);N=7.40(7.29)

### Intermédiaire 56 : 3-{4-[Bis(tert-butoxycarbonyl)amino]butyl}-1-[(3-chloro-4-hydroxyphényl)méthyl]-4-éthoxy-4-oxo-1,4-azaphosphinane-3-carboxylate de tert-butyle

L'intermédiaire 56 est obtenu à partir de l'intermédiaire 21 et du 3-chloro-4-hydroxybenzaldéhyde selon la procédure F précédemment décrite.
RMN ¹H : (400 MHz, dmso-d6) δ ppm 10.05 (m, 1 H), 7.22 (df, 1 H), 7.02 (dd, 1 H), 6.9 (d, 1 H), 3.98 (m, 2 H), 3.5/3.31 (2d, 2 H), 3.4 (m, 2 H), 3-2.25 (m, 4 H), 2-1.8 (m, 4 H), 1.4 (2s+m, 29 H), 1.2 (2t, 3 H), 1.1-0.8 (2m, 2 H)

### EXEMPLE 66 : Acide 3-(4-aminobutyl)-1-[(3-chloro-4-hydroxyphényl)méthyl]-4-hydroxy-4-oxo-1,4-azaphosphinane-3-carboxylique

L'exemple 66 est obtenu à partir de l'intermédiaire 56 selon la procédure D précédemment décrite.
RMN ¹H : (400 MHz, D2O) δ ppm 7.42 (df, 1 H), 7.2 (dd, 1 H), 6.97 (d, 1 H), 4.27/4.07 (2*d, 2 H), 3.62/3.22 (2*m, 2 H), 3.39/3 (2*m, 2 H), 2.85 (m, 2 H), 2.18/1.7 (2*m, 2 H), 1.85/1.52 (2*m, 2 H), 1.52/1.4 (2*m, 2 H), 1.18/1.05 (2*m, 2 H)
ESI/FIA/HR et MS/MS : [M+H]+ = 391.1189 (391.1189)
Analyse Elémentaire : C=49.09(49.17);H=6.02(6.19);N=7.18(7.17)

### Intermédiaire 57 : 3-{4-[Bis(tert-butoxycarbonyl)amino]butyl}-4-éthoxy-1-[[4-hydroxy-3-(trifluoromethoxy)phényl]méthyl]-4-oxo-1,4-azaphosphinane-3-carboxylate de tert-butyle

L'intermédiaire 57 est obtenu à partir de l'intermédiaire 21 et du 4-hydroxy-3-(trifluorométhyl)benzaldéhyde selon la procédure F précédemment décrite.
RMN ¹H : (400 MHz, dmso-d6) δ ppm 10.1 (m, 1 H), 7.15 (df, 1 H), 7.08 (dd, 1 H), 6.95 (d, 1 H), 3.98 (m, 2 H), 3.5/3.4 (2d, 2 H), 3.4 (m, 2 H), 3-2.2 (m, 4 H), 2-1.8 (m, 4 H), 1.4 (2s+m, 29 H), 1.2 (2t, 3 H), 1.1-0.8 (2m, 2 H)

### EXEMPLE 67 : Acide 3-(4-aminobutyl)-4-hydroxy-1-[[4-hydroxy-3-(trifluoromethoxy)phényl]méthyl]-4-oxo-1,4-azaphosphinane-3-carboxylique

L'exemple 67 est obtenu à partir de l'intermédiaire 57 selon la procédure D précédemment décrite.
RMN ¹H : (400 MHz, D2O) δ ppm 7.44 (df, 1 H), 7.33 (dd, 1 H), 7.09 (d, 1 H), 4.38/4.13 (2*d, 2 H), 3.7/3.31 (2*dd, 2 H), 3.44/3.07 (2*dd, 2 H), 2.92 (m, 2 H), 2.23/1.78 (2*m, 2 H), 1.94/1.59 (2*m, 2 H), 1.59/1.46 (2*m, 2 H), 1.21/1.1 (2*m, 2 H)
RMN ¹⁹F : (400 MHz, D2O) δ ppm -58.3
RMN ³¹P : (400 MHz, D2O) δ ppm 26
ESI/FIA/HR et MS/MS : [M+H]+ = 441.1403 (441.1402)
Analyse Elémentaire : C=46.46(46.37);H=4.98(5.49);N=6.42(6.36)

### Intermédiaire 58 : 3-{4-[Bis(tert-butoxycarbonyl)amino]butyl}-4-éthoxy-1-[(4-hydroxy-3,5-diméthylphényl)méthyl]-4-oxo-1,4-azaphosphinane-3-carboxylate de tert-butyle

L'intermédiaire 58 est obtenu à partir de l'intermédiaire 21 et du 3,5-diméthyl-4-hydroxybenzaldéhyde selon la procédure F précédemment décrite.
RMN ¹H : (400 MHz, dmso-d6) δ ppm 8.09 (s, 1 H), 6.8 (s, 2 H), 3.98 (m, 2 H), 3.49/3.21 (2d, 2 H), 3.35 (m, 2 H), 3-2.2 (m, 4 H), 2.15 (s, 6 H), 2-1.8 (m, 4 H), 1.4 (2s+m, 29 H), 1.2 (2t, 3 H), 0.95-0.82 (2m, 2 H)

### EXEMPLE 68 : Acide 3-(4-aminobutyl)-4-hydroxy-1-[(4-hydroxy-3,5-diméthylphényl)méthyl]-4-oxo-1,4-azaphosphinane-3-carboxylique

L'exemple 68 est obtenu à partir de l'intermédiaire 58 selon la procédure D précédemment décrite.
RMN ¹H : (400 MHz, D2O) δ ppm 7.1 (s, 2 H), 4.25-4.1 (d, 2 H), 3.7-3.5 (m, 2 H), 3.25 (m, 1 H), 3.1 (m, 1 H), 2.95 (t, 2 H), 2.2 (s, 6 H), 2.2/1.75 (m, 2 H), 1.95 (m, 1 H), 1.6 (m, 2 H), 1.5 (m, 1 H), 1.3-1.1 (m, 2 H)
ESI/FIA/HR et MS/MS : [M+H]+ = 385.1889 (385.1892)
Analyse Elémentaire : C=55.50(56.24);H=7.07(7.60);N=7.16(7.29)

### Intermédiaire 59 : 3-{4-[Bis(tert-butoxycarbonyl)amino]butyl}-4-éthoxy-4-oxo-1-(pyridin-4-ylméthyl)-1,4-azaphosphinane-3-carboxylate de tert-butyle

L'intermédiaire 59 est obtenu à partir de l'intermédiaire 21 et de la 4-formylpyridine selon la procédure F précédemment décrite.
RMN ¹H : (400 MHz, dmso-d6) δ ppm 8.5 (d, 2 H), 7.3 (d, 2 H), 4.1-3.9 (2m, 2 H), 3.65/3.52 (2x2d, 2 H), 3.45 (2m, 2 H), 2.95-2.3 (m, 4 H), 1.98 (m, 4 H), 1.5-1.35 (m+s, 29 H), 1.22 (2t, 3 H), 1.05 (m, 2 H)

### EXEMPLE 69 : Acide 3-(4-aminobutyl)-4-hydroxy-4-oxo-1-(pyridin-4-ylméthyl)-1,4-azaphosphinane-3-carboxylique

L'exemple 69 est obtenu à partir de l'intermédiaire 59 selon la procédure D précédemment décrite.
RMN ¹H : (400 MHz, dmso-d6) δ ppm 8.5 (d, 2 H), 8.05 (m, 3 H), 7.3 (d, 2 H), 3.55 (AB, 2 H), 3.05/2.3 (2m, 2 H), 2.7 (m, 3 H), 2.5 (m, 1 H), 1.75 (m, 1 H), 1.65-1.15 (m, 7 H) ESI/FIA/HR et MS/MS : [M+H]+ = 342.1577 (342.1582)
Analyse Elémentaire : C=52.56(52.78);H=6.70(7.09);N=12.22(12.31)

### Intermédiaire 60 : 3-{4-[Bis(tert-butoxycarbonyl)amino]butyl}-4-éthoxy-4-oxo-1-(pyridin-3-ylméthyl)-1,4-azaphosphinane-3-carboxylate de tert-butyle

L'intermédiaire 60 est obtenu à partir de l'intermédiaire 21 et de la 3-formylpyridine selon la procédure F précédemment décrite.
RMN ¹H : (400 MHz, dmso-d6) δ ppm 8.5 (d+s, 2 H), 7.7 (2dd, 1 H), 7.35 (m, 1 H), 4.1-3.9 (2m, 2 H), 3.65/3.52 (2x2d, 2 H), 3.45 (2m, 2 H), 3-2.3 (m, 4 H), 1.9 (m, 4 H), 1.4 (m+s, 30 H), 1.22 (2t, 3 H), 0.82 (m, 1H)

### EXEMPLE 70 : Acide 3-(4-aminobutyl)-4-hydroxy-4-oxo-1-(pyridin-3-ylméthyl)-1,4-azaphosphinane-3-carboxylique

L'exemple 70 est obtenu à partir de l'intermédiaire 60 selon la procédure D précédemment décrite.
RMN ¹H : (400 MHz, D2O) δ ppm 8.6 (s+d, 2 H), 8 (dd, 1 H), 7.52 (dd, 1 H), 4.4 (AB, 2 H), 3.7 (m, 1 H), 3.4 (m, 2 H), 3.18 (dd, 1 H), 2.9 (m, 2 H), 2.28/1.8 (2m, 2 H), 1.91 (m, 1 H), 1.6 (m, 2 H), 1.5 (m, 1 H), 1.21/1.1 (2m, 2 H)
ESI/FIA/HR et MS/MS : [M+H]+ = 342.1568 (342.1582)
Analyse Elémentaire : C=52.32(52.78);H=6.66(7.09);N=12.32(12.31)

### Intermédiaire 61 : 3-{4-[Bis(tert-butoxycarbonyl)amino]butyl}-4-éthoxy-4-oxo-1-(pyridin-2-ylméthyl)-1,4-azaphosphinane-3-carboxylate de tert-butyle

L'intermédiaire 61 est obtenu à partir de l'intermédiaire 21 et de la 2-formylpyridine selon la procédure F précédemment décrite.
RMN ¹H : (400 MHz, dmso-d6) δ ppm 8.5 (df, 1 H), 7.75 (t, 1 H), 7.42/7.25 (2dd, 2 H), 4.1-3.9 (2m, 2 H), 3.71/3.6 (2AB, 2 H), 3.5-3.35 (m, 2 H), 3-2.3 (m, 4 H), 2.0-2.9 (m, 4 H), 1.45/1.35 (3s, 27 H), 1.4/1-0.8 (3m, 4 H), 1.22 (2t, 3 H)

### EXEMPLE 71 : Acide 3-(4-aminobutyl)-4-hydroxy-4-oxo-1-(pyridin-2-ylméthyl)-1,4-azaphosphinane-3-carboxylique

L'exemple 71 est obtenu à partir de l'intermédiaire 61 selon la procédure D précédemment décrite.
RMN ¹H : (400 MHz, D2O) δ ppm 8.6 (dd, 1 H), 7.91 (t, 1 H), 7.55 (d, 1 H), 7.45 (dd, 1 H), centré à 4.41 (AB, 2 H), 3.75/3.4 (2m, 2 H), 3.55/3.3 (2dd, 2 H), 2.95 (m, 2 H), 2.3/1.78 (2m, 2 H), 1.98 (m, 1 H), 1.65 (m, 2 H), 1.5 (m, 1 H), 1.3/1.2 (2m, 2 H)
ESI/FIA/HR et MS/MS : [M+H]+ = 342.1592 (342.1582)
Analyse Elémentaire : C=52.72(52.78);H=6.92(7.09);N=12.25(12.31)

### Intermédiaire 63 : 3-{4-[Bis(tert-butoxycarbonyl)amino]butyl}-1-(2-cyclohexyléthyl)-4-éthoxy-4-oxo-1,4-azaphosphinane-3-carboxylate de tert-butyle

L'intermédiaire 63 est obtenu à partir de l'intermédiaire 21 et du 2-cyclohexylacétaldéhyde selon la procédure F précédemment décrite.
RMN ¹H : (400 MHz, dmso-d6) δ ppm 3.98 (m, 2 H), 3.48 (t, 2 H), 2.8 (m, 2 H), 2.5-2.2 (m, 4 H), 2-0.8 (m, 19 H), 1.9 (m, 2 H), 1.45/1.4 (2s, 27 H), 1.2 (t, 3 H)

### EXEMPLE 73 : Acide 3-(4-aminobutyl)-1-(2-cyclohexyléthyl)-4-hydroxy-4-oxo-1,4-azaphosphinane-3-carboxylique

L'exemple 73 est obtenu à partir de l'intermédiaire 63 selon la procédure D précédemment décrite.
RMN ¹H : (400 MHz, D2O) δ ppm 3.7-3.5 (m, 2 H), 3.3 (m, 1 H), 3.15 (m, 3 H), 3 (t, 2 H), 2.22/1.8 (2m, 2 H), 2 (m, 1 H), 1.7-0.85 (m, 17 H), 1.65 (m, 1 H)
ESI/FIA/HR et MS/MS : [M+H]+ = 361.2257 (361.2256)
Analyse Elémentaire : C=56.25(56.65);H=8.92(9.23);N=7.49(7.77)

### Intermédiaire 64 : 3-{4-[Bis(tert-butoxycarbonyl)amino]butyl}-4-éthoxy-4-oxo-1-[(4-phénylphényl)méthyl]-1,4-azaphosphinane-3-carboxylate de tert-butyle

L'intermédiaire 64 est obtenu à partir de l'intermédiaire 21 et du 4-phénylbenzaldéhyde selon la procédure F précédemment décrite.
RMN ¹H : (400 MHz, dmso-d6) δ ppm 7.69 (2d, 4 H), 7.5-7.3 (m, 5 H), 4 (m, 2 H), 3.7/3.45 (2d, 2 H), 3.4 (m, 2 H), 3.05-2.3 (m, 4 H), 2.05-1.65 (m, 4 H), 1.4 (m+2s, 29 H), 1.22 (2t, 3 H), 1-0.7 (m, 2 H)

### EXEMPLE 74 : Acide 3-(4-aminobutyl)-4-hydroxy-4-oxo-1-[(4-phénylphényl)méthyl]-1,4-azaphosphinane-3-carboxylique

L'exemple 74 est obtenu à partir de l'intermédiaire 64 selon la procédure D précédemment décrite.
RMN ¹H : (400 MHz, D2O) δ ppm 7.8 (d, 2 H), 7.7 (d, 2 H), 7.55 (d, 2 H), 7.5 (t, 2 H), 7.41 (t, 1 H), 4.45/4.28 (2d, 2 H), 3.75/3.35 (2m, 2 H), 3.5/3.15 (2dd, 2 H), 2.9 (m, 2 H), 2.25/1.8 (2m, 2 H), 1.95/1.45 (2m, 2 H), 1.6 (m, 2 H), 1.25/1.1 (2m, 2 H)
ESI/FIA/HR et MS/MS : [M+H]+ = 417.1932 (417.1943)
Analyse Elémentaire : C=63.25(63.45);H=6.64(7.02);N=6.55(6.73)

### Intermédiaire 65 : 3-{4-[Bis(tert-butoxycarbonyl)amino]butyl}-4-éthoxy-4-oxo-1-[(3-phénoxyphényl)méthyl]-1,4-azaphosphinane-3-carboxylate de tert-butyle

L'intermédiaire 65 est obtenu à partir de l'intermédiaire 21 et du 3-phénoxybenzaldéhyde selon la procédure F précédemment décrite.
RMN ¹H : (400 MHz, dmso-d6) δ ppm 7.38 (2t, 3 H), 7.15 (t, 1 H), 7.05 (d, 1 H), 7.05 (d, 2 H), 6.95 (si, 1 H), 6.9 (d, 1 H), 3.98 (m, 2 H), 3.6/3.45 (2d, 2 H), 3.4 (m, 2 H), 3-2.3 (m, 4 H), 2-1.6 (m, 6 H), 1.4 (2s, 27 H), 1.2 (2t, 3 H), 1-0.75 (m, 2 H)

### EXEMPLE 75 : Acide 3-(4-aminobutyl)-4-hydroxy-4-oxo-1-[(3-phénoxyphényl)méthyl]-1,4-azaphosphinane-3-carboxylique

L'exemple 75 est obtenu à partir de l'intermédiaire 65 selon la procédure D précédemment décrite.
RMN ¹H : (400 MHz, D2O) δ ppm 7.5-7.3 (2t, 3 H), 7.2-6.95 (m, 6 H), centré à 4.2 (AB, 2 H), 3.65/3.25 (2m, 2 H), 3.4/3 (2dd, 2 H), 2.82 (m, 2 H), 2.19/1.7 (2m, 2 H), 1.88/1.4 (2m, 2 H), 1.52 (m, 2 H), 1.2-1 (2m, 2 H)
ESI/FIA/HR et MS/MS : [M+H]+ = 433.1891 (433.1892)
Analyse Elémentaire : C=60.90(61.10);H=6.58(6.76);N=6.34(6.48)

### Intermédiaire 66 : 3-{4-[Bis(tert-butoxycarbonyl)amino]butyl}-4-éthoxy-4-oxo-1-(3-phénylpropyl)-1,4-azaphosphinane-3-carboxylate de tert-butyle

L'intermédiaire 66 est obtenu à partir de l'intermédiaire 21 et du 3-phénylpropanal selon la procédure F précédemment décrite.
RMN ¹H : (400 MHz, dmso-d6) δ ppm 7.29 (t, 2 H), 7.15 (d+t, 3 H), 3.98 (m, 2 H), 3.49 (t, 2 H), 2.9-2.2 (m, 4 H), 2.59 (t, 2 H), 2.35 (m, 2 H), 2-1.6 (m, 7 H), 1.5 (m, 1 H), 1.4 (2s, 27 H), 1.25/1 (2m, 2 H), 1.2 (2t, 3 H)

### EXEMPLE 76 : Acide 3-(4-aminobutyl)-4-hydroxy-4-oxo-1-(3-phénylpropyl)-1,4-azaphosphinane-3-carboxylique

L'exemple 76 est obtenu à partir de l'intermédiaire 66 selon la procédure D précédemment décrite.
RMN ¹H : (400 MHz, D2O) δ ppm 7.38 (t, 2 H), 7.27 (m, 3 H), 3.62/3.28 (2m, 2 H), 3.52 (dd, 1 H), 3.11 (m, 3 H), 3 (td, 2 H), 2.7 (t, 2 H), 2.25/1.75 (2m, 2 H), 2.09 (m, 2 H), 1.95/1.5 (2m,
2 H), 1.65 (m, 2 H), 1.38/1.22 (2m, 2 H)
ESI/FIA/HR et MS/MS : [M+H]+ = 369.195 (369.1943)
Analyse Elémentaire : C=58.46(58.68);H=7.45(7.93);N=7.54(7.60)

### Intermédiaire 68 : 3-14-[Bis(tert-butoxycarbonyl)amino]butyl}-4-éthoxy-4-oxo-1-[(4-phénoxyphényl)méthyl]-1,4-azaphosphinane-3-carboxylate de tert-butyle

L'intermédiaire 68 est obtenu à partir de l'intermédiaire 21 et du 4-phénoxybenzaldéhyde selon la procédure F précédemment décrite.
RMN ¹H : (400 MHz, dmso-d6) δ ppm 7.4 (t, 2 H), 7.3 (d, 2 H), 7.12 (t, 1 H), 6.98 (2d, 4 H), 3.99 (m, 2 H), 3.6/3.4 (2d, 2 H), 3.38 (m, 2 H), 3-2.25 (m, 4 H), 2-1.65 (m, 6 H), 1.4 (2s, 27 H), 1.2 (2t, 3 H), 1-0.75 (m, 2 H)

### EXEMPLE 78 : Acide 3-(4-aminobutyl)-4-hydroxy-4-oxo-1-[(4-phénoxyphényl)méthyl]-1,4-azaphosphinane-3-carboxylique

L'exemple 78 est obtenu à partir de l'intermédiaire 68 selon la procédure D précédemment décrite.
RMN ¹H : (400 MHz, D2O) δ ppm 7.44 (d, 2 H), 7.41 (t, 2 H), 7.21 (t, 1 H), 7.08 (d, 2 H), 7.08 (d, 2 H), 4.38/4.21 (2d, 1+1 H), 3.7/3.31 (m+m, 1+1 H), 3.5/3.1 (2d, 2 H), 2.93 (m, 2 H), 2.23/1.77 (m+m, 1+1 H), 1.93/1.48 (m+m, 1+1 H), 1.6 (quint., 2 H), 1.24/1.13 (m+m, 1+1 H) RMN ¹³C : (400 MHz, D2O) δ ppm 177.6, 158.2, 155.7, 132.1, 130, 124.3, 124.1, 119.3, 118.7, 59.4, 51.6, 50.9, 38.8, 27, 26.6, 24.8, 20
RMN ³¹P : (400 MHz, D2O) δ ppm 25
ESI/FIA/HR et MS/MS : [M+H]+ = 433.1884 (433.1892)
Analyse Elémentaire : C=60.77(61.10);H=6.27(6.76);N=6.42(6.48)

### EXEMPLE 79 : Acide 3-(4-aminobutyl)-4-hydroxy-4-oxo-1-(2-phénylméthoxyéthyl)-1,4-azaphosphinane-3-carboxylique

L'exemple 79 est obtenu à partir de l'intermédiaire 21 et du benzyloxyacétaldéhyde selon les procédures F et D précédemment décrites.
RMN ¹H : (400 MHz, D2O) δ ppm 7.42 (m, 5 H), 4.63 (d, 1 H), 4.55 (d, 1 H), 3.82 (t, 2 H), 3.61 (dd, 1 H), 3.5 (m, 1 H), 3.33 (m, 2 H), 3.26 (m, 1 H), 3.16 (dd, 1 H), 2.92 (t, 2 H), 2.24 (m, 1 H), 1.94 (m, 1 H), 1.71 (m, 1 H), 1.61 (quint, 2 H), 1.48 (m, 1 H), 1.21 (m, 2 H) RMN ³¹P : (400 MHz, D2O) δ ppm 25.6
ESI/FIA/HR et MS/MS : [M+H]+ = 385.1883 (385.1892)

### EXEMPLE 80 : Acide 3-(4-aminobutyl)-4-hydroxy-1-(2-hydroxyéthyl)-4-oxo-1,4-azaphosphinane-3-carboxylique

L'exemple 80 est obtenu à partir de l'exemple 79 selon la procédure E précédemment décrite.
RMN ¹H : (400 MHz, D2O) δ ppm 3.9 (m, 2 H), 3.72 (dd, 1 H), 3.62 (dd, 1 H), 3.32 (m, 1 H), 3.29 (t, 2 H), 3.25 (dd, 1 H), 2.99 (m, 2 H), 2.29 (m, 1 H), 1.99 (m, 1 H), 1.79 (m, 1 H), 1.67 (quint, 2 H), 1.52 (m, 1 H), 1.41 (m, 1 H), 1.29 (m, 1 H)
ESI/FIA/HR et MS/MS : [M+H]+ = 295.1424 (295.1422)
Analyse Elémentaire : C=44.08(44.90);H=7.66(7.88);N=9.23(9.52)

### Intermédiaire 69 : 3-{4-[Bis(tert-butoxycarbonyl)amino]butyl}-1-[(3-chlorophényl)méthyl]-4-éthoxy-4-oxo-1,4-azaphosphinane-3-carboxylate de tert-butyle

L'intermédiaire 69 est obtenu à partir de l'intermédiaire 21 et du 3-chlorobenzaldéhyde selon la procédure F précédemment décrite.
RMN ¹H : (400 MHz, dmso-d6) δ ppm 7.4-7.2 (m, 4 H), 3.99 (m, 2 H), 3.62/3.43 (AB, 1+1 H), 3.38 (m, 2 H), 2.92/2.36 (m)+(m, 1+1 H), 2.73/2.47 (m)+(m, 1+1 H), 1.96 (m, 2 H), 1.89 (m, 2 H), 1.41 (m, 2 H), 1.37/1.33 (2*(s, 27 H), 1.2 (t, 3 H), 0.81 (m, 2 H)
RMN ¹³C : (400 MHz, dmso-d6) δ ppm 127-130, 152.3, 60.9, 60.6, 54.9, 51.6, 45.6, 28.9, 28.9, 27.6, 24.7, 20.6, 133.2, 81.8/81.1

### EXEMPLE 81 : Acide 3-(4-aminobutyl)-1-[(3-chlorophényl)méthyl]-4-hydroxy-4-oxo-1,4-azaphosphinane-3-carboxylique

L'exemple 81 est obtenu à partir de l'intermédiaire 69 selon la procédure D précédemment décrite.
RMN ¹H : (400 MHz, D2O) δ ppm 7.52-7.35 (m, 4 H), centré à 4.32 (AB, 2 H), 3.7/3.32 (2m, 2 H), 3.5/3.12 (2dd, 2 H), 2.93 (m, 2 H), 2.25/1.8 (2m, 2 H), 1.95/1.48 (2m, 2 H), 1.6 (m, 2 H), 1.25/1.11 (2m, 2 H)
ESI/FIA/HR et MS/MS : [M+H]+ = 375.1256 (375.1240)
Analyse Elémentaire : C=51.26(51.27);H=6.32(6.45);N=7.43(7.47)

### Intermédiaire 70 : 3-{4-[Bis(tert-butoxycarbonyl)amino]butyl}-4-éthoxy-1-[(3-hydroxyphényl)méthyl]-4-oxo-1,4-azaphosphinane-3-carboxylate de tert-butyle

L'intermédiaire 70 est obtenu à partir de l'intermédiaire 21 et du 3-hydroxybenzaldéhyde selon la procédure F précédemment décrite.
RMN ¹H : (400 MHz, dmso-d6) δ ppm 9.25 (s, 1 H), 7.1 (t, 1 H), 6.7-6.55 (m, 3 H), 3.98 (m, 2 H), 3.52/3.4 (2d, 2 H), 3.4 (m, 2 H), 3-2.2 (m, 4 H), 2-1.65 (m, 6 H), 1.4 (2s, 27 H), 1.2 (2t, 3 H), 0.95/0.82 (2m, 2 H)

### EXEMPLE 82 : Acide 3-(4-aminobutyl)-4-hydroxy-1-[(3-hydroxyphényl)méthyl]-4-oxo-1,4-azaphosphinane-3-carboxylique

L'exemple 82 est obtenu à partir de l'intermédiaire 70 selon la procédure D précédemment décrite.
RMN ¹H : (400 MHz, D2O) δ ppm 7.38 (t, 1 H), 7-6.9 (m, 3 H), centré à 4.18 (AB, 2 H), 3.7/3.32 (2m, 2 H), 3.45/3.1 (2dd, 2 H), 2.91 (m, 2 H), 2.25/1.8 (2m, 2 H), 1.92/1.48 (2m, 2 H), 1.6 (m, 2 H), 1.25/1.1 (2m, 2 H)
ESI/FIA/HR et MS/MS : [M+H]+ = 357.1573 (357.1579)
Analyse Elémentaire : C=53.22(53.93);H=6.98(7.07);N=7.72(7.86)

### Intermédiaire 71 : 3-{4-[Bis(tert-butoxycarbonyl)amino]butyl}-4-éthoxy-1-[(4-hydroxyphényl)méthyl]-4-oxo-1,4-azaphosphinane-3-carboxylate de tert-butyle

L'intermédiaire 71 est obtenu à partir de l'intermédiaire 21 et du 4-hydroxybenzaldéhyde selon la procédure F précédemment décrite.
RMN ¹H : (400 MHz, dmso-d6) δ ppm 9.25 (s, 1 H), 7.05 (d, 2 H), 6.7 (d, 2 H), 3.98 (m, 2 H), 3.49/3.3 (2d, 2 H), 3.4 (m, 2 H), 3-2.2 (m, 4 H), 2-1.8 (m, 4 H), 1.4 (2s+m, 29 H), 1.2 (2t, 3 H), 0.95/0.82 (2m, 2 H)

### EXEMPLE 83 : Acide 3-(4-aminobutyl)-4-hydroxy-1-[(4-hydroxyphényl)méthyl]-4-oxo-1,4-azaphosphinane-3-carboxylique

L'exemple 83 est obtenu à partir de l'intermédiaire 71 selon la procédure D précédemment décrite.
RMN ¹H : (400 MHz, D2O) δ ppm 7.38 (d, 2 H), 6.95 (d, 2 H), centré à 4.25 (AB, 2 H), 3.7/3.3 (2m, 2 H), 3.48/3.05 (2dd, 2 H), 2.95 (m, 2 H), 2.21/1.75 (2m, 2 H), 1.95/1.5 (2m, 2 H), 1.6 (m, 2 H), 1.25/1.11 (2m, 2 H)
ESI/FIA/HR et MS/MS : [M+H]+ = 357.1572 (357.1579)
Analyse Elémentaire : C=53.24(53.93);H=6.89(7.07);N=7.57(7.86)

### Intermédiaire 72 : 3-{4-[Bis(tert-butoxycarbonyl)amino]butyl}-1-(furan-3-ylméthyl)-4-éthoxy-4-oxo-1,4-azaphosphinane-3-carboxylate de tert-butyle

L'intermédiaire 72 est obtenu à partir de l'intermédiaire 21 et du 3-furaldéhyde selon la procédure F précédemment décrite.
RMN ¹H : (400 MHz, dmso-d6) δ ppm 7.61/7.55 (2sl, 2 H), 6.39 (si, 1 H), 3.98 (m, 2 H), 3.49/3.33 (2d, 2 H), 3.45 (m, 2 H), 3-2.2 (m, 4 H), 2-1.8 (m, 4 H), 1.4 (2s+m, 29 H), 1.2 (2t, 3 H), 1.05/0.9 (2m, 2 H)

### EXEMPLE 84 : Acide 3-(4-aminobutyl)-1-(furan-3-ylméthyl)-4-hydroxy-4-oxo-1,4-azaphosphinane-3-carboxylique

L'exemple 84 est obtenu à partir de l'intermédiaire 72 selon la procédure D précédemment décrite.
RMN ¹H : (400 MHz, D2O) δ ppm 7.71 (si, 1 H), 7.59 (si, 1 H), 6.58 (si, 1 H), centré à 4.22 (AB, 2 H), 3.7/3.35 (2m, 2 H), 3.55/3.08 (2dd, 2 H), 2.95 (m, 2 H), 2.25/1.8 (2m, 2 H), 1.95/1.5 (2m, 2 H), 1.62 (m, 2 H), 1.31/1.18 (2m, 2 H)
ESI/FIA/HR et MS/MS : [M+H]+ = 331.1431 (331.1422)
Analyse Elémentaire : C=50.45(50.91);H=6.72(7.02);N=8.39(8.48)

### Intermédiaire 73 : 3-{4-[Bis(tert-butoxycarbonyl)amino]butyl}-4-éthoxy-1-[(2-hydroxyphényl)méthyl]-4-oxo-1,4-azaphosphinane-3-carboxylate de tert-butyle

L'intermédiaire 73 est obtenu à partir de l'intermédiaire 21 et du 2-hydroxybenzaldéhyde selon la procédure F précédemment décrite.
RMN ¹H : (400 MHz, dmso-d6) δ ppm 9.45 (s, 1 H), 7.1 (d+t, 2 H), 6.78 (d+t, 2 H), 4 (m, 2 H), 3.62/3.52 (2d, 2 H), 3.4 (m, 2 H), 3-2.3 (m, 4 H), 2-1.8 (m, 4 H), 1.42/1.38 (m+2s, 29 H), 1.21 (2t, 3 H), 0.98/0.85 (2m, 2 H)

### EXEMPLE 85 : Acide 3-(4-aminobutyl)-4-hydroxy-1-[(2-hydroxyphényl)méthyl]-4-oxo-1,4-azaphosphinane-3-carboxylique

L'exemple 85 est obtenu à partir de l'intermédiaire 73 selon la procédure D précédemment décrite.
RMN ¹H : (400 MHz, D2O) δ ppm 7.39 (t, 1 H), 7.3 (d, 1 H), 6.95 (d+t, 2 H), 4.28 (AB, 2 H), 3.65/3.28 (2m, 2 H), 3.52/3.2 (2dd, 2 H), 2.95 (m, 2 H), 2.2/1.72 (2m, 2 H), 1.98/1.5 (2m, 2 H), 1.62 (m, 2 H), 1.3/1.18 (2m, 2 H)
ESI/FIA/HR et MS/MS : [M+H]+ = 357.1591 (357.1579)
Analyse Elémentaire : C=53.22(53.93);H=6.97(7.07);N=7.71(7.86)

### Intermédiaire 74 : 3-{4-[Bis(tert-butoxycarbonyl)amino]butyl}-4-éthoxy-1-[[2-(4-hydroxyphényl)phényl]méthyl]-4-oxo-1,4-azaphosphinane-3-carboxylate de tert-butyle

L'intermédiaire 74 est obtenu à partir de l'intermédiaire 21 et du 2-(4-hydroxyphényl)benzaldéhyde selon la procédure F précédemment décrite.
RMN ¹H : (400 MHz, dmso-d6) δ ppm 9.45 (s, 1 H), 7.45 (dd, 1 H), 7.3 (td, 2 H), 7.16 (dd, 1 H), 7.13 (d, 2 H), 6.81 (d, 2 H), 3.93 (quad., 2 H), 3.54/3.36 (d, 2 H), 3.28 (t, 2 H), 2.85-2.15 (m, 2 H), 2.85-2.15 (m, 2 H), 1.84 (m, 2 H), 1.84 (m, 2 H), 1.47-1.3 (s, 27 H), 1.47-1.3 (s, 2 H), 1.17 (t, 3 H), 0.7 (m, 2 H)
RMN ³¹P : (400 MHz, dmso-d6) δ ppm 45.3

### EXEMPLE 86 : Acide 3-(4-aminobutyl)-4-hydroxy-1-[[2-(4-hydroxyphényl)phényl]méthyl]-4-oxo-1,4-azaphosphinane-3-carboxylique

L'exemple 86 est obtenu à partir de l'intermédiaire 74 selon la procédure D précédemment décrite.
RMN ¹H : (400 MHz, D2O) δ ppm 7.56 (dd, 1 H), 7.55-7.45 (2td, 2 H), 7.37 (dd, 1 H), 7.25 (d, 2 H), 7 (d, 2 H), 4.36 (dd, 2 H), 3.35/3.1 (2m, 2 H), 3.2/2.85 (2m, 2 H), 2.95 (m, 2 H), 2.05/1.65 (2m, 2 H), 1.85/1.35 (2m, 2 H), 1.6 (m, 2 H), 1.2-0.95 (m, 2 H)
ESI/FIA/HR et MS/MS : [M+H]+ = 433.1893 (433.1892)
Analyse Elémentaire : C=61.11(61.10);H=6.10(6.76);N=6.68(6.48)

### Intermédiaire 75 : 3-{4-[Bis(tert-butoxycarbonyl)amino]butyl}-4-éthoxy-4-oxo-1-[(3-phényl-1H-pyrazol-4-yl)méthyl]-1,4-azaphosphinane-3-carboxylate de tert-butyle

L'intermédiaire 75 est obtenu à partir de l'intermédiaire 21 et du 3-phényl-1H-pyrazole-4-carboxaldéhyde selon la procédure F précédemment décrite.
RMN ¹H : (400 MHz, dmso-d6) δ ppm 13-12.5 (m, 1 H), 7.9-7.3 (m, 6 H), 4.05-3.9 (m, 2 H), 3.45 (dd, 2 H), 3.3 (m, 2 H), 3.1-2.2 (m, 6 H), 2-1.7 (m, 2 H), 1.4 (s, 18 H), 1.35 (s, 9 H), 1.2 (m, 2 H), 1.2 (t, 3 H), 1-0.7 (m, 2 H)
ESI/FIA/HR et MS/MS : [M+H]+ = 691.3829 (691.3835)

### EXEMPLE 87 : Acide 3-(4-aminobutyl)-4-hydroxy-4-oxo-1-[(3-phényl-1H-pyrazol-4-yl)méthyl]-1,4-azaphosphinane-3-carboxylique

L'exemple 87 est obtenu à partir de l'intermédiaire 75 selon la procédure D précédemment décrite.
RMN ¹H : (400 MHz, D2O) δ ppm 7.95 (si, 1 H), 7.6-7.5 (m, 5 H), 4.45 (dd, 2 H), 3.55/3.15 (2m, 2 H), 3.05/2.7 (2m, 2 H), 2.9 (m, 2 H), 2.2/1.7 (m, 2 H), 1.8/1.25 (2m, 2 H), 1.5 (m, 2 H), 0.95/0.7 (2m, 2 H)
ESI/FIA/HR et MS/MS : [M+H]+ = 407.1865 (407.1848)
Analyse Elémentaire : C=55.99(56.15);H=6.40(6.70);N=13.79(13.79)

### Intermédiaire 76 : 3-{4-[Bis(tert-butoxycarbonyl)amino]butyl}-4-éthoxy-1-[[2-(4-méthoxyphényl)phényl]méthyl]-4-oxo-1,4-azaphosphinane-3-carboxylate de tert-butyle

L'intermédiaire 76 est obtenu à partir de l'intermédiaire 21 et du 2-(4-méthoxyphényl)benzaldéhyde selon la procédure F précédemment décrite.
RMN ¹H : (400 MHz, dmso-d6) δ ppm 7.47 (d, 1 H), 7.32 (m, 2 H), 7.27 (d, 2 H), 7.19 (d, 1 H), 7 (d, 2 H), 3.93 (m, 2 H), 3.8 (s, 3 H), 3.54/3.37 (2*d, 2 H), 3.32 (m, 2 H), 2.74/2.22 (m, 2 H), 2.74/2.33 (m, 2 H), 1.95-1.75 (m, 2 H), 1.95-1.75 (m, 2 H), 1.41 (s, 18 H), 1.37 (m, 2 H), 1.34 (s, 9 H), 1.18 (t, 3 H), 0.7 (m, 2 H)

### EXEMPLE 88 : Acide 3-(4-aminobutyl)-4-hydroxy-1-[[2-(4-méthoxyphényl)phényl]méthyl]-4-oxo-1,4-azaphosphinane-3-carboxylique

L'exemple 88 est obtenu à partir de l'intermédiaire 76 selon la procédure D précédemment décrite.

RMN ¹H : (400 MHz, D2O) δ ppm 7.59 (dd, 1 H), 7.51 (m, 2 H), 7.38 (dd, 1 H), 7.31 (d, 2 H), 7.1 (d, 2 H), 4.44/4.29 (2*d, 2 H), 3.36 (s, 3 H), 3.19/2.94 (m, 2 H), 3.19/2.84 (m, 2 H), 2.94 (m, 2 H), 2.08/1.65 (m, 2 H), 1.85/1.35 (m, 2 H), 1.58 (m, 2 H), 1.13/1.05 (m, 2 H)
ESI/FIA/HR et MS/MS : [M+H]+ = 447.2046 (447.2048)
Analyse Elémentaire : C=62.04(61.87);H=6.38(7.00);N=6.05(6.27)

### Intermédiaire 77 : 3-{4-[Bis(tert-butoxycarbonyl)amino]butyl}-4-éthoxy-4-oxo-1-[(4-phényl-1H-pyrazol-3-yl)méthyl]-1,4-azaphosphinane-3-carboxylate de tert-butyle

L'intermédiaire 77 est obtenu à partir de l'intermédiaire 21 et du 4-phényl-1H-pyrazole-3-carboxaldéhyde selon la procédure F précédemment décrite.
RMN ¹H : (400 MHz, dmso-d6) δ ppm 13-12.5 (m, 1 H), 8 (m, 1 H), 7.62 (d, 2 H), 7.35 (t, 2 H), 7.2 (t, 1 H), 4.1-3.9 (m, 2 H), 3.8-3.35 (m, 2 H), 3.25 (m, 2 H), 3.15-2.3 (m, 4 H), 2.1-1.9 (m, 2 H), 1.8 (m, 2 H), 1.4-1.2 (m, 2 H), 1.4 (s, 18 H), 1.35 (s, 9 H), 1.2 (t, 3 H), 0.7 (m, 2 H)

### EXEMPLE 89 : Acide 3-(4-aminobutyl)-4-hydroxy-4-oxo-1-[(4-phényl-1H-pyrazol-3-yl)méthyl]-1,4-azaphosphinane-3-carboxylique

L'exemple 89 est obtenu à partir de l'intermédiaire 77 selon la procédure D précédemment décrite.
RMN ¹H : (400 MHz, D2O) δ ppm 7.9 (s, 1 H), 7.55-7.35 (m, 5 H), 4.65-4.4 (dd, 2 H), 3.55/3.15 (2m, 2 H), 3.15/2.9 (2m, 2 H), 2.9 (m, 2 H), 2.15/1.65 (2m, 2 H), 1.8/1.25 (m, 2 H), 1.5 (m, 2 H), 0.95/0.75 (2m, 2 H)
ESI/FIA/HR et MS/MS : [M+H]+ = 407.1844 (407.1848)
Analyse Elémentaire : C=56.58(56.15);H=6.24(6.70);N=13.79(13.79)

### Intermédiaire 78 : 3-{4-[Bis(tert-butoxycarbonyl)amino]butyl}-4-éthoxy-4-oxo-1-[(5-phényl-1,3-oxazol-4-yl)méthyl]-1,4-azaphosphinane-3-carboxylate de tert-butyle

L'intermédiaire 78 est obtenu à partir de l'intermédiaire 21 et du 5-phényl-1,3-oxazole-4-carboxaldéhyde selon la procédure F précédemment décrite.
RMN ¹H : (300 MHz, dmso-d6) δ ppm 8.29 (s, 1 H), 7.78 (m, 2 H), 7.5 (m, 2 H), 7.4 (m, 1 H), 4 (m, 2 H), 3.68 (dd, 2 H), 3.3 (m, 2 H), 3.05/2.5 (2m, 2 H), 2.95/2.65 (2m, 2 H), 2.05-1.7 (m, 4 H), 1.45 (s, 18 H), 1.35 (s, 9 H), 1.25 (m, 2 H), 1.25 (t, 3 H), 0.9 (m, 2 H)

### EXEMPLE 90 : Acide 3-(4-aminobutyl)-4-hydroxy-4-oxo-1-[(5-phényl-1,3-oxazol-4-yl)méthyl]-1,4-azaphosphinane-3-carboxylique

L'exemple 90 est obtenu à partir de l'intermédiaire 78 selon la procédure D précédemment décrite.
RMN ¹H : (400 MHz, D2O) δ ppm 8.28 (s, 1 H), 7.68 (d, 2 H), 7.6-7.5 (m, 3 H), 4.55 (dd, 2 H), 3.65/3.3 (2m, 2 H), 3.4/3.1 (2dd, 2 H), 2.9 (m, 2 H), 2.2/1.71 (2m, 2 H), 1.9/1.35 (2m, 2 H), 1.55 (m, 2 H), 1.05/0.95 (2m, 2 H)
ESI/FIA/HR et MS/MS : [M+H]+ = 408.1689 (408.1688)
Analyse Elémentaire : C=55.52(56.02);H=6.16(6.43);N=10.20(10.31)

### Intermédiaire 79 : 3-{4-[Bis(tert-butoxycarbonyl)amino]butyl}-4-éthoxy-1-[[2-(1,2-oxazol-5-yl)phényl]méthyl]-4-oxo-1,4-azaphosphinane-3-carboxylate de tert-butyle

L'intermédiaire 79 est obtenu à partir des intermédiaires 21 et 218 selon la procédure F précédemment décrite.
RMN ¹H : (300 MHz, dmso-d6) δ ppm 8.6 (d, 1 H), 7.65 (m, 1 H), 7.55-7.4 (m, 3 H), 6.75 (d, 1 H), 4 (m, 2 H), 3.85/3.5 (dd, 2 H), 3.3 (m, 2 H), 2.95/2.4 (2m, 2 H), 2.8/2.55 (2m, 2 H), 2.1-1.6 (2m, 4 H), 1.45 (s, 18 H), 1.35 (m, 2 H), 1.35 (s, 9 H), 1.22 (t, 3 H), 0.9-0.5 (m, 2 H)

### EXEMPLE 91 : Acide 3-(4-aminobutyl)-4-hydroxy-1-[[2-(1,2-oxazol-5-yl)phényl]méthyl]-4-oxo-1,4-azaphosphinane-3-carboxylique

L'exemple 91 est obtenu à partir de l'intermédiaire 79 selon la procédure D précédemment décrite.
RMN ¹H : (400 MHz, D2O) δ ppm 8.55 (d, 1 H), 7.85 (m, 1 H), 7.65-7.55 (m, 3 H), 6.85 (d, 1 H), 4.7/4.4 (dd, 2 H), 3.85/3.2 (2m, 2 H), 3.5/3.2 (2dd, 2 H), 2.9 (m, 2 H), 2.3/1.8 (2m, 2 H), 1.9/1.45 (2m, 2 H), 1.6 (m, 2 H), 1.2/1.05 (2m, 2 H)
ESI/FIA/HR et MS/MS : [M+H]+ = 408.1685 (408.1688)
Analyse Elémentaire : C=55.14(56.02);H=5.95(6.43);N=10.14(10.31)

### Intermédiaire 80 : 3-{4-[Bis(tert-butoxycarbonyl)amino]butyl}-4-éthoxy-4-oxo-1-[(2-pyrazol-1-ylphényl)méthyl]-1,4-azaphosphinane-3-carboxylate de tert-butyle

L'intermédiaire 80 est obtenu à partir de l'intermédiaire 21 et du 2-(1H-pyrazol-1-yl)-benzaldéhyde selon la procédure F précédemment décrite.
RMN ¹H : (400 MHz, dmso-d6) δ ppm 8.04 (d, 1 H), 7.7 (d, 1 H), 7.55-7.35 (m, 4 H), 6.5 (m, 1 H), 3.95 (m, 2 H), 3.65/3.35 (dd, 2 H), 3.3 (m, 2 H), 2.8-2.6 (2m, 2 H), 2.4-2.15 (2m, 2 H), 2-1.65 (m, 4 H), 1.5-1.3 (m, 2 H), 1.41 (s, 18 H), 1.35 (s, 9 H), 1.19 (t, 3 H), 0.7 (m, 2 H)

### EXEMPLE 92 : Acide 3-(4-aminobutyl)-4-hydroxy-4-oxo-1-[(2-pyrazol-1-ylphényl)méthyl]-1,4-azaphosphinane-3-carboxylique

L'exemple 92 est obtenu à partir de l'intermédiaire 80 selon la procédure D précédemment décrite.
RMN ¹H : (400 MHz, D2O) δ ppm 8.05 (d, 1 H), 7.9 (d, 1 H), 7.7-7.45 (m, 4 H), 6.6 (t, 1 H), 4.25 (dd, 2 H), 3.5/3.1 (2dd, 2 H), 3.35/3.15 (2m, 2 H), 2.95 (m, 2 H), 2.3/1.8 (2m, 2 H), 1.9/1.5 (2m, 2 H), 1.6 (m, 2 H), 1.3/1.1 (2m, 2 H)
ESI/FIA/HR et MS/MS : [M+H]+ = 407.1869 (407.1848)
Analyse Elémentaire : C=56.00(56.15);H=6.16(6.70);N=13.77(13.79)

### Intermédiaire 81 : 3-{4-[Bis(tert-butoxycarbonyl)amino]butyl}-4-éthoxy-1-[[2-fluoro-6-(4-méthoxyphényl)phényl]méthyl]-4-oxo-1,4-azaphosphinane-3-carboxylate de tert-butyle

L'intermédiaire 81 est obtenu à partir de l'intermédiaire 21 et du 2-fluoro-6-(4-méthoxyphényl)benzaldéhyde selon la procédure F précédemment décrite.
RMN ¹H : (400 MHz, D2O) δ ppm 7.4 (d, 2 H), 7.4 (dd, 1 H), 7.18 (t, 1 H), 7.08 (d, 1 H), 7 (d, 2 H), 3.92 (m, 2 H), 3.8 (s, 3 H), 3.46 (s, 2 H), 3.22 (m, 2 H), 2.7/2.38 (dd, 2 H), 2.65/2.25 (2*m, 2 H), 2-1.6 (m, 4 H), 1.4 (s, 18 H), 1.34 (s, 9 H), 1.25 (m, 2 H), 1.18 (t, 3 H), 0.63/0.5 (2*m, 2 H)

### EXEMPLE 93 : Acide 3-(4-aminobutyl)-1-[[2-fluoro-6-(4-méthoxyphényl)phényl]méthyl]-4-hydroxy-4-oxo-1,4-azaphosphinane-3-carboxylique

L'exemple 93 est obtenu à partir de l'intermédiaire 81 selon la procédure D précédemment décrite.
RMN ¹H : (400 MHz, D2O) δ ppm 7.54 (m, 1 H), 7.3 (d, 2 H), 7.28 (m, 1 H), 7.21 (d, 1 H), 7.11 (d, 2 H), 4.42 (dd, 2 H), 3.86 (s, 3 H), 3.46/3.09 (m, 2 H), 3.2/2.9 (m, 2 H), 2.95 (m, 2 H), 2.11/1.67 (m, 2 H), 1.87/1.37 (m, 2 H), 1.6 (m, 2 H), 1.11 (m, 2 H)
ESI/FIA/HR et MS/MS : [M+H]+ = 465.1955 (465.1954)
Analyse Elémentaire : C=59.31(59.48);H=5.75(6.51);N=6.10(6.03)

### Intermédiaire 82 : 3-{4-[Bis(tert-butoxycarbonyl)amino]butyl}-4-éthoxy-1-[[2-(1-méthylpyrazol-4-yl)phényl]méthyl]-4-oxo-1,4-azaphosphinane-3-carboxylate de tert-butyle

L'intermédiaire 82 est obtenu à partir des intermédiaires 21 et 235 selon la procédure F précédemment décrite.
RMN ¹H : (400 MHz, dmso-d6) δ ppm 7.9 (s, 1 H), 7.65 (s, 1 H), 7.4-7.35 (m, 2 H), 7.3-7.2 (m, 2 H), 4 (m, 2 H), 3.9 (s, 3 H), 3.61/3.4 (dd, 2 H), 3.3-3.15 (m, 2 H), 2.9/2.35 (2m, 2 H), 2.8/2.45 (2dd, 2 H), 2.05-1.9 (m, 2 H), 1.8 (m, 2 H), 1.4 (s, 18 H), 1.35 (s, 9 H), 1.25 (m, 2 H), 1.2 (t, 3 H), 0.65 (m, 2 H)

### EXEMPLE 94 : Acide 3-(4-aminobutyl)-4-hydroxy-1-[[2-(1-méthylpyrazol-4-yl)phényl]méthyl]-4-oxo-1,4-azaphosphinane-3-carboxylique

L'exemple 94 est obtenu à partir de l'intermédiaire 82 selon la procédure D précédemment décrite.
RMN ¹H : (400 MHz, D2O) δ ppm 7.75 (s, 1 H), 7.6 (s, 1 H), 7.55-7.35 (m, 4 H), 4.4 (dd, 2 H), 3.9 (s, 3 H), 3.6-3.1 (m, 3 H), 3-2.8 (m, 3 H), 2.05/1.7 (m, 2 H), 1.85/1.35 (m, 2 H), 1.55 (m, 2 H), 1.1 (m, 2 H)
ESI/FIA/HR et MS/MS : [M+H]+ = 421.2016 (421.2004)
Analyse Elémentaire : C=56.61(57.13);H=6.36(6.95);N=13.08(13.33)

### Intermédiaire 83 : 3-{4-[Bis(tert-butoxycarbonyl)amino]butyl}-4-éthoxy-4-oxo-1-[(2-pyrimidin-5-ylphényl)méthyl]-1,4-azaphosphinane-3-carboxylate de tert-butyle

L'intermédiaire 83 est obtenu à partir des intermédiaires 21 et 236 selon la procédure F précédemment décrite.
RMN ¹H : (400 MHz, dmso-d6) δ ppm 9.2 (s, 1 H), 8.86 (s, 2 H), 7.5-7.3 (m, 4 H), 3.93 (m, 2 H), 3.46 (dd, 2 H), 3.3 (m, 2 H), 2.78-2.6 (2m, 2 H), 2.4 (dd, 1 H), 2.2 (m, 1 H), 2-1.75 (m, 2 H), 1.65 (m, 2 H), 1.4 (m, 2 H), 1.4 (s, 18 H), 1.33 (s, 9 H), 1.18 (t, 3 H), 0.6 (m, 2 H)

### EXEMPLE 95 : Acide 3-(4-aminobutyl)-4-hydroxy-4-oxo-1-[(2-pyrimidin-5-ylphényl)méthyl]-1,4-azaphosphinane-3-carboxylique

L'exemple 95 est obtenu à partir de l'intermédiaire 83 selon la procédure D précédemment décrite.
RMN ¹H : (400 MHz, D2O) δ ppm 9.19 (s, 1 H), 8.85 (s, 2 H), 7.7 (m, 1 H), 7.68-7.59 (m, 2 H), 7.45 (m, 1 H), 4.38 (dd, 2 H), 3.52/3.15 (2m, 2 H), 3.25/2.9 (2m, 2 H), 2.9 (m, 2 H), 2.1/1.7 (2m, 2 H), 1.85/1.4 (2m, 2 H), 1.6 (m, 2 H), 1.1 (m, 2 H)
ESI/FIA/HR et MS/MS : [M+H]+ = 419.1856 (419.1848)
Analyse Elémentaire : C=57.94(57.41);H=6.37(6.50);N=13.40(13.39)

### Intermédiaire 84 : 3-{4-[Bis(tert-butoxycarbonyl)amino]butyl}-4-éthoxy-1-[[2-(2-méthylpyrazol-3-yl)phényl]méthyl]-4-oxo-1,4-azaphosphinane-3-carboxylate de tert-butyle

L'intermédiaire 84 est obtenu à partir des intermédiaires 21 et 280 selon la procédure F précédemment décrite.
RMN ¹H : (400 MHz, dmso-d6) δ ppm 7.56 (dd, 1 H), 7.5 (d, 1 H), 7.47 (td, 1 H), 7.39 (td, 1 H), 7.28 (dd, 1 H), 6.25 (d, 1 H), 3.95 (m, 2 H), 3.58 (s, 3 H), 3.45-3.25 (m, 4 H), 2.8/2.2 (2m, 2 H), 2.7/2.35 (2dd, 2 H), 2-1.75 (m, 4 H), 1.4 (m, 2 H), 1.4 (s, 18 H), 1.35 (s, 9 H), 1.2 (t, 3 H), 0.7 (m, 2 H)

### EXEMPLE 96 : Acide 3-(4-aminobutyl)-4-hydroxy-1-[[2-(2-méthylpyrazol-3-yl)phényl]méthyl]-4-oxo-1,4-azaphosphinane-3-carboxylique

L'exemple 96 est obtenu à partir de l'intermédiaire 84 selon la procédure D précédemment décrite.
RMN ¹H : (400 MHz, D2O) δ ppm 7.7-7.4 (m, 4 H), 7.63 (d, 1 H), 6.45 (d, 1 H), 4.2 (dd, 2 H), 3.58 (s, 3 H), 3.55-3.05 (m, 4 H), 2.8 (m, 2 H), 2.1/1.7 (2m, 2 H), 1.85/1.4 (2m, 2 H), 1.6 (m, 2 H), 1.3-1 (m, 2 H)
ESI/FIA/HR et MS/MS : [M+H]+ = 421.2019 (421.2004)
Analyse Elémentaire : C=57.47(57.13);H=6.44(6.95);N=13.24(13.33)

### Intermédiaire 85 : 3-{4-[Bis(tert-butoxycarbonyl)amino]butyl}-1-[[2-(2-chlorophényl)phényl]méthyl]-4-éthoxy-4-oxo-1,4-azaphosphinane-3-carboxylate de tert-butyle

L'intermédiaire 85 est obtenu à partir des intermédiaires 21 et 214 selon la procédure F précédemment décrite.
RMN ¹H : (400 MHz, dmso-d6) δ ppm 7.56 (m, 1 H), 7.56/7.49 (d, 1 H), 7.42 (m, 2 H), 7.42 (m, 1 H), 7.34 (m, 1 H), 7.27 (m, 1 H), 7.12 (m, 1 H), 3.92 (m, 2 H), 3.5-3.15 (m, 2 H), 3.5-3.15 (m, 2 H), 2.68/2.1 (m, 2 H), 2.68/2.31 (m, 2 H), 2-1.7 (m, 2 H), 2-1.7 (m, 2 H), 1.42 (s, 18 H), 1.39 (m, 2 H), 1.34 (s, 9 H), 1.16 (2*t, 3 H), 0.73 (m, 2 H)

### EXEMPLE 97 : Acide 3-(4-aminobutyl)-1-[[2-(2-chlorophényl)phényl]méthyl]-4-hydroxy-4-oxo-1,4-azaphosphinane-3-carboxylique

L'exemple 97 est obtenu à partir de l'intermédiaire 85 selon la procédure D précédemment décrite.
RMN ¹H : (400 MHz, D2O) δ ppm 7.7-7.25 (m, 4 H), 7.7-7.25 (m, 4 H), 4.45-3.9 (m, 2 H), 3.75-3 (m, 2 H), 3.45-2.75 (m, 2 H), 2.95 (m, 2 H), 2.15/1.69 (m, 2 H), 1.87/1.38 (m, 2 H), 1.59 (m, 2 H), 1.3-1 (m, 2 H)
ESI/FIA/HR et MS/MS : [M+H]+ = 451.1545 (451.1553)
Analyse Elémentaire : C=58.81(58.60);H=6.24(6.26);N=6.36(6.21)

### Intermédiaire 86 : 3-{4-[Bis(tert-butoxycarbonyl)amino]butyl}-4-éthoxy-1-[(2-imidazol-1-ylphényl)méthyl]-4-oxo-1,4-azaphosphinane-3-carboxylate de tert-butyle

L'intermédiaire 86 est obtenu à partir de l'intermédiaire 21 et du 2-imidazol-1-ylbenzaldéhyde selon la procédure F précédemment décrite.
RMN ¹H : (400 MHz, dmso-d6) δ ppm 7.81 (t, 1 H), 7.53 (m, 1 H), 7.5-7.42 (2m, 2 H), 7.41 (t, 1 H), 7.35 (m, 1 H), 7.1 (t, 1 H), 4.05-4 (m, 2 H), 3.4 (dd, 2 H), 3.3 (m, 2 H), 2.9/2.4 (2m, 2 H), 2.68/2.6 (2dd, 2 H), 1.9-1.6 (m, 4 H), 1.45 (s, 18 H), 1.35 (m, 2 H), 1.35 (s, 9 H), 1.21 (t, 3 H), 1-0.65 (2m, 2 H)

### EXEMPLE 98 : Acide 3-(4-aminobutyl)-4-hydroxy-1-[(2-imidazol-1-ylphényl)méthyl]-4-oxo-1,4-azaphosphinane-3-carboxylique

L'exemple 98 est obtenu à partir de l'intermédiaire 86 selon la procédure D précédemment décrite.
RMN ¹H : (400 MHz, D2O) δ ppm 7.9 (si, 1 H), 7.7-7.45 (m, 4 H), 7.36 (si, 1 H), 7.25 (si, 1 H), 4.25 (dd, 2 H), 3.5-3 (m, 4 H), 2.95 (m, 2 H), 2.1/1.7 (2m, 2 H), 1.85/1.4 (2m, 2 H), 1.6 (m, 2 H), 1.15 (m, 2 H)
ESI/FIA/HR et MS/MS : [M+H]+ = 407.1852 (407.1848)
Analyse Elémentaire : C=56.41(56.15);H=5.89(6.70);N=13.55(13.79)

### Intermédiaire 87 : 3-{4-[Bis(tert-butoxycarbonyl)amino]butyl}-4-éthoxy-4-oxo-1-[(2-piperazin-1-ylphényl)méthyl]-1,4-azaphosphinane-3-carboxylate de tert-butyle

L'intermédiaire 87 est obtenu à partir de l'intermédiaire 21 et 1-Boc-4(2-formylphényl)pipérazine selon la procédure F précédemment décrite.
RMN ¹H : (400 MHz, dmso-d6) δ ppm 7.35 (dd, 1 H), 7.24 (td, 1 H), 7.11 (dd, 1 H), 7.07 (td, 1 H), 4.1-4 (m, 2 H), 3.58 (dd, 2 H), 3.46 (m, 4 H), 3.3 (m, 2 H), 2.9-2.5 (m, 8 H), 1.9 (m, 2 H), 1.75 (m, 2 H), 1.4 (s, 18 H), 1.4 (s, 9 H), 1.35 (s, 9 H), 1.35 (m, 2 H), 1.25 (t, 3 H), 0.85 (m, 2 H)

### EXEMPLE 99 : Acide 3-(4-aminobutyl)-4-hydroxy-4-oxo-1-[(2-piperazin-1-ylphényl)méthyl]-1,4-azaphosphinane-3-carboxylique

L'exemple 99 est obtenu à partir de l'intermédiaire 87 selon la procédure D précédemment décrite.
RMN ¹H : (400 MHz, D2O) δ ppm 7.6-7.25 (m, 4 H), 4.3 (dd, 2 H), 3.75-3.05 (m, 12 H), 2.9 (m, 2 H), 2.2/1.75 (2m, 2 H), 1.9/1.5 (2m, 2 H), 1.6 (m, 2 H), 1.3/1.15 (2m, 2 H)
ESI/FIA/HR et MS/MS : [M+H]+ = 425.2317 (425.231768)
Analyse Elémentaire : C=47.64(47.53);H=6.01(6.78);N=11.07(11.09);Br-=15.70(15.81)

### Intermédiaire 88 : 3-{4-[Bis(tert-butoxycarbonyl)amino]butyl}-4-éthoxy-4-oxo-1-[[2-(2-oxo-1,3-oxazolidin-3-yl)phényl]méthyl]-1,4-azaphosphinane-3-carboxylate de tert-butyle

L'intermédiaire 88 est obtenu à partir de l'intermédiaire 21 et du 2-(2-oxooxazolidin-3-yl)benzaldéhyde selon la procédure F précédemment décrite.
RMN ¹H : (400 MHz, dmso-d6) δ ppm 7.5-7.3 (m, 4 H), 4.5 (t, 2 H), 4.15-4 (m, 2 H), 4-3.85 (2m, 2 H), 3.5 (dd, 2 H), 3.35 (m, 2 H), 2.9-2.5 (m, 4 H), 2-1.7 (m, 4 H), 1.42 (s, 18 H), 1.4 (m, 2 H), 1.38 (s, 9 H), 1.25 (t, 3 H), 0.85 (m, 2 H)

### EXEMPLE 100 : Acide 3-(4-aminobutyl)-4-hydroxy-4-oxo-1-[[2-(2-oxo-1,3-oxazolidin-3-yl)phényl]méthyl]-1,4-azaphosphinane-3-carboxylique

L'exemple 100 est obtenu à partir de l'intermédiaire 88 selon la procédure D précédemment décrite.
RMN ¹H : (400 MHz, D2O) δ ppm 7.71-7.42 (m, 4 H), 4.64 (t, 2 H), 4.38 (s, 2 H), 4.12/4.03 (m, 2 H), 3.65/3.38 (dd, 2 H), 3.46/3.16 (dd, 2 H), 2.94 (t, 2 H), 2.24/1.76 (m, 2 H), 1.93/1.47 (m, 2 H), 1.6 (t, 2 H), 1.26/1.12 (m, 2 H)
ESI/FIA/HR et MS/MS : [M+H]+ = 426.1782 (426.1793)
Analyse Elémentaire : C=53.51(53.64);H=6.34(6.63);N=9.71(9.88)

### Intermédiaire 89 : 3-{4-[Bis(tert-butoxycarbonyl)amino]butyl}-4-éthoxy-1-[[2-(3-méthylimidazol-4-yl)phényl]méthyl]-4-oxo-1,4-azaphosphinane-3-carboxylate de tert-butyle

L'intermédiaire 89 est obtenu à partir de l'intermédiaire 21 et du 3-(méthylimidazol-4-yl)benzaldéhyde selon la procédure F précédemment décrite.
RMN ¹H : (400 MHz, dmso-d6) δ ppm 7.7 (s, 1 H), 7.52 (dd, 1 H), 7.4/7.35 (2t, 2 H), 7.25 (dd, 1 H), 6.86 (s, 1 H), 4.1-4 (m, 2 H), 3.45-3.25 (m, 2 H), 3.4 (s, 3 H), 3.35 (m, 2 H), 2.95/2.4 (2m, 2 H), 2.85-2.6 (2m, 2 H), 1.95-1.8 (m, 2 H), 1.75 (m, 2 H), 1.45 (s, 18 H), 1.4 (m, 2 H), 1.35 (s, 9 H), 1.25 (t, 3 H), 0.9-0.7 (2m, 2 H)

### EXEMPLE 101 : Acide 3-(4-aminobutyl)-4-hydroxy-1-[[2-(3-méthylimidazol-4-yl)phényl]méthyl]-4-oxo-1,4-azaphosphinane-3-carboxylique

L'exemple 101 est obtenu à partir de l'intermédiaire 89 selon la procédure D précédemment décrite.
RMN ¹H : (400 MHz, D2O) δ ppm 7.8 (s, 1 H), 7.65 (m, 1 H), 7.59 (m, 2 H), 7.45 (m, 1 H), 7.08 (d, 1 H), 4.24 (dl, 2 H), 3.43 (s, 3 H), 3.36/3.02 (dd, 2 H), 3.19 (m, 2 H), 2.94 (m, 2 H), 2.15/1.73 (m, 2 H), 1.9/1.44 (m, 2 H), 1.6 (m, 2 H), 1.15 (dl, 2 H)
ESI/FIA/HR et MS/MS : [M+H]+ = 421.1999 (421.2004)
Analyse Elémentaire : C=58.05(57.13);H=6.09(6.95);N=13.62(13.33)

### Intermédiaire 90 : 3-{4-[Bis(tert-butoxycarbonyl)amino]butyl}-4-éthoxy-1-[[2-(1-méthylimidazol-2-yl)phényl]méthyl]-4-oxo-1,4-azaphosphinane-3-carboxylate de tert-butyle

L'intermédiaire 90 est obtenu à partir des intermédiaires 21 et 223 selon la procédure F précédemment décrite.
RMN ¹H : (400 MHz, dmso-d6) δ ppm 7.55 (dd, 1 H), 7.45/7.35 (2t, 2 H), 7.31 (dd, 1 H), 7.25 (d, 1 H), 6.98 (d, 1 H), 4 (m, 2 H), 3.5 (dd, 2 H), 3.48 (s, 3 H), 3.4 (m, 2 H), 2.95/2.35 (2m, 2 H), 2.7-2.4 (2m, 2 H), 1.85-1.75 (m, 2 H), 1.7 (m, 2 H), 1.45 (s, 18 H), 1.4 (m, 2 H), 1.35 (s, 9 H), 1.2 (t, 3 H), 0.85 (m, 2 H)

### EXEMPLE 102 : Acide 3-(4-aminobutyl)-4-hydroxy-1-[[2-(1-méthylimidazol-2-yl)phényl]méthyl]-4-oxo-1,4-azaphosphinane-3-carboxylique

L'exemple 102 est obtenu à partir de l'intermédiaire 90 selon la procédure D précédemment décrite.
RMN ¹H : (400 MHz, D2O) δ ppm 7.7-7.5 (m, 4 H), 7.23 (d, 1 H), 7.16 (d, 1 H), 4.17 (dd, 2 H), 3.61 (s, 3 H), 3.46/3.26 (2m, 2 H), 3.37/3.09 (dd, 2 H), 2.8 (m, 2 H), 2.11/1.83 (2m, 2 H), 1.94/1.4 (2m, 2 H), 1.5 (m, 2 H), 1 (m, 2 H)
ESI/FIA/HR et MS/MS : [M+H]+ = 421.2013 (421.2004)
Analyse Elémentaire : C=57.28(57.13);H=7.10(6.95);N=13.21(13.33)

### Intermédiaire 91 : 3-{4-[Bis(tert-butoxycarbonyl)amino]butyl}-4-éthoxy-1-[[4-fluoro-2-(4-méthoxyphényl)phényl]méthyl]-4-oxo-1,4-azaphosphinane-3-carboxylate de tert-butyle

L'intermédiaire 91 est obtenu à partir des intermédiaires 21 et 221 selon la procédure F précédemment décrite.
RMN ¹H : (400 MHz, dmso-d6) δ ppm 7.48 (dd, 1 H), 7.33 (d, 2 H), 7.16 (td, 1 H), 7.02 (dd, 1 H), 7.02 (d, 2 H), 4.03 (m, 2 H), 3.8 (s, 3 H), 3.4 (AB, 2 H), 3.32 (m, 2 H), 2.66/2.51/2.41 (3m, 4 H), 1.91-1.66 (m, 4 H), 1.41 (s, 18 H), 1.37 (m, 2 H), 1.34 (s, 9 H), 1.22 (t, 3 H), 0.88/0.76 (2m, 2 H)

### EXEMPLE 103 : Acide 3-(4-aminobutyl)-1-[[4-fluoro-2-(4-méthoxyphényl)phényl]méthyl]-4-hydroxy-4-oxo-1,4-azaphosphinane-3-carboxylique

L'exemple 103 est obtenu à partir de l'intermédiaire 91 selon la procédure D précédemment décrite.
RMN ¹H : (300 MHz, D2O) δ ppm 7.62 (dd, 1 H), 7.35 (d, 2 H), 7.24 (td, 1 H), 7.17 (dd, 1 H), 7.13 (d, 2 H), 4.36 (AB, 2 H), 3.89 (s, 3 H), 3.37/3.14 (2m, 2 H), 3.14/2.85 (2m, 2 H), 2.97 (m, 2 H), 2.09/1.66 (2m, 2 H), 1.88/1.38 (2m, 2 H), 1.62 (m, 2 H), 1.09 (m, 2 H)
ESI/FIA/HR et MS/MS : [M+H]+ = 465.1965 (465.1954)
Analyse Elémentaire : C=59.91(59.48);H=6.23(6.51);N=6.25(6.03)

### EXEMPLE 104 : Acide 3-(4-aminobutyl)-1-[[4-fluoro-2-(4-hydroxyphényl)phényl]méthyl]-4-hydroxy-4-oxo-1,4-azaphosphinane-3-carboxylique

L'exemple 104 est obtenu à partir de l'intermédiaire 91 selon la procédure D précédemment décrite.
RMN ¹H : (300 MHz, D2O) δ ppm 7.62 (dd, 1 H), 7.3 (d, 2 H), 7.25 (td, 1 H), 7.18 (dd, 1 H), 7.03 (d, 2 H), 4.39 (AB, 2 H), 3.4/3.13 (2m, 2 H), 3.18/2.88 (2m, 2 H), 2.99 (m, 2 H), 2.12/1.68 (2m, 2 H), 1.89/1.38 (2m, 2 H), 1.63 (m, 2 H), 1.12 (m, 2 H)
ESI/FIA/HR et MS/MS : [M+H]+ = 451.1816 (451.1798)
Analyse Elémentaire : C=58.99(58.66);H=6.33(6.27);N=6.27(6.22)

### Intermédiaire 92 : 3-{4-[Bis(tert-butoxycarbonyl)amino]butyl}-4-éthoxy-4-oxo-1-[(2-phénylthiophen-3-yl)méthyl]-1,4-azaphosphinane-3-carboxylate de tert-butyle

L'intermédiaire 92 est obtenu à partir des intermédiaires 21 et 224 selon la procédure F précédemment décrite.
RMN ¹H : (400 MHz, dmso-d6) δ ppm 7.55-7.34 (m, 5 H), 7.55-7.34 (m, 1 H), 7.09 (d, 1 H), 4.04 (m, 2 H), 3.51 (dd, 2 H), 3.34 (m, 2 H), 2.88-2.52 (m, 4 H), 1.91 (m, 2 H), 1.74 (m, 2 H), 1.41 (s, 18 H), 1.38 (m, 2 H), 1.35 (s, 9 H), 1.23 (t, 3 H), 0.89 (m, 2 H)

### EXEMPLE 105 : Acide 3-(4-aminobutyl)-4-hydroxy-4-oxo-1-[(2-phénylthiophen-3-yl)méthyl]-1,4-azaphosphinane-3-carboxylique

L'exemple 105 est obtenu à partir de l'intermédiaire 92 selon la procédure D précédemment décrite.
RMN ¹H : (300 MHz, D2O) δ ppm 7.52 (d, 1 H), 7.4 (m, 5 H), 7.2 (d, 1 H), 4.4-4.25 (m, 2 H), 3.6-3.4 (m, 1 H), 3.2-3.1 (m, 2 H), 2.9 (m, 2 H), 2.7 (m, 1 H), 2.15 (m, 1 H), 1.85 (m, 1 H), 1.65-1.5 (m, 3 H), 1.3 (m, 1 H), 1 (m, 2 H)
ESI/FIA/HR et MS/MS : [M+H]+ = 423.1511 (423.1507)
Analyse Elémentaire : C=56.99(56.86);H=6.31(6.44);N=6.79(6.63);S=7.29(7.59)

### Intermédiaire 93 : 3-{4-[Bis(tert-butoxycarbonyl)amino]butyl}-4-éthoxy-4-oxo-1-[(4-phénylthiophen-3-yl)méthyl]-1,4-azaphosphinane-3-carboxylate de tert-butyle

L'intermédiaire 93 est obtenu à partir des intermédiaires 21 et 252 selon la procédure F précédemment décrite.
RMN ¹H : (400 MHz, dmso-d6) δ ppm 7.53 (d, 2 H), 7.5/7.47 (2d, 2 H), 7.41 (t, 2 H), 7.33 (t, 1 H), 4.04 (m, 2 H), 3.47 (AB, 2 H), 3.32 (m, 2 H), 2.92-2.4 (m, 4 H), 1.94-1.6 (m, 4 H), 1.43/1.41 (2s, 18 H), 1.34 (2s, 9 H), 1.33 (m, 2 H), 1.22 (t, 3 H), 0.8 (m, 2 H)

### EXEMPLE 106 : Acide 3-(4-aminobutyl)-4-hydroxy-4-oxo-1-[(4-phénylthiophen-3-yl)méthyl]-1,4-azaphosphinane-3-carboxylique

L'exemple 106 est obtenu à partir de l'intermédiaire 93 selon la procédure D précédemment décrite.
RMN ¹H : (300 MHz, D2O) δ ppm 7.75 (d, 1 H), 7.45 (m, 5 H), 7.42 (d, 1 H), 4.4-4.3 (2*d, 2 H), 3.45/3.15 (m, 2 H), 3.15/2.75 (m, 2 H), 2.9 (m, 2 H), 2.1 (m, 1 H), 1.8 (m, 1 H), 1.7-1.5 (m, 3 H), 1.25 (m, 1 H), 0.95 (m, 2 H)
ESI/FIA/HR et MS/MS : [M+H]+ = 423.1503 (423.1507)
Analyse Elémentaire : C=57.35(56.86);H=6.30(6.44);N=6.53(6.63);S=7.56(7.59)

### Intermédiaire 94 : 3-{4-[Bis(tert-butoxycarbonyl)amino]butyl}-4-éthoxy-4-oxo-1-(thiophen-2-ylméthyl)-1,4-azaphosphinane-3-carboxylate de tert-butyle

L'intermédiaire 94 est obtenu à partir de l'intermédiaire 21 et du 2-formylthiophène selon la procédure F précédemment décrite.
RMN ¹H : (400 MHz, dmso-d6) δ ppm 7.43 (m, 1 H), 6.97 (m, 2 H), 4.05 (m, 2 H), 3.77 (AB, 2 H), 3.41 (m, 2 H), 2.85/2.55 (2m, 2 H), 2.85/2.66 (2m, 2 H), 2-1.7 (m, 4 H), 1.43 (s, 18 H), 1.41 (m, 2 H), 1.39 (s, 9 H), 1.24 (t, 3 H), 1.01 (m, 2 H)

### EXEMPLE 107 : Acide 3-(4-aminobutyl)-4-hydroxy-4-oxo-1-(thiophen-2-ylméthyl)-1,4-azaphosphinane-3-carboxylique

L'exemple 107 est obtenu à partir de l'intermédiaire 94 selon la procédure D précédemment décrite.
RMN ¹H : (400 MHz, D2O) δ ppm 7.6 (dl, 1 H), 7.25 (dl, 1 H), 7.12 (t, 1 H), 4.6/4.5 (d, 2 H), 3.75/3.3 (m, 2 H), 3.6/3.12 (m, 2 H), 2.95 (m, 2 H), 2.25/1.8 (m, 2 H), 1.95/1.5 (m, 2 H), 1.6 (m, 2 H), 1.3-1.1 (m, 2 H)
ESI/FIA/HR et MS/MS : [M+H]+ = 347.1205 (347.1194)
Analyse Elémentaire : C=48.94(48.55);H=5.79(6.69);N=7.90(8.09);S=9.22(9.26)

### Intermédiaire 95 : 3-{4-[Bis(tert-butoxycarbonyl)amino]butyl}-4-éthoxy-4-oxo-1-[[2-(1,3-thiazol-2-yl)phényl]méthyl]-1,4-azaphosphinane-3-carboxylate de tert-butyle

L'intermédiaire 95 est obtenu à partir des intermédiaires 21 et 225 selon la procédure F précédemment décrite.
RMN ¹H : (400 MHz, dmso-d6) δ ppm 7.98 (d, 1 H), 7.85 (d, 1 H), 7.7 (d, 1 H), 4.45 (m, 3 H), 4.05 (quad., 2 H), 3.45/3.35 (d, 2 H), 3.4/2.5 (m, 2 H), 3.3 (m, 2 H), 2.7/2.5 (m, 2 H), 1.8-1.55 (m, 4 H), 1.45-1.35 (m, 2 H), 1.4/1.3 (s, 27 H), 1.2 (t, 3 H), 0.65-0.3 (m, 2 H)
ESI/FIA/HR et MS/MS : [M+H]+ = 708.3453 (708.3447)

### EXEMPLE 108 : Acide 3-(4-aminobutyl)-4-hydroxy-4-oxo-1-[[2-(1,3-thiazol-2-yl)phényl]méthyl]-1,4-azaphosphinane-3-carboxylique

L'exemple 108 est obtenu à partir de l'intermédiaire 95 selon la procédure D précédemment décrite.
RMN ¹H : (300 MHz, D2O) δ ppm 7.98 (m, 2 H), 7.85 (m, 4 H), 4.6 (d, 1 H), 4.2 (d, 1 H), 3.85 (m, 1 H), 3.45 (m, 2 H), 3.15 (dd, 1 H), 2.9 (m, 2 H), 2.3 (m, 1 H), 1.85 (m, 2 H), 1.55 (m, 3 H), 1.2 (m, 1 H), 1.0 (m, 1 H)
ESI/FIA/HR et MS/MS : [M+H]+ = 424.1443 (424.1459)
Analyse Elémentaire : C=54.17(53.89);H=5.57(6.19);N=9.91(9.92);S=7.38(7.57)

### Intermédiaire 96 : 3-{4-[Bis(tert-butoxycarbonyl)amino]butyl}-4-éthoxy-1-[(2-naphthalen-1-ylphényl)méthyl]-4-oxo-1,4-azaphosphinane-3-carboxylate de tert-butyle

L'intermédiaire 96 est obtenu à partir des intermédiaires 21 et 228 selon la procédure F précédemment décrite.
RMN ¹H : (400 MHz, dmso-d6) δ ppm 8-7.15 (m, 11 H), 4.05-3.8 (m, 2 H), 3.5-3.3 (m, 2 H), 3.3-3 (dd, 2 H), 2.7-2.1 (m, 4 H), 2-1.5 (m, 4 H), 1.5-1.3 (m, 2 H), 1.45 (s, 18 H), 1.35 (s, 9 H), 1.2 (t, 3 H), 1-0.5 (m, 2 H)

### EXEMPLE 109 : Acide 3-(4-aminobutyl)-4-hydroxy-1-[(2-naphthalen-1-ylphényl)méthyl]-4-oxo-1,4-azaphosphinane-3-carboxylique

L'exemple 109 est obtenu à partir de l'intermédiaire 96 selon la procédure D précédemment décrite.
RMN ¹H : (400 MHz, D2O) δ ppm 8 (d, 2 H), 7.75-7.3 (m, 9 H), 4.3/4.05 (2*d, 1 H), 3.95/3.8 (2*d, 1 H), 3.55-2.8 (m, 5 H), 2.65 (m, 1 H), 2.2-1.9 (m, 1 H), 1.75 (m, 1 H), 1.7-1.4 (m, 3 H), 1.35-0.7 (m, 3 H)
ESI/FIA/HR et MS/MS : [M+H]+ = 467.2109 (467.2099)
Analyse Elémentaire : C=67.08(66.94);H=6.21(6.70);N=5.75(6.00)

### Intermédiaire 97 : 3-{4-[Bis(tert-butoxycarbonyl)amino]butyl}-4-éthoxy-4-oxo-1-[(4-phénylthiophen-2-yl)méthyl]-1,4-azaphosphinane-3-carboxylate de tert-butyle

L'intermédiaire 97 est obtenu à partir de l'intermédiaire 21 et du 4-phényl-2-thiophènecarboxaldehyde selon la procédure F précédemment décrite.
RMN ¹H : (500 MHz, dmso-d6) δ ppm 7.75 (d, 1 H), 7.67 (d, 2 H), 7.41 (d, 1 H), 7.39 (t, 2 H), 7.27 (t, 1 H), 4.07 (m, 2 H), 3.8 (AB, 2 H), 3.4 (m, 2 H), 2.95/2.58 (2m, 2 H), 2.89/2.71 (2m, 2 H), 2-1.74 (m, 4 H), 1.43/1.4/1.39/1.37 (5s, 27 H), 1.4 (m, 2 H), 1.24/1.2 (2t, 3 H), 1.1/1 (2m, 2 H)

### EXEMPLE 110 : Acide 3-(4-aminobutyl)-4-hydroxy-4-oxo-1-[(4-phénylthiophen-2-yl)méthyl]-1,4-azaphosphinane-3-carboxylique

L'exemple 110 est obtenu à partir de l'intermédiaire 97 selon la procédure D précédemment décrite.
RMN ¹H : (400 MHz, D2O) δ ppm 7.75 (s, 1 H), 7.65 (d, 2 H), 7.6 (s, 1 H), 7.42 (d, 2 H), 7.3 (t, 1 H), 4.6/4.5 (2*d, 2 H), 3.8-3.35 (m, 3 H), 3.1 (m, 1 H), 2.85 (m, 2 H), 2.25/1.8 (m, 2 H), 1.95/1.45 (m, 2 H), 1.55 (m, 2 H), 1.25-1.05 (m, 2 H)
ESI/FIA/HR et MS/MS : [M+H]+ = 423.151 (423.1507)
Analyse Elémentaire : C=57.05(56.86);H=6.10(6.44);N=6.67(6.63);S=7.51(7.59)

### Intermédiaire 98 : 3-{4-[Bis(tert-butoxycarbonyl)amino]butyl}-1-[(2-bromo-4-hydroxyphényl)méthyl]-4-éthoxy-4-oxo-1,4-azaphosphinane-3-carboxylate de tert-butyle

L'intermédiaire 98 est obtenu à partir de l'intermédiaire 21 et du 2-bromo-4-méthoxybenzaldéhyde selon la procédure F précédemment décrite.
RMN ¹H : (300 MHz, dmso-d6) δ ppm 7.3 (d, 1 H), 7.2 (s, 1 H), 6.95 (dd, 1 H), 4.05 (m, 2 H), 3.75 (s, 3 H), 3.6-3.5 (d, 2 H), 3.4-3.3 (m, 2 H), 3-2.6 (m, 4 H), 2.9-1.5 (m, 4 H), 1.42/1.35 (s, 27 H), 1.4 (m, 2 H), 1.22 (t, 3 H), 0.75 (m, 2 H)
RMN ³¹P : (300 MHz, dmso-d6) δ ppm 47.5

### EXEMPLE 111 : Acide 3-(4-aminobutyl)-1-[(2-bromo-4-hydroxyphényl)méthyl]-4-hydroxy-4-oxo-1,4-azaphosphinane-3-carboxylique

L'exemple 111 est obtenu à partir de l'intermédiaire 98 selon la procédure D précédemment décrite.
RMN ¹H : (400 MHz, D2O) δ ppm 7.38 (d, 1 H), 7.2 (s, 1 H), 6.9 (dd, 1 H), 4.4-4.3 (2*d, 2 H), 3.8-3.7 (m, 1 H), 3.6-3.4 (m, 2 H), 3.25 (dd, 1 H), 2.95 (m, 2 H), 2.25 (m, 1 H), 1.95 (m, 1 H), 1.8 (m, 1 H), 1.65-1.5 (m, 3 H), 1.3 (m, 1 H), 1.15 (m, 1 H)
ESI/FIA/HR et MS/MS : [M+H]+ = 435.067 (435.0684)
Analyse Elémentaire : C=44.16(44.15);H=5.06(5.56);N=6.05(6.44)

### Intermédiaire 99 : 3-{4-[Bis(tert-butoxycarbonyl)amino]butyl}-1-[[2-(4-chlorophényl)pyridin-3-yl]méthyl]-4-éthoxy-4-oxo-1,4-azaphosphinane-3-carboxylate de tert-butyle

L'intermédiaire 99 est obtenu à partir des intermédiaires 21 et 291 selon la procédure F précédemment décrite.
RMN ¹H : (400 MHz, dmso-d6) δ ppm 8.58 (m, 1 H), 7.9/7.89 (2dd, 1 H), 7.63/7.61 (2d, 2 H), 7.53 (d, 2 H), 7.4 (dd, 1 H), 4.04/3.94 (2m, 2 H), 3.56/3.53 (2AB, 2 H), 3.32 (m, 2 H), 2.76-2.25 (m, 4 H), 1.95-1.65 (m, 4 H), 1.43/1.41 (2s, 18 H), 1.35 (m, 2 H), 1.33/1.32 (2s, 9 H), 1.23/1.18 (2t, 3 H), 0.9/0.74 (2m, 2 H)

### EXEMPLE 112 : Acide 3-(4-aminobutyl)-1-[[2-(4-chlorophényl)pyridin-3-yl]méthyl]-4-hydroxy-4-oxo-1,4-azaphosphinane-3-carboxylique

L'exemple 112 est obtenu à partir de l'intermédiaire 99 selon la procédure D précédemment décrite.
RMN ¹H : (300 MHz, D2O) δ ppm 8.61 (dd, 1 H), 8.09 (dd, 1 H), 7.56/7.43 (2d, 4 H), 7.55 (dd, 1 H), 4.42 (AB, 2 H), 3.45/3.1 (2m, 2 H), 3.1/2.87 (2m, 2 H), 2.9 (m, 2 H), 2.1/1.65 (2m, 2 H), 1.82/1.33 (2m, 2 H), 1.55 (m, 2 H), 1.03 (m, 2 H)
ESI/FIA/HR et MS/MS : [M+H]+ = 452.1508 (452.1505)
Analyse Elémentaire : C=55.90(55.82);H=6.11(6.02);N=8.95(9.30)

### Intermédiaire 100 : 3-{4-[Bis(tert-butoxycarbonyl)amino]butyl}-1-[[4-chloro-2-(4-chlorophényl)phényl]méthyl]-4-éthoxy-4-oxo-1,4-azaphosphinane-3-carboxylate de tert-butyle

L'intermédiaire 100 est obtenu à partir des intermédiaires 21 et 292 selon la procédure F précédemment décrite.
RMN ¹H : (300 MHz, dmso-d6) δ ppm 7.55-7.35 (m, 6 H), 7.25 (s, 1 H), 4.15-4 (m, 2 H), 3.5-3.3 (m, 4 H), 2.95-2.4 (m, 4 H), 1.95-1.6 (m, 6 H), 1.5-1.3 (3t, 27 H), 1.25 (t, 3 H), 1.15-0.75 (m, 2 H)

### EXEMPLE 113 : Acide 3-(4-aminobutyl)-1-[[4-chloro-2-(4-chlorophényl)phényl]méthyl]-4-hydroxy-4-oxo-1,4-azaphosphinane-3-carboxylique

L'exemple 113 est obtenu à partir de l'intermédiaire 100 selon la procédure D précédemment décrite.
RMN ¹H : (400 MHz, D2O) δ ppm 7.58 (d, 1 H), 7.54 (d, 2 H), 7.54 (dd, 1 H), 7.45 (d, 1 H), 7.33 (d, 2 H), 4.33 (dd, 2 H), 3.41/3.1 (2m, 2 H), 3.15/2.85 (dd, 2 H), 2.95 (m, 2 H), 2.1/1.65 (2m, 2 H), 1.85/1.35 (2m, 2 H), 1.6 (m, 2 H), 1.1 (m, 2 H)
ESI/FIA/HR et MS/MS : [M+H]+ = 485.1172 (485.1163)
Analyse Elémentaire : C=54.57(54.44);H=5.31(5.61);N=5.84(5.77)

### Intermédiaire 101 : 3-{4-[Bis(tert-butoxycarbonyl)amino]butyl}-4-éthoxy-4-oxo-1-[[2-(2-phénylphényl)phényl]méthyl]-1,4-azaphosphinane-3-carboxylate de tert-butyle

L'intermédiaire 101 est obtenu à partir des intermédiaires 21 et 238 selon la procédure F précédemment décrite.
RMN ¹H : (300 MHz, dmso-d6) δ ppm 7.6-7 (m, 13 H), 4.15-3.9 (m, 2 H), 3.5-3.3 (m, 2 H), 3.3-2.9 (m, 2 H), 2.75-2.2 (m, 4 H), 1.95-1.65 (m, 4 H), 1.6-1.3 (m, 2 H), 1.45 (3s, 27 H), 1.25 (t, 3 H), 1.2-0.7 (m, 2 H)

### EXEMPLE 114 : Acide 3-(4-aminobutyl)-4-hydroxy-4-oxo-1-[[2-(2-phénylphényl)phényl]méthyl]-1,4-azaphosphinane-3-carboxylique

L'exemple 114 est obtenu à partir de l'intermédiaire 101 selon la procédure D précédemment décrite.
RMN ¹H : (300 MHz, D2O) δ ppm 7.6-7.1 (m, 13 H), 3.8/3.6/3.55 (2*d, 2 H), 3.3/3 (2*m, 2 H), 3.15/2.6 (2*m, 2 H), 2.92 (m, 2 H), 2.05/1.6 (2*m, 2 H), 1.8/1.3 (2*m, 2 H), 1.6 (m, 2 H), 1.02 (m, 2 H)
ESI/FIA/HR et MS/MS : [M+H]+ = 493.2273 (493.2256)
Analyse Elémentaire : C=68.12(68.28);H=6.31(6.75);N=5.77(5.69)

### Intermédiaire 102 : 3-{4-[Bis(tert-butoxycarbonyl)amino]butyl}-4-éthoxy-4-oxo-1-[[2-[3-(trifluoromethoxy)phényl]phényl]méthyl]-1,4-azaphosphinane-3-carboxylate de tert-butyle

L'intermédiaire 102 est obtenu à partir de l'intermédiaire 21 et du 2-[3-(trifluorométhoxyphényl)]benzaldéhyde selon la procédure F précédemment décrite.
RMN ¹H : (400 MHz, dmso-d6) δ ppm 7.59 (t, 1 H), 7.42 (m, 3 H), 7.42 (m, 3 H), 7.28 (m, 1 H), 3.99 (m, 2 H), 3.54/3.32 (m, 2 H), 3.32 (m, 2 H), 3.1-2.2 (m, 2 H), 3.1-2.2 (m, 2 H), 2-1.6 (m, 2 H), 2-1.6 (m, 2 H), 1.5-1.3 (m, 27 H), 1.3 (m, 2 H), 1.21 (t, 3 H), 0.9-0.5 (m, 2 H)

### EXEMPLE 115 : Acide 3-(4-aminobutyl)-4-hydroxy-4-oxo-1-[[2-[3-(trifluoromethoxy)phényl]phényl]méthyl]-1,4-azaphosphinane-3-carboxylique

L'exemple 115 est obtenu à partir de l'intermédiaire 102 selon la procédure D précédemment décrite.
RMN ¹H : (400 MHz, dmso-d6) δ ppm 7.63 (m, 1 H), 7.59 (t, 1 H), 7.55 (m, 2 H), 7.41 (m, 1 H), 7.41 (m, 1 H), 7.34 (m, 2 H), 4.41/3.2 (2*d, 2 H), 3.43/3.1 (m, 2 H), 3.2/2.84 (m, 2 H), 2.94 (m, 2 H), 2.11/1.64 (m, 2 H), 1.86/1.36 (m, 2 H), 1.59 (m, 2 H), 1.13/1.06 (m, 2 H)
ESI/FIA/HR et MS/MS : [M+H]+ = 501.1759 (501.1766)
Analyse Elémentaire : C=54.63(55.20);H=5.24(5.64);N=5.43(5.60)

### Intermédiaire 103 : 3-{4-[Bis(tert-butoxycarbonyl)amino]butyl}-4-éthoxy-1-[(4-fluoro-2-phénylphényl)méthyl]-4-oxo-1,4-azaphosphinane-3-carboxylate de tert-butyle

L'intermédiaire 103 est obtenu à partir des intermédiaires 21 et 257 selon la procédure F précédemment décrite.
RMN ¹H : (400 MHz, dmso-d6) δ ppm 22/2.7 (2*m, 2 H), 7.52 (dd, 1 H), 7.47 (t, 2 H), 7.4 (t, 1 H), 7.37 (d, 2 H), 7.2 (dt, 1 H), 7.04 (dd, 1 H), 4-3.7 (m, 2 H), 3.52/3.32 (2*d, 2 H), 3.32 (m,
2 H), 2.7/2.32 (2*m, 2 H), 1.95-1.72 (m, 2 H), 1.95-1.72 (m, 2 H), 1.41/1.35 (2*s, 27 H), 1.38 (m, 2 H), 1.17 (t, 3 H), 0.8-0.6 (m, 2 H)

### EXEMPLE 116 : Acide 3-(4-aminobutyl)-1-[(4-fluoro-2-phénylphényl)méthyl]-4-hydroxy-4-oxo-1,4-azaphosphinane-3-carboxylique

L'exemple 116 est obtenu à partir de l'intermédiaire 103 selon la procédure D précédemment décrite.
RMN ¹H : (300 MHz, D2O) δ ppm 7.6 (dd, 1 H), 7.49 (m, 3 H), 7.34 (m, 2 H), 7.22 (td, 1 H), 7.15 (dd, 1 H), 4.31 (AB, 2 H), 3.32/3.06 (2m, 2 H), 3.16/2.8 (2m, 2 H), 2.92 (m, 2 H), 2.05/1.6 (2m, 2 H), 1.82/1.33 (2m, 2 H), 1.56 (m, 2 H), 1.07 (m, 2 H)
RMN ¹⁹F: (300 MHz, D2O) δ ppm -111.5
ESI/FIA/HR et MS/MS : [M+H]+ = 435.1854 (435.1848)
Analyse Elémentaire : C=60.79(60.82);H=6.07(6.50);N=6.19(6.45)

### Intermédiaire 104 : 3-{4-[Bis(tert-butoxycarbonyl)amino]butyl}-4-éthoxy-1-[(4-hydroxy-2-phénylphényl)méthyl]-4-oxo-1,4-azaphosphinane-3-carboxylate de tert-butyle

L'intermédiaire 104 est obtenu à partir des intermédiaires 21 et 285 selon la procédure F précédemment décrite.
RMN ¹H : (400 MHz, dmso-d6) δ ppm 7.45-7.32 (m, 5 H), 7.42 (d, 1 H), 6.95 (dd, 1 H), 6.74 (df, 1 H), 3.92 (m, 2 H), 3.77 (s, 3 H), 3.46/3.27 (2*d, 2 H), 3.34 (m, 2 H), 2.75/2.15 (2*m, 2 H), 2.68/2.28 (2*m, 2 H), 1.85-1.65 (m, 2 H), 1.85-1.65 (m, 2 H), 1.69 (m, 2 H), 1.41/1.34 (2*s, 27 H), 1.39 (m, 2 H), 1.17 (t, 3 H)

### EXEMPLE 117 : Acide 3-(4-aminobutyl)-4-hydroxy-1-[(4-hydroxy-2-phénylphényl)méthyl]-4-oxo-1,4-azaphosphinane-3-carboxylique

L'exemple 117 est obtenu à partir de l'intermédiaire 104 selon la procédure D précédemment décrite.
RMN ¹H : (400 MHz, D2O) δ ppm 7.5-7.25 (m, 5 H), 7.3 (d, 1 H), 6.92 (dd, 1 H), 6.82 (d, 1 H), 4.3/4.15 (2*d, 2 H), 3.3/3 (2*m, 2 H), 3.15/2.72 (dd, 2 H), 2.9 (m, 2 H), 2/1.55 (2*m, 2 H), 1.8/1.55 (2*m, 2 H), 1.55/1.3 (2*m, 2 H), 1.05 (m, 2 H)
ESI/FIA/HR et MS/MS : [M+H]+ = 433.1888 (433.1892)
Analyse Elémentaire : C=60.92(61.10);H=6.44(6.76);N=6.43(6.48)

### Intermédiaire 105 : 3-{4-[Bis(tert-butoxycarbonyl)amino]butyl}-4-éthoxy-1-[[2-(furan-2-yl)phényl]méthyl]-4-oxo-1,4-azaphosphinane-3-carboxylate de tert-butyle

L'intermédiaire 105 est obtenu à partir de l'intermédiaire 21 et du 2-(2-furyl)benzaldéhyde selon la procédure F précédemment décrite.
RMN ¹H : (400 MHz, dmso-d6) δ ppm 7.77 (d, 1 H), 7.62 (d, 1 H), 7.41 (d, 1 H), 7.37/7.31 (2*m, 2 H), 6.77 (d, 1 H), 6.61 (dd, 1 H), 3.96 (m, 2 H), 3.84/3.49 (2*d, 2 H), 3.3-3.15 (m, 2 H), 2.95/2.38 (2*m, 2 H), 2.77/2.45 (2*m, 2 H), 2.05-1.6 (m, 2 H), 2.05-1.6 (m, 2 H), 1.4/1.32 (2*s, 27 H), 1.3-1.15 (m, 2 H), 1.2 (t, 3 H), 0.7-0.48 (4*m, 2 H)

### EXEMPLE 118 : Acide 3-(4-aminobutyl)-1-[[2-(furan-2-yl)phényl]méthyl]-4-hydroxy-4-oxo-1,4-azaphosphinane-3-carboxylique

L'exemple 118 est obtenu à partir de l'intermédiaire 105 selon la procédure D précédemment décrite.
RMN ¹H : (400 MHz, D2O) δ ppm 7.75 (d, 1 H), 7.73 (s, 1 H), 7.52 (t, 1 H), 7.48 (d, 1 H), 7.4 (t, 1 H), 6.88 (d, 1 H), 6.63 (d, 1 H), 4.63/4.33 (2*d, 2 H), 3.82/3.5 (2*m, 2 H), 3.48/3.18 (dd, 2 H), 2.9 (m, 2 H), 2.26/1.8 (2*m, 2 H), 1.9/1.48 (2*m, 2 H), 1.6 (m, 2 H), 1.2/1.05 (2*m, 2 H)
ESI/FIA/HR et MS/MS : [M+H]+ = 407.1729 (407.1735)
Analyse Elémentaire : C=59.18(59.11);H=6.65(6.70);N=6.86(6.89)

### Intermédiaire 106 : 3-{4-[Bis(tert-butoxycarbonyl)amino]butyl}-4-éthoxy-4-oxo-1-[(2-thiophen-2-ylphényl)méthyl]-1,4-azaphosphinane-3-carboxylate de tert-butyle

L'intermédiaire 106 est obtenu à partir de l'intermédiaire 21 et du 2-(2-thiényl)benzaldéhyde selon la procédure F précédemment décrite.
RMN ¹H : (400 MHz, dmso-d6) δ ppm 7.62 (dd, 1 H), 7.46-7.33 (m, 4 H), 7.23 (dd, 1 H), 7.14 (dd, 1 H), 4.03-3.88 (m, 2 H), 3.65/3.42 (2*d, 2 H), 3.32-3.15 (m, 2 H), 3-2.7/2.4 (2*m, 2 H), 3-2.7/2.4 (2*m, 2 H), 2.05-1.75 (m, 2 H), 2.05-1.75 (m, 2 H), 1.4/1.34 (2*s, 27 H), 1.39 (m, 2 H), 1.2 (t, 3 H), 0.7-0.45 (m, 2 H)

### EXEMPLE 119 : Acide 3-(4-aminobutyl)-4-hydroxy-4-oxo-1-[(2-thiophen-2-ylphényl)méthyl]-1,4-azaphosphinane-3-carboxylique

L'exemple 119 est obtenu à partir de l'intermédiaire 106 selon la procédure D précédemment décrite.
RMN ¹H : (400 MHz, D2O) δ ppm 7.6 (m, 1 H), 7.57 (d, 1 H), 7.52 (m, 2 H), 7.52 (m, 1 H), 7.2 (d, 1 H), 7.15 (d, 1 H), 4.52/4.42 (2*d, 2 H), 3.48/3.22 (2*m, 2 H), 3.3/2.98 (dd, 2 H), 2.93 (m, 2 H), 2.13/1.68 (2*m, 2 H), 1.88/1.4 (2*m, 2 H), 1.6 (m, 2 H), 1.2/1.05 (2*m, 2 H) ESI/FIA/HR et MS/MS : [M+H]+ = 423.1493 (423.1507)
Analyse Elémentaire : C=56.66(56.86);H=6.32(6.44);N=6.64(6.63);S=7.48(7.59)

### Intermédiaire 107 : 3-{4-[Bis(tert-butoxycarbonyl)amino]butyl}-4-éthoxy-4-oxo-1-[(2-pyridin-4-ylphényl)méthyl]-1,4-azaphosphinane-3-carboxylate de tert-butyle

L'intermédiaire 107 est obtenu à partir des intermédiaires 21 et 258 selon la procédure F précédemment décrite.
RMN ¹H : (400 MHz, dmso-d6) δ ppm 8.62 (d, 2 H), 7.5 (dd, 1 H), 7.46-7.37 (m, 2 H), 7.4 (d, 2 H), 7.26 (dd, 1 H), 3.92 (m, 2 H), 3.58/3.38 (2*d, 2 H), 3.3 (m, 2 H), 2.82/2.22 (2*m, 2 H), 2.68/2.37 (2*m, 2 H), 1.85-1.65 (m, 2 H), 1.85-1.65 (m, 2 H), 1.41/1.33 (2*s, 27 H), 1.38 (m, 2 H), 1.18 (t, 3 H), 0.63 (m, 2 H)
RMN ¹³C : (400 MHz, dmso-d6) δ ppm 107, 67, 29/23

### EXEMPLE 120 : Acide 3-(4-aminobutyl)-4-hydroxy-4-oxo-1-[(2-pyridin-4-ylphényl)méthyl]-1,4-azaphosphinane-3-carboxylique

L'exemple 120 est obtenu à partir de l'intermédiaire 107 selon la procédure D précédemment décrite.
RMN ¹H : (400 MHz, D2O) δ ppm 8.59 (d, 2 H), 7.62 (m, 1 H), 7.55 (m, 2 H), 7.41 (d, 2 H), 7.41 (m, 1 H), 4.4/4.29 (2*d, 2 H), 3.42/3.09 (2*m, 2 H), 3.18/2.85 (2*m, 2 H), 2.91 (m, 2 H), 2.08/1.65 (2*m, 2 H), 1.82/1.55 (2*m, 2 H), 1.55/1.32 (2*m, 2 H), 1.05 (m, 2 H)
ESI/FIA/HR et MS/MS : [M+H]+ = 418.1891 (418.1895)
Analyse Elémentaire : C=59.87(60.42);H=6.51(6.76);N=9.86(10.07)

### Intermédiaire 108 : 3-{4-[Bis(tert-butoxycarbonyl)amino]butyl}-4-éthoxy-4-oxo-1-[(2-pyridin-3-ylphényl)méthyl]-1,4-azaphosphinane-3-carboxylate de tert-butyle

L'intermédiaire 108 est obtenu à partir de l'intermédiaire 21 et du 2-(3-pyridyl)benzaldéhyde selon la procédure F précédemment décrite.
RMN ¹H : (400 MHz, dmso-d6) δ ppm 8.58 (dd, 1 H), 8.56 (d, 1 H), 7.81 (dt, 1 H), 7.48 (m, 1 H), 7.48 (m, 1 H), 7.41 (m, 2 H), 7.27 (d, 1 H), 3.93 (m, 2 H), 3.55/3.35 (2*d, 2 H), 3.29 (m, 2 H), 2.71/2.2 (m, 2 H), 2.71/2.35 (m, 2 H), 1.95-1.7 (m, 2 H), 1.95-1.7 (m, 2 H), 1.41 (s, 18 H), 1.36 (m, 2 H), 1.34 (s, 9 H), 1.18 (t, 3 H), 0.64 (m, 2 H)

### EXEMPLE 121 : Acide 3-(4-aminobutyl)-4-hydroxy-4-oxo-1-[(2-pyridin-3-ylphényl)méthyl]-1,4-azaphosphinane-3-carboxylique

L'exemple 121 est obtenu à partir de l'intermédiaire 108 selon la procédure D précédemment décrite.
RMN ¹H : (400 MHz, D2O) δ ppm 8.58 (dd, 1 H), 8.51 (d, 1 H), 7.86 (dt, 1 H), 7.67 (m, 1 H), 7.58 (m, 1 H), 7.58 (m, 2 H), 7.43 (m, 1 H), 4.42/4.3 (2*d, 2 H), 3.46/3.11 (m, 2 H), 3.2/2.87 (m, 2 H), 2.94 (m, 2 H), 2.11/1.65 (m, 2 H), 1.86/1.36 (m, 2 H), 1.59 (m, 2 H), 1.08 (m, 2 H)
ESI/FIA/HR et MS/MS : [M+H]+ = 418.1898 (418.1895)
Analyse Elémentaire : C=60.58(60.42);H=6.51(6.76);N=10.09(10.07)

### Intermédiaire 109 : 3-{4-[Bis(tert-butoxycarbonyl)amino]butyl}-4-éthoxy-4-oxo-1-[[2-[4-(trifluorométhyl)phényl]phényl]méthyl]-1,4-azaphosphinane-3-carboxylate de tert-butyle

L'intermédiaire 109 est obtenu à partir de l'intermédiaire 21 et du 2-[4-(trifluorométhyl)phényl]benzaldéhyde selon la procédure F précédemment décrite.
RMN ¹H : (400 MHz, dmso-d6) δ ppm 7.81 (d, 2 H), 7.62 (d, 2 H), 7.5 (d, 1 H), 7.41 (m, 2 H), 7.27 (d, 1 H), 3.93 (m, 2 H), 3.58/3.34 (2*d, 2 H), 3.28 (m, 2 H), 2.74/2.36 (m, 2 H), 2.74/2.24 (m, 2 H), 2-1.7 (m, 2 H), 2-1.7 (m, 2 H), 1.4 (s, 18 H), 1.36 (m, 2 H), 1.34 (s, 9 H), 1.17 (t, 3 H), 0.65 (m, 2 H)

### EXEMPLE 122 : Acide 3-(4-aminobutyl)-4-hydroxy-4-oxo-1-[[2-[4-(trifluorométhyl)phényl]phényl]méthyl]-1,4-azaphosphinane-3-carboxylique

L'exemple 122 est obtenu à partir de l'intermédiaire 109 selon la procédure D précédemment décrite.
RMN ¹H : (400 MHz, D2O) δ ppm 7.83 (d, 2 H), 7.64 (m, 1 H), 7.56 (m, 2 H), 7.53 (d, 2 H), 7.43 (m, 1 H), 4.42/4.28 (2*d, 2 H), 3.42/3.09 (m, 2 H), 3.18/2.84 (m, 2 H), 2.94 (m, 2 H), 2.1/1.64 (m, 2 H), 1.85/1.35 (m, 2 H), 1.59 (m, 2 H), 1.13/1.05 (m, 2 H)
ESI/FIA/HR et MS/MS : [M+H]+ = 485.1800 (485.1817)
Analyse Elémentaire : C=56.24(57.02);H=5.26(5.83);N=5.65(5.78)

### Intermédiaire 110 : 3-{4-[Bis(tert-butoxycarbonyl)amino]butyl}-1-[(2-cyclohexylphényl)méthyl]-4-éthoxy-4-oxo-1,4-azaphosphinane-3-carboxylate de tert-butyle

L'intermédiaire 110 est obtenu à partir de l'intermédiaire 21 et du 2-cyclohéxylbenzaldéhyde selon la procédure F précédemment décrite.
RMN ¹H : (400 MHz, dmso-d6) δ ppm 7.28/7.17 (2*d, 2 H), 7.22/7.1 (2*m, 2 H), 3.98 (m, 2 H), 3.5 (s, 2 H), 3.31 (m, 2 H), 3-2.8/2.28 (2*m, 2 H), 3-2.8/2.5 (2*m, 2 H), 3-2.8 (m, 1 H), 2-1.78 (2*m, 2 H), 1.9-1.65 (m, 2 H), 1.9-1.65 (m, 6 H), 1.45-1.25 (m, 2 H), 1.45-1.25 (m, 4 H), 1.41/1.37 (2*s, 27 H), 1.21 (t, 3 H), 0.85-0.65 (m, 2 H)

### EXEMPLE 123 : Acide 3-(4-aminobutyl)-1-[(2-cyclohexylphényl)méthyl]-4-hydroxy-4-oxo-1,4-azaphosphinane-3-carboxylique

L'exemple 123 est obtenu à partir de l'intermédiaire 110 selon la procédure D précédemment décrite.
RMN ¹H : (400 MHz, D2O) δ ppm 7.52-7.4 (m, 2 H), 7.52-7.4/7.3 (2*m, 2 H), 4.4 (t, 2 H), 3.6/3.4 (2*m, 2 H), 3.49/3.24 (2*m, 2 H), 2.93 (m, 2 H), 2.66 (m, 1 H), 2.21/1.75 (2*m, 2 H), 1.95/1.5 (2*m, 2 H), 1.85-1.1 (m, 10 H), 1.85-1.1 (m, 4 H)
ESI/FIA/HR et MS/MS : [M+H]+ = 423.2408 (423.2412)
Analyse Elémentaire : C=62.46(62.54);H=8.23(8.35);N=6.61(6.63)

### Intermédiaire 111 : 3-{4-[Bis(tert-butoxycarbonyl)amino]butyl}-4-éthoxy-1-(1H-indazol-5-ylméthyl)-4-oxo-1,4-azaphosphinane-3-carboxylate de tert-butyle

L'intermédiaire 111 est obtenu à partir de l'intermédiaire 21 et du 1H-indazole-5-carbaldéhyde selon la procédure F précédemment décrite.
RMN ¹H : (400 MHz, dmso-d6) δ ppm 13 (si, 1 H), 8 (s, 1 H), 7.62 (df, 1 H), 7.48 (d, 1 H), 7.3 (dd, 1 H), 3.97 (m, 2 H), 3.72/3.52 (2*d, 2 H), 3.4-3.2 (m, 2 H), 2.96/2.32 (2*m, 2 H), 2.83/2.47 (2*m, 2 H), 2-1.8 (m, 2 H), 2-1.8 (m, 2 H), 1.39/1.33 (2*s, 27 H), 1.36 (m, 2 H), 1.2 (t, 3 H), 0.9-0.7 (m, 2 H)

### EXEMPLE 124 : Acide 3-(4-aminobutyl)-4-hydroxy-1-(1H-indazol-5-ylméthyl)-4-oxo-1,4-azaphosphinane-3-carboxylique

L'exemple 124 est obtenu à partir de l'intermédiaire 111 selon la procédure D précédemment décrite.
RMN ¹H : (400 MHz, D2O) δ ppm 8.16 (s, 1 H), 7.91 (s, 1 H), 7.68 (d, 1 H), 7.49 (d, 1 H), 4.51/4.32 (2*d, 2 H), 3.64/3.35 (2*m, 2 H), 3.48/3.11 (2*m, 2 H), 2.89 (m, 2 H), 2.25/1.77 (2*m, 2 H), 1.91/1.55 (2*m, 2 H), 1.55/1.45 (2*m, 2 H), 1.19/1.05 (2*m, 2 H)
ESI/FIA/HR et MS/MS : [M+H]+ = 381.1697 (381.1691)
Analyse Elémentaire : C=53.27(53.68);H=6.55(6.62);N=14.52(14.73)

### Intermédiaire 112 : 3-{4-[Bis(tert-butoxycarbonyl)amino]butyl}-4-éthoxy-4-oxo-1-[(2-thiophen-3-ylphényl)méthyl]-1,4-azaphosphinane-3-carboxylate de tert-butyle

L'intermédiaire 112 est obtenu à partir des intermédiaires 21 et 259 selon la procédure F précédemment décrite.
RMN ¹H : (400 MHz, dmso-d6) δ ppm 7.61 (dd, 1 H), 7.59 (dd, 1 H), 7.44/7.32 (2*m, 4 H), 7.25 (dd, 1 H), 3.95 (m, 2 H), 3.6/3.4 (2*d, 2 H), 3.3 (m, 2 H), 2.8/2.29 (2*m, 2 H), 2.8/2.4 (2*m, 2 H), 2-1.79 (m, 2 H), 2-1.79 (m, 2 H), 1.7 (m, 2 H), 1.4/1.34 (2*s, 27 H), 1.35 (m, 2 H), 1.19 (t, 3 H)

### EXEMPLE 125 : Acide 3-(4-aminobutyl)-4-hydroxy-4-oxo-1-[(2-thiophen-3-ylphényl)méthyl]-1,4-azaphosphinane-3-carboxylique

L'exemple 125 est obtenu à partir de l'intermédiaire 112 selon la procédure D précédemment décrite.
RMN ¹H : (400 MHz, D2O) δ ppm 7.63-7.43 (m, 2 H), 7.63-7.43 (m, 4 H), 7.19 (dd, 1 H), 4.49/4.34 (2*d, 2 H), 3.39/3.17 (2*m, 2 H), 3.25/2.94 (2*m, 2 H), 2.94 (m, 2 H), 2.1/1.68 (2*m, 2 H), 1.87/1.59 (2*m, 2 H), 1.59/1.39 (2*m, 2 H), 1.18/1.05 (2*m, 2 H)
ESI/FIA/HR et MS/MS : [M+H]+ = 423.1504 (423.1507)
Analyse Elémentaire : C=57.17(56.86);H=6.48(6.44);N=6.67(6.63);S=7.11(7.59)

### Intermédiaire 113 : 3-{4-[Bis(tert-butoxycarbonyl)amino]butyl}-4-éthoxy-1-[[2-(3-fluorophényl)phényl]méthyl]-4-oxo-1,4-azaphosphinane-3-carboxylate de tert-butyle

L'intermédiaire 113 est obtenu à partir de l'intermédiaire 21 et du 2-(3-fluorophényl)benzaldéhyde selon la procédure F précédemment décrite.
RMN ¹H : (400 MHz, dmso-d6) δ ppm 7.48 (m, 1 H), 7.48 (m, 1 H), 7.38 (m, 2 H), 7.23 (m, 3 H), 7.23 (m, 1 H), 3.93 (m, 2 H), 3.56/3.36 (2*d, 2 H), 3.29 (m, 2 H), 2.73/2.36 (m, 2 H), 2.73/2.23 (m, 2 H), 2-1.7 (m, 2 H), 2-1.7 (m, 2 H), 1.41 (s, 18 H), 1.36 (m, 2 H), 1.33 (s, 9 H), 1.17 (t, 3 H), 0.66 (m, 2 H)

### EXEMPLE 126 : Acide 3-(4-aminobutyl)-1-[[2-(3-fluorophényl)phényl]méthyl]-4-hydroxy-4-oxo-1,4-azaphosphinane-3-carboxylique

L'exemple 126 est obtenu à partir de l'intermédiaire 113 selon la procédure D précédemment décrite.
RMN ¹H : (400 MHz, D2O) δ ppm 7.62 (m, 1 H), 7.54 (m, 2 H), 7.5 (m, 1 H), 7.41 (m, 1 H), 7.21 (m, 1 H), 7.16 (m, 1 H), 7.14 (m, 1 H), 4.44/4.31 (2*d, 2 H), 3.43/3.11 (m, 2 H), 3.21/2.86 (m, 2 H), 2.94 (m, 2 H), 2.11/1.64 (m, 2 H), 1.86/1.36 (m, 2 H), 1.59 (m, 2 H), 1.09 (m, 2 H)
ESI/FIA/HR et MS/MS : [M+H]+ = 435.1841 (435.1848)
Analyse Elémentaire : C=61.58(60.82);H=5.84(6.50);N=6.51(6.45)

### Intermédiaire 114 : 3-{4-[Bis(tert-butoxycarbonyl)amino]butyl}-4-éthoxy-4-oxo-1-[(5-phényl-1,2-oxazol-4-yl)méthyl]-1,4-azaphosphinane-3-carboxylate de tert-butyle

L'intermédiaire 114 est obtenu à partir des intermédiaires 21 et 219 selon la procédure F précédemment décrite.
RMN ¹H : (400 MHz, dmso-d6) δ ppm 8.6 (s, 1 H), 7.9 (m, 2 H), 7.55 (m, 3 H), 4 (m, 2 H), 3.6 (dd, 2 H), 3.3 (m, 2 H), 3/2.35 (2m, 2 H), 2.8/2.5 (2dd, 2 H), 2 (m, 2 H), 1.8 (m, 2 H), 1.4 (s, 18 H), 1.35 (s, 9 H), 1.35 (m, 2 H), 1.2 (t, 3 H), 0.8 (m, 2 H)

### EXEMPLE 127 : Acide 3-(4-aminobutyl)-4-hydroxy-4-oxo-1-[(5-phényl-1,2-oxazol-4-yl)méthyl]-1,4-azaphosphinane-3-carboxylique

L'exemple 127 est obtenu à partir de l'intermédiaire 114 selon la procédure D précédemment décrite.
RMN ¹H : (400 MHz, D2O) δ ppm 8.66 (s, 1 H), 7.75-7.55 (m, 5 H), 4.5 (dd, 2 H), 3.6/3.28 (2m, 2 H), 3.2/2.9 (dd, 2 H), 2.85 (m, 2 H), 2.2/1.7 (2m, 2 H), 1.85/1.25 (2m, 2 H), 1.5 (m, 2 H), 0.9/0.7 (2m, 2 H)
ESI/FIA/HR et MS/MS : [M+H]+ = 408.1661 (408.1688)

### Intermédiaire 115 : 3-{4-[Bis(tert-butoxycarbonyl)amino]butyl}-1-[(5-bromo-2-phénylphényl)méthyl]-4-éthoxy-4-oxo-1,4-azaphosphinane-3-carboxylate de tert-butyle

L'intermédiaire 115 est obtenu à partir de l'intermédiaire 21 et du 5-bromo-2-phénylbenzaldéhyde selon la procédure F précédemment décrite.
RMN ¹H : (400 MHz, dmso-d6) δ ppm 7.71 (df, 1 H), 7.53 (dd, 1 H), 7.45 (t, 2 H), 7.4 (dd, 1 H), 7.3 (d, 2 H), 7.16 (t, 1 H), 3.92 (m, 2 H), 3.58/3.35 (2*d, 2 H), 3.4 (m, 2 H), 2.74/2.22 (2*m, 2 H), 2.67/2.34 (2*m, 2 H), 2-1.7 (m, 2 H), 2-1.7 (m, 2 H), 1.42 (m, 2 H), 1.4/1.35 (2*s, 27 H), 1.17 (t, 3 H), 0.78 (m, 2 H)

### EXEMPLE 128 : Acide 3-(4-aminobutyl)-1-[(5-bromo-2-phénylphényl)méthyl]-4-hydroxy-4-oxo-1,4-azaphosphinane-3-carboxylique

L'exemple 128 est obtenu à partir de l'intermédiaire 115 selon la procédure D précédemment décrite.
RMN ¹H : (400 MHz, D2O) δ ppm 7.81 (d, 1 H), 7.7 (dd, 1 H), 7.52 (t, 2 H), 7.49 (t, 1 H), 7.37 (d, 2 H), 7.31 (d, 1 H), 4.4/4.28 (2*d, 2 H), 3.32/2.88 (2*m, 2 H), 3.2/3.1 (2*m, 2 H), 2.95 (m, 2 H), 2.08/1.67 (2*m, 2 H), 1.88/1.6 (2*m, 2 H), 1.6/1.37 (2*m, 2 H), 1.17/1.08 (2*m, 2 H)
ESI/FIA/HR et MS/MS : [M+H]+ = 495.1041 (495.1048)
Analyse Elémentaire : C=53.71(53.34);H=5.72(5.70);N=5.89(5.66)

### Intermédiaire 116 : 3-{4-[Bis(tert-butoxtcarbonyl)amino]butyl}-4-éthoxy-4-oxo-1-[[2-[4-(trifluoromethoxy)phényl]phényl]méthyl]-1,4-azaphosphinane-3-carboxylate de tert-butyle

L'intermédiaire 116 est obtenu à partir des intermédiaires 21 et 286 selon la procédure F précédemment décrite.
RMN ¹H : (400 MHz, dmso-d6) δ ppm 7.65-7.33 (m, 4 H), 7.65-7.33 (m, 3 H), 7.25 (dd, 1 H), 3.94 (m, 2 H), 3.57/3.3 (2*d, 2 H), 3.3 (m, 2 H), 2.72/2.23 (2*m, 2 H), 2.72/2.37 (2*m, 2 H), 2-1.7 (m, 2 H), 2-1.7 (m, 2 H), 1.4/1.34 (2*s, 24 H), 1.35 (m, 2 H), 1.17 (t, 3 H), 0.65 (m, 2 H)

### EXEMPLE 129 : Acide 3-(4-aminobutyl)-4-hydroxy-4-oxo-1-[[2-[4-(trifluoromethoxy)phényl]phényl]méthyl]-1,4-azaphosphinane-3-carboxylique

L'exemple 129 est obtenu à partir de l'intermédiaire 116 selon la procédure D précédemment décrite.
RMN ¹H : (400 MHz, D2O) δ ppm 7.62 (m, 1 H), 7.54 (m, 2 H), 7.44 (s, 4 H), 7.4 (m, 1 H), 4.42/4.28 (2*d, 2 H), 3.4/3.12 (2*m, 2 H), 3.2/2.88 (2*m, 2 H), 2.94 (m, 2 H), 2.1/1.64 (2*m, 2 H), 1.86/1.59 (2*m, 2 H), 1.59/1.38 (2*m, 2 H), 1.15/1.05 (2*m, 2 H)
ESI/FIA/HR et MS/MS : [M+H]+ = 501.1773 (501.1766)
Analyse Elémentaire : C=55.71(55.20);H=5.27(5.64);N=5.62(5.60)

### Intermédiaire 117 : 3-{4-[Bis(tert-butoxycarbonyl)amino]butyl}-4-éthoxy-1-[[4-fluoro-2-(4-fluorophényl)phényl]méthyl]-4-oxo-1,4-azaphosphinane-3-carboxylate de tert-butyle

L'intermédiaire 117 est obtenu à partir des intermédiaires 21 et 271 selon la procédure F précédemment décrite.
RMN ¹H : (400 MHz, dmso-d6) δ ppm 7.49 (dd, 1 H), 7.42 (dd, 2 H), 7.29 (t, 2 H), 7.21 (dt, 1 H), 7.06 (dd, 1 H), 3.93 (m, 2 H), 3.49/3.3 (2*d, 2 H), 3.3 (m, 2 H), 2.72/2.21 (2*m, 2 H), 2.69/2.34 (2*m, 2 H), 1.95-1.7 (massif, 2 H), 1.95-1.7 (massif, 2 H), 1.68 (m, 2 H), 1.41/1.33 (2*s, 27 H), 1.38 (m, 2 H), 1.18 (t, 3 H)

### EXEMPLE 130 : Acide 3-(4-aminobutyl)-1-[[4-fluoro-2-(4-fluorophényl)phényl]méthyl]-4-hydroxy-4-oxo-1,4-azaphosphinane-3-carboxylique

L'exemple 130 est obtenu à partir de l'intermédiaire 117 selon la procédure D précédemment décrite.
RMN ¹H : (400 MHz, D2O) δ ppm 7.66 (dd, 1 H), 7.38 (dd, 2 H), 7.27 (t, 2 H), 7.25 (df, 1 H), 7.18 (dd, 1 H), 4.39/4.28 (2*d, 2 H), 3.38/3.09 (2*m, 2 H), 3.18/2.87 (2*m, 2 H), 2.95 (m, 2 H), 2.09/1.68 (2*m, 2 H), 1.86/1.6 (2*m, 2 H), 1.6/1.37 (2*m, 2 H), 1.15/1.07 (2*m, 2 H)
ESI/FIA/HR et MS/MS : [M+H]+ = 453.1741 (453.1754)
Analyse Elémentaire : C=58.67(58.40);H=5.99(6.01);N=6.46(6.19)

### Intermédiaire 118 : 3-{4-[Bis(tert-butoxycarbonyl)amino]butyl}-1-[[2-[4-chloro-3-(trifluorométhyl)phényl]phényl]méthyl]-4-éthoxy-4-oxo-1,4-azaphosphinane-3-carboxylate de tert-butyle

L'intermédiaire 118 est obtenu à partir des intermédiaires 21 et 278 selon la procédure F précédemment décrite.
RMN ¹H : (400 MHz, dmso-d6) δ ppm 7.9 (d, 1 H), 7.81 (d, 1 H), 7.75 (dd, 1 H), 7.42/7.32 (2m, 3 H), 3.95 (m, 2 H), 3.41 (AB, 2 H), 3.2 (m, 2 H), 2.79/2.37 (2m, 2 H), 2.69/2.27 (2m, 2 H), 1.95/1.7 (2m, 2 H), 1.8/1.7 (2m, 2 H), 1.39 (s, 18 H), 1.33 (s, 9 H), 1.27 (m, 2 H), 1.19 (t, 3 H), 0.61/0.48 (2m, 2 H)

### EXEMPLE 131 : Acide 3-(4-aminobutyl)-1-[[2-[4-chloro-3-(trifluorométhyl)phényl]phényl]méthyl]-4-hydroxy-4-oxo-1,4-azaphosphinane-3-carboxylique

L'exemple 131 est obtenu à partir de l'intermédiaire 118 selon la procédure D précédemment décrite.
RMN ¹H : (300 MHz, D2O) δ ppm 7.76 (si, 1 H), 7.71 (d, 1 H), 7.63 (m, 1 H), 7.54 (dl, 1 H), 7.54 (m, 2 H), 7.38 (m, 1 H), 4.31 (AB, 2 H), 3.46/3.08 (2m, 2 H), 3.13/2.83 (2m, 2 H), 2.92 (m, 2 H), 2.11/1.64 (2m, 2 H), 1.83/1.34 (2m, 2 H), 1.57 (m, 2 H), 1.06 (m, 2 H)
RMN ¹⁹F: (300 MHz, D2O) δ ppm -62.3
ESI/FIA/HR et MS/MS : [M+H]+ = 519.1442 (519.1427)
Analyse Elémentaire : C=53.40(53.24);H=4.76(5.24);N=5.43(5.40)

### Intermédiaire 119 : 3-{4-[Bis(tert-butoxycarbonyl)amino]butyl}-1-[[2-(3-chlorophényl)phényl]méthyl]-4-éthoxy-4-oxo-1,4-azaphosphinane-3-carboxylate de tert-butyle

L'intermédiaire 119 est obtenu à partir des intermédiaires 21 et 213 selon la procédure F précédemment décrite.
RMN ¹H : (400 MHz, dmso-d6) δ ppm 7.52 (t, 1 H), 7.45 (m, 3 H), 7.4/7.37 (2td, 2 H), 7.33 (dt, 1 H), 7.25 (dd, 1 H), 3.94 (m, 2 H), 3.53/3.32 (AB, 2 H), 3.25 (m, 2 H), 2.76/2.37 (2m, 2 H), 2.7/2.25 (2m, 2 H), 1.95/1.78 (2m, 2 H), 1.82/1.75 (2m, 2 H), 1.41 (s, 18 H), 1.33 (s, 9 H), 1.33 (m, 2 H), 1.18 (t, 3 H), 0.66/0.57 (2m, 2 H)

### EXEMPLE 132 : Acide 3-(4-aminobutyl)-1-[[2-(3-chlorophényl)phényl]méthyl]-4-hydroxy-4-oxo-1,4-azaphosphinane-3-carboxylique

L'exemple 132 est obtenu à partir de l'intermédiaire 119 selon la procédure D précédemment décrite.
RMN ¹H : (300 MHz, D2O) δ ppm 7.6 (m, 1 H), 7.52 (m, 2 H), 7.5-7.35 (m, 4 H), 7.26 (m, 1 H), 4.33 (AB, 2 H), 3.41/3.09 (2m, 2 H), 3.16/2.81 (2m, 2 H), 2.92 (m, 2 H), 2.09/1.64 (2m, 2 H), 1.83/1.34 (2m, 2 H), 1.56 (m, 2 H), 1.05 (m, 2 H)
ESI/FIA/HR et MS/MS : [M+H]+ = 451.1556 (451.1553)
Analyse Elémentaire : C=58.91(58.60);H=5.83(6.26);N=6.32(6.21)

### Intermédiaire 120 : 3-{4-[Bis(tert-butoxycarbonyl)amino]butyl}-4-éthoxy-1-[[2-(4-méthylphényl)phényl]méthyl]-4-oxo-1,4-azaphosphinane-3-carboxylate de tert-butyle

L'intermédiaire 120 est obtenu à partir des intermédiaires 21 et 288 selon la procédure F précédemment décrite.
RMN ¹H : (400 MHz, dmso-d6) δ ppm 7.49 (dd, 1 H), 7.33 (2*m, 2 H), 7.23 (2*d, 4 H), 7.18 (dd, 1 H), 3.92 (m, 2 H), 3.54/3.35 (2*d, 2 H), 3.3 (m, 2 H), 2.74/2.2 (2*m, 2 H), 2.74/2.32 (2*m, 2 H), 2.36 (s, 3 H), 1.95-1.75 (m, 2 H), 1.95-1.75 (m, 2 H), 1.41/1.33 (2*s, 27 H), 1.38 (m, 2 H), 1.17 (t, 3 H), 0.71 (m, 2 H)

### EXEMPLE 133 : Acide 3-(4-aminobutyl)-4-hydroxy-1-[[2-(4-méthylphényl)phényl]méthyl]-4-oxo-1,4-azaphosphinane-3-carboxylique

L'exemple 133 est obtenu à partir de l'intermédiaire 120 selon la procédure D précédemment décrite.
RMN ¹H : (400 MHz, D2O) δ ppm 7.6 (dd, 1 H), 7.52/7.5 (2*m, 2 H), 7.39 (dd, 1 H), 7.35 (d, 2 H), 7.25 (d, 2 H), 4.43/4.28 (2*d, 2 H), 3.36/3.1 (2*dd, 2 H), 3.22/2.83 (2*dd, 2 H), 2.94 (m, 2 H), 2.38 (s, 3 H), 2.09/1.64 (2*m, 2 H), 1.86/1.6 (2*m, 2 H), 1.6/1.35 (2*m, 2 H), 1.15/1.05 (2*m, 2 H)
ESI/FIA/HR et MS/MS : [M+H]+ = 431.2103 (431.2099)
Analyse Elémentaire : C=64.12(64.17);H=7.45(7.26);N=6.50(6.51)

### Intermédiaire 121 : 3-{4-[Bis(tert-butoxycarbonyl)amino]butyl}-1-[[2-(2,4-dichlorophényl)phényl]méthyl]-4-éthoxy-4-oxo-1,4-azaphosphinane-3-carboxylate de tert-butyle

L'intermédiaire 121 est obtenu à partir des intermédiaires 21 et 251 selon la procédure F précédemment décrite.
RMN ¹H : (400 MHz, dmso-d6) δ ppm 7.73 (m, 1 H), 7.51 (m, 1 H), 7.51 (m, 1 H), 7.42/7.35 (m, 2 H), 7.31 (m, 1 H), 7.12 (m, 1 H), 3.92 (m, 2 H), 3.5-3.2 (m, 2 H), 3.5-3.2 (m, 2 H), 2.68/2.12 (m, 2 H), 2.68/2.35 (m, 2 H), 1.81 (m, 2 H), 1.81 (m, 2 H), 1.42 (2*s, 18 H), 1.38 (m, 2 H), 1.35 (2*s, 9 H), 1.17 (2*t, 3 H), 0.74 (m, 2 H)

### EXEMPLE 134 : Acide 3-(4-aminobutyl)-1-[[2-(2,4-dichlorophényl)phényl]méthyl]-4-hydroxy-4-oxo-1,4-azaphosphinane-3-carboxylique

L'exemple 134 est obtenu à partir de l'intermédiaire 121 selon la procédure D précédemment décrite.
RMN ¹H : (400/500 MHz, D2O) δ ppm 7.7-7.63 (m, 2 H), 7.6-7.5 (m, 2 H), 7.46 (dd, 1 H), 7.33 (m, 1 H), 7.3/7.28 (2*d, 1 H), 4.42/4.19/3.99 (m, 2 H), 3.64/3.41/3.14 (m, 2 H), 3.38/3.12/2.99/2.84 (m, 2 H), 2.95 (m, 2 H), 2.14/1.67 (m, 2 H), 1.9/1.4 (m, 2 H), 1.58 (m, 2 H), 1.19/1.11 (m, 2 H)
RMN ¹³C : (400/500 MHz, D2O) δ ppm 132.1, 130.6, 129.5, 127.3, 57, 57, 51.8, 38.6, 27, 26.4, 24.5, 20
ESI/FIA/HR et MS/MS : [M+H]+ = 485.1166 (485.1163)
Analyse Elémentaire : C=54.26(54.44);H=5.33(5.61);N=5.83(5.77)

### Intermédiaire 122 : 3-{4-[Bis(tert-butoxycarbonyl)amino]butyl}-4-éthoxy-1-[[2-(2-méthoxyphényl)phényl]méthyl]-4-oxo-1,4-azaphosphinane-3-carboxylate de tert-butyle

L'intermédiaire 122 est obtenu à partir des intermédiaires 21 et 282 selon la procédure F précédemment décrite.
RMN ¹H : (300 MHz, dmso-d6) δ ppm 7.5 (d, 1 H), 7.4-7.2 (m, 2 H), 7.4-7.2 (m, 1 H), 7.09 (m, 1 H), 7.09 (m, 2 H), 7.01 (t, 1 H), 3.96 (m, 2 H), 3.7 (s, 3 H), 3.42 (m, 2 H), 3.34 (dd, 2 H), 2.73/2.23 (m, 2 H), 2.73/2.46 (m, 2 H), 2-1.7 (m, 2 H), 2-1.7 (m, 2 H), 1.47 (s, 18 H), 1.42 (m, 2 H), 1.4 (s, 9 H), 1.21 (t, 3 H), 0.96 (m, 2 H)

### EXEMPLE 135 : Acide 3-(4-aminobutyl)-4-hydroxy-1-[[2-(2-méthoxyphényl)phényl]méthyl]-4-oxo-1,4-azaphosphinane-3-carboxylique

L'exemple 135 est obtenu à partir de l'intermédiaire 122 selon la procédure D précédemment décrite.
RMN ¹H : (400 MHz, D2O) δ ppm 7.7-7.1 (m, 4 H), 7.7-7.1 (m, 4 H), 4.4-3.9 (m, 2 H), 3.74 (s, 3 H), 3.7-3 (m, 2 H), 3.25-2.65 (m, 2 H), 2.94 (m, 2 H), 2.3-1.3 (m, 2 H), 2.3-1.3 (m, 2 H), 1.61 (m, 2 H), 1.3-0.9 (m, 2 H)
ESI/FIA/HR et MS/MS : [M+H]+ = 447.2031 (447.2048)
Analyse Elémentaire : C=62.34(61.87);H=6.65(7.00);N=6.46(6.27)

### Intermédiaire 123 : 3-{4-[Bis(tert-butoxycarbonyl)amino]butyl}-4-éthoxy-1-[[2-(3-méthoxyphényl)phényl]méthyl]-4-oxo-1,4-azaphosphinane-3-carboxylate de tert-butyle

L'intermédiaire 123 est obtenu à partir des intermédiaires 21 et 281 selon la procédure F précédemment décrite.
RMN ¹H : (400 MHz, dmso-d6) δ ppm 7.49 (d, 1 H), 7.35 (m, 2 H), 7.35 (m, 1 H), 7.21 (d, 1 H), 6.95 (dd, 1 H), 6.89 (d, 1 H), 6.86 (si, 1 H), 3.93 (m, 2 H), 3.79 (s, 3 H), 3.55/3.37 (2*d, 2 H), 3.29 (m, 2 H), 2.74/2.21 (m, 2 H), 2.74/2.33 (m, 2 H), 2-1.7 (m, 2 H), 2-1.7 (m, 2 H), 1.41 (s, 18 H), 1.37 (m, 2 H), 1.33 (s, 9 H), 1.17 (t, 3 H), 0.68 (m, 2 H)

### EXEMPLE 136 : Acide 3-(4-aminobutyl)-4-hydroxy-1-[[2-(3-méthoxyphényl)phényl]méthyl]-4-oxo-1,4-azaphosphinane-3-carboxylique

L'exemple 136 est obtenu à partir de l'intermédiaire 123 selon la procédure D précédemment décrite.
RMN ¹H : (400 MHz, D2O) δ ppm 7.6 (m, 1 H), 7.53 (m, 2 H), 7.46 (t, 1 H), 7.4 (m, 1 H), 7.07 (dd, 1 H), 6.95 (m, 2 H), 4.43/4.28 (2*d, 2 H), 3.84 (s, 3 H), 3.38/3.1 (m, 2 H), 3.2/2.85 (m, 2 H), 2.94 (m, 2 H), 2.1/1.64 (m, 2 H), 1.86/1.36 (m, 2 H), 1.59 (m, 2 H), 1.13/1.06 (m, 2 H)
ESI/FIA/HR et MS/MS : [M+H]+ = 447.2064 (447.2048)
Analyse Elémentaire : C=61.90(61.87);H=6.25(7.00);N=6.35(6.27)

### Intermédiaire 124 : 3-{4-[Bis(tert-butoxycarbonyl)amino]butyl}-4-éthoxy-4-oxo-1-[[2-(4-propan-2-ylphényl)phényl]méthyl]-1,4-azaphosphinane-3-carboxylate de tert-butyle

L'intermédiaire 124 est obtenu à partir des intermédiaires 21 et 289 selon la procédure F précédemment décrite.
RMN ¹H : (400 MHz, dmso-d6) δ ppm 7.49 (dd, 1 H), 7.33 (2*m, 2 H), 7.31 (d, 2 H), 7.27 (d, 2 H), 7.21 (dd, 1 H), 3.93 (m, 2 H), 3.6/3.35 (2*d, 2 H), 3.33 (m, 2 H), 2.95 (hept., 1 H), 2.73/2.23 (2*m, 2 H), 2.73/2.34 (2*m, 2 H), 1.9-1.75 (m, 2 H), 1.9-1.75 (m, 2 H), 1.41/1.32 (2*s, 27 H), 1.38 (m, 2 H), 1.25 (d, 6 H), 1.18 (t, 3 H), 0.7 (m, 2 H)

### EXEMPLE 137 : Acide 3-(4-aminobutyl)-4-hydroxy-4-oxo-1-[[2-(4-propan-2-ylphényl)phényl]méthyl]-1,4-azaphosphinane-3-carboxylique

L'exemple 137 est obtenu à partir de l'intermédiaire 124 selon la procédure D précédemment décrite.
RMN ¹H : (400 MHz, D2O) δ ppm 7.61 (dd, 1 H), 7.52 (2*t, 2 H), 7.43 (d, 2 H), 7.38 (dd, 1 H), 7.29 (d, 2 H), 4.42/4.24 (2*d, 2 H), 3.35/3.11 (2*m, 2 H), 3.22/2.83 (2*m, 2 H), 2.97 (m, 1 H), 2.95 (m, 2 H), 2.09/1.67 (2*m, 2 H), 1.85/1.6 (2*m, 2 H), 1.6/1.35 (2*m, 2 H), 1.24 (d, 6 H), 1.15/1.05 (2*m, 2 H)
ESI/FIA/HR et MS/MS : [M+H]+ = 459.2418 (459.2412)
Analyse Elémentaire : C=65.54(65.49);H=7.39(7.69);N=6.12(6.11)

### Intermédiaire 125 : 3-14-[Bis(tert-butoxycarbonyl)amino]butyl]-4-éthoxy-1-(1H-indazol-4-ylméthyl)-4-oxo-1,4-azaphosphinane-3-carboxylate de tert-butyle

L'intermédiaire 125 est obtenu à partir de l'intermédiaire 21 et du 1H-indazole-4-carbaldéhyde selon la procédure F précédemment décrite.
RMN ¹H : (400 MHz, dmso-d6) δ ppm 13.01 (s, 1 H), 8.19 (s, 1 H), 7.44 (d, 1 H), 7.27 (dd, 1 H), 6.99 (d, 1 H), 3.98 (m, 2 H), 3.93/3.71 (2*d, 2 H), 3.29/3.16 (m, 2 H), 3.03/2.37 (m, 2 H), 2.83/2.52 (m, 2 H), 2-1.7 (m, 2 H), 2-1.7 (m, 2 H), 1.38 (s, 18 H), 1.33 (s, 9 H), 1.24/1.1 (m, 2 H), 1.21 (t, 3 H), 0.65/0.51 (m, 2 H)

### EXEMPLE 138 : Acide 3-(4-aminobutyl)-4-hydroxy-1-(1H-indazol-4-ylméthyl)-4-oxo-1,4-azaphosphinane-3-carboxylique

L'exemple 138 est obtenu à partir de l'intermédiaire 125 selon la procédure D précédemment décrite.
RMN ¹H : (300 MHz, D2O) δ ppm 8.29 (s, 1 H), 7.71 (dl, 1 H), 7.49 (dd, 1 H), 7.3 (dl, 1 H), 4.65 (AB, 2 H), 3.71/3.39 (2m, 2 H), 3.56/3.21 (2m, 2 H), 2.87 (m, 2 H), 2.19/1.74 (2m, 2 H), 1.91/1.45 (2m, 2 H), 1.54 (quint., 2 H), 1.08 (m, 2 H)
ESI/FIA/HR et MS/MS : [M+H]+ = 381.1707 (381.1691)
Analyse Elémentaire : C=53.52(53.68);H=6.73(6.62);N=14.77(14.73)

### Intermédiaire 126 : 3-{4-[Bis(tert-butoxycarbonyl)amino]butyl}-1-[[3-(4-chlorophényl)pyridin-4-yl]méthyl]-4-éthoxy-4-oxo-1,4-azaphosphinane-3-carboxylate de tert-butyle

L'intermédiaire 126 est obtenu à partir de l'intermédiaire 21 et du 3-(4-chlorophényl)pyridine-4-carbaldéhyde selon la procédure F précédemment décrite.
RMN ¹H : (400 MHz, dmso-d6) δ ppm 8.57 (d, 1 H), 8.42 (s, 1 H), 7.56 (d, 2 H), 7.54 (d, 1 H), 7.44 (d, 2 H), 3.94 (m, 2 H), 3.53 (AB, 2 H), 3.36 (m, 2 H), 2.7/2.45 (2m, 2 H), 2.7/2.26 (2m, 2 H), 1.89 (m, 4 H), 1.41 (s, 18 H), 1.41 (m, 2 H), 1.35 (s, 9 H), 1.17 (t, 3 H), 0.78 (m, 2 H)

### EXEMPLE 139 : Acide 3-(4-aminobutyl)-1-[[3-(4-chlorophényl)pyridin-4-yl]méthyl]-4-hydroxy-4-oxo-1,4-azaphosphinane-3-carboxylique

L'exemple 139 est obtenu à partir de l'intermédiaire 126 selon la procédure D précédemment décrite.
RMN ¹H : (400 MHz, D2O) δ ppm 8.62 (d, 1 H), 8.54 (s, 1 H), 7.67 (d, 1 H), 7.58 (d, 2 H), 7.38 (d, 2 H), 4.46/4.35 (2*d, 2 H), 3.47/3.12 (2*m, 2 H), 3.17/2.95 (2*m, 2 H), 2.95 (m, 2 H), 2.15/1.68 (2*m, 2 H), 1.87/1.61 (2*m, 2 H), 1.59/1.38 (2*m, 2 H), 1.08 (m, 2 H)
RMN ³¹P : (400 MHz, D2O) δ ppm 25.1
RMN ¹³C : (400 MHz, D2O) δ ppm 147.4, 146.1, 128.3, 126.4, 122.2, 55, 53.2, 49.9, 36, 24.3, 23.9, 22.1, 17.3
ESI/FIA/HR et MS/MS : [M+H]+ = 452.1516 (452.1505)
Analyse Elémentaire : C=55.97(55.82);H=5.86(6.02);N=9.28(9.30)

### Intermédiaire 127 : 3-14-[Bis(tert-butoxycarbonyl)amino]butyl]-4-éthoxy-4-oxo-1-[(3-phényl-1-benzothiophen-2-yl)méthyl]-1,4-azaphosphinane-3-carboxylate de tert-butyle

L'intermédiaire 127 est obtenu à partir des intermédiaires 21 et 283 selon la procédure F précédemment décrite.
RMN ¹H : (400 MHz, dmso-d6) δ ppm 7.95 (m, 1 H), 7.55 (t, 2 H), 7.47 (t, 1 H), 7.45-7.3 (m, 3 H), 7.45-7.3 (m, 2 H), 4.05 (m, 2 H), 3.81 (dd, 2 H), 3.42/3.33 (m, 2 H), 2.88/2.53 (m, 2 H), 2.88/2.7 (m, 2 H), 2-1.7 (m, 2 H), 2-1.7 (m, 2 H), 1.49 (m, 2 H), 1.39 (s, 27 H), 1.23 (t, 3 H), 1.14/1.02 (m, 2 H)

### EXEMPLE 140 : Acide 3-(4-aminobutyl)-4-hydroxy-4-oxo-1-[(3-phényl-1-benzothiophen-2-yl)méthyl]-1,4-azaphosphinane-3-carboxylique

L'exemple 140 est obtenu à partir de l'intermédiaire 127 selon la procédure D précédemment décrite.
RMN ¹H : (400 MHz, D2O) δ ppm 7.97 (d, 1 H), 7.56 (m, 3 H), 7.49 (d, 1 H), 7.45 (t, 1 H), 7.35 (m, 1 H), 7.35 (m, 2 H), 4.6/4.51 (dd, 2 H), 3.55/3.18 (m, 2 H), 3.26/2.9 (m, 2 H), 2.9 (m, 2 H), 2.18/1.68 (m, 2 H), 1.86/1.34 (m, 2 H), 1.56 (m, 2 H), 1.04 (m, 2 H)
ESI/FIA/HR et MS/MS : [M+H]+ = 473.1669 (473.1663)
Analyse Elémentaire : C=61.52(61.00);H=5.76(6.19);N=5.51(5.93);S=6.56(6.79)

### EXEMPLE 141 : Acide 3-(4-aminobutyl)-4-hydroxy-4-oxo-1-[(3-phénylphényl)méthyl]-1,4-azaphosphinane-3-carboxylique

L'exemple 141 est obtenu à partir de l'intermédiaire 21 et du 3-phénylbenzaldéhyde selon les procédures F et D précédemment décrites.
RMN ¹H : (400 MHz, D2O) δ ppm 7.79-7.4 (m, 9 H), 4.49 (m, 1 H), 4.29 (d, 1 H), 3.76 (m, 1 H), 3.51 (dd, 1 H), 3.39 (m, 1 H), 3.13 (dd, 1 H), 2.89 (m, 2 H), 2.27 (m, 1 H), 1.92 (m, 1 H), 1.79 (m, 1 H), 1.58 (massif, 2 H), 1.48 (m, 1 H), 1.21 (m, 1 H), 1.09 (m, 1 H)
ESI/FIA/HR et MS/MS : [M+H]+ = 417.1937 (417.1943)
Analyse Elémentaire : C=64.07(63.45);H=6.72(7.02);N=6.86(6.73)

### EXEMPLE 142 : Acide 3-(4-aminobutyl)-4-hydroxy-4-oxo-1-(4-phénylbutyl)-1,4-azaphosphinane-3-carboxylique

L'exemple 142 est obtenu à partir de l'intermédiaire 21 et du 4-phénylbutanal selon les procédures F et D précédemment décrites.
RMN ¹H : (400 MHz, D2O) δ ppm 7.29 (t, 2 H), 7.21 (d, 2 H), 7.19 (t, 1 H), 3.5 (m, 1 H), 3.41 (dd, 1 H), 3.2 (m, 1 H), 3.07 (m, 2 H), 3 (m, 1 H), 2.92 (m, 2 H), 2.61 (m, 2 H), 2.19 (m, 1 H), 1.9 (m, 1 H), 1.75-1.1 (m, 10 H)
ESI/FIA/HR et MS/MS : [M+H]+ = 383.2092 (383.2099)
Analyse Elémentaire : C=59.51(59.67);H=8.12(8.17);N=7.34(7.33)

### EXEMPLE 143 : Acide 3-(4-aminobutyl)-4-hydroxy-4-oxo-1-(5-phénylpentyl)-1,4-azaphosphinane-3-carboxylique

L'exemple 143 est obtenu à partir de l'intermédiaire 21 et du 5-phénylpentanal selon les procédures F et D précédemment décrites.
RMN ¹H : (400 MHz, D2O) δ ppm 7.33 (t, 2 H), 7.26 (d, 1 H), 7.21 (t, 1 H), 3.6 (m, 1 H), 3.52 (dd, 1 H), 3.28 (m, 1 H), 3.1 (m, 3 H), 2.99 (m, 2 H), 2.62 (t, 2 H), 2.22 (m, 1 H), 1.98 (m, 1 H), 1.8-1.6 (massif, 2 H), 1.8-1.15 (m, 10 H)
ESI/FIA/HR et MS/MS : [M+H]+ = 397.2251 (397.2256)
Analyse Elémentaire : C=61.01(60.59);H=7.98(8.39);N=7.12(7.07)

### EXEMPLE 145 : Acide 3-(4-aminobutyl)-4-hydroxy-1-[(4-méthylsulfanylphényl)méthyl]-4-oxo-1,4-azaphosphinane-3-carboxylique

L'exemple 145 est obtenu à partir de l'intermédiaire 21 et du 4-formylthioanisole selon les procédures F et D précédemment décrites.
RMN ¹H : (400 MHz, D2O) δ ppm 7.41/7.37 (2*d, 4 H), 4.38/4.17 (2*d, 2 H), 3.7/3.31 (2*m, 2 H), 3.45/3.09 (2*m, 2 H), 2.92 (m, 2 H), 2.49 (s, 3 H), 2.22/1.78 (2*m, 2 H), 1.92/1.59 (2*m, 2 H), 1.58/1.47 (2*m, 2 H), 1.22/1.1 (2*m, 2 H)
RMN³¹P : (400 MHz, D2O) δ ppm 25.9
ESI/FIA/HR et MS/MS : [M+H]+ = 387.1512 (387.1507)
Analyse Elémentaire : C=52.52(52.84);H=6.70(7.04);N=7.35(7.25);S=8.34(8.30)

### EXEMPLE 146 : Acide 3-(4-aminobutyl)-1-[(3-carboxyphényl)méthyl]-4-hydroxy-4-oxo-1,4-azaphosphinane-3-carboxylique

L'exemple 146 est obtenu à partir de l'intermédiaire 21 et du 3-formylbenzoate de méthyle selon les procédures F et D précédemment décrites.
RMN ¹H : (400 MHz, D2O) δ ppm 8.03 (2*m, 2 H), 7.7 (d, 1 H), 7.59 (t, 1 H), 4.52/4.3 (2*d, 2 H), 3.75/3.4 (2*m, 2 H), 3.4/3.12 (2*m, 2 H), 2.9 (m, 2 H), 2.25/1.81 (2*m, 2 H), 1.95/1.58 (2*m, 2 H), 1.62-1.43 (massif, 2 H), 1.2/1.1 (2*m, 2 H)
RMN ³¹P : (400 MHz, D2O) δ ppm 25
ESI/FIA/HR et MS/MS : [M+H]+ = 385.1522 (385.1528)
Analyse Elémentaire : C=53.60(53.12);H=6.54(6.56);N=7.42(7.29)

### EXEMPLE 147 : Acide 3-(4-aminobutyl)-1-[(4-carboxyphényl)méthyl]-4-hydroxy-4-oxo-1,4-azaphosphinane-3-carboxylique

L'exemple 147 est obtenu à partir de l'intermédiaire 21 et du 4-formylbenzoate de méthyle selon les procédures F et D précédemment décrites.
RMN ¹H : (400 MHz, D2O) δ ppm 8 (d, 2 H), 7.6 (d, 2 H), 4.52/4.3 (2*d, 2 H), 3.75/3.4 (2*m, 2 H), 3.45/3.13 (dd, 2 H), 2.9 (m, 2 H), 2.28/1.8 (2*m, 2 H), 1.9/1.55 (2*m, 2 H), 1.55/1.5 (m, 2 H), 1.2/1.1 (m, 2 H)
ESI/FIA/HR et MS/MS : [M+H]+ = 385.1524 (385.1528)
Analyse Elémentaire : C=52.66(53.12);H=6.04(6.56);N=7.33(7.29)

### EXEMPLE 148 : Acide 3-(4-aminobutyl)-1-[[4-(difluorométhoxy)phényl]méthyl]-4-hydroxy-4-oxo-1,4-azaphosphinane-3-carboxylique

L'exemple 148 est obtenu à partir de l'intermédiaire 21 et du 4-(difluorométhoxy)benzaldéhyde selon les procédures F et D précédemment décrites.
RMN ¹H : (400 MHz, D2O) δ ppm 7.5 (d, 2 H), 7.25 (d, 2 H), 6.83 (t, 1 H), 4.4/4.22 (2*d, 2 H), 3.7/3.32 (dd, 2 H), 3.45/3.1 (dd, 2 H), 2.92 (m, 2 H), 2.25/1.78 (2*m, 2 H), 1.9/1.6 (2*m, 2 H), 1.6/1.46 (2*m, 2 H), 1.22/1.1 (2*m, 2 H)
ESI/FIA/HR et MS/MS : [M+H]+ = 407.1551 (407.1547)
Analyse Elémentaire : C=50.33(50.25);H=5.97(6.20);N=7.02(6.89)

### EXEMPLE 149 : Acide 3-(4-aminobutyl)-4-hydroxy-4-oxo-1-[(1S)-1-phényléthyl]-1,4-azaphosphinane-3-carboxylique

L'exemple 149 est obtenu à partir de l'intermédiaire 21 et de l'acétophénone selon les procédures F et D précédemment décrites.
RMN ¹H : (400 MHz, D2O) δ ppm 7.5 (m, 5 H), 4.67 (quad., 1 H), 3.72/3.53 (2*m, 1 H), 3.62/3.41 (2*m, 1 H), 3.29/3.13 (2*m, 1 H), 3.09-2.9 (m, 1 H), 3.09-2.9 (m, 2 H), 2.28/2.11 (2*m, 1 H), 2-1.65 (m, 1 H), 2-1.65 (m, 2 H), 1.7 (2*d, 3 H), 1.7-1.05 (m, 2 H), 1.7-1.05 (m, 2 H)
RMN ³¹P : (400 MHz, D2O) δ ppm 26.4/26.2
ESI/FIA/HR et MS/MS : [M+H]+ = 355.1807 (355.1781)
Analyse Elémentaire : C=57.43(57.62);H=7.57(7.68);N=7.96(7.90)

### EXEMPLE 150 : Acide 3-(4-aminobutyl)-1-[(3,4-difluorophényl)méthyl]-4-hydroxy-4-oxo-1,4-azaphosphinane-3-carboxylique

L'exemple 150 est obtenu à partir de l'intermédiaire 21 et du 3,4-difluorobenzaldéhyde selon les procédures F et D précédemment décrites.
RMN ¹H : (300 MHz, D2O) δ ppm 7.46-7.22 (m, 3 H), 4.28 (AB, 2 H), 3.69/3.42 (2m, 2 H), 3.34/3.1 (m+dd, 2 H), 2.92 (m, 2 H), 2.23/1.77 (2m, 2 H), 1.92/1.46 (2m, 2 H), 1.59 (quint., 2 H), 1.22/1.09 (2m, 2 H)
RMN ¹⁹F : (300 MHz, D2O) δ ppm -135.5
RMN ³¹P : (300 MHz, D2O) δ ppm 23
ESI/FIA/HR et MS/MS : [M+H]+ = 377.1416 (377.1441)
Analyse Elémentaire : C=50.40(51.06);H=6.20(6.16);N=7.38(7.44)

### EXEMPLE 151 : Acide 3-(4-aminobutyl)-1-[[3-[(diméthylamino)méthyl]-4-hydroxyphényl]méthyl]-4-hydroxy-4-oxo-1,4-azaphosphinane-3-carboxylique

L'exemple 151 est obtenu à partir de l'intermédiaire 21 et du 3-(diméthylaminométhyl)-4-hydroxy-benzaldéhyde selon les procédures F et D précédemment décrites.
RMN ¹H : (400 MHz, D2O) δ ppm 7.45 (d, 1 H), 7.43 (s, 1 H), 7.02 (d, 1 H), 4.35/4.18 (2*d, 2 H), 4.32/4.28 (2*d, 2 H), 3.7/3.3 (2*m, 2 H), 3.44/3.08 (dd, 2 H), 2.9 (m, 2 H), 2.8 (d, 6 H), 2.2/1.75 (2*m, 2 H), 1.9/1.45 (2*m, 2 H), 1.6 (m, 2 H), 1.25/1.1 (2*m, 2 H)
ESI/FIA/HR et MS/MS : [M+H]+ = 414.2155 (414.2157)
Analyse Elémentaire : C=46.97(47.82);H=5.90(6.31);N=7.67(7.97)

### EXEMPLE 152 : Acide 3-(4-aminobutyl)-1-[(2-fluorophényl)méthyl]-4-hydroxy-4-oxo-1,4-azaphosphinane-3-carboxylique

L'exemple 152 est obtenu à partir de l'intermédiaire 21 et du 2-fluorobenzaldéhyde selon les procédures F et D précédemment décrites.
RMN ¹H : (300 MHz, D2O) δ ppm 7.5 (m, 2 H), 7.26 (m, 2 H), 4.41 (si, 2 H), 3.7/3.35 (2m, 2 H), 3.52/3.21 (2m, 2 H), 2.94 (m, 2 H), 2.22/1.77 (2m, 2 H), 1.95/1.49 (2m, 2 H), 1.61 (quint., 2 H), 1.28/1.14 (2m, 2 H)
RMN ¹⁹F : (300 MHz, D2O) δ ppm -115
RMN ³¹P : (300 MHz, D2O) δ ppm 25.6
ESI/FIA/HR et MS/MS : [M+H]+ = 359.1530 (359.1535)
Analyse Elémentaire : C=53.34(53.63);H=6.46(6.75);N=7.77(7.82)

### EXEMPLE 153 : Acide 3-(4-aminobutyl)-1-[(3-fluorophényl)méthyl]-4-hydroxy-4-oxo-1,4-azaphosphinane-3-carboxylique

L'exemple 153 est obtenu à partir de l'intermédiaire 21 et du 3-fluorobenzaldéhyde selon les procédures F et D précédemment décrites.
RMN ¹H : (300 MHz, D2O) δ ppm 7.48 (m, 1 H), 7.24 (m, 3 H), 4.31 (AB, 2 H), 3.71/3.35 (2m, 2 H), 3.45/3.12 (2m, 2 H), 2.92 (m, 2 H), 2.24/1.77 (2m, 2 H), 1.93/1.46 (2m, 2 H), 1.58 (quint., 2 H), 1.22/1.1 (2m, 2 H)
RMN ¹⁹F : (300 MHz, D2O) δ ppm -112
RMN ³¹P : (300 MHz, D2O) δ ppm 22.9
ESI/FIA/HR et MS/MS : [M+H]+ = 359.1536 (359.1535)
Analyse Elémentaire : C=53.15(53.63);H=6.41(6.75);N=7.83(7.82)

### EXEMPLE 154 : Acide 3-(4-aminobutyl)-1-[(6-aminopyridin-3-yl)méthyl]-4-hydroxy-4-oxo-1,4-azaphosphinane-3-carboxylique

L'exemple 154 est obtenu à partir de l'intermédiaire 21 et du 2-(Boc-amino)pyridine-5-carboxaldéhyde selon les procédures F et D précédemment décrites.
RMN ¹H : (400 MHz, D2O) δ ppm 7.97 (d, 1 H), 7.61 (dd, 1 H), 6.71 (d, 1 H), 4.18 (AB, 2 H), 3.67/3.29 (2m, 2 H), 3.42/3.05 (2m, 2 H), 2.93 (m, 2 H), 2.22/1.77 (2m, 2 H), 1.93/1.47 (2m, 2 H), 1.59 (quint., 2 H), 1.25/1.11 (2m, 2 H)
RMN ³¹P : (400 MHz, D2O) δ ppm 23.2
ESI/FIA/HR et MS/MS : [M+H]+ = 357.1696 (357.1691)
Analyse Elémentaire : C=51.09(50.56);H=6.51(7.07);N=15.78(15.72)

### EXEMPLE 155 : Acide 3-(4-aminobutyl)-4-hydroxy-4-oxo-1-[(2-phénoxyphényl)-méthyl]-1,4-azaphosphinane-3-carboxylique

L'exemple 155 est obtenu à partir de l'intermédiaire 21 et du 2-phénoxybenzaldéhyde selon les procédures F et D précédemment décrites.
RMN ¹H : (300 MHz, D2O) δ ppm 7.47 (t, 1 H), 7.41 (t, 2 H), 7.27-7.09 (m, 4 H), 7.05 (d, 2 H), 4.27 (AB, 2 H), 3.68/3.3 (2m, 2 H), 3.46/3.07 (2m, 2 H), 2.91 (m, 2 H), 2.23/1.76 (2m, 2 H), 1.93/1.46 (2m, 2 H), 1.6 (quint., 2 H), 1.15 (m, 2 H)
RMN ³¹P : (300 MHz, D2O) δ ppm 25.8
ESI/FIA/HR et MS/MS : [M+H]+ = 433.1894 (433.1892)
Analyse Elémentaire : C=61.51(61.10);H=6.57(6.76);N=6.61(6.48)

### EXEMPLE 156 : Acide 3-(4-aminobutyl)-1-[[2-(4-chlorophényl)phényl]méthyl]-4-hydroxy-4-oxo-1,4-azaphosphinane-3-carboxylique

L'exemple 156 est obtenu à partir de l'intermédiaire 21 et du 2-(4-chlorophényl)benzaldéhyde selon les procédures F et D précédemment décrites.
RMN ¹H : (300 MHz, D2O) δ ppm 7.6/7.52/7.4 (m, 3 H), 7.52/7.34 (2d, 4 H), 4.35 (AB, 2 H), 3.4/3.1 (2m, 2 H), 3.17/2.86 (2m, 2 H), 2.94 (m, 2 H), 2.09/1.64 (2m, 2 H), 1.85/1.35 (2m, 2 H), 1.58 (m, 2 H), 1.08 (m, 2 H)
ESI/FIA/HR et MS/MS : [M+H]+ = 451.1559 (451.1553)
Analyse Elémentaire : C=58.24(58.60);H=5.82(6.26);N=6.48(6.21)

### EXEMPLE 157 : Acide 3-(4-aminobutyl)-1-[(2-bromophényl)méthyl]-4-hydroxy-4-oxo-1,4-azaphosphinane-3-carboxylique

L'exemple 157 est obtenu à partir de l'intermédiaire 21 et du 2-bromobenzaldéhyde selon les procédures F et D précédemment décrites.
RMN ¹H : (300 MHz, D2O) δ ppm 7.75 (dl, 1 H), 7.53 (dd, 1 H), 7.45 (tl, 1 H), 7.39 (td, 1 H), 4.44 (AB, 2 H), 3.74/3.43 (2m, 2 H), 3.5/3.28 (2m, 2 H), 2.94 (m, 2 H), 2.24/1.79 (2m, 2 H), 1.95/1.5 (2m, 2 H), 1.6 (m, 2 H), 1.26/1.1 (2m, 2 H)
ESI/FIA/HR et MS/MS : [M+H]+ = 419.0732 (419.0735)
Analyse Elémentaire : C=45.33(45.84);H=5.29(5.77);N=7.00(6.68)

### EXEMPLE 159 : Acide 3-(4-aminobutyl)-4-hydroxy-4-oxo-1-[(2-oxo-1,3-dihydroindol-5-yl)méthyl]-1,4-azaphosphinane-3-carboxylique

L'exemple 159 est obtenu à partir de l'intermédiaire 21 et du oxindole-5-carboxaldéhyde selon les procédures F et D précédemment décrites.
RMN ¹H : (300 MHz, D2O) δ ppm 7.35 (d, 1 H), 7.3 (dd, 1 H), 7 (d, 1 H), 4.35/4.15 (dd, 2 H), 3.7/3.3 (2m, 2 H), 3.6 (dd, 2 H), 3.4/3.1 (2dd, 2 H), 2.9 (m, 2 H), 2.2/1.75 (2m, 2 H), 1.9/1.45 (2m, 2 H), 1.58 (m, 2 H), 1.3-1 (2m, 2 H)
ESI/FIA/HR et MS/MS : [M+H]+ = 396.1692 (396.1688)
Analyse Elémentaire : C=54.70(54.68);H=6.42(6.63);N=10.57(10.63)

### EXEMPLE 160 : Acide 3-(4-aminobutyl)-4-hydroxy-1-[[2-(3-méthyl-1,2,4-oxadiazol-5-yl)phényl]méthyl]-4-oxo-1,4-azaphosphinane-3-carboxylique

L'exemple 160 est obtenu à partir de l'intermédiaire 21 et du 2-(3-méthyl-1,2,4-oxadiazol-5-yl)benzaldéhyde selon les procédures F et D précédemment décrites.
RMN ¹H : (400 MHz, D2O) δ ppm 8.26 (d, 1 H), 7.72 (m, 2 H), 7.65 (d, 1 H), 4.78/4.36 (2*d, 2 H), 4/3.55 (m, 2 H), 3.44/3.25 (m, 2 H), 2.91 (m, 2 H), 2.5 (s, 3 H), 2.31/1.88 (m, 2 H), 1.88/1.49 (m, 2 H), 1.58 (m, 2 H), 1.22/1.02 (m, 2 H)
ESI/FIA/HR et MS/MS : [M+H]+ = 423.1801 (423.1797)
Analyse Elémentaire : C=53.57(54.02);H=6.25(6.44);N=12.77(13.26)

### EXEMPLE 161 : Acide 3-(4-aminobutyl)-4-hydroxy-1-[[2-[2-méthyl-5-(trifluorométhyl)pyrazol-3-yl]phényl]méthyl]-4-oxo-1,4-azaphosphinane-3-carboxylique

L'exemple 156 est obtenu à partir de l'intermédiaire 21 et du 2-[1-méthyl-3(trifluorométhyl)-1H-pyrazol-5-yl]benzaldéhyde selon les procédures F et D précédemment décrites.
RMN ¹H : (400 MHz, D2O) δ ppm 7.71 (d, 1 H), 7.67 (t, 1 H), 7.63 (t, 1 H), 7.48 (d, 1 H), 6.83 (s, 1 H), 4.3/4.18 (dl, 2 H), 3.53/3.21 (m, 2 H), 3.27 (s, 3 H), 3.27/3.03 (m, 2 H), 2.94 (m, 2 H), 2.18/1.71 (m, 2 H), 2.18/1.43 (m, 2 H), 1.6 (m, 2 H), 1.18/1.1 (m, 2 H)
ESI/FIA/HR et MS/MS : [M+H]+ = 489.1903 (489.1878)
Analyse Elémentaire : C=51.66(51.64);H=5.61(5.78);N=11.25(11.47)

### EXEMPLE 162 : Acide 3-(4-aminobutyl)-1-[[2-(2,4-difluorophényl)phényl]méthyl]-4-hydroxy-4-oxo-1,4-azaphosphinane-3-carboxylique

L'exemple 162 est obtenu à partir des intermédiaires 21 et 287 selon les procédures F et D précédemment décrites.
RMN ¹H : (400 MHz, D2O) δ ppm 7.65 (m, 1 H), 7.57 (m, 2 H), 7.4 (m, 1 H), 7.34 (m, 1 H), 7.1 (2*m, 2 H), 4.45-4 (m, 2 H), 3.7-2.8 (m, 2 H), 3.7-2.8 (m, 2 H), 2.93 (m, 2 H), 2.15/1.66 (2*m, 2 H), 1.88/1.6 (2*m, 2 H), 1.6/1.38 (2*m, 2 H), 1.15/1.05 (2*m, 2 H)
ESI/FIA/HR et MS/MS : [M+H]+ = 453.1745 (453.1754)
Analyse Elémentaire : C=58.27(58.40);H=6.09(6.01);N=6.17(6.19)

### EXEMPLE 163 : Acide 3-(4-aminobutyl)-1-[[2-fluoro-6-(4-hydroxyphényl)phényl]-méthyl]-4-hydroxy-4-oxo-1,4-azaphosphinane-3-carboxylique

L'exemple 163 est obtenu à partir de l'intermédiaire 21 et du 2-fluoro-6-(4-hydroxyphényl)benzaldéhyde selon les procédures F et D précédemment décrites.
RMN ¹H : (400 MHz, D2O) δ ppm 7.52 (m, 1 H), 7.27 (m, 1 H), 7.22 (d, 2 H), 7.19 (m, 1 H), 6.98 (d, 2 H), 4.41 (dd, 2 H), 3.45/3.09 (m, 2 H), 3.16/2.9 (m, 2 H), 2.94 (m, 2 H), 2.09/1.64 (m, 2 H), 1.86/1.35 (m, 2 H), 1.59 (m, 2 H), 1.08 (m, 2 H)
ESI/FIA/HR et MS/MS : [M+H]+ = 451.1813 (451.1798)
Analyse Elémentaire : C=58.33(58.66);H=5.35(6.27);N=6.49(6.22)

### EXEMPLE 164 : Acide 3-(4-aminobutyl)-1-[[2-(1,3-benzodioxol-5-yl)phényl]méthyl]-4-hydroxy-4-oxo-1,4-azaphosphinane-3-carboxylique

L'exemple 164 est obtenu à partir des intermédiaires 21 et 279 selon les procédures F et D précédemment décrites.
RMN ¹H : (400 MHz, dmso-d6) δ ppm 8.2-7.9 (s, 3 H), 7.45 (d, 1 H), 7.3 (m, 2 H), 7.15 (d, 1 H), 6.95 (dd, 1 H), 6.95 (s, 1 H), 6.8 (dd, 1 H), 6.05 (s, 2 H), 3.5 (d, 1 H), 3.4 (d, 1 H), 2.9 (m, 1 H), 2.7 (t, 2 H), 2.55 (m, 1 H), 2.35 (m, 1 H), 2.25 (m, 1 H), 1.7 (m, 1 H), 1.6-1 (m, 7 H)
ESI/FIA/HR et MS/MS : [M+H]+ = 461.1854 (461.1841)
Analyse Elémentaire : C=60.04(59.99);H=6.06(6.35);N=5.62(6.08)

### EXEMPLE 165 : Acide 3-(4-aminobutyl)-4-hydroxy-1-[[2-(6-méthoxypyridin-3-yl)phényl] méthyl] -4-oxo-1,4-azaphosphinane-3-carboxylique

L'exemple 165 est obtenu à partir de l'intermédiaire 21 et du 2-(6-méthoxy-3-pyridinyl)benzaldéhyde selon les procédures F et D précédemment décrites.
RMN ¹H : (300 MHz, D2O) δ ppm 8.05 (s, 1 H), 7.75 (dd, 1 H), 7.6 (m, 1 H), 7.5 (m, 2 H), 7.35 (m, 1 H), 6.95 (d, 1 H), 4.4 (d, 1 H), 4.25 (d, 1 H), 3.9 (s, 3 H), 3.55-3.3 (m, 1 H), 3.3-3.1 (m, 1 H), 3.1 (m, 1 H), 2.95 (t, 2 H), 2.85 (m, 1 H), 2.1 (m, 1 H), 1.85 (m, 1 H), 1.75-1.5 (m, 3 H), 1.35 (m, 1 H), 1.2-0.95 (m, 2 H)
ESI/FIA/HR et MS/MS : [M+H]+ = 448.1996 (448.2001)
Analyse Elémentaire : C=59.45(59.05);H=6.75(6.76);N=9.12(9.39)

### EXEMPLE 166 : Acide 3-(4-aminobutyl)-4-hydroxy-4-oxo-1-[[2-(6-oxo-1H-pyridin-3-yl)phényl]méthyl]-1,4-azaphosphinane-3-carboxylique

L'exemple 166 est obtenu à partir des intermédiaires 21 et 237 selon les procédures F et D précédemment décrites.
RMN ¹H : (300/400 MHz, D2O) δ ppm 7.7 (dd, 1 H), 7.6 (s, 1 H), 7.6-7.3 (2*m, 2 H), 6.7 (d, 1 H), 4.35 (m, 2 H), 3.6-3.4 (m, 1 H), 3.4-3.05 (m, 2 H), 3 (m, 1 H), 2.9 (t, 2 H), 2.1 (m, 1 H), 1.85 (m, 1 H), 1.7 (m, 1 H), 1.6 (m, 2 H), 1.4 (m, 1 H), 1.25-0.95 (m, 2 H)
ESI/FIA/HR et MS/MS : [M+H]+ = 434.1860 (434.1844)
Analyse Elémentaire : C=58.86(58.19);H=6.27(6.51);N=9.89(9.69)

### EXEMPLE 167 : Acide 3-(4-aminobutyl)-4-hydroxy-1-[(2-imidazo[1,2-a]pyridin-3-ylphényl)méthyl]-4-oxo-1,4-azaphosphinane-3-carboxylique

L'exemple 167 est obtenu à partir des intermédiaires 21 et 227 selon les procédures F et D précédemment décrites.
RMN ¹H : (300 MHz, D2O) δ ppm 7.98 (d, 1 H), 7.68 (s, 1 H), 7.65 (d, 1 H), 7.65 (m, 2 H), 7.52 (m, 1 H), 7.45 (dd, 1 H), 7.22 (m, 1 H), 6.98 (t, 1 H), 4.25 (m, 2 H), 3.45/3.1 (2*m, 2 H), 2.9 (m, 2 H), 2.85 (m, 2 H), 2.12/1.55 (2*m, 2 H), 1.8/1.55 (2*m, 2 H), 1.55/0.95 (2*m, 2 H), 1.28/0.95 (2*m, 2 H)
ESI/FIA/HR et MS/MS : [M+H]+ = 457.2004 (457.20046)
Analyse Elémentaire : C=59.82(60.52);H=5.48(6.40);N=11.98(12.27)

### EXEMPLE 168 : Acide 3-(4-aminobutyl)-1-[(2-chlorophényl)méthyl]-4-hydroxy-4-oxo-1,4-azaphosphinane-3-carboxylique

L'exemple 168 est obtenu à partir de l'intermédiaire 21 et du 2-chlorobenzaldéhyde selon les procédures F et D précédemment décrites.
RMN ¹H : (400 MHz, D2O) δ ppm 7.58/7.53 (2*d, 2 H), 7.48/7.4 (2*m, 2 H), 4.44 (m, 2 H), 3.71/3.41 (2*m, 2 H), 3.51/3.28 (2*m, 2 H), 2.94 (m, 2 H), 2.22/1.78 (2*m, 2 H), 1.95/1.61 (2*m, 2 H), 1.6/1.49 (2*m, 2 H), 1.28/1.11 (2*m, 2 H)
RMN ³¹P : (400 MHz, D2O) δ ppm -25.5
ESI/FIA/HR et MS/MS : [M+H]+ = 375.1235 (375.1240)
Analyse Elémentaire : C=51.58(51.27);H=6.22(6.45);N=7.65(7.47)

### EXEMPLE 169 : Acide 3-(4-aminobutyl)-4-hydroxy-4-oxo-1-[(1R)-1-phényléthyl]-1,4-azaphosphinane-3-carboxylique

L'exemple 169 est obtenu à partir de l'intermédiaire 21 et de l'acétophénone selon les procédures F et D précédemment décrites.
RMN ¹H : (400 MHz, D2O) δ ppm 7.5 (m, 5 H), 4.67 (quad., 1 H), 3.72/3.53 (2*m, 1 H), 3.62/3.41 (2*m, 1 H), 3.29/3.13 (2*m, 1 H), 3.09-2.9 (m, 1 H), 3.09-2.9 (m, 2 H), 2.28/2.11 (2*m, 1 H), 2-1.65 (m, 1 H), 2-1.65 (m, 2 H), 1.7 (2*d, 3 H), 1.7-1.05 (m, 2 H), 1.7-1.05 (m, 2 H)
RMN ³¹P : (400 MHz, D2O) δ ppm 26.4/26.2
ESI/FIA/HR et MS/MS : [M+H]+ = 355.1783 (355.1786)
Analyse Elémentaire : C=58.22(57.62);H=7.85(7.68);N=8.04(7.90)

### EXEMPLE 170 : Acide 3-(4-aminobutyl)-4-hydroxy-4-oxo-1-[(2-phénylphényl)méthyl]-1,4-azaphosphinane-3-carboxylique

L'exemple 170 est obtenu à partir de l'intermédiaire 21 et du 2-phénylbenzaldéhyde selon les procédures F et D précédemment décrites.
RMN ¹H : (400 MHz, dmso-d6) δ ppm 7.65-7.3 (m, 6 H), 4.33 (AB, 2 H), 3.43-3 (m, 3 H), 2.92 (dd, 1 H), 2.82 (m, 2 H), 2.06/1.57 (2m, 2 H), 1.83/1.33 (2m, 2 H), 1.57 (m, 2 H), 1.08 (m, 2 H)
ESI/FIA/HR et MS/MS : [M+H]+ = 417.1945 (417.1943)
Analyse Elémentaire : C=63.68(63.45);H=6.84(7.02);N=6.85(6.73)

### EXEMPLE 171 : Acide 3-(4-aminobutyl)-1-[[2-(furan-3-yl)phényl]méthyl]-4-hydroxy-4-oxo-1,4-azaphosphinane-3-carboxylique

L'exemple 171 est obtenu à partir des intermédiaires 21 et 290 selon les procédures F et D précédemment décrites.
RMN ¹H : (400 MHz, D2O) δ ppm 7.68 (m, 1 H), 7.65 (m, 1 H), 7.58-7.44 (m, 4 H), 6.63 (m, 1 H), 4.49/4.4 (2*d, 2 H), 3.5/3.22 (2*m, 2 H), 3.34/3.01 (2*m, 2 H), 2.94 (m, 2 H), 2.1/1.68 (2*m, 2 H), 1.69/1.6 (2*m, 2 H), 1.59/1.41 (2*m, 2 H), 1.18/1.08 (2*m, 2 H)
ESI/FIA/HR et MS/MS : [M+H]+ = 407.1736 (407.1735)
Analyse Elémentaire : C=59.20(59.11);H=7.12(6.70);N=7.00(6.89)

### EXEMPLE 172 : Acide 3-(4-aminobutyl)-4-hydroxy-1-[[2-(3-hydroxyphényl)phényl]-méthyl]-4-oxo-1,4-azaphosphinane-3-carboxylique

L'exemple 172 est obtenu à partir des intermédiaires 21 et 281 selon les procédures F et D précédemment décrites.
RMN ¹H : (400 MHz, D2O) δ ppm 7.52 (m, 2 H), 7.39 (m, 1 H), 7.39 (m, 1 H), 7.29 (d, 1 H), 6.95 (dd, 1 H), 6.9 (dl, 1 H), 6.85 (si, 1 H), 4.44/4.29 (d, 2 H), 3.38/3.13 (m, 2 H), 3.2/2.85 (m, 2 H), 3.13/1.36 (m, 2 H), 2.94 (m, 2 H), 2.1-1.65 (m, 2 H), 1.59 (m, 2 H), 1.13/1.05 (m, 2 H)
ESI/FIA/HR et MS/MS : [M+H]+ = 433.1895 (433.1892)
Analyse Elémentaire : C=61.64(61.10);H=6.51(6.76);N=6.73(6.48)

### EXEMPLE 173 : Acide 3-(4-aminobutyl)-1-[[2-(4-chlorophényl)-4-fluorophényl]-méthyl]-4-hydroxy-4-oxo-1,4-azaphosphinane-3-carboxylique

L'exemple 173 est obtenu à partir des intermédiaires 21 et 220 selon les procédures F et D précédemment décrites.
RMN ¹H : (400 MHz, D2O) δ ppm 7.59 (dd, 1 H), 7.51 (d, 2 H), 7.32 (d, 2 H), 7.23 (td, 1 H), 7.14 (dd, 1 H), 4.3 (AB, 2 H), 3.37/3.06 (2m, 2 H), 3.11/2.81 (2m, 2 H), 2.92 (m, 2 H), 2.06/1.62 (2m, 2 H), 1.83/1.33 (2m, 2 H), 1.56 (m, 2 H), 1.05 (m, 2 H)
ESI/FIA/HR et MS/MS : [M+H]+ = 469.1470 (469.1459)
Analyse Elémentaire : C=56.07(56.35);H=5.39(5.80);N=6.03(5.97)

### EXEMPLE 174 : Acide 3-(4-aminobutyl)-4-hydroxy-4-oxo-1-[(2-propan-2-ylphényl)méthyl]-1,4-azaphosphinane-3-carboxylique

L'exemple 174 est obtenu à partir de l'intermédiaire 21 et du 2-isopropylbenzaldéhyde selon les procédures F et D précédemment décrites.
RMN ¹H : (500 MHz, D2O) δ ppm 7.49 (d, 1 H), 7.45 (t, 1 H), 7.38 (d, 1 H), 7.28 (t, 1 H), 4.39 (dd, 2 H), 3.65 (dd, 1 H), 3.5 (dd, 1 H), 3.35 (m, 1 H), 3.21 (dd, 1 H), 3.09 (m, 1 H), 2.91 (m, 2 H), 2.17 (m, 1 H), 1.93 (m, 1 H), 1.74 (m, 1 H), 1.58 (m, 2 H), 1.47 (m, 1 H), 1.24 (m, 1 H), 1.2/1.18 (2*s, 6 H), 1.1 (m, 1 H)
ESI/FIA/HR et MS/MS : [M+H]+ = 383.2097 (383.2099)
Analyse Elémentaire : C=59.45(59.67);H=8.00(8.17);N=7.60(7.33)

### EXEMPLE 175 : Acide 3-(4-aminobutyl)-1-[[2-(3,4-dichlorophényl)phényl]méthyl]-4-hydroxy-4-oxo-1,4-azaphosphinane-3-carboxylique

L'exemple 175 est obtenu à partir des intermédiaires 21 et 215 selon les procédures F et D précédemment décrites.
RMN ¹H : (300 MHz, D2O) δ ppm 7.62 (d, 1 H), 7.6 (m, 1 H), 7.54 (d, 1 H), 7.52 (m, 2 H), 7.37 (m, 1 H), 7.24 (dd, 1 H), 4.34 (AB, 2 H), 3.45/3.07 (2m, 2 H), 3.12/2.83 (2m, 2 H), 2.93 (m, 2 H), 2.1/1.63 (2m, 2 H), 1.84/1.33 (2m, 2 H), 1.57 (m, 2 H), 1.04 (m, 2 H)
ESI/FIA/HR et MS/MS : [M+H]+ = 485.1158 (485.1163)
Analyse Elémentaire : C=54.91(54.44);H=5.37(5.61);N=5.35(5.77)

### EXEMPLE 176 : Acide 3-(4-aminobutyl)-4-hydroxy-4-oxo-1-[(3-phénylthiophen-2-yl)méthyl]-1,4-azaphosphinane-3-carboxylique

L'exemple 176 est obtenu à partir des intermédiaires 21 et 284 selon les procédures F et D précédemment décrites.
RMN ¹H : (300 MHz, D2O) δ ppm 7.62 (d, 1 H), 7.49 (t, 2 H), 7.44 (t, 1 H), 7.4 (d, 2 H), 7.17 (d, 1 H), 4.6 (AB, 2 H), 3.52/3.13 (2m, 2 H), 3.13/2.73 (2m, 2 H), 2.91 (m, 2 H), 2.12/1.66 (2m, 2 H), 1.81/1.26 (2m, 2 H), 1.55 (m, 2 H), 0.97 (m, 2 H)
ESI/FIA/HR et MS/MS : [M+H]+ = 423.1525 (423.1507)
Analyse Elémentaire : C=56.76(56.86);H=6.46(6.44);N=7.19(6.63);S=7.30(7.59)

### Procédure G : Séparation Chirale des diastéréoisomères de l'intermédiaire 20b

### Intermédiaire 128: (3S)-3-{4-[Bis(tert-butoxycarbonyl)amino]butyl}-1-benzyl-4-éthoxy-4-oxo-1,4-azaphosphinane-3-carboxylate de tert-butyle

Dans un ballon tricol de 1L équipé d'une agitation mécanique sont introduits successivement sous une atmosphère d'argon du DMSO (30 mL) et du NaH à 60% (6.78 g, 186.6 mmol, 1.6 eq). Le ballon est maintenu à température ambiante à l'aide d'un bain d'eau. Une solution de l'intermédiaire 204 (41.1 g, 116.7 mmol, 1.1 eq) dans du DMSO (25 mL) est alors ajoutée goutte à goutte sur 5 minutes. Une solution d'intermédiaire 19 (37.54 g, 106 mmol) dans du DMSO (100 mL) est alors additionnée goutte à goutte en maintenant la température en dessous de 20°C. En effet, l'addition provoque un important échauffement ainsi qu'un fort épaississement du milieu réactionnel. Après la fin de l'addition, 100 mL de THF anhydre sont alors ajoutés pour pouvoir maintenir une agitation. Après 3h, le mélange réactionnel est refroidi à l'aide d'un bain d'eau glacée et hydrolysé par ajout de 500 mL d'une solution saturée de NH₄Cl. Le mélange est ensuite extrait avec de l'AcOEt (3 x 300 mL). Les phases organiques sont alors rassemblées, lavées avec une solution saturée de NaCl (2 x 300 mL), et séchées sur MgSO₄ avant d'être concentrées sous pression réduite pour conduire à un solide jaunâtre (69.94 g), mélange de 4 diastéréoisomères. Les 4 diastéréoisomères de l'intermédiaire 20b sont séparés sur une colonne chirale de type (*R,R*)-Whelk-O-1 de 2.5 kg par lot de 8 g, chaque lot nécessitant 2 passages dans les conditions suivantes :

### 1^{er} passage :

Le mélange contenant les 4 diastéréoisomères de l'intermédiaire 20b (8 g de brut) est déposé sur une colonne chirale de type (*R,R*)-Whelk-O-1 de 2.5 kg en utilisant comme phase mobile DCM/Heptane (55:45) + 10% NEt₃ afin d'isoler le diastéréoisomère 4 (numéro attribué suivant l'ordre de sortie de colonne) de l'intermédiaire 20b.

### 2^{eme} passage :

Le mélange contenant les 3 diastéréoisomères restants de l'intermédiaire 20b est déposé sur une colonne chirale de type (*R,R*)-Whelk-O-1 de 2.5 kg en utilisant comme phase mobile MTBE + 10% DEA afin d'isoler le diastéréoisomère 2.

Après 9 injections, les fractions contenant les diastéréoisomères 2 et 4 de l'intermédiaire 20b sont alors rassemblées et évaporées sous pression réduite pour conduire à l'intermédiaire 128 (25.2 g, 40.3 mmol), carbone quaternaire de configuration *(S)* avec un rendement de 38%.
RMN ¹H : (400 MHz, dmso-d6) δ ppm 7.3 (m, 5 H), 4 (m, 2 H), 3.6 (d, 1 H), 3.4 (d, 1 H), 3.35 (m, 2 H), 2.9 (m, 1H), 2.8 (dd, 1 H), 2.45 (dd, 1 H), 2.3 (m, 1 H), 1.9 (m, 4 H), 1.4 (m, 2 H), 1.38 (s, 18 H), 1.35 (s, 9 H), 1.2 (t, 3 H), 0.8 (3, 2 H).
IR (cm⁻¹) : 1760-1680 cm⁻¹ (C=O), 1124 cm⁻¹ (P=O), 1124 cm⁻¹(C-O-C).

### Intermédiaire 129: (3S)-3-{4-[Bis(tert-butoxycarbonyl)amino]butyl}-4-éthoxy-4-oxo-1,4-azaphosphinane-3-carboxylate de tert-butyle

Dans un ballon de 500 mL à température ambiante et sous flux argon sont introduits successivement de l'intermédiaire 128 (30.35 g, 48.6 mol), de l'éthanol (200 mL), du Pd/C (3.03 g, 10% massique) et de l'HCl à 37% (3 mL, 0.8eq). L'argon est ensuite remplacé par une atmosphère d'hydrogène. La réaction est suivie par LC/MS. Après 4h, la réaction est totale et le catalyseur est filtré sur fibre de verre. Le filtrat est évaporé à sec afin d'obtenir une huile jaune qui est reprise par de l'AcOEt (200 mL) et par une solution de NaHCO₃ à 10% (200 mL). Après décantation, la phase aqueuse est extraite avec de l'AcOEt (3 x 100 mL). Les phases organiques sont réunies puis lavées avec une solution saturée en NaCl (400 mL), séchées sur MgSO₄ et concentrées pour conduire à l'intermédiaire 129 sous forme d'un solide blanc (23.12 g, 43.24 mmol) avec un rendement de 89%.
RMN ¹H : (DMSO-d₆, 400 MHz) δ 4.01 à 3.88 (m, 2 H), 3.47 (m, 2 H), 2.95-2.68 (2m, 2 H), 2.95 (m, 2 H), 2.23 (m, 1 H), 1.92-1.68 (m, 4 H), 1.48 (m, 2 H), 1.44 (s, 9 H), 1.41 (s, 9 H), 1.3 (s, 9 H), 1.27/0.97 (2m, 2 H), 1.24/0.97 (2t, 3 H).
IR (cm⁻¹) : 1715-1692 cm⁻¹ (C=O), 1125 cm⁻¹(C-O-C).

### EXEMPLE 177: Acide (3S)-3-(4-aminobutyl)-1-benzyl-4-hydroxy-4-oxo-1,4-azaphosphinane-3-carboxylique

L'exemple 177 est obtenu à partir de l'intermédiaire 128 selon la procédure D précédemment décrite.
RMN ¹H : (400 MHz, D2O) δ ppm 7.49 (m, 5 H), 4.41/4.21 (2d, 2 H), 3.7/3.32 (2m, 2 H), 3.5/3.1 (2m, 2 H), 2.91 (m, 2 H), 2.22/1.78 (2m, 2 H), 1.92 (m, 1 H), 1.6 (m, 2 H), 1.45 (m, 1 H), 1.22/1.1 (2m, 2 H)
ESI/FIA/HR et MS/MS : [M+H]+ = 341.1638 (341.1630)
Analyse Elémentaire : C=56.43(56.46);H=7.33(7.40);N=8.20(8.23)
PR: -45.630 (589 nm,T=20°C,C=1.1)

### Intermédiaire 131: (3S)-3-{4-[Bis(tert-butoxycarbonyl)amino]butyl}-1-[[2-(3,4-diméthoxyphényl)-4-fluorophényl]méthyl]-4-éthoxy-4-oxo-1,4-azaphosphinane-3-carboxylate de tert-butyle

L'intermédiaire 131 est obtenu à partir des intermédiaires 129 et 229 selon la procédure F précédemment décrite.
RMN ¹H : (400 MHz, dmso-d6) δ ppm 7.5 (dd, 1 H), 7.15 (m, 1 H), 7.05 (m, 2 H), 6.9 (m, 2 H), 3.95 (m, 2 H), 3.8 (2s, 6 H), 3.55 (d, 1 H), 3.35 (d, 1 H), 3.3 (m, 2 H), 2.85-2.65 (m, 2 H), 2.35 (dd, 1 H), 2.25 (m, 1 H), 2-1.75 (m, 4 H), 1.45-1.3 (m, 2 H), 1.4 (s, 18 H), 1.35 (s, 9 H), 1.2 (t, 3 H), 0.8-0.6 (m, 2 H).

### EXEMPLE 179: Acide (3S)-3-(4-aminobutyl)-1-[[2-(3,4-diméthoxyphényl)-4-fluorophényl]méthyl]-4-hydroxy-4-oxo-1,4-azaphosphinane-3-carboxylique

L'exemple 179 est obtenu à partir de l'intermédiaire 131 selon la procédure D précédemment décrite.
RMN ¹H : (300 MHz, D2O) δ ppm 7.6 (dd, 1 H), 7.2 (td, 1 H), 7.1 (m, 1 H), 7 (dd, 1 H), 6.9 (dd, 1 H), 4.4/4.25 (2 d, 2 H), 3.8 (2 s, 6 H), 3.35 (m, 1 H), 3.2-2.75 (m, 5 H), 2.05 (m, 1 H), 1.8 (m, 1 H), 1.6 (m, 3 H), 1.3 (m, 1 H), 1 (m, 2 H)
ESI/FIA/HR et MS/MS : [M+H]+ = 495.2056 (495.2060)
Analyse Elémentaire : C=58.64(58.29);H=6.44(6.52);N=5.72(5.67)
PR : -23.170 (589 nm, T=21°C, C=0.8)

### Intermédiaire 132: (3S)-3-14-[Bis(tert-butoxycarbonyl)amino]butyl}-4-éthoxy-1-[[4-fluoro-2-(4-méthylphényl)phényl]méthyl]-4-oxo-1,4-azaphosphinane-3-carboxylate de tert-butyle

L'intermédiaire 132 est obtenu à partir des intermédiaires 129 et 272 selon la procédure F précédemment décrite.
RMN ¹H : (400 MHz, dmso-d6) δ ppm 7.51 (dd, 1 H), 7.26 (s, 4 H), 7.18 (td, 1 H), 7.01 (dd, 1 H), 3.93 (m, 2 H), 3.42 (AB, 2 H), 3.34 (m, 2 H), 2.72/2.2 (2m, 2 H), 2.72/2.32 (2m, 2 H), 2.36 (s, 3 H), 1.9-1.7 (m, 4 H), 1.41 (s, 18 H), 1.37 (m, 2 H), 1.34 (s, 9 H), 1.17 (t, 3 H), 0.7 (m, 2 H)

### EXEMPLE 180: Acide (3S)-3-(4-aminobutyl)-1-[[4-fluoro-2-(4-méthylphényl)phényl]-méthyl]-4-hydroxy-4-oxo-1,4-azaphosphinane-3-carboxylique

L'exemple 180 est obtenu à partir de l'intermédiaire 132 selon la procédure D précédemment décrite.
RMN ¹H : (400 MHz, D2O) δ ppm 7.61 (dd, 1 H), 7.36 (d, 2 H), 7.26 (d, 2 H), 7.23 (td, 1 H), 7.15 (dd, 1 H), 4.41/4.26 (2*d, 2 H), 3.35/3.09 (m, 2 H), 3.17/2.81 (m, 2 H), 2.95 (m, 2 H), 2.37 (s, 3 H), 2.08/1.64 (m, 2 H), 1.85/1.35 (m, 2 H), 1.6 (m, 2 H), 1.13/1.05 (m, 2 H)
ESI/FIA/HR et MS/MS : [M+H]+ = 449.2005 (449.2005)
Analyse Elémentaire : C=61.57(61.60);H=6.53(6.74);N=6.45(6.25)
PR : -15.640 (589 nm, T=20°C, C=1.0)

### Intermédiaire 133: (3S)-3-{4-[Bis(tert-butoxycarbonyl)amino]butyl}-4-éthoxy-4-oxo-1-[(2-phénylphényl)méthyl]-1,4-azaphosphinane-3-carboxylate de tert-butyle

L'intermédiaire 133 est obtenu à partir de l'intermédiaire 129 et du 2-phénylbenzaldéhyde selon la procédure F précédemment décrite.
RMN ¹H : (400 MHz, dmso-d6) δ ppm 7.52-7.2 (m, 9 H), 4.06-3.86 (m, 2 H), 3.55/3.36 (AB, 2 H), 3.36 (m, 2 H), 2.71/2.32/2.19 (3m, 4 H), 1.95-1.65 (m, 4 H), 1.42/1.34 (2s, 27 H), 1.37 (m, 2 H), 1.21/1.17 (2t, 3 H), 0.68 (m, 2 H)

### EXEMPLE 181: Acide (3S)-3-(4-aminobutyl)-4-hydroxy-4-oxo-1-[(2-phénylphényl)méthyl]-1,4-azaphosphinane-3-carboxylique

L'exemple 181 est obtenu à partir de l'intermédiaire 133 selon la procédure D précédemment décrite.
RMN ¹H : (400 MHz, dmso-d6) δ ppm 7.65-7.3 (m, 9 H), 4.3 (AB, 2 H), 3.43/3 (m, 3 H), 2.92 (dd, 1 H), 2.82 (m, 2 H), 2.06-1.57 (2m, 2 H), 1.83/1.33 (2m, 2 H) 1.57 (m, 2 H), 1.08 (m, 2 H)
ESI/FIA/HR et MS/MS : [M+H]+ = 417.1938 (417.1943)
Analyse Elémentaire : C=62.99(63.45);H=6.45(7.02);N=6.93(6.73)

### Intermédiaire 134: (3S)-3-{4-[Bis(tert-butoxycarbonyl)amino]butyl}-4-éthoxy-4-oxo-1-[(2-pyrimidin-5-ylphényl)méthyl]-1,4-azaphosphinane-3-carboxylate de tert-butyle

L'intermédiaire 134 est obtenu à partir des intermédiaires 129 et 236 selon la procédure F précédemment décrite.
RMN ¹H : (300 MHz, dmso-d6) δ ppm 9.2 (s, 1 H), 8.85 (s, 2 H), 7.45 (m, 3 H), 7.35 (d, 1 H), 3.95 (m, 2 H), 3.55 (d, 1 H), 3.4 (d, 1 H), 3.3 (m, 2 H), 2.8-2.55 (m, 2 H), 2.45-2.15 (2*m, 2 H), 2.15-1.7 (m, 2 H), 2-1.55 (m, 4 H), 1.5-1.25 (3s, 27 H), 1.2 (t, 3 H), 0.6 (m, 2 H)

### EXEMPLE 182: Acide (3S)-3-(4-aminobutyl)-4-hydroxy-4-oxo-1-[(2-pyrimidin-5-ylphényl)méthyl]-1,4-azaphosphinane-3-carboxylique

L'exemple 182 est obtenu à partir de l'intermédiaire 134 selon la procédure D précédemment décrite.
RMN ¹H : (400 MHz, D2O) δ ppm 9.15 (s, 1 H), 8.82 (s, 2 H), 7.7-7.55 (m, 3 H), 7.43 (d, 1 H), 4.4/4.3 (2d, 2 H), 3.6-3.35 (m, 1 H), 3.3-3.1 (m, 1 H), 3.1 (m, 1 H), 2.95 (m, 3 H), 2.1 (m, 1 H), 1.85 (m, 1 H), 1.65 (m, 1 H), 1.62-1.45 (m, 2 H), 1.35 (m, 1 H), 1.15-1 (m, 2 H)
ESI/FIA/HR et MS/MS : [M+H]+ = 419.1855 (419.1848)
Analyse Elémentaire : C=57.01(57.41);H=6.57(6.50);N=13.45(13.39)
PR: -20.520 (589 nm, T=20°C, C=1.0)

### Intermédiaire 135: (3S)-3-{4-[Bis(tert-butoxycarbonyl)amino]butyl}-4-éthoxy-4-oxo-1-[(2-thiophen-2-ylphényl)méthyl]-1,4-azaphosphinane-3-carboxylate de tert-butyle

L'intermédiaire 135 est obtenu à partir de l'intermédiaire 129 et du 2-(2-thiényl)benzaldéhyde selon la procédure F précédemment décrite.
RMN ¹H : (300 MHz, dmso-d6) δ ppm 7.62 (d, 1 H), 7.45-4.3 (m, 4 H), 7.25 (d, 1 H), 7.15 (t, 1 H), 4.1-3.9 (quad., 2 H), 3.65/3.45 (d, 2 H), 3.4-3.2 (m, 2 H), 2.9-2.7 (m, 2 H), 2.4-2.3 (m, 2 H), 1.9-1.7 (m, 4 H), 1.5-1.3 (m, 2 H), 1.4/1.35 (2*s, 27 H), 1.2 (t, 3 H), 0.7-0.5 (m, 2 H)

### EXEMPLE 183: Acide (3S)-3-(4-aminobutyl)-4-hydroxy-4-oxo-1-[(2-thiophen-2-ylphényl)méthyl]-1,4-azaphosphinane-3-carboxylique

L'exemple 183 est obtenu à partir de l'intermédiaire 135 selon la procédure D précédemment décrite.
RMN ¹H : (400 MHz, D2O) δ ppm 7.6 (d, 1 H), 7.55 (d, 1 H), 7.48 (m, 2 H), 7.45 (d, 1 H), 7.15 (dd, 1 H), 7.1 (t, 1 H), 4.5/1.4 (2*d, 2 H), 3.5-3.2 (m, 3 H), 3-2.85 (m, 3 H), 2.15/1.7 (m, 4 H), 1.88 (m, 1 H), 1.55 (m, 2 H), 1.2-1 (m, 2 H)
ESI/FIA/HR et MS/MS : [M+H]+ = 423.1505 (423.1507)
Analyse Elémentaire : C=57.14(56.86);H=6.12(6.44);N=6.61(6.63);S=7.33(7.59)
PR : -18.340 (589 nm, T=20°C, C=0.9)

### Intermédiaire 136: (3S)-3-{4-[Bis(tert-butoxtcarbonyl)amino]butyl}-4-éthoxy-4-oxo-1-[(2-pyridin-3-ylphényl)méthyl]-1,4-azaphosphinane-3-carboxylate de tert-butyle

L'intermédiaire 136 est obtenu à partir des intermédiaires 129 et 260 selon la procédure F précédemment décrite.
RMN ¹H : (400 MHz, dmso-d6) δ ppm 8.59 (dd, 1 H), 8.56 (dd, 1 H), 7.82 (dt, 1 H), 7.49 (dd, 1 H), 7.48 (ddd, 1 H), 7.42/7.39 (2td, 2 H), 7.27 (dd, 1 H), 3.92 (m, 2 H), 3.44 (AB, 2 H), 3.3 (m, 2 H), 2.7/2.2 (2m, 2 H), 2.7/2.35 (2m, 2 H), 1.95-1.66 (m, 4 H), 1.42 (s, 18 H), 1.35 (m, 2 H), 1.33 (s, 9 H), 1.17 (t, 3 H), 0.63 (m, 2 H)

### EXEMPLE 184: Acide (3S)-3-(4-aminobutyl)-4-hydroxy-4-oxo-1-[(2-pyridin-3-ylphényl)méthyl]-1,4-azaphosphinane-3-carboxylique

L'exemple 184 est obtenu à partir de l'intermédiaire 136 selon la procédure D précédemment décrite.
RMN ¹H : (400 MHz, dmso-d6) δ ppm 8.57 (d, 1 H), 8.48 (s, 1 H), 7.83 (d, 1 H), 7.65 (m, 1 H), 7.57 (m, 1 H), 7.57 (m, 2 H), 7.4 (m, 1 H), 4.41/4.29 (dd, 2 H), 3.44/3.12 (dd, 2 H), 3.2/2.86 (dd, 2 H), 2.93 (m, 2 H), 2.1/1.67 (2*m, 2 H), 1.85/1.35 (2*m, 2 H), 1.59 (m, 2 H), 1.08 (m, 2 H)
ESI/FIA/HR et MS/MS : [M+H]+ = 418.1871 (418.1895)
Analyse Elémentaire : C=60.35(60.42);H=6.74(6.76);N=9.70(10.07)
PR: -20.940 (589 nm, T=28°C, C=0.9)

### Intermédiaire 137: (3S)-3-{4-[Bis(tert-butoxycarbonyl)amino]butyl}-4-éthoxy-1-[[4-fluoro-2-(3-méthoxyphényl)phényl]méthyl]-4-oxo-1,4-azaphosphinane-3-carboxylate de tert-butyle

L'intermédiaire 137 est obtenu à partir des intermédiaires 129 et 222 selon la procédure F précédemment décrite.
RMN ¹H : (400 MHz, dmso-d6) δ ppm 7.51 (dd, 1 H), 7.36 (t, 1 H), 7.2 (td, 1 H), 7.05 (dd, 1 H), 6.97 (d, 1 H), 6.91 (m, 1 H), 6.9 (m, 1 H), 3.93 (m, 2 H), 3.79 (s, 3 H), 3.52/3.33 (2*d, 2 H), 3.33 (m, 2 H), 2.72/2.21 (m, 2 H), 2.72/2.32 (m, 2 H), 2-1.75 (m, 4 H), 1.4 (s, 18 H), 1.37 (m, 2 H), 1.34 (s, 9 H), 1.17 (t, 3 H), 0.69 (m, 2 H)

### EXEMPLE 185: Acide (3S)-3-(4-aminobutyl)-1-[[4-fluoro-2-(3-méthoxyphényl)phényl]méthyl]-4-hydroxy-4-oxo-1,4-azaphosphinane-3-carboxylique

L'exemple 185 est obtenu à partir de l'intermédiaire 137 selon la procédure D précédemment décrite.
RMN ¹H : (400 MHz, D2O) δ ppm 7.62 (dd, 1 H), 7.46 (t, 1 H), 7.25 (td, 1 H), 7.16 (dd, 1 H), 7.08 (dd, 1 H), 6.97 (dl, 1 H), 6.96 (si, 1 H), 4.41/4.27 (2*d, 2 H), 3.84 (s, 3 H), 3.37/3.09 (m, 2 H), 3.16/2.83 (m, 2 H), 2.95 (m, 2 H), 2.09/1.66 (m, 2 H), 1.86/1.35 (m, 2 H), 1.66 (m, 2 H), 1.13/1.06 (m, 2 H)
ESI/FIA/HR et MS/MS : [M+H]+ = 465.1941 (465.1949)
Analyse Elémentaire : C=59.94(59.48);H=6.44(6.51);N=6.11(6.03)
PR : -19.240 (589 nm, T=20°C, C=0.6)

### Intermédiaire 138: (3S)-3-{4-[Bis(tert-butoxycarbonyl)amino]butyl}-1-[[3-(4-chlorophényl)pyridin-2-yl]méthyl]-4-éthoxy-4-oxo-1,4-azaphosphinane-3-carboxylate de tert-butyle

L'intermédiaire 138 est obtenu à partir des intermédiaires 129 et 291 selon la procédure F précédemment décrite.
RMN ¹H : (400 MHz, dmso-d6) δ ppm 8.55 (d, 1 H), 7.68 (d, 1 H), 7.55 (m, 4 H), 7.42 (dd, 1 H), 3.94 (m, 2 H), 3.63/3.44 (2*d, 2 H), 3.19 (m, 2 H), 2.98/2.51 (2*m, 2 H), 2.64/2.36 (2*m, 2 H), 1.91/1.75 (2*m, 2 H), 1.75/1.62 (2*m, 2 H), 1.4 (s, 18 H), 1.33 (s, 9 H), 1.23 (m, 2 H), 1.18 (t, 3 H), 0.59/0.34 (m, 2 H)

### EXEMPLE 186: Acide (3S)-3-(4-aminobutyl)-1-[[3-(4-chlorophényl)pyridin-2-yl]méthyl]-4-hydroxy-4-oxo-1,4-azaphosphinane-3-carboxylique

L'exemple 186 est obtenu à partir de l'intermédiaire 138 selon la procédure D précédemment décrite.
RMN ¹H : (400 MHz, D2O) δ ppm 8.6 (d, 1 H), 7.8 (d, 1 H), 7.5 (m, 3 H), 7.3 (d, 2 H), 4.4 (m, 2 H), 3.5-3.3 (m, 2 H), 3.2 (m, 2 H), 2.95 (m, 2 H), 2.2 (m, 1 H), 1.9 (m, 1 H), 1.65 (m, 1 H), 1.6 (m, 2 H), 1.45 (m, 1 H), 1.3-1.1 (m, 2 H)
ESI/FIA/HR et MS/MS : [M+H]+ = 452.1502 (452.1500)
Analyse Elémentaire : C=55.89(55.82);H=5.56(6.02);N=9.17(9.30)
PR: -5.260 (589 nm, T=20°C, C=1.0)

### Intermédiaire 139 : (3S)-3-{4-[Bis(tert-butoxycarbonyl)amino]butyl}-4-éthoxy-1-[[4-fluoro-2-(4-méthoxyphényl)phényl]méthyl]-4-oxo-1,4-azaphosphinane-3-carboxylate de tert-butyle

L'intermédiaire 139 est obtenu à partir des intermédiaires 129 et 221 selon la procédure F précédemment décrite.
RMN ¹H : (400 MHz, dmso-d6) δ ppm 7.49 (dd, 1 H), 7.31 (d, 2 H), 7.17 (m, 1 H), 7.01 (m, 1 H), 7.01 (d, 2 H), 3.93 (m, 2 H), 3.8 (s, 3 H), 3.5/3.32 (2*d, 2 H), 3.32 (m, 2 H), 2.75/2.21 (2*m, 2 H), 2.72/2.33 (2*m, 2 H), 2-1.78 (massif, 2 H), 2-1.78 (massif, 2 H), 1.4/1.33 (2*s, 27 H), 1.38 (m, 2 H), 1.18 (t, 3 H), 0.7 (m, 2 H)

### EXEMPLE 187: Acide (3S)-3-(4-aminobutyl)-1-[[4-fluoro-2-(4-méthoxyphényl)phényl]méthyl]-4-hydroxy-4-oxo-1,4-azaphosphinane-3-carboxylique

L'exemple 187 est obtenu à partir de l'intermédiaire 139 selon la procédure D précédemment décrite.
RMN ¹H : (300 MHz, D2O) δ ppm 7.57 (dd, 1 H), 7.3 (d, 2 H), 7.19 (td, 1 H), 7.12 (dd, 1 H), 7.08 (d, 2 H), 4.32 (AB, 2 H), 3.84 (s, 3 H), 3.33/3.07 (2m, 2 H), 3.13/2.8 (2m, 2 H), 2.92 (m, 2 H), 2.05/1.62 (2m, 2 H), 1.83/1.32 (2m, 2 H), 1.57 (m, 2 H), 1.05 (m, 2 H)
ESI/FIA/HR et MS/MS : [M+H]+ = 465.1960 (465.1954)
Analyse Elémentaire : C=59.41(59.48);H=6.76(6.51);N=6.09(6.03)
PR: -13.770 (589 nm, T=20°C, C=0.9)

### Intermédiaire 140: (3S)-3-{4-[Bis(tert-butoxycarbonyl)amino]butyl}-4-éthoxy-1-[[2-(4-méthylphényl)phényl]méthyl]-4-oxo-1,4-azaphosphinane-3-carboxylate de tert-butyle

L'intermédiaire 140 est obtenu à partir des intermédiaires 129 et 288 selon la procédure F précédemment décrite.
RMN ¹H : (400 MHz, dmso-d6) δ ppm 7.49 (dd, 1 H), 7.33 (2*m, 2 H), 7.23 (2*d, 4 H), 7.18 (dd, 1 H), 3.92 (m, 2 H), 3.54/3.35 (2*d, 2 H), 3.3 (m, 2 H), 2.74/2.2 (2*m, 2 H), 2.74/2.32 (2*m, 2 H), 2.36 (s, 3 H), 1.95-1.75 (m, 2 H), 1.95-1.75 (m, 2 H), 1.41/1.33 (2*s, 27 H), 1.38 (m, 2 H), 1.17 (t, 3 H), 0.71 (m, 2 H)

### EXEMPLE 188: Acide (3S)-3-(4-aminobutyl)-4-hydroxy-1-[[2-(4-méthylphényl)phényl]méthyl]-4-oxo-1,4-azaphosphinane-3-carboxylique

L'exemple 188 est obtenu à partir de l'intermédiaire 140 selon la procédure D précédemment décrite.
RMN ¹H : (300 MHz, D2O) δ ppm 7.6-7.35 (m, 4 H), 7.33/7.22 (2d, 4 H), 4.33 (AB, 2 H), 3.33/3.07 (2m, 2 H), 3.19/2.8 (2m, 2 H), 2.91 (m, 2 H), 2.35 (s, 3 H), 2.06/1.58 (2m, 2 H), 1.82/1.32 (2m, 2 H), 1.57 (m, 2 H), 1.07 (m, 2 H)
ESI/FIA/HR et MS/MS : [M+H]+ = 431.2097 (431.2099)
Analyse Elémentaire : C=64.03(64.17);H=6.92(7.26);N=6.45(6.51)
PR: -13.150 (589 nm, T=20°C, C=0.9)

### Intermédiaire 141: (3S)-3-{4-[Bis(tert-butoxycarbonyl)amino]butyl}-4-éthoxy-1-[[4-fluoro-2-(4-fluorophényl)phényl]méthyl]-4-oxo-1,4-azaphosphinane-3-carboxylate de tert-butyle

L'intermédiaire 141 est obtenu à partir des intermédiaires 129 et 271 selon la procédure F précédemment décrite.
RMN ¹H : (400 MHz, dmso-d6) δ ppm 7.49 (dd, 1 H), 7.42 (dd, 2 H), 7.29 (t, 2 H), 7.21 (td, 1 H), 7.06 (dd, 1 H), 3.92 (m, 2 H), 3.49/3.32 (2*d, 2 H), 3.32 (m, 2 H), 2.71/2.2 (2*m, 2 H), 2.7/2.34 (2*m, 2 H), 1.9-1.65 (m, 2 H), 1.9-1.65 (m, 2 H), 1.4/1.33 (2*s, 27 H), 1.38 (m, 2 H), 1.18 (t, 3 H), 0.67 (m, 2 H)

### EXEMPLE 189: Acide (3S)-3-(4-aminobutyl)-1-[[4-fluoro-2-(4-fluorophényl)phényl]-méthyl]-4-hydroxy-4-oxo-1,4-azaphosphinane-3-carboxylique

L'exemple 189 est obtenu à partir de l'intermédiaire 141 selon la procédure D précédemment décrite.
RMN ¹H : (300 MHz, D2O) δ ppm 7.59 (dd, 1 H), 7.35 (m, 2 H), 7.23 (td, 1 H), 7.23 (t, 2 H), 7.14 (dd, 1 H), 4.31 (AB, 2 H), 3.36/3.07 (2m, 2 H), 3.16/2.83 (2m, 2 H), 2.92 (m, 2 H), 2.06/1.58 (2m, 2 H), 1.84/1.33 (2m, 2 H), 1.58 (m, 2 H), 1.07 (m, 2 H)
RMN ¹⁹F : (300 MHz, D2O) δ ppm -111.4/-114.1
ESI/FIA/HR et MS/MS : [M+H]+ = 453.1750 (453.1754)
Analyse Elémentaire : C=58.26(58.40);H=5.79(6.01);N=6.18(6.19)
PR: -17.770 (589 nm, T=20°C, C=1.2)

### Intermédiaire 142: (3S)-3-{4-[Bis(tert-butoxycarbonyl)amino]butyl}-4-éthoxy-1-[(4-fluoro-2-pyridin-3-ylphényl)méthyl]-4-oxo-1,4-azaphosphinane-3-carboxylate de tert-butyle

L'intermédiaire 142 est obtenu à partir des intermédiaires 129 et 261 selon la procédure F précédemment décrite.
RMN ¹H : (400 MHz, dmso-d6) δ ppm 8.6 (dd, 1 H), 8.58 (dl, 1 H), 7.84 (dt, 1 H), 7.51 (m, 2 H), 7.26 (td, 1 H), 7.15 (dd, 1 H), 3.93 (m, 2 H), 3.42 (AB, 2 H), 3.33 (m, 2 H), 2.67/2.2 (2m, 2 H), 2.67/2.35 (2m, 2 H), 1.96-1.62 (m, 4 H), 1.41 (s, 18 H), 1.36 (m, 2 H), 1.34 (s, 9 H), 1.17 (t, 3 H), 0.65 (m, 2 H)

### EXEMPLE 190: Acide (3S)-3-(4-aminobutyl)-1-[(4-fluoro-2-pyridin-3-ylphényl)méthyl]-4-hydroxy-4-oxo-1,4-azaphosphinane-3-carboxylique

L'exemple 190 est obtenu à partir de l'intermédiaire 142 selon la procédure D précédemment décrite.
RMN ¹H : (300 MHz, D2O) δ ppm 8.62 (dd, 1 H), 8.54 (d, 1 H), 7.89 (dt, 1 H), 7.7 (dd, 1 H), 7.6 (dd, 1 H), 7.34 (td, 1 H), 7.23 (dd, 1 H), 4.35 (AB, 2 H), 3.46/3.12 (2m, 2 H), 3.18/2.87 (2m, 2 H), 2.97 (m, 2 H), 2.12/1.67 (2m, 2 H), 1.88/1.37 (2m, 2 H), 1.61 (m, 2 H), 1.11 (m, 2 H)
RMN ¹⁹F : (300 MHz, D2O) δ ppm -111
ESI/FIA/HR et MS/MS : [M+H]+ = 436.1794 (436.1801)
Analyse Elémentaire : C=57.69(57.93);H=5.69(6.25);N=9.60(9.65)
PR: -19.680 (589 nm, T=20°C, C=0.7)

### Intermédiaire 143: (3S)-3-{4-[Bis(tert-butoxycarbonyl)amino]butyl}-4-éthoxy-1-[(4-méthoxy-2-phénylphényl)méthyl]-4-oxo-1,4-azaphosphinane-3-carboxylate de tert-butyle

L'intermédiaire 143 est obtenu à partir des intermédiaires 129 et 285 selon la procédure F précédemment décrite.
RMN ¹H : (400 MHz, dmso-d6) δ ppm 7.47-7.32 (m, 5 H), 7.35 (d, 1 H), 6.95 (dd, 1 H), 6.75 (d, 1 H), 3.92 (quad., 2 H), 3.77 (s, 3 H), 3.47/3.27 (dd, 2 H), 3.32 (t, 2 H), 2.75/2.28 (dd, 2 H), 2.67/2.16 (dd, 2 H), 1.85/1.72 (dd, 2 H), 1.79 (t, 2 H), 1.41 (s, 18 H), 1.36 (m, 2 H), 1.34 (s, 9 H), 1.16 (t, 3 H), 0.68 (m, 2 H)

### EXEMPLE 191: Acide (3S)-3-(4-aminobutyl)-4-hydroxy-1-[(4-hydroxy-2-phénylphényl)méthyl]-4-oxo-1,4-azaphosphinane-3-carboxylique

L'exemple 191 est obtenu à partir de l'intermédiaire 143 selon la procédure D précédemment décrite.
RMN ¹H : (400 MHz, D2O) δ ppm 7.54-7.44 (m, 3 H), 7.54-7.44 (m, 1 H), 7.34 (d, 2 H), 6.97 (dd, 1 H), 6.86 (df, 1 H), 4.34/4.19 (2*d, 2 H), 3.33/3.05 (2*m, 2 H), 3.19/2.78 (2*m, 2 H), 2.95 (m, 2 H), 2.07/1.63 (2*m, 2 H), 1.85/1.35 (2*m, 2 H), 1.59 (m, 2 H), 1.2-1 (2*m, 2 H)
ESI/FIA/HR et MS/MS : [M+H]+ = 433.1887 (433.1887)
Analyse Elémentaire : C=60.81(61.10);H=6.31(6.76);N=6.49(6.48)
PR : -39.130 (589 nm, T=20°C, C=0.9)

### Intermédiaire 144: (3S)-3-{4-[Bis(tert-butoxycarbonyl)amino]butyl}-4-éthoxy-1-[(4-fluoro-2-pyridin-4-ylphényl)méthyl]-4-oxo-1,4-azaphosphinane-3-carboxylate de tert-butyle

L'intermédiaire 144 est obtenu à partir des intermédiaires 129 et 262 selon la procédure F précédemment décrite.
RMN ¹H : (400 MHz, dmso-d6) δ ppm 8.65 (m, 2 H), 7.53 (dd, 1 H), 7.44 (m, 2 H), 7.28 (td, 1 H), 7.14 (dd, 1 H), 3.93 (m, 2 H), 3.44 (AB, 2 H), 3.32 (m, 2 H), 2.69/2.21 (2m, 2 H), 2.69/2.35 (2m, 2 H), 1.96-1.62 (m, 4 H), 1.41 (s, 18 H), 1.36 (m, 2 H), 1.33 (s, 9 H), 1.17 (t, 3 H), 0.66/0.58 (2m, 2 H)

### EXEMPLE 192: Acide (3S)-3-(4-aminobutyl)-1-[(4-fluoro-2-pyridin-4-ylphényl)méthyl]-4-hydroxy-4-oxo-1,4-azaphosphinane-3-carboxylique

L'exemple 192 est obtenu à partir de l'intermédiaire 144 selon la procédure D précédemment décrite.
RMN ¹H : (400 MHz, D2O) δ ppm 8.63 (d, 2 H), 7.67 (dd, 1 H), 7.45 (d, 2 H), 7.33 (td, 1 H), 7.21 (dd, 1 H), 4.4/4.29 (2*d, 2 H), 3.44/3.08 (m, 2 H), 3.16/2.88 (m, 2 H), 2.95 (m, 2 H), 2.1/1.65 (m, 2 H), 1.86/1.36 (m, 2 H), 1.6 (m, 2 H), 1.08 (m, 2 H)
ESI/FIA/HR et MS/MS : [M+H]+ = 436.1804 (436.1801)
Analyse Elémentaire : C=58.11(57.93);H=5.71(6.25);N=9.79(9.65)
PR : -16.250 (589 nm, T=20°C, C=1.0)

### Intermédiaire 146: (3S)-3-{4-[Bis(tert-butoxycarbonyl)amino]butyl}-1-[[2-(3-chlorophényl)phényl]méthyl]-4-éthoxy-4-oxo-1,4-azaphosphinane-3-carboxylate de tert-butyle

L'intermédiaire 146 est obtenu à partir des intermédiaires 129 et 213 selon la procédure F précédemment décrite.
RMN ¹H : (400 MHz, dmso-d6) δ ppm 7.52 (si, 1 H), 7.45 (m, 2 H), 7.45/7.38/7.25 (3m, 4 H), 7.33 (dt, 1 H), 3.94 (m, 2 H), 3.53/3.32 (AB, 2 H), 3.25 (m, 2 H), 2.76/2.37 (2m, 2 H), 2.7/2.25 (2m, 2 H), 1.95/1.78 (2m, 2 H), 1.82/1.75 (2m, 2 H), 1.41 (s, 18 H), 1.33 (s, 9 H), 1.33 (m, 2 H), 1.18 (t, 3 H), 0.66/0.57 (2m, 2 H)

### EXEMPLE 194: Acide (3S)-3-(4-aminobutyl)-1-[[2-(3-chlorophényl)phényl]méthyl] -4-hydroxy-4-oxo-1,4-azaphosphinane-3-carboxylique

L'exemple 194 est obtenu à partir de l'intermédiaire 146 selon la procédure D précédemment décrite.
RMN ¹H : (300 MHz, D2O) δ ppm 7.63-7.23 (m, 8 H), 4.33 (AB, 2 H), 3.42/3.1 (2m, 2 H), 3.17/2.82 (2m, 2 H), 2.93 (m, 2 H), 2.1/1.63 (2m, 2 H), 1.84/1.34 (2m, 2 H), 1.57 (m, 2 H), 1.07 (m, 2 H)
ESI/FIA/HR et MS/MS : [M+H]+ = 451.1539 (451.1548)
Analyse Elémentaire : C=58.76(58.60);H=6.46(6.26);N=6.38(6.21)
PR : -15.780 (589 nm, T=20°C, C=1.2)

### Intermédiaire 147: (3S)-3-{4-[Bis(tert-butoxycarbonyl)amino]butyl}-1-[[4-chloro-2-(4-chlorophényl)phényl]méthyl]-4-éthoxy-4-oxo-1,4-azaphosphinane-3-carboxylate de tert-butyle

L'intermédiaire 147 est obtenu à partir des intermédiaires 129 et 292 selon la procédure F précédemment décrite.
RMN ¹H : (400 MHz, dmso-d6) δ ppm 7.52 (d, 2 H), 7.5 (d, 1 H), 7.45 (dd, 1 H), 7.41 (d, 2 H), 7.27 (d, 1 H), 3.94 (m, 2 H), 3.5/3.33 (dd, 2 H), 3.3 (m, 2 H), 2.8-2.6 (2m, 2 H), 2.35 (dd, 1 H), 2.2 (m, 1 H), 2-1.7 (m, 4 H), 1.41 (s, 18 H), 1.4-1.3 (m, 2 H), 1.33 (s, 9 H), 1.18 (t, 3 H), 0.68 (m, 2 H)

### EXEMPLE 195: Acide (3S)-3-(4-aminobutyl)-1-[[4-chloro-2-(4-chlorophényl)phényl]méthyl]-4-hydroxy-4-oxo-1,4-azaphosphinane-3-carboxylique

L'exemple 195 est obtenu à partir de l'intermédiaire 147 selon la procédure D précédemment décrite.
RMN ¹H : (300 MHz, D2O) δ ppm 7.55-7.5 (2d, 2 H), 7.5 (d, 2 H), 7.4 (d, 1 H), 7.28 (d, 2 H), 4.3 (dd, 2 H), 3.35/3.05 (2m, 2 H), 3.15/2.8 (2m, 2 H), 2.9 (m, 2 H), 2.05/1.65 (2m, 2 H), 1.8/1.3 (2m, 2 H), 1.55 (m, 2 H), 1.05 (m, 2 H)
ESI/FIA/HR et MS/MS : [M+H]+ = 485.1162 (485.1163)
Analyse Elémentaire : C=55.11(54.44);H=5.26(5.61);N=5.87(5.77)
PR : -17.410 (589 nm, T=19°C, C=1.0)

### Intermédiaire 148: (3S)-3-{4-[Bis(tert-butoxycarbonyl)amino]butyl}-4-éthoxy-1-[2-(6-méthoxypyridin-3-yl)benzyl]-4-oxo-1,4-azaphosphinane-3-carboxylate de tert-butyle

L'intermédiaire 148 est obtenu à partir des intermédiaires 129 et 237 selon la procédure F précédemment décrite.
RMN ¹H : (400 MHz, dmso-d6) δ ppm 8.1 (d, 1 H), 7.75 (dd, 1 H), 7.5-7.2 (m, 4 H), 6.9 (d, 1 H), 3.9 (s, 3 H), 3.9 (m, 2 H), 3.55/3.35 (dd, 2 H), 3.3 (m, 2 H), 2.75/2.2 (2m, 2 H), 2.7/2.35 (2dd, 2 H), 1.9/1.8 (2m, 2 H), 1.8 (m, 2 H), 1.4 (s, 18 H), 1.35 (m, 2 H), 1.35 (s, 9 H), 1.2 (t, 3 H), 0.65 (m, 2 H)

### EXEMPLE 196: Acide (3S)-3-(4-aminobutyl)-4-hydroxy-1-[2-(6-méthoxypyridin-3-yl)benzyl]-4-oxo-1,4-azaphosphinane-3-carboxylique

L'exemple 196 est obtenu à partir de l'intermédiaire 148 selon la procédure D précédemment décrite.
RMN ¹H : (400 MHz, D2O) δ ppm 8.05 (d, 1 H), 7.75 (dd, 1 H), 7.6 (m, 1 H), 7.5 (m, 2 H), 7.4 (m, 1 H), 7 (d, 1 H), 4.3 (dd, 2 H), 3.9 (s, 3 H), 3.45/3.15 (2m, 2 H), 3.2/2.85 (2dd, 2 H), 2.9 (m, 2 H), 2.1/1.7 (2m, 2 H), 1.8/1.35 (2m, 2 H), 1.6 (m, 2 H), 1.1 (m, 2 H)
ESI/FIA/HR et MS/MS : [M+H]+ = 448.2009 (448.2001)
Analyse Elémentaire : C=58.81(59.05);H=6.79(6.76);N=9.31(9.39)
PR : -11.510 (589 nm, T=19°C, C=0.9)

### Intermédiaire 149: (3S)-3-{4-[Bis(tert-butoxycarbonyl)amino]butyl}-4-éthoxy-1-[[2-(4-fluorophényl)phényl]méthyl]-4-oxo-1,4-azaphosphinane-3-carboxylate de tert-butyle

L'intermédiaire 149 est obtenu à partir de l'intermédiaire 129 et du 2-(4-fluorophényl)benzaldéhyde selon la procédure F précédemment décrite.
RMN ¹H : (400 MHz, dmso-d6) δ ppm 7.47 (dd, 1 H), 7.39/7.33 (2*m, 2 H), 7.39 (dd, 2 H), 7.27 (t, 2 H), 7.21 (dd, 1 H), 3.93 (m, 2 H), 3.52/3.34 (2*d, 2 H), 3.38-3.22 (m, 2 H), 2.75/2.21 (2*m, 2 H), 2.7/2.35 (2*m, 2 H), 1.98-1.72 (m, 2 H), 1.98-1.72 (m, 2 H), 1.41/1.34 (2*s, 27 H), 1.37 (m, 2 H), 1.19 (t, 3 H), 0.68 (m, 2 H)

### EXEMPLE 197: Acide (3S)-3-(4-aminobutyl)-1-[[2-(4-fluorophényl)phényl]méthyl]-4-hydroxy-4-oxo-1,4-azaphosphinane-3-carboxylique

L'exemple 197 est obtenu à partir de l'intermédiaire 149 selon la procédure D précédemment décrite.
RMN ¹H : (400 MHz, D2O) δ ppm 7.6 (d, 1 H), 7.52 (m, 2 H), 7.4 (d, 1 H), 7.35 (dd, 2 H), 7.24 (dd, 2 H), 4.41/4.29 (dd, 2 H), 3.39/3.1 (2*m, 2 H), 3.19/2.88 (2*m, 2 H), 2.93 (m, 2 H), 2.09/1.65 (2*m, 2 H), 1.85/1.59 (2*m, 2 H), 1.59/1.35 (2*m, 2 H), 1.2-1 (m, 2 H)
ESI/FIA/HR et MS/MS : [M+H]+ = 435.1843 (435.1843)
Analyse Elémentaire : C=60.70(60.82);H=6.56(6.50);N=6.49(6.45)
PR : -23.350 (589 nm, T=20°C, C=0.7)

### Intermédiaire 150: (3S)-3-{4-[Bis(tert-butoxycarbonyl)amino]butyl}-1-[[2-(4-chlorophényl)-4-fluorophényl]méthyl]-4-éthoxy-4-oxo-1,4-azaphosphinane-3-carboxylate de tert-butyle

L'intermédiaire 150 est obtenu à partir des intermédiaires 129 et 220 selon la procédure F précédemment décrite.
RMN ¹H : (400 MHz, dmso-d6) δ ppm 7.52 (d, 2 H), 7.5 (m, 1 H), 7.42 (d, 2 H), 7.22 (dt, 1 H), 7.07 (dd, 1 H), 4-3.86 (m, 2 H), 3.5/3.32 (2*d, 2 H), 3.4-3.25 (m, 2 H), 2.71/2.21 (2*m, 2 H), 2.71/2.35 (2*m, 2 H), 1.99-1.72 (m, 2 H), 1.99-1.72 (m, 2 H), 1.41/1.33 (2*s, 27 H), 1.38 (m, 2 H), 1.19 (t, 3 H), 0.68 (m, 2 H)

### EXEMPLE 198: Acide (3S)-3-(4-aminobutyl)-1-[[2-(4-chlorophényl)-4-fluorophényl]méthyl]-4-hydroxy-4-oxo-1,4-azaphosphinane-3-carboxylique

L'exemple 198 est obtenu à partir de l'intermédiaire 150 selon la procédure D précédemment décrite.
RMN ¹H : (400 MHz, D2O) δ ppm 7.62 (dd, 1 H), 7.54 (d, 2 H), 7.35 (d, 2 H), 7.25 (td, 1 H), 7.17 (dd, 1 H), 4.39/4.28 (2*d, 2 H), 3.4/3.09 (2*m, 2 H), 3.13/2.84 (2*m, 2 H), 2.95 (m, 2 H), 2.09/1.65 (2*m, 2 H), 1.86/1.36 (2*m, 2 H), 1.6 (m, 2 H), 1.09 (m, 2 H)
ESI/FIA/HR et MS/MS : [M+H]+ = 469.1452 (469.1454)
Analyse Elémentaire : C=56.19(56.35);H=5.57(5.80);N=5.97(5.97)
PR : -12.560 (589 nm, T=20°C, C=0.7)

### Intermédiaire 151: (3S)-3-{4-[Bis(tert-butoxycarbonyl)amino]butyl}-4-éthoxy-1-[(2-naphthalen-1-ylphényl)méthyl]-4-oxo-1,4-azaphosphinane-3-carboxylate de tert-butyle

L'intermédiaire 151 est obtenu à partir des intermédiaires 129 et 228 selon la procédure F précédemment décrite.
RMN ¹H : (400 MHz, dmso-d6) δ ppm 8 (m, 2 H), 7.65-7.2 (m, 9 H), 3.85 (m, 2 H), 3.4 (m, 2 H), 3.35-3 (2dd, 2 H), 2.65/2.2 (2m, 2 H), 2.5/2 (2m, 2 H), 1.9-1.5 (m, 4 H), 1.4 (2s, 18 H), 1.35 (m, 2 H), 1.3 (2s, 9 H), 1.1 (t, 3 H), 0.65 (m, 2 H)

### EXEMPLE 199: Acide (3S)-3-(4-aminobutyl)-4-hydroxy-1-[(2-naphthalen-1-ylphényl)méthyl]-4-oxo-1,4-azaphosphinane-3-carboxylique

L'exemple 199 est obtenu à partir de l'intermédiaire 151 selon la procédure D précédemment décrite.
RMN ¹H : (400 MHz, D2O) δ ppm 8 (m, 2 H), 7.75-7.3 (m, 9 H), 4.35-3.8 (2dd, 2 H), 3.4/2.95 (2m, 2 H), 3.2/2.7 (2m, 2 H), 2.9 (m, 2 H), 2.05/1.55 (2m, 2 H), 1.75/1.25 (2m, 2 H), 1.5 (m, 2 H), 1.2-0.7 (m, 2 H)
ESI/FIA/HR et MS/MS : [M+H]+ = 467.2109 (467.2099)
Analyse Elémentaire : C=66.50(66.94);H=6.26(6.70);N=6.08(6.00)
PR : -25.420 (589 nm, T=19°C, C=1.0)

### Intermédiaire 152: (3S)-3-{4-[Bis(tert-butoxycarboxy)amino]butyl}-1-[(2-tert-butylphényl)méthyl]-4-éthoxy-4-oxo-1,4-azaphosphinane-3-carboxylate de tert-butyle

L'intermédiaire 152 est obtenu à partir de l'intermédiaire 129 et du 2-*tert*-butylbenzaldéhyde selon la procédure F précédemment décrite.
RMN ¹H : (400 MHz, dmso-d6) δ ppm 7.6-7.15 (3m, 4 H), 4 (m, 2 H), 3.75 (dd, 2 H), 3.45-3.3 (m, 2 H), 3-2.75 (2m, 2 H), 2.5 (dd, 2 H), 2.4 (m, 2 H), 2-1.85 (m, 2 H), 1.4 (m, 2 H), 1.4 (s, 18 H), 1.4 (t, 9 H), 1.35 (s, 9 H), 1.2 (t, 3 H), 0.95-0.7 (m, 2 H)

### EXEMPLE 200: Acide (3S)-3-(4-aminobutyl)-1-[(2-tert-butylphényl)méthyl]-4-hydroxy-4-oxo-1,4-azaphosphinane-3-carboxylique

L'exemple 200 est obtenu à partir de l'intermédiaire 152 selon la procédure D précédemment décrite.
RMN ¹H : (300 MHz, D2O) δ ppm 7.57/7.46 (2m, 2 H), 7.36 (m, 2 H), 4.65 (AB, 2 H), 3.68/3.41 (2m, 2 H), 3.46/3.22 (2m, 2 H), 2.89 (m, 2 H), 2.25/1.75 (2m, 2 H), 1.9/1.46 (2m, 2 H), 1.56 (quint., 2 H), 1.35 (s, 9 H), 1.13 (m, 2 H)
ESI/FIA/HR et MS/MS : [M+H]+ = 397.2253 (397.2256)
Analyse Elémentaire : C=61.19(60.59);H=8.40(8.39);N=7.29(7.07)
PR : -41.120 (589 nm, T=19°C, C=0.9)

### Intermédiaire 153: (3S)-3-{4-[Bis(tert-butoxycarbonyl)amino]butyl}-4-éthoxy-1-[[4-fluoro-2-(4-fluoro-3-méthoxyphényl)phényl]méthyl]-4-oxo-1,4-azaphosphinane-3-carboxylate de tert-butyle

L'intermédiaire 153 est obtenu à partir des intermédiaires 129 et 273 selon la procédure F précédemment décrite.
RMN ¹H : (400 MHz, dmso-d6) δ ppm 7.51 (dd, 1 H), 7.28 (dd, 1 H), 7.21 (dt, 1 H), 7.09 (m, 1 H), 7.09 (m, 1 H), 6.93 (m, 1 H), 3.93 (m, 2 H), 3.88 (s, 3 H), 3.52/3.35 (2*d, 2 H), 3.4-3.25 (m, 2 H), 2.72/2.21 (2*m, 2 H), 2.72/2.34 (2*m, 2 H), 1.9-1.65 (m, 2 H), 1.9-1.65 (m, 2 H), 1.41/1.33 (2*s, 27 H), 1.38 (m, 2 H), 1.18 (t, 3 H), 0.7 (m, 2 H)

### EXEMPLE 201: Acide (3S)-3-(4-aminobutyl)-1-[[4-fluoro-2-(4-fluoro-3-méthoxyphényl)phényl]méthyl]-4-hydroxy-4-oxo-1,4-azaphosphinane-3-carboxylique

L'exemple 201 est obtenu à partir de l'intermédiaire 153 selon la procédure D précédemment décrite.
RMN ¹H : (400 MHz, dmso-d6) δ ppm 7.6 (dd, 1 H), 7.25 (m, 2 H), 7.15 (m, 2 H), 6.95 (m, 1 H), 4.4/4.3 (2*d, 2 H), 3.9 (s, 3 H), 3.5-3.3 (m, 1 H), 3.2-3.05 (m, 2 H), 2.95 (m, 2 H), 2.9 (dd, 1 H), 2.05 (m, 1 H), 1.85 (m, 1 H), 1.65 (m, 1 H), 1.6 (m, 2 H), 1.35 (m, 1 H), 1.2-1 (m, 2 H) ESI/FIA/HR et MS/MS : [M+H]+ = 483.1854 (483.1855)
Analyse Elémentaire : C=57.37(57.26);H=5.95(6.06);N=5.86(5.81)
PR : -21.910 (589 nm, T=21°C, C=1.1)

### Intermédiaire 154: (3S)-3-{4-[Bis(tert-butoxycarbonyl)amino]butyl}-4-éthoxy-1-[(2-isoquinolin-4-ylphényl)méthyl]-4-oxo-1,4-azaphosphinane-3-carboxylate de tert-butyle

L'intermédiaire 154 est obtenu à partir des intermédiaires 129 et 264 selon la procédure F précédemment décrite.
RMN ¹H : (400 MHz, dmso-d6) δ ppm 9.35 (d, 1 H), 8.32 (d, 1 H), 8.21 (m, 1 H), 7.71 (m, 2 H), 7.6 (2*d, 1 H), 7.5 (2*t, 1 H), 7.42 (t, 1 H), 7.31 (m, 1 H), 7.25 (m, 1 H), 3.87 (m, 2 H), 3.45-3.3 (m, 2 H), 3.32/3.2/3.05 (m, 2 H), 2.62/2.2 (m, 2 H), 2.58/2 (m, 2 H), 1.8-1.25 (m, 6 H), 1.6 (m, 2 H), 1.42/1.4 (2*s, 18 H), 1.32/1.3 (2*s, 9 H), 1.1 (m, 3 H)
RMN ³¹P : (400 MHz, dmso-d6) δ ppm 44.94

### EXEMPLE 202: Acide (3S)-3-(4-aminobutyl)-4-hydroxy-1-[(2-isoquinolin-4-ylphényl)méthyl]-4-oxo-1,4-azaphosphinane-3-carboxylique

L'exemple 202 est obtenu à partir de l'intermédiaire 154 selon la procédure D précédemment décrite.
RMN ¹H : (400 MHz, D2O) δ ppm 9.3 (s, 1 H), 8.35/8 (2*s, 1 H), 8.2 (m, 1 H), 7.8-7.4 (m, 7 H), 4.4-3.8 (2AB, 2 H), 3.6-3.2/3 (m, 2 H), 3.1/2.7 (m, 2 H), 2.91 (m, 2 H), 2.1/1.55 (m, 2 H), 1.75/1.25 (m, 2 H), 1.6 (m, 2 H), 1.15-0.75 (m, 2 H)
RMN ³¹P : (400 MHz, D2O) δ ppm 25.29
ESI/FIA/HR et MS/MS : [M+H]+ = 468.2046 (468.2047)
Analyse Elémentaire : C=64.83(64.23);H=5.86(6.47);N=9.17(8.99)

### Intermédiaire 155: (3S)-3-4-[Bis(tert-butoxycarbonyl)amino]butyl}-4-éthoxy-1-[[2-(2-méthoxypyridin-4-yl)phényl]méthyl]-4-oxo-1,4-azaphosphinane-3-carboxylate de tert-butyle

L'intermédiaire 155 est obtenu à partir des intermédiaires 129 et 244 selon la procédure F précédemment décrite.
RMN ¹H : (400 MHz, dmso-d6) δ ppm 8.2 (d, 1 H), 7.5 (m, 1 H), 7.45-7.3 (2m, 2 H), 7.25 (m, 1 H), 7 (dd, 1 H), 6.8 (si, 1 H), 3.9 (m, 2 H), 3.9 (s, 3 H), 3.6/3.35 (dd, 2 H), 3.3 (m, 2 H), 2.75/2.35 (dd, 2 H), 2.7/2.25 (2m, 2 H), 1.95/1.75 (2m, 2 H), 1.75 (m, 2 H), 1.4 (s, 18 H), 1.35 (s, 9 H), 1.3 (m, 2 H), 1.2 (t, 3 H), 0.7-0.5 (m, 2 H)

### EXEMPLE 203: Acide (3S)-3-(4-aminobutyl)-4-hydroxy-1-[[2-(2-méthoxypyridin-4-yl)phényl]méthyl]-4-oxo-1,4-azaphosphinane-3-carboxylique

L'exemple 203 est obtenu à partir de l'intermédiaire 155 selon la procédure D précédemment décrite.
RMN ¹H : (400 MHz, D2O) δ ppm 8.18 (d, 1 H), 7.65-7.35 (m, 4 H), 7.02 (d, 1 H), 6.85 (s, 1 H), 4.35 (dd, 2 H), 3.9 (s, 3 H), 3.65-3 (m, 3 H), 3-2.8 (m, 3 H), 2.1/1.7 (2m, 2 H), 1.85/1.35 (2m, 2 H), 1.6 (m, 2 H), 1.1 (m, 2 H)
ESI/FIA/HR et MS/MS : [M+H]+ = 448.1989 (448.2001)
Analyse Elémentaire : C=59.15(59.05);H=6.25(6.76);N=9.64(9.39)
PR : -11.440 (589 nm, T=19.5°C, C=0.9)

### EXEMPLE 204: Acide (3S)-3-(4-aminobutyl)-4-hydroxy-4-oxo-1-[[2-(2-oxo-1H-pyridin-4-yl)phényl]méthyl]-1,4-azaphosphinane-3-carboxylique

L'exemple 204 est obtenu à partir de l'intermédiaire 155 selon la procédure D précédemment décrite.
RMN ¹H : (300 MHz, D2O) δ ppm 7.65-7.3 (m, 4 H), 7.6 (d, 1 H), 6.55 (s, 1 H), 6.5 (d, 1 H), 4.35 (dd, 2 H), 3.5 (2m, 2 H), 3.25/2.9 (2m, 2 H), 2.9 (m, 2 H), 2.15/1.7 (2m, 2 H), 1.85/1.4 (2m, 2 H), 1.6 (m, 2 H), 1.1 (m, 2 H)
ESI/FIA/HR et MS/MS : [M+H]+ = 434.1838 (434.1839)
Analyse Elémentaire : C=58.88(58.19);H=6.31(6.51);N=9.95(9.69)
PR : -10.530 (589 nm, T=19°C, C=1.0)

### Intermédiaire 156: (3S)-3-{4-[Bis(tert-butoxycarbonyl)amino]butyl}-4-éthoxy-4-oxo-1-{2-[1-(tétrahydro-2H-pyran-2-yl)-1H-pyrazol-5-yl]benzyl}-1,4-azaphosphinane-3-carboxylate de tert-butyle

L'intermédiaire 156 est obtenu à partir des intermédiaires 129 et 293 selon la procédure F précédemment décrite.
RMN ¹H : (400 MHz, dmso-d6) δ ppm 7.6 (d, 1 H), 7.6-7.2 (m, 4 H), 6.3 (d, 1 H), 4.9-4.8 (dd, 1 H), 4-3.8 (m, 4 H), 3.5-3.2 (m, 4 H), 2.85-2.6 (m, 2 H), 2.5-2.1 (m, 3 H), 2-1.65 (m, 6 H), 1.5-1.25 (m, 5 H), 1.4 (s, 18 H), 1.35 (s, 9 H), 1.2 (t, 3 H), 0.85-0.6 (m, 2 H)

### EXEMPLE 205: Acide (3S)-3-(4-aminobutyl)-4-hydroxy-4-oxo-1-[2-(1H-pyrazol-3-yl)benzyl]-1,4-azaphosphinane-3-carboxylique

L'exemple 205 est obtenu à partir de l'intermédiaire 156 selon la procédure D précédemment décrite.
RMN ¹H : (300/400/500 MHz, dmso-d6) δ ppm 7.62 (d, 1 H), 7.6 (d, 1 H), 7.38 (t, 1 H), 7.33 (d, 1 H), 7.26 (t, 1 H), 6.55 (d, 1 H), 4.32/4.05 (dd, 2 H), 3.59/3.19 (dd, 2 H), 3.33/2.93 (dd, 2 H), 2.74 (m, 2 H), 2.11/1.61 (2*m, 2 H), 1.73/1.29 (2*m, 2 H), 1.41 (m, 2 H), 1.04/0.88 (2*m, 2 H)
ESI/FIA/HR et MS/MS : [M+H]+ = 407.1843 (407.1848)
Analyse Elémentaire : C=56.28(56.15);H=6.42(6.70);N=13.82(13.79)
PR : -84.390 (589 nm, T=19°C, C=1.1)

### Intermédiaire 157: (3S)-3-{4-[Bis(tert-butoxycarbonyl)amino]butyl}-1-[[2-(2-chlorophényl)-4-fluorophényl]méthyl]-4-éthoxy-4-oxo-1,4-azaphosphinane-3-carboxylate de tert-butyle

L'intermédiaire 157 est obtenu à partir des intermédiaires 129 et 253 selon la procédure F précédemment décrite.
RMN ¹H : (300/400 MHz, dmso-d6) δ ppm 7.65-7.4 (m, 4 H), 7.25 (m, 2 H), 7 (m, 1 H), 3.9 (m, 2 H), 3.35 (d, 1 H), 3.3 (m, 2 H), 3.15 (d, 1 H), 2.8-2.55 (m, 2 H), 2.3 (m, 1 H), 2.1 (m, 1 H), 1.95-1.7 (m, 4 H), 1.45 (m, 2 H), 1.45/1.35 (2*s, 27 H), 1.15 (t, 3 H), 0.75 (m, 2 H) RMN ¹⁹F : (300/400 MHz, dmso-d6) δ ppm -114.5
ESI/FIA/HR et MS/MS : [M+H]+ = 753.3436 (753.3441)

### EXEMPLE 206: Acide (3S)-3-(4-aminobutyl)-1-[[2-(2-chlorophényl)-4-fluorophényl]méthyl]-4-hydroxy-4-oxo-1,4-azaphosphinane-3-carboxylique

L'exemple 206 est obtenu à partir de l'intermédiaire 157 selon la procédure D précédemment décrite.
RMN ¹H : (300/400 MHz, dmso-d6) δ ppm 7.7-7.35 (m, 4 H), 7.35-7.2 (m, 2 H), 7.1 (m, 1 H), 4.1 (si, 2 H), 3.7-3 (m, 3 H), 3-2.7 (m, 3 H), 2.2-2 (m, 1 H), 1.8 (m, 1 H), 1.65 (m, 1 H), 1.55 (m, 2 H), 1.45-1 (m, 3 H)
ESI/FIA/HR et MS/MS : [M+H]+ = 469.1456 (469.1454)
Analyse Elémentaire : C=56.86(56.35);H=5.21(5.80);N=5.91(5.97)
PR : -15.060 (589 nm, T=20°C, C=1.0)

### Intermédiaire 158: (3S)-3-{4-[Bis(tert-butoxycarbonyl)amino]butyl}-1-[[2-(2,3-diméthoxyphényl)phényl]méthyl]-4-hydroxy-4-oxo-1,4-azaphosphinane-3-carboxylate de tert-butyle

L'intermédiaire 158 est obtenu à partir de l'intermédiaire 129 et du 2-(2,3-diméthoxyphénylbezaldéhyde) selon la procédure F précédemment décrite.
RMN ¹H : (400 MHz, dmso-d6) δ ppm 7.5 (m, 1 H), 7.35-7.2 (2m, 2 H), 7.1 (m, 1 H), 7.05-7 (2m, 2 H), 6.7 (dd, 1 H), 3.9 (m, 2 H), 3.85 (s, 3 H), 3.45 (s, 3 H), 3.4 (m, 2 H), 3.35 (dd, 2 H), 2.7/2.4 (2m, 2 H), 2.65/2.2 (2m, 2 H), 1.95-1.7 (m, 4 H), 1.45 (s, 18 H), 1.4 (m, 2 H), 1.35 (s, 9 H), 1.2 (t, 3 H), 0.9 (m, 2 H)
ESI/FIA/HR et MS/MS : [M+H]+ = 761.4136 (761.4142)

### EXEMPLE 207: Acide (3S)-3-(4-aminobutyl)-1-[[2-(2,3-diméthoxyphényl)phényl]méthyl]-4-hydroxy-4-oxo-1,4-azaphosphinane-3-carboxylique

L'exemple 207 est obtenu à partir de l'intermédiaire 158 selon la procédure D précédemment décrite.
RMN ¹H : (400 MHz, D2O) δ ppm 7.65-7.35 (m, 4 H), 7.25 (m, 1 H), 7.2 (m, 1 H), 6.9-6.8 (2dd, 1 H), 4.35-3.9 (2dd, 2 H), 3.86 (s, 3 H), 3.7-2.65 (m, 4 H), 3.4-3.35 (2s, 3 H), 2.9 (m, 2 H), 2.5-1.6 (m, 4 H), 1.6 (m, 2 H), 1.5-0.9 (m, 2 H)
ESI/FIA/HR et MS/MS : [M+H]+ = 477.2149 (477.2154)
Analyse Elémentaire : C=61.06(60.50);H=6.99(6.98);N=5.97(5.88)

### Intermédiaire 159: (3S)-3-{4-[Bis(tert-butoxycarbonyl)amino]butyl)-4-hydroxy-1-[[2-(2-méthylsulfonylphényl)phényl]méthyl]-4-oxo-1,4-azaphosphinane-3-carboxylate de tert-butyle

L'intermédiaire 159 est obtenu à partir de l'intermédiaire 129 et du 2-(2-méthylsulfonylphényl)benzaldéhyde selon la procédure F précédemment décrite.
RMN ¹H : (300/400 MHz, dmso-d6) δ ppm 8.1 (d, 1 H), 7.8-7.65 (2*m, 2 H), 7.55 (d, 1 H), 7.45 (m, 1 H), 7.3 (d, 1 H), 7.25 (d, 1 H), 7.25 (m, 1 H), 3.95 (m, 2 H), 3.5-3.25 (m, 4 H), 3.1 (dd, 1 H), 2.9-2.6 (2*m, 2 H), 2.85 (2*s, 3 H), 2.45 (m, 1 H), 2.3 (m, 1 H), 2.1 (m, 1 H), 2-1.65 (m, 4 H), 1.5-1.3 (4s, 27 H), 1.2 (m, 3 H), 0.75 (m, 2 H)
ESI/FIA/HR et MS/MS : [M+H]+ = 779.3696 (779.3701)

### EXEMPLE 208: Acide (3S)-3-(4-aminobutyl)-4-hydroxy-1-[[2-(2-méthylsulfonylphényl)phényl]méthyl]-4-oxo-1,4-azaphosphinane-3-carboxylique

L'exemple 208 est obtenu à partir de l'intermédiaire 159 selon la procédure D précédemment décrite.
RMN ¹H : (400 MHz, dmso-d6) δ ppm 8.2 (d, 1 H), 7.9/7.8 (2*m, 2 H), 7.8-7.4 (m, 4 H), 7.55 (d, 1 H), 4.4-3.8 (4d, 2 H), 3.8-3.5 (m, 1 H), 3.5-2.85 (m, 5 H), 3.1/3 (2*s, 3 H), 2.4-1.85 (m, 2 H), 1.8 (m, 1 H), 1.7 (m, 2 H), 1.6-1.1 (m, 3 H)
ESI/FIA/HR et MS/MS : [M+H]+ = 495.1716 (495.1713)
Analyse Elémentaire : C=55.39(55.86);H=5.77(6.32);N=5.61(5.66);S=6.24(6.48)

### Intermédiaire 160: (3S)-3-{4-[Bis(tert-butoxycarbonyl)amino]butyl}-4-éthoxy-1-[(2-naphthalen-2-ylphényl)méthyl]-4-oxo-1,4-azaphosphinane-3-carboxylate de tert-butyle

L'intermédiaire 160 est obtenu à partir des intermédiaires 129 et 240 selon la procédure F précédemment décrite.
RMN ¹H : (400 MHz, dmso-d6) δ ppm 8-7.3 (m, 11 H), 3.9 (m, 2 H), 3.6/3.4 (dd, 2 H), 3.3 (m, 2 H), 2.75/2.3 (2dd, 2 H), 2.7/2.2 (2m, 2 H), 1.9/1.7 (2m, 2 H), 1.75 (m, 2 H), 1.4 (s, 18 H), 1.3 (m, 2 H), 1.3 (s, 9 H), 1.15 (t, 3 H), 0.65 (m, 2 H)

### EXEMPLE 209: Acide (3S)-3-(4-aminobutyl)-4-hydroxy-1-[(2-naphthalen-2-ylphényl)méthyl]-4-oxo-1,4-azaphosphinane-3-carboxylique

L'exemple 209 est obtenu à partir de l'intermédiaire 160 selon la procédure D précédemment décrite.
RMN ¹H : (300 MHz, D2O) δ ppm 8-7.4 (m, 11 H), 4.35 (dd, 2 H), 3.35/3 (2m, 2 H), 3.1/2.7 (2dd, 2 H), 2.8 (m, 2 H), 2/1.6 (2m, 2 H), 1.75/1.2 (2m, 2 H), 1.5 (m, 2 H), 0.9 (m, 2 H) ESI/FIA/HR et MS/MS : [M+H]+ = 467.2095 (467.2099)
Analyse Elémentaire : C=67.07(66.94);H=6.46(6.70);N=5.88(6.00)
PR : -8.570 (589 nm, T=19°C, C=0.8)

### Intermédiaire 161: (3S)-3-{4-[Bis(tert-butoxycarbonyl)amino]butyl}-1-[[4-chloro-2-(4-fluorophényl)phényl]méthyl]-4-éthoxy-4-oxo-1,4-azaphosphinane-3-carboxylate de tert-butyle

L'intermédiaire 161 est obtenu à partir de l'intermédiaire 129 et du 4-chloro-2-(4-fluorophényl)benzaldéhyde selon la procédure F précédemment décrite.
RMN ¹H : (400 MHz, dmso-d6) δ ppm 7.5 (d, 1 H), 7.45 (dd, 1 H), 7.4 (dd, 2 H), 7.29 (t, 2 H), 7.25 (d, 1 H), 3.92 (m, 2 H), 3.5/3.3 (AB, 2 H), 3.3 (m, 2 H), 2.72/2.2 (2*m, 2 H), 2.67/2.35 (2*m, 2 H), 2-1.7 (m, 2 H), 1.8 (m, 2 H), 1.4 (s, 18 H), 1.35 (m, 2 H), 1.32 (s, 9 H), 1.18 (t, 3 H), 0.7 (m, 2 H)
RMN ³¹P : (400 MHz, dmso-d6) δ ppm 44.88, -113.8

### EXEMPLE 210: Acide (3S)-3-(4-aminobutyl)-1-[[4-chloro-2-(4-fluorophényl)phényl]méthyl]-4-hydroxy-4-oxo-1,4-azaphosphinane-3-carboxylique

L'exemple 210 est obtenu à partir de l'intermédiaire 161 selon la procédure D précédemment décrite.
RMN ¹H : (400 MHz, D2O) δ ppm 7.57 (d, 1 H), 7.51 (dd, 1 H), 7.45 (d, 1 H), 7.36 (m, 2 H), 7.24 (t, 2 H), 4.4/4.27 (AB, 2 H), 3.38/3.1 (m, 2 H), 3.2/2.85 (m, 2 H), 2.94 (m, 2 H), 2.09/1.65 (m, 2 H), 1.85/1.35 (m, 2 H), 1.59 (m, 2 H), 1.1 (m, 2 H)
RMN ³¹P : (400 MHz, D2O) δ ppm 25.4, -110.5
ESI/FIA/HR et MS/MS : [M+H]+ = 469.1455 (469.1454)
Analyse Elémentaire : C=56.27(56.35);H=5.43(5.80);N=5.97(5.97);C1=7.28(7.56)
PR : -28.440 (589 nm, T=20°C, C=0.9)

### Intermédiaire 162: (3S)-3-{4-[Bis(tert-butoxycarbonyl)amino]butyl}-4-éthoxy-1-[[4-fluoro-2-(4-hydroxyphényl)phényl]méthyl]-4-oxo-1,4-azaphosphinane-3-carboxylate de tert-butyle

L'intermédiaire 162 est obtenu à partir des intermédiaires 129 et 265 selon la procédure F précédemment décrite.
RMN ¹H : (400 MHz, dmso-d6) δ ppm 9.6 (si, 1 H), 7.49 (dd, 1 H), 7.18 (d, 2 H), 7.12 (td, 1 H), 6.98 (dd, 1 H), 6.81 (d, 2 H), 3.92 (m, 2 H), 3.5/3.3 (AB, 2 H), 3.3 (m, 2 H), 2.72/2.2 (2*m, 2 H), 2.67/2.35 (2*m, 2 H), 2-1.7 (m, 2 H), 1.8 (m, 2 H), 1.4 (s, 18 H), 1.35 (m, 2 H), 1.32 (s, 9 H), 1.18 (t, 3 H), 0.7 (m, 2 H)
RMN ³¹P : (400 MHz, dmso-d6) δ ppm 45.1, -114.8

### EXEMPLE 211: Acide (3S)-3-(4-aminobutyl)-1-[[4-fluoro-2-(4-hydroxyphényl)phényl]méthyl]-4-hydroxy-4-oxo-1,4-azaphosphinane-3-carboxylique

L'exemple 211 est obtenu à partir de l'intermédiaire 162 selon la procédure D précédemment décrite.
RMN ¹H : (400 MHz, D2O) δ ppm 7.47 (dd, 1 H), 7.12 (d, 2 H), 7.09 (td, 1 H), 7.01 (dd, 1 H), 6.88 (d, 2 H), 4.3/4.16 (AB, 2 H), 3.23/2.98 (m, 2 H), 3/2.7 (m, 2 H), 2.81 (m, 2 H), 1.96/1.55 (m, 2 H), 1.72/1.21 (m, 2 H), 1.49 (m, 2 H), 0.95 (m, 2 H)
RMN ³¹P : (400 MHz, D2O) δ ppm 25.4, -113
ESI/FIA/HR et MS/MS : [M+H]+ = 451.1794 (451.1793)
Analyse Elémentaire : C=58.16(58.66);H=5.71(6.27);N=6.30(6.22)
PR : -16.520 (589 nm, T=20°C, C=0.9)

### Intermédiaire 163: (3S)-3-{4-[Bis(tert-butoxycarbonyl)amino]butyl}-4-éthoxy-1-[4-fluoro-2-(1-méthyl-1H-pyrazol-4-yl)benzyl]-4-oxo-1,4-azaphosphinane-3-carboxylate de tert-butyle

L'intermédiaire 163 est obtenu à partir des intermédiaires 129 et 250 selon la procédure F précédemment décrite.
RMN ¹H : (300 MHz, dmso-d6) δ ppm 8 (s, 1 H), 7.7 (s, 1 H), 7.4 (dd, 1 H), 7.2 (dd, 1 H), 7.05 (td, 1 H), 3.97 (m, 2 H), 3.9 (s, 3 H), 3.6/3.4 (dd, 2 H), 3.25 (m, 2 H), 3/2.35 (2m, 2 H), 2.8/2.45 (2m, 2 H), 2 (m, 2 H), 1.8 (m, 2 H), 1.4 (s, 18 H), 1.35 (s, 9 H), 1.3 (m, 2 H), 1.2 (t, 3 H), 0.65 (m, 2 H)

### EXEMPLE 212: Acide (3S)-3-(4-aminobutyl)-1-[4-fluoro-2-(1-méthyl-1H-pyrazol-4-yl)benzyl]-4-hydroxy-4-oxo-1,4-azaphosphinane-3-carboxylique

L'exemple 212 est obtenu à partir de l'intermédiaire 163 selon la procédure D précédemment décrite.
RMN ¹H : (300 MHz, D2O) δ ppm 7.81 (s, 1 H), 7.7 (s, 1 H), 7.6 (dd, 1 H), 7.2 (m, 2 H), 4.45 (dd, 2 H), 3.95 (s, 3 H), 3.5/3.25 (2m, 2 H), 3.3/3 (2m, 2 H), 3 (m, 2 H), 2.1/1.75 (2m, 2 H), 1.9/1.4 (2m, 2 H), 1.65 (m, 2 H), 1.15 (m, 2 H)
RMN ¹⁹F : (300 MHz, D2O) δ ppm -110.5
ESI/FIA/HR et MS/MS : [M+H]+ = 439.1905 (439.1910)
Analyse Elémentaire : C=54.79(54.79);H=6.05(6.44);N=12.61(12.78)
PR : -18.620 (589 nm, T=19°C, C=0.9)

### Intermédiaire 164: (3S)-3-{4-[Bis(tert-butoxycarbonyl)amino]butyl}-4-éthoxy-1-[(4-fluoro-2-thiophen-2-ylphényl)méthyl]-4-oxo-1,4-azaphosphinane-3-carboxylate de tert-butyle

L'intermédiaire 164 est obtenu à partir des intermédiaires 129 et 246 selon la procédure F précédemment décrite.
RMN ¹H : (300 MHz, dmso-d6) δ ppm 7.66 (dd, 1 H), 7.47 (dd, 1 H), 7.31 (dd, 1 H), 7.25 (dd, 1 H), 7.2 (dd, 1 H), 7.16 (td, 1 H), 3.97 (m, 2 H), 3.63/3.4 (dd, 2 H), 3.25 (m, 2 H), 2.88/2.32 (2m, 2 H), 2.79/2.42 (dd, 2 H), 2.05-1.9 (m, 2 H), 1.82 (m, 2 H), 1.39 (s, 18 H), 1.33 (s, 9 H), 1.3 (m, 2 H), 1.2 (t, 3 H), 0.7-0.4 (2m, 2 H)
RMN ¹⁹F : (300 MHz, dmso-d6) δ ppm -113.9

### EXEMPLE 213: Acide (3S)-3-(4-aminobutyl)-1-[(4-fluoro-2-thiophen-2-ylphényl)méthyl]-4-hydroxy-4-oxo-1,4-azaphosphinane-3-carboxylique

L'exemple 213 est obtenu à partir de l'intermédiaire 164 selon la procédure D précédemment décrite.
RMN ¹H : (300 MHz, dmso-d6) δ ppm 7.65 (dd, 1 H), 7.55 (dd, 1 H), 7.29 (dd, 1 H), 7.2 (td, 1 H), 7.15 (m, 2 H), 4.5 (dd, 2 H), 3.5/3.2 (2m, 2 H), 3.3/2.9 (2m, 2 H), 2.9 (m, 2 H), 2.1/1.75 (2m, 2 H), 1.85/1.4 (2m, 2 H), 1.6 (m, 2 H), 1.1 (m, 2 H)
RMN ¹⁹F : (300 MHz, dmso-d6) δ ppm -110
ESI/FIA/HR et MS/MS : [M+H]+ = 441.1405 (441.1413)
Analyse Elémentaire : C=54.66(54.54);H=5.90(5.95);N=6.35(6.36);S=7.27(7.28)
PR : -13.110 (589 nm, T=19.5°C, C=0.7)

### Intermédiaire 165: (3S)-3-{4-Bis(tert-butoxycarbonyl)amino]butyl}-4-éthoxy-1-[(2-isoquinolin-5-ylphényl)méthyl]-4-oxo-1,4-azaphosphinane-3-carboxylate de tert-butyle

L'intermédiaire 165 est obtenu à partir des intermédiaires 129 et 216 selon la procédure F précédemment décrite.
RMN ¹H : (400 MHz, dmso-d6) δ ppm 9.4 (s, 1 H), 8.45 (d, 1 H), 8.2 (m, 1 H), 7.75 (m, 1 H), 7.65 (m, 1 H), 7.6-7.4 (m, 3 H), 7.2 (m, 1 H), 7.15 (2*d, 1 H), 3.95-3.7 (m, 2 H), 3.5-3 (m, 4 H), 3.5-3.3 (m, 2 H), 2.7-2.4 (m, 2 H), 2.2 (m, 1 H), 2 (m, 1 H), 1.85 (m, 1 H), 1.8 (m, 2 H), 1.7-1.5 (m, 1 H), 1.5-1.25 (4s, 27 H), 1.1 (t, 3 H), 0.75-0.5 (m, 2 H)

### EXEMPLE 214: Acide (3S)-3-(4-aminobutyl)-4-hydroxy-1-[(2-isoquinolin-5-ylphényl)méthyl]-4-oxo-1,4-azaphosphinane-3-carboxylique

L'exemple 214 est obtenu à partir de l'intermédiaire 165 selon la procédure D précédemment décrite.
RMN ¹H : (400 MHz, D2O) δ ppm 9.25 (s, 1 H), 8.3 (dd, 1 H), 8.2 (m, 1 H), 7.85-7.55 (m, 5 H), 7.35 (m, 1 H), 7.3 (d, 1 H), 4.45-3.8 (4d, 2 H), 3.65-3.05 (m, 2 H), 3.05-2.8 (m, 3 H), 2.7 (m, 1 H), 2.1 (m, 1 H), 1.75 (m, 1 H), 1.7-1.5 (m, 3 H), 1.35-0.6 (m, 3 H)
ESI/FIA/HR et MS/MS : [M+H]+ = 468.2051 (468.2047)
Analyse Elémentaire : C=64.96(64.23);H=6.08(6.47);N=9.03(8.99)

### Intermédiaire 166: (3S)-3-{4-[Bis(tert-butoxycarbonyl)amino]butyl}-4-éthoxy-1-[(4-fluoro-2-naphthalen-2-ylphényl)méthyl]-4-oxo-1,4-azaphosphinane-3-carboxylate de tert-butyle

L'intermédiaire 166 est obtenu à partir des intermédiaires 129 et 247 selon la procédure F précédemment décrite.
RMN ¹H : (400 MHz, dmso-d6) δ ppm 8-7.9 (m, 3 H), 7.9 (si, 1 H), 7.6-7.5 (m, 4 H), 7.25 (td, 1 H), 7.15 (dd, 1 H), 3.9 (m, 2 H), 3.6/3.4 (dd, 2 H), 3.3 (m, 2 H), 2.75/2.3 (2m, 2 H), 2.7/2.2 (2m, 2 H), 1.9/1.75 (2m, 2 H), 1.75 (m, 2 H), 1.4 (s, 18 H), 1.3 (s, 9 H), 1.3 (m, 2 H), 1.15 (t, 3 H), 0.7 (m, 2 H)

### EXEMPLE 215: Acide (3S)-3-(4-aminobutyl)-1-[(4-fluoro-2-naphthalen-2-ylphényl)méthyl]-4-hydroxy-4-oxo-1,4-azaphosphinane-3-carboxylique

L'exemple 215 est obtenu à partir de l'intermédiaire 166 selon la procédure D précédemment décrite.
RMN ¹H : (400 MHz, D2O) δ ppm 8.08 (d, 1 H), 8 (m, 2 H), 7.95 (d, 1 H), 7.7 (dd, 1 H), 7.65 (m, 2 H), 7.54 (dd, 1 H), 7.3 (m, 1 H), 7.3 (dd, 1 H), 4.65-4.2 (m, 2 H), 3.35/3.05 (2m, 2 H), 3.05/2.8 (2m, 2 H), 2.85 (m, 2 H), 2/1.65 (2m, 2 H), 1.85/1.35 (2m, 2 H), 1.55 (m, 2 H), 0.9 (m, 2 H)
ESI/FIA/HR et MS/MS : [M+H]+ = 485.2002 (485.2005)
Analyse Elémentaire : C=63.97(64.45);H=6.21(6.24);N=5.93(5.78)
PR : -6.790 (589 nm, T=19°C, C=0.7)

### Intermédiaire 167: (3S)-3-{4-[Bis(tert-butyloxycarbonyl)amino]butyl}-1-[[2-(1-benzothiophen-2-yl)-4-fluorophényl]méthyl]-4-éthoxy-4-oxo-1,4-azaphosphinane-3-carboxylate de tert-butyle

L'intermédiaire 167 est obtenu à partir des intermédiaires 129 et 217 selon la procédure F précédemment décrite.
RMN ¹H : (400 MHz, dmso-d6) δ ppm 8.05 (d, 1 H), 7.9 (d, 1 H), 7.6 (s, 1 H), 7.55 (dd, 1 H), 7.4 (m, 2 H), 7.35 (d, 1 H), 7.3 (m, 1 H), 3.95 (m, 2 H), 3.75 (d, 1 H), 3.5 (d, 1 H), 3.25 (m, 2 H), 2.95 (m, 1 H), 2.8 (m, 1 H), 2.45 (dd, 1 H), 2.35 (m, 1 H), 2.05-1.6 (m, 4 H), 1.42 (m, 2 H), 1.4 (s, 18 H), 1.35 (s, 9 H), 1.2 (t, 3 H), 0.65 (m, 1 H), 0.55 (m, 1 H)

### EXEMPLE 216: Acide (3S)-3-(4-aminobutyl)-1-[[2-(1-benzothiophen-2-yl)-4-fluorophényl]méthyl]-4-hydroxy-4-oxo-1,4-azaphosphinane-3-carboxylique

L'exemple 216 est obtenu à partir de l'intermédiaire 167 selon la procédure D précédemment décrite.
RMN ¹H : (400 MHz, D2O) δ ppm 7.95/7.9 (2*d, 2 H), 7.65 (m, 1 H), 7.45 (m, 2 H), 7.4 (s, 1 H), 7.3 (m, 2 H), 4.55/4.4 (2*d, 2 H), 3.6-3.4 (m, 1 H), 3.25 (m, 1 H), 3.15 (m, 1 H), 2.9 (dd, 1 H), 2.85 (m, 2 H), 2.1 (m, 1 H), 1.85 (m, 1 H), 1.65 (m, 1 H), 1.6-0.85 (m, 5 H)
ESI/FIA/HR et MS/MS : [M+H]+ = 491.1565 (491.1564)
Analyse Elémentaire : C=59.10(58.77);H=5.38(5.75);N=5.84(5.71);S=6.42(6.54)
PR : 0.930 (589 nm, T=20.5°C, C=1.0)

### Intermédiaire 168: (3S)-3-{4-[Bis(tert-butoxycarbonyl)amino]butyl}-4-éthoxy-1-[4-fluoro-2-(1-méthyl-1H-imidazol-5-yl)benzyl]-4-oxo-1,4-azaphosphinane-3-carboxylate de tert-butyle

L'intermédiaire 168 est obtenu à partir des intermédiaires 129 et 248 selon la procédure F précédemment décrite.
RMN ¹H : (400 MHz, dmso-d6) δ ppm 7.75 (s, 1 H), 7.56 (2d, 1 H), 7.29 (2t, 1 H), 7.15 (2d, 1 H), 6.9 (s, 1 H), 3.95 (m, 2 H), 3.5-3.25 (m, 4 H), 3.4 (s, 3 H), 2.9/2.2 (2m, 2 H), 2.65/2.3 (2m, 2 H), 1.95/1.85 (2m, 2 H), 1.85 (m, 2 H), 1.4 (m, 2 H), 1.4 (2s, 18 H), 1.35 (2s, 9 H), 1.2 (t, 3 H), 1.1-0.65 (m, 2 H)

### EXEMPLE 217: Acide (3S)-3-(4-aminobutyl)-1-[4-fluoro-2-(1-méthyl-1H-imidazol-5-yl)benzyl]-4-hydroxy-4-oxo-1,4-azaphosphinane-3-carboxylique

L'exemple 217 est obtenu à partir de l'intermédiaire 168 selon la procédure D précédemment décrite.
RMN ¹H : (400 MHz, D2O) δ ppm 7.8 (s, 1 H), 7.68 (dd, 1 H), 7.33 (td, 1 H), 7.22 (dd, 1 H), 7.1 (s, 1 H), 4.25/4.18 (AB, 2 H), 3.6/3.2 (2m, 2 H), 3.45 (s, 3 H), 3.3/3 (2m, 2 H), 2.95 (m, 2 H), 2.12/1.71 (2m, 2 H), 1.9/1.42 (2m, 2 H), 1.6 (m, 2 H), 1.3-1 (m, 2 H)
RMN ¹⁹F : (400 MHz, D2O) δ ppm -109.75
ESI/FIA/HR et MS/MS : [M+H]+ = 439.1905 (439.1905)
Analyse Elémentaire : C=54.41(54.79);H=6.12(6.44);N=12.74(12.78)
PR : -27.720 (589 nm, T=20°C, C=0.7)

### Intermédiaire 169: (3S)-3-{4-[Bis(tert-butoxycarbonyl)amino]butyl}-1-[[4-chloro-2-(4-méthylphényl)phényl]méthyl]-4-éthoxy-4-oxo-1,4-azaphosphinane-3-carboxylate de tert-butyle

L'intermédiaire 169 est obtenu à partir de l'intermédiaire 129 et du 4-chloro-2-(4-méthylphényl)benzaldéhyde selon la procédure F précédemment décrite.
RMN ¹H : (400 MHz, dmso-d6) δ ppm 7.52 (d, 1 H), 7.4 (dd, 1 H), 7.25 (dd, 4 H), 7.2 (d, 1 H), 3.9 (m, 2 H), 3.5/2.8 (dd, 2 H), 2.8 (m, 2 H), 2.75/2.2 (2m, 2 H), 2.65/2.3 (2dd, 2 H), 2.35 (s, 3 H), 1.95/1.8 (2m, 2 H), 1.8 (m, 2 H), 1.4 (s, 18 H), 1.35 (m, 2 H), 1.35 (s, 9 H), 1.18 (t, 3 H), 0.7 (m, 2 H)

### EXEMPLE 218: Acide (3S)-3-(4-aminobutyl)-1-[[4-chloro-2-(4-méthylphényl)phényl]méthyl]-4-hydroxy-4-oxo-1,4-azaphosphinane-3-carboxylique

L'exemple 218 est obtenu à partir de l'intermédiaire 169 selon la procédure D précédemment décrite.
RMN ¹H : (400 MHz, D2O) δ ppm 7.58 (d, 1 H), 7.5 (dd, 1 H), 7.42 (d, 1 H), 7.38 (d, 2 H), 7.23 (d, 2 H), 4.4/4.25 (AB, 2 H), 3.35/3.1 (2m, 2 H), 3.2/2.81 (2m, 2 H), 2.95 (m, 2 H), 2.39 (s, 3 H), 2.09/1.68 (2m, 2 H), 1.85/1.35 (2m, 2 H), 1.6 (m, 2 H), 1.2-1 (m, 2 H)
ESI/FIA/HR et MS/MS : [M+H]+ = 465.1707 (465.1704)
Analyse Elémentaire : C=60.11(59.42);H=6.34(6.50);N=6.09(6.03)
PR : -26.100 (589 nm, T=20°C, C=0.3)

### Intermédiaire 170: (3S)-3-{4-[Bis(tert-butoxycarbonyl)amino]butyl}-1-[2-(1,2-diméthyl-1H-imidazol-5-yl)-4-fluorobenzyl]-4-éthoxy-4-oxo-1,4-azaphosphinane-3-carboxylate de tert-butyle

L'intermédiaire 170 est obtenu à partir des intermédiaires 129 et 274 selon la procédure F précédemment décrite.
RMN ¹H : (300/400 MHz, dmso-d6) δ ppm 7.55 (dd, 1 H), 7.2 (m, 1 H), 7 (d, 1 H), 6.75 (s, 1 H), 4.05-3.7 (m, 4 H), 3.5-3.3 (2*d, 2 H), 3.25 (s, 3 H), 3-2.2 (m, 4 H), 2.35 (s, 3 H), 2-1.3 (m, 6 H), 1.4 (3s, 27 H), 1.25 (m, 3 H), 0.85 (m, 2 H)

### EXEMPLE 219: Acide (3S)-3-(4-aminobutyl)-1-[2-(1,2-diméthyl-1H-imidazol-5-yl)-4-fluorobenzyl]-4-hydroxy-4-oxo-1,4-azaphosphinane-3-carboxylique

L'exemple 219 est obtenu à partir de l'intermédiaire 170 selon la procédure D précédemment décrite.
RMN ¹H : (400 MHz, D2O) δ ppm 7.75 (dd, 1 H), 7.55 (s, 1 H), 7.5 (m, 1 H), 7.3 (dd, 1 H), 4.25 (s, 2 H), 3.55 (m, 1 H), 3.45 (s, 3 H), 3.4 (m, 1 H), 3.25 (m, 1 H), 3.15 (dd, 1 H), 2.95 (t, 2 H), 2.65 (s, 3 H), 2.15 (m, 1 H), 1.9 (m, 1 H), 1.75 (m, 1 H), 1.65 (m, 2 H), 1.45 (m, 1 H), 1.3 (m, 1 H), 1.15 (m, 1 H)
ESI/FIA/HR et MS/MS : [M+H]+ = 453.2058 (453.2061)
Analyse Elémentaire : C=46.18(47.29);H=5.24(5.86);N=10.02(10.50)
PR : -28.200 (589 nm, T=20°C, C=1.0)

### Intermédiaire 171: (3S)-3-{4-[Bis(tert-butoxycarbonyl)amino]butyl}-4-éthoxy-1-[(4-fluoro-2-imidazo[1,2-a]pyridin-3-ylphényl)méthyl]-4-oxo-1,4-azaphosphinane-3-carboxylate de tert-butyle

L'intermédiaire 171 est obtenu à partir des intermédiaires 129 et 234 selon la procédure F précédemment décrite.
RMN ¹H : (300 MHz, dmso-d6) δ ppm 7.9 (d, 1 H), 7.65 (m, 2 H), 7.65 (s, 1 H), 7.35-7.2 (m, 3 H), 6.9 (m, 1 H), 3.9 (quad., 2 H), 3.35 (t, 2 H), 3.35/3.25 (2*d, 2 H), 2.8-2.4 (m, 3 H), 2.4 (dd, 1 H), 2.2 (m, 1 H), 1.9-1.6 (m, 3 H), 1.5-1.3 (m, 2 H), 1.45 (s, 18 H), 1.35 (s, 9 H), 1.15 (t, 3 H), 1-0.75 (m, 2 H)

### EXEMPLE 220: Acide (3S)-3-(4-aminobutyl)-1-[(4-fluoro-2-imidazo[1,2-a]pyridin-3-ylphényl)méthyl]-4-hydroxy-4-oxo-1,4-azaphosphinane-3-carboxylique

L'exemple 220 est obtenu à partir de l'intermédiaire 171 selon la procédure D précédemment décrite.
RMN ¹H : (400 MHz, D2O) δ ppm 8.05 (d, 1 H), 7.75 (dd, 1 H), 7.7 (d, 1 H), 7.7 (s, 1 H), 7.45 (m, 1 H), 7.4 (m, 1 H), 7.3 (d, 1 H), 7 (m, 1 H), 4.6-4 (m, 2 H), 3.45 (m, 1 H), 3.05 (m, 2 H), 2.9 (m, 2 H), 2.8 (dd, 1 H), 2.1 (m, 1 H), 1.8 (m, 1 H), 1.65 (m, 1 H), 1.55 (m, 2 H), 1.3 (m, 1 H), 0.95 (m, 2 H)
ESI/FIA/HR et MS/MS : [M+H]+ = 475.1906 (475.1905)
Analyse Elémentaire : C=58.69(58.22);H=5.71(5.95);N=11.94(11.81)
PR : -27.190 (589 nm, T=20°C, C=1.0)

### Intermédiaire 172: (3S)-3-{4-[Bis(tert-butoxycarbonyl)amino]butyl}-4-éthoxy-1-[[4-fluoro-2-(2-méthoxypyridin-4-yl)phényl]méthyl]-4-oxo-1,4-azaphosphinane-3-carboxylate de tert-butyle

L'intermédiaire 172 est obtenu à partir des intermédiaires 129 et 249 selon la procédure F précédemment décrite.
RMN ¹H : (400 MHz, dmso-d6) δ ppm 8.23 (d, 1 H), 7.52 (dd, 1 H), 7.26 (td, 1 H), 7.11 (dd, 1 H), 7.01 (dd, 1 H), 6.85 (si, 1 H), 3.94 (m, 2 H), 3.9 (s, 3 H), 3.53/3.34 (dd, 2 H), 3.31 (m, 2 H), 2.74/2.24 (2m, 2 H), 2.68/2.37 (2dd, 2 H), 1.93/1.75 (2m, 2 H), 1.86-1.66 (m, 2 H), 1.41 (s, 18 H), 1.36 (m, 2 H), 1.35 (s, 9 H), 1.19 (2m, 2 H), 0.69/0.61 (t, 3 H)

### EXEMPLE 221: Acide (3S)-3-(4-aminobutyl)-1-[[4-fluoro-2-(2-méthoxypyridin-4-yl)phényl]méthyl]-4-hydroxy-4-oxo-1,4-azaphosphinane-3-carboxylique

L'exemple 221 est obtenu à partir de l'intermédiaire 172 selon la procédure D précédemment décrite.
RMN ¹H : (400 MHz, D2O) δ ppm 8.17 (d, 1 H), 7.64 (dd, 1 H), 7.29 (td, 1 H), 7.17 (dd, 1 H), 7.01 (dd, 1 H), 6.86 (s, 1 H), 4.32 (dd, 2 H), 3.9 (s, 3 H), 3.45/3.07 (2m, 2 H), 3.17/2.84 (2m, 2 H), 2.9 (m, 2 H), 2.1/1.68 (2m, 2 H), 1.85/1.35 (2m, 2 H), 1.6 (m, 2 H), 1.07 (m, 2 H)
ESI/FIA/HR et MS/MS : [M+H]+ = 466.1905 (466.1902)
Analyse Elémentaire : C=56.79(56.77);H=6.15(6.28);N=8.94(9.03)
PR : -9.530 (589 nm, T=19°C, C=1.0)

### EXEMPLE 222: Acide (3S)-3-(4-aminobutyl)-1-[[4-fluoro-2-(2-oxo-1H-pyridin-4-yl)phényl]méthyl]-4-hydroxy-4-oxo-1,4-azaphosphinane-3-carboxylique

L'exemple 222 est obtenu à partir de l'intermédiaire 172 selon la procédure D précédemment décrite.
RMN ¹H : (400 MHz, D2O) δ ppm 7.64 (dd, 1 H), 7.62 (d, 1 H), 7.29 (td, 1 H), 7.18 (dd, 1 H), 6.57 (d, 1 H), 6.53 (dd, 1 H), 4.3 (dd, 2 H), 3.51/3.15 (2m, 2 H), 3.24/2.95 (2m, 2 H), 2.92 (m, 2 H), 2.13/1.7 (2m, 2 H), 1.86/1.38 (2m, 2 H), 1.58 (m, 2 H), 1.1 (m, 2 H)
RMN ¹⁹F : (400 MHz, D2O) δ ppm -109.6
ESI/FIA/HR et MS/MS : [M+H]+ = 452.1745 (452.1745)
Analyse Elémentaire : C=56.06(55.87);H=5.96(6.03);N=9.21(9.31)
PR : -10.270 (589 nm, T=19°C, C=0.9)

### Intermédiaire 173: (3S)-3-{4-[Bis(tert-butoxycarbonyl)amino]butyl}-4-éthoxy-1-[[4-hydroxy-2-(4-méthylphényl)phényl]méthyl]-4-oxo-1,4-azaphosphinane-3-carboxylate de tert-butyle

L'intermédiaire 173 est obtenu à partir des intermédiaires 129 et 266 selon la procédure F précédemment décrite.
RMN ¹H : (400 MHz, dmso-d6) δ ppm 9.4 (s, 1 H), 7.2 (s, 4 H), 7.2 (d, 1 H), 6.75 (dd, 1 H), 6.7 (t, 1 H), 6.55 (s, 1 H), 3.95 (m, 2 H), 3.4 (d, 1 H), 3.25 (d, 1 H), 2.8 (m, 3 H), 2.7 (dd, 1 H), 2.35 (s, 3 H), 2.25 (dd, 1 H), 2.15 (m, 1 H), 1.9 (m, 1 H), 1.85-1.7 (m, 3 H), 1.35 (2s, 18 H), 1.25 (m, 2 H), 1.2 (t, 3 H), 0.75 (m, 2 H)

### EXEMPLE 223: Acide (3S)-3-(4-aminobutyl)-4-hydroxy-1-[[4-hydroxy-2-(4-méthylphényl)phényl]méthyl]-4-oxo-1,4-azaphosphinane-3-carboxylique

L'exemple 223 est obtenu à partir de l'intermédiaire 173 selon la procédure D précédemment décrite.
RMN ¹H : (400 MHz, D2O) δ ppm 7.47 (d, 1 H), 7.35 (d, 2 H), 7.22 (d, 2 H), 6.95 (dd, 1 H), 6.83 (d, 1 H), 4.32/4.19 (AB, 2 H), 3.32/3.05 (2m, 2 H), 3.18/2.77 (2m, 2 H), 2.95 (m, 2 H), 2.38 (s, 3 H), 2.09/1.68 (2m, 2 H), 1.85/1.35 (2m, 2 H), 1.6 (m, 2 H), 1.2-1 (m, 2 H)
ESI/FIA/HR et MS/MS : [M+H]+ = 447.2045 (447.2043)
Analyse Elémentaire : C=62.20(61.87);H=6.77(7.00);N=6.19(6.27)
PR : -32.320 (589 nm, T=20°C, C=0.7)

### Intermédiaire 174: (3S)-3-{4-[Bis(tert-butoxycarbonyl)amino]butyl}-4-éthoxy-1-[4-méthoxy-2-(imidazo[1,2-a]pyridin-3-yl)benzyl]-4-oxo-1,4-azaphosphinane-3-carboxylate de tert-butyle

L'intermédiaire 174 est obtenu à partir des intermédiaires 129 et 267 selon la procédure F précédemment décrite.
RMN ¹H : (400 MHz, dmso-d6) δ ppm 7.98 (d, 1 H), 7.66 (d, 1 H), 7.63 (d, 1 H), 7.5 (s, 1 H), 7.29 (t, 1 H), 7.1 (dd, 1 H), 6.98 (d, 1 H), 6.9 (t, 1 H), 3.9 (m, 2 H), 3.8 (s, 3 H), 3.35 (m, 2 H), 3.35/3.19 (AB, 2 H), 2.7/2.25 (m, 2 H), 2.62/2.1 (m, 2 H), 1.85/1.7 (m, 2 H), 1.65 (m, 2 H), 1.4 (s, 18 H), 1.35 (m, 2 H), 1.3 (s, 9 H), 1.15 (t, 3 H), 0.65 (m, 2 H)

### EXEMPLE 224: Acide (3S)-3-(4-aminobutyl)-4-hydroxy-1-[4-hydroxy-2-(imidazo[1,2-a]pyridin-3-yl)benzyl]-4-oxo-1,4-azaphosphinane-3-carboxylique

L'exemple 224 est obtenu à partir de l'intermédiaire 174 selon la procédure D précédemment décrite.
RMN ¹H : (400 MHz, D2O) δ ppm 8 (d, 1 H), 7.67 (s, 1 H), 7.65 (d, 1 H), 7.59 (d, 1 H), 7.42 (t, 1 H), 7.09 (dd, 1 H), 6.99 (t, 1 H), 6.96 (df, 1 H), 4.15 (m, 2 H), 3.4/3.02 (m, 2 H), 3.02/2.72 (m, 2 H), 2.9 (m, 2 H), 2.1/1.6 (m, 2 H), 1.75/1.22 (m, 2 H), 1.5 (m, 2 H), 0.92 (m, 2 H)
ESI/FIA/HR et MS/MS : [M+H]+ = 473.1950 (473.1948)
Analyse Elémentaire : C=58.82(58.47);H=6.00(6.19);N=11.80(11.86)
PR : -34.930 (589 nm, T=18°C, C=0.9)

### Intermédiaire 175: (3S)-3-{4-[Bis(tert-butoxycarbonyl)amino]butyl}-4-éthoxy-1-[(4-méthoxy-2-pyridin-4-ylphényl)méthyl]-4-oxo-1,4-azaphosphinane-3-carboxylate de tert-butyle

L'intermédiaire 175 est obtenu à partir des intermédiaires 129 et 268 selon la procédure F précédemment décrite.
RMN ¹H : (400 MHz, dmso-d6) δ ppm 8.61 (d, 2 H), 7.41 (d, 2 H), 7.38 (d, 1 H), 7 (dd, 1 H), 6.8 (d, 1 H), 3.92 (m, 2 H), 3.79 (s, 3 H), 3.49/3.3 (AB, 2 H), 2.73/2.18 (m, 2 H), 2.73 (m, 2 H), 2.67/2.3 (m, 2 H), 1.95-1.8 (m, 2 H), 1.7 (m, 2 H), 1.35 (2s, 18 H), 1.22 (m, 2 H), 1.18 (t, 3 H), 0.69/0.55 (m, 2 H)

### EXEMPLE 225: Acide (3S)-3-(4-aminobutyl)-4-hydroxy-1-[(4-hydroxy-2-pyridin-4-ylphényl)méthyl]-4-oxo-1,4-azaphosphinane-3-carboxylique

L'exemple 225 est obtenu à partir de l'intermédiaire 175 selon la procédure D précédemment décrite.
RMN ¹H : (400 MHz, D2O) δ ppm 8.6 (d, 2 H), 7.5 (d, 1 H), 7.41 (d, 2 H), 7.01 (dd, 1 H), 6.88 (d, 1 H), 4.31/4.2 (AB, 2 H), 3.41/3.05 (m, 2 H), 3.15/2.8 (m, 2 H), 2.97 (m, 2 H), 2.09/1.65 (m, 2 H), 1.85/1.35 (m, 2 H), 1.6 (m, 2 H), 1.09 (m, 2 H)
ESI/FIA/HR et MS/MS : [M+H]+ = 434.1841 (434.1839)
Analyse Elémentaire : C=58.51(58.19);H=5.87(6.51);N=9.77(9.69)
PR : -28.730 (589 nm, T=18°C, C=0.6)

### Intermédiaire 176: (3S)-3-{4-[Bis(tert-butoxycarbonyl)amino]butyl}-4-éthoxy-1-[[4-fluoro-2-(3-fluorophényl)phényl]méthyl]-4-oxo-1,4-azaphosphinane-3-carboxylate de tert-butyle

L'intermédiaire 176 est obtenu à partir des intermédiaires 129 et 269 selon la procédure F précédemment décrite.
RMN ¹H : (400 MHz, dmso-d6) δ ppm 7.52-7.08 (m, 7 H), 3.92 (m, 2 H), 3.51/3.32 (AB, 2 H), 3.3 (m, 2 H), 2.72/2.21 (m, 2 H), 2.69/2.36 (m, 2 H), 2-1.85 (m, 2 H), 1.78 (m, 2 H), 1.4 (s, 18 H), 1.34 (m, 2 H), 1.31 (s, 9 H), 1.18 (m, 3 H), 0.65 (t, 2 H)
RMN ¹⁹F : (400 MHz, dmso-d6) δ ppm -112/-114

### EXEMPLE 226: Acide (3S)-3-(4-aminobutyl)-1-[[4-fluoro-2-(3-fluorophényl)phényl]méthyl]-4-hydroxy-4-oxo-1,4-azaphosphinane-3-carboxylique

L'exemple 226 est obtenu à partir de l'intermédiaire 176 selon la procédure D précédemment décrite.
RMN ¹H : (400 MHz, D2O) δ ppm 7.65 (dd, 1 H), 7.5 (m, 1 H), 7.25 (m, 1 H), 7.25 (d, 1 H), 7.25-7.1 (m, 3 H), 4.4 (d, 1 H), 4.3 (d, 1 H), 3.5-3.35 (m, 1 H), 3.2 (m, 1 H), 3.15 (m, 1 H), 2.95 (m, 2 H), 2.85 (dd, 1 H), 2.1 (m, 1 H), 1.85 (m, 1 H), 1.7 (m, 1 H), 1.6 (m, 2 H), 1.35 (m, 1 H), 1.2-1 (m, 2 H)
ESI/FIA/HR et MS/MS : [M+H]+ = 453.1748 (453.1749)
Analyse Elémentaire : C=58.06(58.40);H=6.00(6.01);N=6.14(6.19)
PR : -15.830 (589 nm, T=20.5°C, C=1.0)

### Intermédiaire 177: (3S)-3-{4-[Bis(tert-butoxycarbonyl)amino]butyl}-4-éthoxy-1-[(4-hydroxy-2-thiophen-2-ylphényl)méthyl]-4-oxo-1,4-azaphosphinane-3-carboxylate de tert-butyle

L'intermédiaire 177 est obtenu à partir des intermédiaires 129 et 255 selon la procédure F précédemment décrite.
RMN ¹H : (400 MHz, dmso-d6) δ ppm 9.55 (s, 1 H), 7.55 (d, 1 H), 7.25 (d, 1 H), 7.2 (d, 1 H), 7.1 (m, 1 H), 6.8 (s, 1 H), 6.75 (dd, 1 H), 4 (m, 2 H), 3.5 (d, 1 H), 3.3 (d, 1 H), 3.25 (m, 2 H), 2.95-2.8 (m, 2 H), 2.4 (dd, 1 H), 2.25 (m, 1 H), 2.05-1.8 (m, 4 H), 1.4 (s, 18 H), 1.35 (s, 9 H), 1.3 (m, 2 H), 1.2 (t, 3 H), 0.65 (m, 2 H)

### EXEMPLE 227: Acide (3S)-3-(4-aminobutyl)-4-hydroxy-1-[(4-hydroxy-2-thiophen-2-ylphényl)méthyl]-4-oxo-1,4-azaphosphinane-3-carboxylique

L'exemple 227 est obtenu à partir de l'intermédiaire 177 selon la procédure D précédemment décrite.
RMN ¹H : (400 MHz, D2O) δ ppm 7.55 (d, 1 H), 7.45 (d, 1 H), 7.2 (m, 1 H), 7.15 (d, 1 H), 6.95 (m, 2 H), 4.45 (d, 1 H), 4.35 (d, 1 H), 3.55-3.4 (m, 1 H), 3.35-3.25 (m, 1 H), 3.15 (m, 1 H), 2.95 (m, 2 H), 2.9 (dd, 1 H), 2.15 (m, 1 H), 1.85 (m, 1 H), 1.7 (m, 1 H), 1.6 (m, 2 H), 1.4 (m, 1 H), 1.2 (m, 1 H), 1.1 (m, 1 H)
ESI/FIA/HR et MS/MS : [M+H]+ = 439.1450 (439.1451)
Analyse Elémentaire : C=55.29(54.78);H=6.41(6.21);N=6.46(6.39);S=7.06(7.31)
PR : -36.090 (589 nm, T=20.5°C, C=1.0)

### Intermédiaire 178: (3S)-3-{4-[Bis(tert-butoxycarbonyl)amino]butyl}-4-éthoxy-1-[[2-(4-fluorophényl)-4-hydroxyphényl]méthyl]-4-oxo-1,4-azaphosphinane-3-carboxylate de tert-butyle

L'intermédiaire 178 est obtenu à partir des intermédiaires 129 et 270 selon la procédure F précédemment décrite.
RMN ¹H : (400 MHz, dmso-d6) δ ppm 9.5 (si, 1 H), 7.39 (dd, 1 H), 7.24 (dd, 1 H), 7.2 (d, 1 H), 6.75 (dd, 2 H), 6.6 (d, 2 H), 3.92 (m, 2 H), 3.31/3.21 (AB, 2 H), 3.3 (m, 2 H), 2.72/2.12 (2*m, 2 H), 2.67/2.3 (2*m, 2 H), 2-1.8 (m, 2 H), 1.8 (m, 2 H), 1.4 (s, 18 H), 1.35 (m, 2 H), 1.32 (s, 9 H), 1.18 (t, 3 H), 0.7 (m, 2 H).
RMN ³¹P : (400 MHz, dmso-d6) δ ppm -114.8

### EXEMPLE 228: Acide (3S)-3-(4-aminobutyl)-1-[[2-(4-fluorophényl)-4-hydroxyphényl]méthyl]-4-hydroxy-4-oxo-1,4-azaphosphinane-3-carboxylique

L'exemple 228 est obtenu à partir de l'intermédiaire 178 selon la procédure D précédemment décrite.
RMN ¹H : (400 MHz, D2O) δ ppm 7.47 (d, 1 H), 7.32 (dd, 2 H), 7.22 (dd, 2 H), 6.98 (dd, 1 H), 6.85 (d, 1 H), 4.32/4.2 (AB, 2 H), 3.37/3.05 (m, 2 H), 3.15/2.8 (m, 2 H), 2.95 (m, 2 H), 2.09/1.65 (m, 2 H), 1.85/1.35 (m, 2 H), 1.59 (m, 2 H), 1.1 (m, 2 H)
RMN ¹⁹F : (400 MHz, D2O) δ ppm -113.5
ESI/FIA/HR et MS/MS : [M+H]+ = 451.1792 (451.1793)
Analyse Elémentaire : C=58.29(58.66);H=6.12(6.27);N=6.20(6.22)
PR : -36.630 (589 nm, T=21°C, C=1.0)

### Intermédiaire 179: (3S)-3-{4-[Bis(tert-butoxycarbonyl)amino]butyl}-1-[[2-(3,4-diméthoxyphényl)-4-hydroxyphényl]méthyl]-4-éthoxy-4-oxo-1,4-azaphosphinane-3-carboxylate de tert-butyle

L'intermédiaire 179 est obtenu à partir des intermédiaires 129 et 256 selon la procédure F précédemment décrite.
RMN ¹H : (400 MHz, dmso-d6) δ ppm 9.4 (m, 1 H), 7.2 (d, 1 H), 7 (d, 1 H), 6.85 (m, 2 H), 6.7 (dd, 1 H), 6.6 (dd, 1 H), 3.95 (m, 2 H), 3.8 (2s, 6 H), 3.4/3.25 (2d, 2 H), 3.3 (m, 2 H), 2.8 (m, 1 H), 2.7 (m, 1 H), 2.3 (dd, 1 H), 2.15 (m, 1 H), 1.9 (m, 1 H), 1.8 (m, 3 H), 1.4/1.25 (2m, 2 H), 1.4 (s, 18 H), 1.35 (s, 9 H), 1.2 (t, 3 H), 0.75 (quint, 2 H)

### EXEMPLE 229: Acide (3S)-3-(4-aminobutyl)-1-[[2-(3,4-diméthoxyphényl)-4-hydroxyphényl]méthyl]-4-hydroxy-4-oxo-1,4-azaphosphinane-3-carboxylique

L'exemple 229 est obtenu à partir de l'intermédiaire 179 selon la procédure D précédemment décrite.
RMN ¹H : (400 MHz, dmso-d6) δ ppm 7.05 (s, 1 H), 7-6.85 (dd;d, 2 H), 6.85 (d, 1 H), 6.25 (dd, 1 H), 6.15 (s, 1 H), 3.75 (s, 6 H), 3.2 (d, 1 H), 3.05 (d, 1 H), 3-2.85 (m, 1 H), 2.8-2.65 (m, 1 H), 2.45-2.25 (m, 3 H), 2.15 (m, 1 H), 1.85-1.7 (m, 2 H), 1.65 (m, 1 H), 1.3-1.15 (m, 3 H), 0.9 (m, 1 H), 0.8 (m, 1 H)
ESI/FIA/HR et MS/MS : ESI-HR +/- : [M+H]+ = 493.2098 (493.2098)
Analyse Elémentaire : C=58.52(58.53);H=6.32(6.75);N=5.48(5.69)
PR : -32.470 (589 nm, T=20°C, C=0.9)

### EXEMPLE 230: Acide (3S)-3-(4-aminobutyl)-4-hydroxy-1-[(4-hydroxyphényl)méthyl]-4-oxo-1,4-azaphosphinane-3-carboxylique

L'exemple 230 est obtenu à partir de l'intermédiaire 129 et du 4-hydroxybenzaldéhyde selon les procédures F et D précédemment décrites.
RMN ¹H : (400 MHz, D2O) δ ppm 7.32 (d, 2 H), 6.9 (d, 2 H), centré à 4.2 (AB, 2 H), 3.7/3.28 (2m, 2 H), 3.45/3.05 (2dd, 2 H), 2.9 (m, 2 H), 2.21/1.75 (2m, 2 H), 1.9/1.45 (2m, 2 H), 1.59 (m, 2 H), 1.22/1.1 (2m, 2 H)
ESI/FIA/HR et MS/MS : [M+H]+ = 357.1577 (357.1574)
Analyse Elémentaire : C=54.12(53.93);H=6.96(7.07);N=7.93(7.86)
PR : -56.580 (589 nm, T=20°C, C=1.0)

### EXEMPLE 231: Acide (3S)-3-(4-aminobutyl)-1-[[2-(4-chlorophényl)phényl]méthyl]-4-hydroxy-4-oxo-1,4-azaphosphinane-3-carboxylique

L'exemple 231 est obtenu à partir de l'intermédiaire 129 et du 2-(4-chlorophényl)benzaldéhyde selon les procédures F et D précédemment décrites.
RMN ¹H : (300 MHz, D2O) δ ppm 7.59 (m, 1 H), 7.51 (m, 2 H), 7.51 (d, 2 H), 7.38 (m, 1 H), 7.31 (d, 2 H), 4.33 (AB, 2 H), 3.39/3.08 (2m, 2 H), 3.15/2.83 (2m, 2 H), 2.92 (m, 2 H), 2.07/1.62 (2m, 2 H), 1.83/1.34 (2m, 2 H), 1.56 (m, 2 H), 1.06 (m, 2 H)
ESI/FIA/HR et MS/MS : [M+H]+ = 451.1541 (451.1553)
Analyse Elémentaire : C=58.91(58.60);H=6.12(6.26);N=5.85(6.21)
PR : -10.140 (589 nm,T=20°C,C=0.8)

### EXEMPLE 232: Acide (3S)-3-(4-aminobutyl)-4-hydroxy-1-[[2-[2-méthyl-5-(trifluorométhyl)pyrazol-3-yl]phényl]méthyl]-4-oxo-1,4-azaphosphinane-3-carboxylique

L'exemple 232 est obtenu à partir de l'intermédiaire 129 et du 2-[1-méthyl-3(trifluorométhyl)-1H-pyrazol-5-yl]benzaldéhyde selon les procédures F et D précédemment décrites.
RMN ¹H : (300 MHz, D2O) δ ppm 7.63/7.46 (m, 4 H), 6.81 (s, 1 H), 4.21 (AB, 2 H), 3.65 (s, 3 H), 3.5/3.19 (2m, 2 H), 3.26/3 (2m, 2 H), 2.91 (m, 2 H), 2.15/1.69 (2m, 2 H), 1.87/1.4 (2m, 2 H), 1.58 (m, 2 H), 1.11 (m, 2 H)
RMN ¹⁹F: (300 MHz, D2O) δ ppm -61.8
ESI/FIA/HR et MS/MS : [M+H]+ = 489.1869 (489.1878)
Analyse Elémentaire : C=51.64(51.64);H=6.03(5.78);N=11.47(11.47)
PR : -27.030 (589 nm,T=20°C,C=0.9)

### EXEMPLE 233: Acide (3S)-3-(4-aminobutyl)-4-hydroxy-4-oxo-1-[[2-(trifluorométhyl)phényl]méthyl]-1,4-azaphosphinane-3-carboxylique

L'exemple 233 est obtenu à partir de l'intermédiaire 129 et du 2-trifluorométhylbenzaldéhyde selon les procédures F et D précédemment décrites.
RMN ¹H : (300 MHz, D2O) δ ppm 7.88-7.6 (m, 4 H), 4.51 (AB, 2 H), 3.69/3.43 (2m, 2 H), 3.48/3.25 (2m, 2 H), 2.92 (m, 2 H), 2.22/1.78 (2m, 2 H), 1.93/1.49 (2m, 2 H), 1.59 (m, 2 H), 1.23/1.09 (2m, 2 H)
RMN ¹⁹F : (300 MHz, D2O) δ ppm -58.3
ESI/FIA/HR et MS/MS : [M+H]+ = 409.1492 (409.1499)
Analyse Elémentaire : C=49.52(50.00);H=5.85(5.92);N=7.00(6.86)
PR : -38.120 (589 nm,T=20°C,C=1.0

### EXEMPLE 234: Acide (3S)-3-(4-aminobutyl)-1-[(4-fluoro-2-pyrimidin-5-ylphényl)méthyl]-4-hydroxy-4-oxo-1,4-azaphosphinane-3-carboxylique

L'exemple 234 est obtenu à partir de l'intermédiaire 129 et du 4-fluoro-(2-pyrimidin-5-yl)benzaldéhyde selon les procédures F et D précédemment décrites.
RMN ¹H : (300 MHz, D2O) δ ppm 9.2 (s, 1 H), 8.82 (s, 2 H), 7.7 (dd, 1 H), 7.35 (td, 1 H), 7.2 (dd, 1 H), 4.38/4.28 (2*d, 2 H), 3.5/3.15 (2*m, 2 H), 3.25/2.9 (2*m, 2 H), 2.9 (t, 2 H), 2.1/1.6 (2*m, 2 H), 1.85/1.6 (2*m, 2 H), 1.6/1.1 (2*m, 2 H), 1.38/1.1 (2*m, 2 H)
ESI/FIA/HR et MS/MS : [M+H]+ = 437.1740 (437.1753)
Analyse Elémentaire : C=54.96(55.04);H=4.82(6.00);N=12.78(12.84)
PR: -23.070 (589 nm, T=20°C, C=1.1)

### EXEMPLE 235: Acide (3S)-3-(4-aminobutyl)-4-hydroxy-1-[[2-(2-méthylpyrazol-3-yl)phényl]méthyl]-4-oxo-1,4-azaphosphinane-3-carboxylique

L'exemple 235 est obtenu à partir des intermédiaires 129 et 280 selon les procédures F et D précédemment décrites.
RMN ¹H : (300 MHz, D2O) δ ppm 7.7-7.35 (m, 4 H), 7.65 (d, 1 H), 6.45 (d, 1 H), 4.25/4.15 (dd, 2 H), 3.6 (s, 3 H), 3.5/3.2 (2m, 2 H), 3.3/3 (dd, 2 H), 2.9 (m, 2 H), 2.15/1.7 (2m, 2 H), 1.9/1.4 (2m, 2 H), 1.6 (m, 2 H), 1.3-1 (m, 2 H)
ESI/FIA/HR et MS/MS : [M+H]+ = 421.1998 (421.2004)
Analyse Elémentaire : C=57.19(57.13);H=7.08(6.95);N=13.43(13.33)
PR : -29.970 (589 nm,T=19.5°C,C=1.0)

### EXEMPLE 236: Acide (3S)-3-(4-aminobutyl)-1-[[4-fluoro-2-(2-méthylpyrazol-3-yl)phényl] méthyl] -4-hydroxy-4-oxo-1,4-azaphosphinane-3-carboxylique

L'exemple 236 est obtenu à partir des intermédiaires 129 et 277 selon les procédures F et D précédemment décrites.
RMN ¹H : (400 MHz, D2O) δ ppm 7.7 (dd, 1 H), 7.65 (d, 1 H), 7.39 (td, 1 H), 7.24 (dd, 1 H), 6.5 (d, 1 H), 4.25/4.13 (AB, 2 H), 3.61 (s, 3 H), 3.48/3.18 (m, 2 H), 3.29/3 (m, 2 H), 2.94 (m, 2 H), 2.17/1.7 (m, 2 H), 1.9/1.41 (m, 2 H), 1.6 (m, 2 H), 1.2/1.1 (m, 2 H)
ESI/FIA/HR et MS/MS : [M+H]+ = 439.1903 (439.1905)
Analyse Elémentaire : C=54.74(54.79);H=6.38(6.44);N=12.57(12.78)
PR : -26.790 (589 nm, T=21°C, C=1.0)

### EXEMPLE 237: Acide (3S)-3-(4-aminobutyl)-4-hydroxy-1-[(2-imidazo[1,2-a]pyridin-3-ylphényl)méthyl]-4-oxo-1,4-azaphosphinane-3-carboxylique

L'exemple 237 est obtenu à partir des intermédiaires 129 et 227 selon les procédures F et D précédemment décrites.
RMN ¹H : (300 MHz, D2O) δ ppm 7.98 (d, 1 H), 7.68 (s, 1 H), 7.65 (d, 1 H), 7.65 (m, 2 H), 7.52 (m, 1 H), 7.45 (dd, 1 H), 7.22 (m, 1 H), 6.98 (t, 1 H), 4.25 (m, 2 H), 3.45/3.1 (2*m, 2 H), 2.9 (m, 2 H), 2.85 (m, 2 H), 2.12/1.55 (2*m, 2 H), 1.8/1.55 (2*m, 2 H), 1.55/0.95 (2*m, 2 H), 1.28/0.95 (2*m, 2 H)
ESI/FIA/HR et MS/MS : [M+H]+ = 457.1993 (457.1999)
Analyse Elémentaire : C=60.73(60.52);H=6.00(6.40);N=12.29(12.27)
PR : -33.550 (589 nm,T=19°C,C=0.9)

### EXEMPLE 238: Acide (3S)-3-(4-aminobutyl)-1-[[4-fluoro-2-(3-hydroxyphényl)phényl]méthyl]-4-hydroxy-4-oxo-1,4-azaphosphinane-3-carboxylique

L'exemple 238 est obtenu à partir des intermédiaires 129 et 254 selon les procédures F et D précédemment décrites.
RMN ¹H : (400 MHz, dmso-d6) δ ppm 7.6 (dd, 1 H), 7.4 (m, 1 H), 7.25 (m, 1 H), 7.15 (d, 1 H), 6.95 (dd, 1 H), 6.9 (dd, 1 H), 6.85 (s, 1 H), 4.4 (d, 1 H), 4.25 (d, 1 H), 3.5-3.3 (m, 1 H), 3.25-3 (m, 2 H), 2.95 (m, 2 H), 2.85 (dd, 1 H), 2.1 (m, 1 H), 1.85 (m, 1 H), 1.65 (m, 1 H), 1.6 (m, 2 H), 1.35 (m, 1 H), 1.2-1 (m, 2 H)
ESI/FIA/HR et MS/MS : [M+H]+ = 451.1794 (451.1793)
Analyse Elémentaire : C=58.81(58.66);H=5.81(6.27);N=6.19(6.22)
PR : -19.520 (589 nm, T=21°C, C=1.0)

### EXEMPLE 239: Acide (3S)-3-(4-aminobutyl)-1-[[4-fluoro-2-(6-méthoxypyridin-3-yl)phényl] méthyl] -4-hydroxy-4-oxo-1,4-azaphosphinane-3-carboxylique

L'exemple 239 est obtenu à partir de l'intermédiaire 129 et du 4-fluoro-2-(6-méthoxy-3-pyridinyl)benzaldéhyde selon les procédures F et D précédemment décrites.
RMN ¹H : (400 MHz, D2O) δ ppm 8.1 (d, 1 H), 7.8 (dd, 1 H), 7.65 (dd, 1 H), 7.3 (td, 1 H), 7.15 (dd, 1 H), 7 (d, 1 H), 4.4/4.3 (2 d, 2 H), 3.9 (s, 3 H), 3.45 (m, 1 H), 3.2 (dd, 1 H), 3.1 (m, 1 H), 2.95 (m, 2 H), 2.85 (dd, 1 H), 2.1 (m, 1 H), 1.85 (m, 1 H), 1.65 (m, 1 H), 1.6 (m, 2 H), 1.35 (m, 1 H), 1.1 (m, 2 H)
ESI/FIA/HR et MS/MS : [M+H]+ = 466.1903 (466.1902)
Analyse Elémentaire : C=57.18(56.77);H=6.17(6.28);N=9.05(9.03)
PR : -11.850 (589 nm,T=20°C,C=0.9)

### EXEMPLE 240: Acide (3S)-3-(4-aminobutyl)-4-hydroxy-1-[[2-(6-hydroxypyridin-3-yl)phényl] méthyl] -4-oxo-1,4-azaphosphinane-3-carboxylique

L'exemple 240 est obtenu à partir de l'intermédiaire 129 et du 2-(6-méthoxy-3-pyridinyl)benzaldéhyde selon les procédures F et D précédemment décrites.
RMN ¹H : (400 MHz, D2O) δ ppm 7.7 (dd, 1 H), 7.6 (d, 1 H), 7.55-7.4 (2 m, 4 H), 6.7 (d, 1 H), 4.4 (2 d coal., 2 H), 3.5 (m, 1 H), 3.3 (dd, 1 H), 3.2 (m, 1 H), 3 (dd, 1 H), 2.95 (m, 2 H), 2.1 (m, 1 H), 1.9 (m, 1 H), 1.7 (m, 1 H), 1.6 (m, 2 H), 1.4 (m, 1 H), 1.1 (m, 2 H)
ESI/FIA/HR et MS/MS : [M+H]+ = 434.1840 (434.1839)
Analyse Elémentaire : C=58.14(58.19);H=6.38(6.51);N=9.60(9.69)
PR : -8.440 (589 nm,T=20°C,C=0.8)

### EXEMPLE 241: Acide (3S)-3-(4-aminobutyl)-1-[[4-fluoro-2-(6-hydroxypyridin-3-yl)phényl]méthyl]-4-hydroxy-4-oxo-1,4-azaphosphinane-3-carboxylique

L'exemple 241 est obtenu à partir de l'intermédiaire 129 et du 4-fluoro-2-(6-méthoxy-3-pyridinyl)benzaldéhyde selon les procédures F et D précédemment décrites.
RMN ¹H : (400 MHz, D2O) δ ppm 7.7 (dd, 1 H), 7.6 (m, 2 H), 7.3 (td, 1 H), 7.2 (dd, 1 H), 6.7 (d, 1 H), 4.35 (2 d coal., 2 H), 3.5 (m, 1 H), 3.25 (m, 1 H), 3.2 (m, 1 H), 3 (dd, 1 H), 2.95 (m, 2 H), 2.1 (m, 1 H), 1.9 (m, 1 H), 1.7 (m, 1 H), 1.6 (quint, 2 H), 1.4 (m, 1 H), 1.1 (m, 2 H)
ESI/FIA/HR et MS/MS : [M+H]+ = 452.1746 (452.1745)
Analyse Elémentaire : C=55.62(55.87);H=5.85(6.03);N=9.16(9.31)
PR : -7.630 (589 nm,T=20°C,C=0.7)

### EXEMPLE 242: Acide (3S)-3-(4-aminobutyl)-4-hydroxy-1-[[4-hydroxy-2-(6-méthoxypyridin-3-yl)phényl]méthyl]-4-oxo-1,4-azaphosphinane-3-carboxylique

L'exemple 242 est obtenu à partir des intermédiaires 129 et 230 selon les procédures F et D précédemment décrites.
RMN ¹H : (400 MHz, D2O) δ ppm 7.95 (d, 1 H), 7.6 (dd, 1 H), 7.4 (d, 1 H), 6.9 (m, 2 H), 6.7 (d, 1 H), 4.2-4.1(2d., 2 H), 3.8 (s, 3 H), 3.3-2.95 (2m, 2 H), 3.1-2.7 (2m, 2 H), 2.85 (m, 2 H), 2.0-1.5 (2m, 2 H), 1.75-1.25 (2m, 2 H), 1.5 (m, 2 H), 1.0 (m, 2 H)
Analyse Elémentaire : C=56.79(57.01);H=6.62(6.52);N=8.99(9.07)
PR : -27.320 (589 nm,T=20°C,C=0.8)

### EXEMPLE 243: Acide (3S)-3-(4-aminobutyl)-4-hydroxy-4-oxo-1-[(2-oxo-1,3-dihydrobenzimidazol-5-yl)méthyl]-1,4-azaphosphinane-3-carboxylique

L'exemple 243 est obtenu à partir de l'intermédiaire 129 et du 2-oxo-1,3-dihydrobenzothiazole-5-carbaldéhyde selon les procédures F et D précédemment décrites.
RMN ¹H : (400 MHz, D2O) δ ppm 7.25 (s, 1 H), 7.2 (m, 2 H), 4.45 (d, 1 H), 4.25 (d, 1 H), 3.8-3.65 (m, 1 H), 3.55-3.4 (m, 1 H), 3.35 (m, 1 H), 3.1 (dd, 1 H), 2.95 (m, 2 H), 2.3-2.15 (m, 1 H), 2-1.85 (m, 1 H), 1.85-1.7 (m, 1 H), 1.6 (m, 2 H), 1.55-1.4 (m, 1 H), 1.3-1 (m, 2 H) ESI/FIA/HR et MS/MS : [M+H]+ = 397.1635 (397.1635)

### EXEMPLE 244: Acide (3S)-3-(4-aminobutyl)-1-[[4-fluoro-2-(6-méthoxypyridin-2-yl)phényl] méthyl] -4-hydroxy-4-oxo-1,4-azaphosphinane-3-carboxylique

L'exemple 244 est obtenu à partir des intermédiaires 129 et 231 selon les procédures F et D précédemment décrites.
RMN ¹H : (300 MHz, D2O) δ ppm 8.2 (t, 1 H), 7.8 (dd, 1 H), 7.7 (dd, 1 H), 7.55 (m, 2 H), 7.2 (d, 1 H), 4.7 (d, 1 H), 4.5 (d, 1 H), 4.2 (s (+m, 3 H), 3.9 (m, 1 H), 3.6 (m, 3 H), 3.2 (t (+m, 2 H), 2.6 (m, 1 H), 2.05 (m, 2 H), 1.8 (m, 2 H), 1.7 (m, 1 H), 1.4/1.3 (2m, 2 H)
RMN ¹⁹F: (300 MHz, D2O) δ ppm -110.9
ESI/FIA/HR et MS/MS : [M+H]+ = 466.1901 (466.1902)
Analyse Elémentaire : C=57.09(56.77);H=6.16(6.28);N=8.87(9.03)
PR : -69.790 (589 nm,T=19°C,C=0.8)

### EXEMPLE 245: Acide (3S)-3-(4-aminobutyl)-1-[[4-fluoro-2-(1,3-thiazol-2-yl)phényl]méthyl]-4-hydroxy-4-oxo-1,4-azaphosphinane-3-carboxylique

L'exemple 245 est obtenu à partir des intermédiaires 129 et 232 selon les procédures F et D précédemment décrites.
RMN ¹H : (300 MHz, D2O) δ ppm 8 (d, 1 H), 7.7 (m, 2 H), 7.7 (dd, 1 H), 7.3 (td, 1 H), 4.5 (d, 1 H), 4.3 (d, 1 H), 3.85 (m, 1 H), 3.45 (m, 2 H), 3.15 (dd, 1 H), 2.9 (m, 2 H), 2.3 (m, 1 H), 1.85 (m, 2 H), 1.55 (m, 3 H), 1.2 (m, 1 H), 1 (m, 1 H)
RMN ¹⁹F: (300 MHz, D2O) δ ppm -109
ESI/FIA/HR et MS/MS : [M+H]+ = 442.1358 (442.1360)
Analyse Elémentaire : C=51.13(51.69);H=5.30(5.71);N=9.42(9.52);S=7.25(7.26)
PR : -99.540 (589 nm,T=18°C,C=0.8)

### EXEMPLE 246: Acide (3S)-3-(4-aminobutyl)-4-hydroxy-1-[[4-hydroxy-2-(3-méthylimidazol-4-yl)phényl]méthyl] -4-oxo-1,4-azaphosphinane-3-carboxylique, bromhydrate

L'exemple 246 est obtenu à partir des intermédiaires 129 et 276 selon les procédures F et D précédemment décrites.
RMN ¹H : (300 MHz, D2O) δ ppm 8.85 (s, 1 H), 7.65 (d, 1 H), 7.6 (s, 1 H), 7.2 (dd, 1 H), 7 (d, 1 H), 4.4 (m, 1 H), 4.15 (m, 1 H), 3.7 (m, 1 H), 3.6 (s, 3 H), 3.55-3.2 (2 m, 2 H), 3.1 (m, 1 H), 2.9 (m, 2 H), 2.15 (m, 1 H), 1.9 (m, 2 H), 1.6 (m, 3 H), 1.25 (2 m, 2 H)
ESI/FIA/HR et MS/MS : [M+H]+ = 437.1947 (437.1948)
Analyse Elémentaire : C=39.13(40.15);H=5.22(5.22);N=8.85(9.37)
PR : -15.390 (589 nm, T=21°C, C=1.0)

### EXEMPLE 247: Acide (3S)-3-(4-aminobutyl)-4-hydroxy-1-[[4-hydroxy-2-(2-méthylpyrazol-3-yl)phényl]méthyl] -4-oxo-1,4-azaphosphinane-3-carboxylique

L'exemple 247 est obtenu à partir de l'intermédiaire 129 et du 4-hydroxy-2-(2-méthylpyrazol-3-yl)benzaldéhyde selon les procédures F et D précédemment décrites.
RMN ¹H : (400 MHz, D2O) δ ppm 7.63 (s, 1 H), 7.52 (dd, 1 H), 7.08 (d, 1 H), 6.9 (s, 1 H), 6.44 (d, 1 H), 4.18/4.05 (2m, 2 H), 3.61 (s, 3 H), 3.45/3.15 (2m, 2 H), 3.26/2.95 (2dd, 2 H), 2.95 (m, 2 H), 2.15/1.68 (2m, 2 H), 1.9/1.4 (2m, 2 H), 1.6 (m, 2 H), 1.2/1.1 (2m, 2 H)
ESI/FIA/HR et MS/MS : [M+H]+ = 437.1947 (437.1948)
Analyse Elémentaire : C=55.22(55.04);H=6.48(6.70);N=12.72(12.84)
PR : -44.210 (589 nm,T=20°C,C=1.0)

### EXEMPLE 248: Acide (3S)-3-(4-aminobutyl)-1-[[2-(3,4-diméthoxyphényl)phényl]méthyl] -4-hydroxy-4-oxo-1,4-azaphosphinane-3-carboxylique

L'exemple 248 est obtenu à partir de l'intermédiaire 129 et du 2-(3,4-diméthoxyphényl)benzaldéhyde selon les procédures F et D précédemment décrites.
RMN ¹H : (300 MHz, D2O) δ ppm 7.6 (dd, 1 H), 7.5 (m, 2 H), 7.35 (dd, 1 H), 7.1 (d, 1 H), 7 (d, 1 H), 6.9 (dd, 1 H), 4.45/4.3 (2 d, 2 H), 3.85 (2 s, 6 H), 3.35/3.1 (2 m, 2 H), 3.1/2.9 (m + dd, 2 H), 2.95 (m, 2 H), 2.1/1.65 (2 m, 2 H), 1.85/1.35 (2 m, 2 H), 1.6 (m, 2 H), 1.05 (m, 2 H)
ESI/FIA/HR et MS/MS : [M+H]+ = 477.2147 (477.2149)
Analyse Elémentaire : C=60.13(60.50);H=6.84(6.98);N=5.68(5.88)
PR : -21.710 (589 nm,T=18°C,C=1.0)

### EXEMPLE 249: Acide (3S)-3-(4-aminobutyl)-1-[[2-(2,3-diméthylimidazol-4-yl)-4-hydroxyphényl]méthyl] -4-hydroxy-4-oxo-1,4-azaphosphinane-3-carboxylique

L'exemple 249 est obtenu à partir des intermédiaires 129 et 275 selon les procédures F et D précédemment décrites.
RMN ¹H : (400 MHz, D2O) δ ppm 7.55 (d, 1 H), 7.45 (s, 1 H), 7.15 (dd, 1 H), 6.95 (s, 1 H), 4.15 (si, 2 H), 3.65-3.5 (m, 1 H), 3.45 (s, 3 H), 3.35 (m, 1 H), 3.2 (m, 1 H), 2.95 (dd, 1 H), 2.95 (t, 2 H), 2.65 (s, 3 H), 2.15 (m, 1 H), 1.9 (m, 1 H), 1.75 (m, 1 H), 1.6 (m, 2 H), 1.45 (m, 1 H), 1.35-1.05 (2m, 2 H)
ESI/FIA/HR et MS/MS : [M+H]+ = 451.2102 (451.2105)
Analyse Elémentaire : C=46.98(47.47);H=6.05(6.07);N=10.40(10.54)
PR : -37.730 (589 nm, T=20.5°C, C=1.0)

### EXEMPLE 250: Acide (3S)-3-(4-aminobutyl)-1-[[2-(2,3-diméthylimidazol-4-yl)phényl] méthyl] -4-hydroxy-4-oxo-1,4-azaphosphinane-3-carboxylique

L'exemple 250 est obtenu à partir des intermédiaires 129 et 226 selon les procédures F et D précédemment décrites.
RMN ¹H : (400 MHz, D2O) δ ppm 7.75-7.45 (m, 4 H), 7.45 (s, 1 H), 4.25 (m, 2 H), 3.5/3.2 (2m, 2 H), 3.4/3.1 (m, 2 H), 3.4 (s, 3 H), 2.9 (m, 2 H), 2.65 (s, 3 H), 2.1/1.7 (2m, 2 H), 1.9/1.45 (2m, 2 H), 1.6 (m, 2 H), 1.25/1.1 (2m, 2 H)
ESI/FIA/HR et MS/MS : [M+H]+ = 435.2179 (435.2161)
Analyse Elémentaire : C=49.57(50.37);H=5.92(5.88);N=9.92(10.21)
PR : -23.310 (589 nm, T=20°C, C=0.9)

### EXEMPLE 251: Acide (3S)-3-(4-aminobutyl)-1-[[4-fluoro-2-(4-fluoro-3-hydroxyphényl)phényl]méthyl]-4-hydroxy-4-oxo-1,4-azaphosphinane-3-carboxylique

L'exemple 251 est obtenu à partir de l'intermédiaire 129 et du 4-fluoro-2-(4-fluoro-3-hydroxyphényl)benzaldéhyde selon les procédures F et D précédemment décrites.
RMN ¹H : (400 MHz, D2O) δ ppm 7.6 (d, 1 H), 7.25 (2*m, 2 H), 7.15 (d, 1 H), 6.95 (d, 1 H), 6.8 (m, 1 H), 4.4 (d, 1 H), 4.3 (d, 1 H), 3.5-3.35 (m, 1 H), 3.2-3.05 (m, 2 H), 2.95 (m, 2 H), 2.85 (dd, 1 H), 2.1 (m, 1 H), 1.85 (m, 1 H), 1.65 (m, 1 H), 1.6 (m, 2 H), 1.35 (m, 1 H), 1.2-1 (m, 2 H)
ESI/FIA/HR et MS/MS : [M+H]+ = 469.1696 (469.1698)
Analyse Elémentaire : C=56.82(56.41);H=5.77(5.81);N=6.02(5.98)
PR : -12.950 (589 nm, T=21°C, C=1.0)

### EXEMPLE 252: Acide (3S)-3-(4-aminobutyl)-4-hydroxy-4-oxo-1-[(2-pyridin-4-ylphényl)méthyl]-1,4-azaphosphinane-3-carboxylique

L'exemple 252 est obtenu à partir de l'intermédiaire 129 et du 2-(4-pyridyl)benzaldéhyde selon les procédures F et D précédemment décrites.
RMN ¹H : (400 MHz, D2O) δ ppm 8.6 (d, 2 H), 7.64 (m, 1 H), 7.57 (m, 2 H), 7.42 (d, 2 H), 7.41 (m, 1 H), 4.42/4.3 (dd, 2 H), 3.44/3.09 (dd, 2 H), 3.18/2.88 (dd, 2 H), 2.93 (m, 2 H), 2.09/1.64 (2*m, 2 H), 1.85/1.35 (2*m, 2 H), 1.59 (m, 2 H), 1.08 (m, 2 H)
ESI/FIA/HR et MS/MS : [M+H]+ = 418.1897 (418.1895)
Analyse Elémentaire : C=61.00(60.42);H=6.62(6.76);N=10.20(10.07)
PR : -17.980 (589 nm,T=28°C,C=0.9)

### Procédure H : Synthèse des azaphosphépanes

### Intermédiaire 180

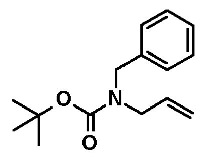

A une suspension de NaH à 60% (11.45 g, 285 mmol, 1.5 eq) dans du THF (100 mL) et sous atmosphère d'Argon, est additionnée goutte à goutte une solution de *N*-Boc allylamine (30 g, 190 mmol) dans du THF (170 mL). Le mélange est laissé en contact 1h30 puis le bromure de benzyle (34 mL, 285 mmol, 1.5 eq) en solution dans du THF (30 mL) est additionné. Le milieu réactionnel est agité à température ambiante pendant 48h. Le THF est évaporé sous pression réduite et l'évaporat est repris dans du DCM (10 mL) refroidi dans un bain glace-eau puis coulé lentement sur H₂O (100 mL). La phase organique est décantée, la phase aqueuse est ré-extraite par DCM (2 x 100 mL). Les phases organiques rassemblées sont lavées par H₂O (2 x 50 mL), séchées sur MgSO₄ puis évaporées. Le résidu obtenu est purifié par flash chromatographie sur gel de silice en utilisant comme éluant un gradient Heptane/DCM (de 50:50 à 0:100). L'intermédiaire 180 est obtenu sous forme d'une huile incolore (39.8 g, 160.92 mmol) avec un rendement de 85%.
RMN ¹H : (400 MHz, DMSO-d6) δ ppm 7.65 (t, 2 H), 7.22 (m, 3 H), 5.75 (m, 1 H), 5.1 (m, 2 H), 4.33 (s, 2 H), 3.75 (m, 2 H), 1.39 (s, 9 H)

### Intermédiaire 181 :

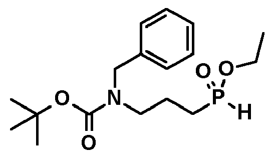

A une solution d'acide hypophosphoreux (11.42 g, 173 mmol, 2 eq) dans de l'acétonitrile (224 mL) à 0°C et sous atmosphère d'Argon, est additionné goutte à goutte le tétraéthoxysilane (38.35 mL, 173 mmol, 2 eq). Après retour à température ambiante, sont ajoutés au milieu réactionnel (dégazé à l'Argon) l'intermédiaire 180 (21.4 g, 86.5 mmol) en solution dans MeCN (44.7 mL), le Xantphos (0.55 g, 11 mmol) puis du Pd₂dba₃ (0.396 g, 5 mmol). Le mélange est chauffé au reflux pendant 16h. Après concentration sous pression réduite, le résidu obtenu est purifié par flash chromatographie sur gel de silice en utilisant comme éluant un gradient AcOEt/EtOH (de 95:5 à 90:10). L'intermédiaire 181 est obtenu sous forme d'une huile incolore (10.1 g, 29.58 mmol) avec un rendement de 34%.
RMN ¹H : (400 MHz, DMSO-d6) δ ppm 7.32 (t, 2 H), 7.22 (m, 3 H), 6.95 (d, 1 H), 4.38 (s, 2 H), 3.99 (m, 2 H), 3.19 (m, 2 H), 1.65 (m, 4 H), 1.4 (m, 9 H), 1.21 (t, 3 H)
RMN ³¹P : (400 MHz, DMSO-d6) δ ppm 40.7

### Intermédiaire 182 :

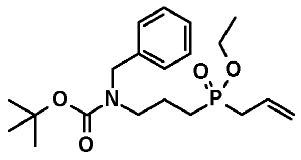

A une solution de l'intermédiaire 181 (10.1 g, 29.6 mmol) dans du THF (100 mL) préalablement dégazée avec de l'Argon, est additionnée une solution de LiHMDS 1M dans du THF (29.6 mL, 29.6 mmol, 1 eq). Après 30 min d'agitation à -70°C, le bromure d'allyle (2.56 mL, 29.6 mmol, 1 eq) est additionné. Le milieu réactionnel est agité 2 h à température ambiante puis coulé sur une solution aqueuse saturée en NH₄Cl. Le mélange est extrait avec DCM (3 x 100 mL), la phase organique est lavée avec H₂O (2 x 100 mL), séchée sur Na₂SO₄. Après concentration sous pression réduite, le résidu obtenu est purifié par flash chromatographie sur gel de silice en utilisant comme éluant un gradient AcOEt/EtOH (de 100% - 90:10). L'intermédiaire 182 attendu est obtenu sous forme d'une huile incolore (9.3 g, 24.3 mmol) avec un rendement de 82%.
RMN ¹H : (400 MHz, DMSO-d6) δ ppm 7.4-7.2 (m, 5 H), 5.2 (m, 1 H), 5.18 (2dd, 2 H), 4.4 (s, 2 H), 3.91 (quad, 2 H), 3.15 (m, 2 H), 2.6 (dd, 2 H), 1.6 (m, 4 H), 1.4 (si, 9 H), 1.2 (t, 3 H)

### Intermédiaire 183 :

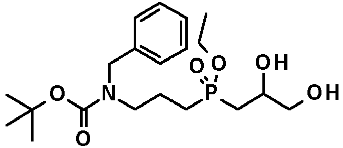

A une solution de l'intermédiaire 182 (9.3 g, 24.3 mmol) et de 4-méthylmorpholine *N*-oxyde (3.14 g, 26.7 mmol, 1.1 eq) dans 100 mL d'un mélange acétone/H₂O (2:1) est additionnée goutte à goutte une solution d'OsO₄ à 4% dans H₂O (4.64 mL, 0.73 mmol) à température ambiante. Le milieu réactionnel est agité à température ambiante pendant 16h puis coulé sur une solution de métabisulfite de sodium à 10% dans H₂O (100 mL). Le mélange est extrait avec de l'AcOEt (2 x 100 mL). Les phases organiques sont rassemblées et lavées avec H₂O (1 x 100 mL), séchées sur MgSO₄ puis concentrées sous vide. L'intermédiaire 183 (9.8 g, 23.58 mmol) est obtenu sans autre purification sous forme d'une huile foncée avec un rendement de 97%.
RMN ¹H : (400 MHz, DMSO-d6) δ ppm 7.35-7.2 (m, 5 H), 4.8/4.78 (2d, 1 H), 4.65 (2t, 1 H), 4.36 (s, 2 H), 3.9 (m, 2 H), 3.75 (m, 1 H), 3.35-3.2 (m, 2 H), 3.15 (m, 2 H), 1.9/1.7 (m, 2 H), 1.63 (m, 4 H), 1.4 (m, 9 H), 1.17 (t, 3 H)
RMN ³¹P : (400 MHz, DMSO-d6) δ ppm 58.6/57.7

### Intermédiaire 184 :

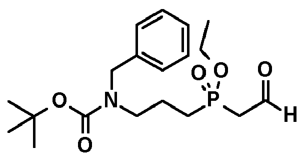

A une solution de l'intermédiaire 183 (9.79 g, 23.5 mmol) dans 320 mL d'un mélange THF/H₂O (3:1) à température ambiante est additionné par portions le sodium periodate (20.16 g, 94.25 mmol, 4 eq). L'agitation est maintenue pendant 72h à température ambiante. Le milieu réactionnel est alors extrait avec de l'AcOEt (2 x 150 mL), les phases organiques sont rassemblées et lavées avec H₂O (1 x 100 mL), séchées sur MgSO₄ puis concentrées sous vide. L'intermédiaire 184 (8.13 g, 21.2 mmol) est obtenu sans autre purification sous forme d'une huile avec un rendement de 90%.
RMN ¹H : (400 MHz, dmso-d6) δ ppm 9.6 (m, 1 H), 7.35-7.2 (m, 5 H), 4.38 (s, 2 H), 4-3.75 (m, 2 H), 3.25 (dd, 2 H), 3.15 (m, 2 H), 1.65 (m, 4 H), 1.4 (m, 9 H), 1.2 (m, 3 H)

### Intermédiaire 186 :

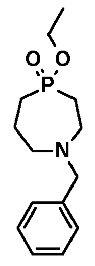

Une solution de l'intermédiaire 184 (8.13 g, 21.2 mmol) dans HCl 2N (53 mL, 106 mmol, 5 eq) est agitée pendant 4h à température ambiante. Le mélange réactionnel est concentré sous vide pour conduire à l'intermédiaire 185 utilisé directement sans autre purification. L'intermédiaire 185 est mis en solution dans du DCM (150 mL) et agité 1h avec du MgSO₄ (10 g). NaBH(OAc)₃ (6.75 g, 31,8 mmol, 1.5 eq) est alors additionné par portions à 0°C et le milieu réactionnel est agité à température ambiante pendant 16h. Après filtration des insolubles, la phase organique est lavée avec une solution aqueuse à 10% de NaHCO₃ (2 x 100 mL) puis par H₂O (1 x 100mL), séchée sur MgSO₄. Après concentration sous pression réduite, le résidu obtenu est purifié par flash chromatographie sur gel de silice en utilisant comme éluant un gradient DCM/EtOH (98:2 à 94:6). Le produit attendu est obtenu sous forme d'une huile incolore (1.9 g, 7.1 mmol) avec un rendement de 33%.
RMN ¹H : (400 MHz, DMSO-d6) δ ppm 7.3 (m, 5 H), 3.9 (quadd, 2 H), 3.7 (s, 2 H), 2.7 (m, 4 H), 2-1.6 (m, 6 H), 1.2 (t, 3 H)
RMN ³¹P : (400 MHz, DMSO-d6) δ ppm 62

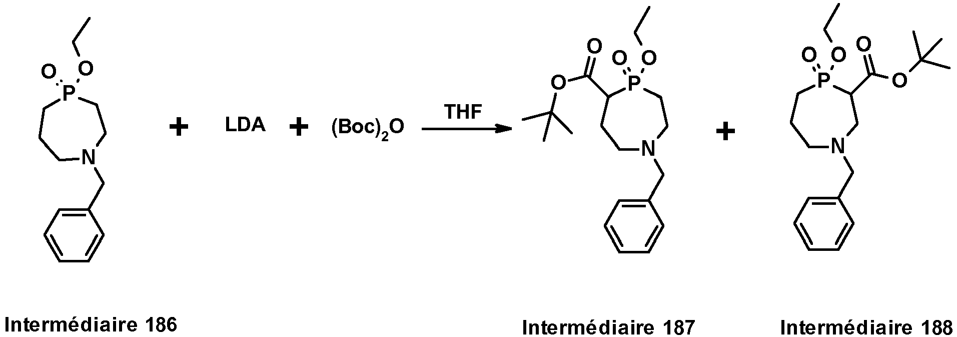

A une solution de l'intermédiaire 186 (1.9 g, 7.1 mmol) dans du THF (18 mL) à -78 °C sous une atmosphère d'Argon est additionné goutte à goutte du LDA 2N dans le THF (5.33 mL, 10.6 mmol, 1.5 eq). Après 30 min, le di*tert*-butyl dicarbonate (2.17 g, 9.95 mmol, 1.4 eq) dissous dans du THF (9 mL) est ajouté. Le milieu réactionnel est agité 1h30 en maintenant la température à -78°C. 1.5 eq de LDA 2N dans le THF (5.33 mL, 10.6 mmol) sont rajoutés goutte à goutte. Après 2h, le milieu réactionnel est hydrolysé à froid avec une solution aqueuse de NH₄Cl (10 mL) suivie par de l'AcOEt (10 mL). Après retour à température ambiante, le milieu réactionnel est extrait avec AcOEt (2 x 100 mL). Les phases organiques sont rassemblées, lavées avec H₂O (100 mL), séchées sur MgSO₄ puis concentrées sous vide. Le résidu obtenu est purifié par flash chromatographie sur gel de silice en utilisant comme éluant un gradient AcOEt/THF (de 100% à 70:30). Les intermédiaires 187 et 188 attendus sont obtenus sous forme d'un mélange huileux (1.7 g, 4.62 mmol) avec un rendement de 63%.

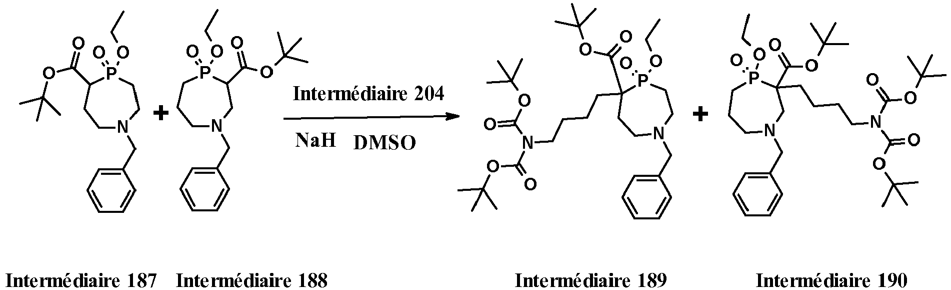

A une solution d'intermédiaire 204 (1.74 g, 4,94 mmol, 1.1 eq) dans du DMSO (10 mL) sous argon, est ajouté à 10°C et par portions NaH à 60% (0.287 g, 7.18 mmol, 1.6 eq). Les intermédiaires 187 et 188 (1.65 g, 4.49 mmol) en solution dans du DMSO (5.9 mL) sont alors additionnés sur la suspension et le mélange est agité pendant 6h à température ambiante. Le milieu réactionnel est alors hydrolysé avec une solution aqueuse de NH₄Cl (30 mL) et extrait avec AcOEt (2 x 50 mL). La phase organique est lavée avec H₂O (2 x 50 mL), séchée sur MgSO₄ et concentrée sous vide. Le résidu obtenu est purifié par flash chromatographie sur gel de silice en utilisant comme éluant un gradient DCM/AcOEt (90:10 à 50:50). L'intermédiaire 189 (0.342 g, 0.54 mmol) et l'intermédiaire 190 (1 g, 1.6 mmol), sont obtenus sous forme d'un mélange de diastéréoisomères avec un rendement respectif de 36% et de 12%.

### EXEMPLE 253 : Acide 5-(4-aminobutyl)-1-benzyl-4-hydroxy-4-oxo-1,4-azaphosphépane-5-carboxylique

A une solution de l'intermédiaire 189 (0.342 g, 0.54 mmol) dans DCM (4 mL) sous Argon et à température ambiante, est additionné goutte à goutte du TMSBr (0.87 mL, 6.6 mmol, 12 eq). Le mélange est agité pendant 16h à température ambiante puis concentré sous vide. Le résidu est repris dans du MeOH (20 mL) et agité 20 min à température ambiante avant d'être évaporé à sec. L'évaporat est mis en solution dans du DCM (4 mL) et de l'acide trifluoroacétique (0.81 mL, 10.9 mmol, 20 eq) est ajouté. Le milieu réactionnel est agité 10h à température ambiante puis concentré sous vide. Le résidu obtenu est purifié par chromatographie en phase inverse en utilisant comme éluant un gradient H₂O/MeCN. L'exemple 253 (0.11 g, 0.31 mmol) est obtenu sous forme d'un solide blanc avec un rendement de 57%.
RMN ¹H : (400 MHz, D2O) δ ppm 7.42 (s, 5 H), 4.32 (AB, 2 H), 3.5-3.2 (m, 4 H), 2.9 (m, 2 H), 2.1-1 (m, 10 H)
RMN ³¹P : (400 MHz, D2O) δ ppm 40.25
ESI/FIA/HR et MS/MS : [M+H]+ = 355.1780 (355.1786)
Analyse Elémentaire : C=57.16(57.62);H=7.28(7.68);N=7.83(7.90)

### EXEMPLE 254 : Acide 3-(4-aminobutyl)-1-benzyl-4-hydroxy-4-oxo-1,4-azaphosphépane-3-carboxylique

A une solution de l'intermédiaire 190 (1 g, 1.61 mmol) en solution dans DCM (5 mL) sous Argon et à température ambiante, est additionné goutte à goutte du TMSBr (2.53 mL, 19.2 mmol, 12 eq). Le mélange est agité pendant 16h à température ambiante puis concentré sous vide. Le résidu est repris dans du MeOH (20 mL) et agité 20 min à température ambiante avant d'être évaporé à sec. L'évaporat est mis en solution dans du DCM (4ml) et de l'acide trifluoroacétique (2.37 mL, 32 mmol, 20 eq) est ajouté. Le milieu réactionnel est agité 10h à température ambiante puis concentré sous vide. Le résidu obtenu est purifié par chromatographie en phase inverse en utilisant comme éluant un gradient H₂O/MeCN. L'exemple 254 (0.34 g, 0.96 mmol) est obtenu sous forme d'un solide blanc avec un rendement de 60%.
RMN ¹H : (400 MHz, D2O) δ ppm 7.5 (m, 5 H), 4.35 (m, 2 H), 3.75-3.1 (m, 4 H), 2.95 (t, 2 H), 2.15-1.4 (m, 4 H), 2.1 (m, 2 H), 1.6 (m, 2 H), 1.35/1.2 (m)+(m, 1+1 H)
ESI/FIA/HR et MS/MS : [M+H]+ = 355.1780 (355.1786)
Analyse Elémentaire : C=57.16(57.62);H=7.28(7.68);N=7.83(7.90)

### EXEMPLE 255 : Acide 5-(5-aminopentyl)-1-benzyl-4-hydroxy-4-oxo-1,4-azaphosphépane-5-carboxylique

L'exemple 255 est obtenu selon la procédure H précédemment décrite en remplaçant l'intermédiaire 204 par l'intermédiaire 206.
RMN ¹H : (500 MHz, D2O) δ ppm 7.54 (m, 5 H), 4.41/4.34 (d)+(d, 1+1 H), 3.7/3.63 (m)+(m, 1+1 H), 3.51/3.22 (m)+(m, 1+1 H), 2.99 (t, 2 H), 2.16/1.58 (m)+(m, 1+1 H), 2.14/1.51 (m)+(m, 1+1 H), 2.11/1.82 (m)+(m, 1+1 H), 1.67 (quint., 2 H), 1.39 (quint., 2 H), 1.35/1.18 (m)+(m, 1+1 H)
RMN ¹³C : (500 MHz, D2O) δ ppm 129.4, 61, 54.7, 51.8, 39.1, 32.3, 29.4, 26, 26, 25.8, 23.
RMN ³¹P : (500 MHz, D2O) δ ppm 37
ESI/FIA/HR et MS/MS : [M+H]+ = 369.1955 (369.1943)
Analyse Elémentaire : C=58.53(58.68);H=7.80(7.93);N=7.51(7.60)

### Intermédiaire 191 : 5-{4-[Bis(tert-butoxycarbonyl)amino]butyl}-4-éthoxy-4-oxo-1-[(3-phénylphényl)méthyl]-1,4-azaphosphépane-5-carboxylate de tert-butyle

L'intermédiaire 191 est obtenu selon la procédure H précédemment décrite en remplaçant le bromure de benzyle par du bromure de 3-phénylbenzyle.
RMN ¹H : (400/500 MHz, dmso-d6) δ ppm 7.65 (d, 2 H), 7.63 (t, 1 H), 7.59 (s, 1 H), 7.53 (d, 1 H), 7.46 (t, 2 H), 7.41 (t, 1 H), 7.31 (d, 1 H), 4.06 (m, 2 H), 3.69/3.63 (2*d, 2 H), 3.44 (t, 2 H), 2.83/2.71 (2*m, 2 H), 2.8/2.67 (2*m, 2 H), 2.21/1.6 (2*m, 2 H), 2.12/1.88 (2*m, 2 H), 1.9/1.67 (2*m, 2 H), 1.42 (m, 2 H), 1.41 (s, 18 H), 1.39 (s, 9 H), 1.22 (t, 3 H), 1.22/0.99 (2*m, 2 H)
RMN ¹³C : (400/500 MHz, dmso-d6) δ ppm 129.1, 128.9, 127.9, 127.6, 127, 126.7, 125.4, 61.5, 60.3, 50.8, 48.2, 45.8, 31.2, 31.2, 29, 28.3, 28, 28, 21.6, 16.6

### EXEMPLE 256 : Acide 5-(4-aminobutyl)-4-hydroxy-4-oxo-1-[(3-phénylphényl)méthyl]-1,4-azaphosphépane-5-carboxylique

L'exemple 256 est obtenu à partir de l'intermédiaire 191 selon la procédure D précédemment décrite.
RMN ¹H : (400 MHz, D2O) δ ppm 7.8-7.4 (m, 9 H), 4.45/4.35 (d, 2 H), 3.7-3.2 (m, 4 H), 2.95 (m, 2 H), 2.2-1.75 (m, 4 H), 1.6-1.2 (m, 6 H)
RMN ³¹P : (400 MHz, D2O) δ ppm 86
ESI/FIA/HR et MS/MS : [M+H]+ = 431.2092 (431.2094)
Analyse Elémentaire : C=63.35(64.17);H=6.85(7.26);N=6.42(6.51)

### Intermédiaire 192 : 3-{4-[Bis(tert-butoxycarbonyl)amino]butyl}-4-éthoxy-4-oxo-1-[(3-phénylphényl)méthyl]-1,4-azaphosphépane-3-carboxylate de tert-butyle

L'intermédiaire 192 est obtenu selon la procédure H précédemment décrite en remplaçant le bromure de benzyle par du bromure de 3-phenylbenzyle.
RMN ¹H : (400 MHz, dmso-d6) δ ppm 7.7 (s, 1 H), 7.66 (d, 2 H), 7.5 (t, 1 H), 7.42 (t, 2 H), 7.35 (m, 2 H), 7.32 (t, 1 H), 4.06 (m, 2 H), 4.01/3.8 (2*d, 2 H), 3.45 (t, 2 H), 3.32/2.76 (dd, 2 H), 2.62-2.45 (m, 2 H), 2-1.3 (m, 8 H), 1.4/1.1 (2*m, 2 H), 1.4 (s, 27 H), 1.25 (t, 3 H)

### EXEMPLE 257 : Acide 3-(4-aminobutyl)-4-hydroxy-4-oxo-1-[(3-phénylphényl)méthyl]-1,4-azaphosphépane-3-carboxylique

L'exemple 257 est obtenu à partir de l'intermédiaire 192 selon la méthode D précédemment décrite.
RMN ¹H : (400 MHz, D2O) δ ppm 7.8-7.4 (m, 9 H), 4.45/4.35 (d, 2 H), 3.6-3.25 (m, 4 H), 2.8 (m, 2 H), 2.1-1.9 (m, 4 H), 1.75-1.1 (m, 6 H)
RMN ³¹P : (400 MHz, D2O) δ ppm 86
ESI/FIA/HR et MS/MS : [M+H]+ = 431.2095 (431.2094)
Analyse Elémentaire : C=63.89(64.17);H=6.92(7.26);N=6.50(6.51)

### Intermédiaire 193 : 3-{4-Bis(tert-butoxycarbonyl)amino]butyl}-4-éthoxy-4-oxo-1-[(4-phénylphényl)méthyl]-1,4-azaphosphépane-3-carboxylate de tert-butyle

L'intermédiaire 193 est obtenu selon la procédure H précédemment décrite en remplaçant le bromure de benzyle par du bromure de 4-phénylbenzyle.
RMN ¹H : (400 MHz, dmso-d6) δ ppm 7.7-7.55 (2d, 4 H), 7.5 (m, 2 H), 7.45 (d, 2 H), 7.35 (m, 1 H), 4.1 (m, 2 H), 3.95 (d, 1 H), 3.8 (d, 1 H), 3.45 (t, 2 H), 3.3 (m, 1 H), 2.8 (m, 1 H), 2.55 (m, 1 H), 2-1.35 (m, 9 H), 1.4 (3s, 27 H), 1.25 (t, 3 H), 1.25 (m, 1 H), 1.1 (m, 1 H)

### EXEMPLE 258 : Acide 3-(4-aminobutyl)-4-hydroxy-4-oxo-1-[(4-phénylphényl)méthyl]-1,4-azaphosphépane-3-carboxylique

L'exemple 258 est obtenu à partir de l'intermédiaire 193 selon la procédure D précédemment décrite.
RMN ¹H : (400 MHz, D2O) δ ppm 7.7 (2d, 4 H), 7.5 (t+d, 4 H), 7.4 (td, 1 H), 4.35 (2d, 2 H), 3.5-3.2 (m, 4 H), 2.85 (m, 2 H), 2.15-1 (m, 10 H)
ESI/FIA/HR et MS/MS : ESI-HR +/- : [M+H]+ = 431.2093 (431.2094)
Analyse Elémentaire : C=63.92(64.17);H=7.15(7.26);N=6.47(6.51)

### Intermédiaire 62 : 5-{4-[Bis(tert-butoxycarbonyl)amino]butyl}-4-éthoxy-4-oxo-1-[(4-phénylphényl)méthyl]-1,4-azaphosphépane-5-carboxylate de tert-butyle

L'intermédiaire 62 est obtenu selon la procédure H précédemment décrite en remplaçant le bromure de benzyle par du bromure de 4-phénylbenzyle.

### EXEMPLE 259 : Acide 5-(4-aminobutyl)-4-hydroxy-4-oxo-1-[(4-phénylphényl)méthyl]-1,4-azaphosphépane-5-carboxylique

L'exemple 259 est obtenu à partir de l'intermédiaire 62 selon la procédure D précédemment décrite.
RMN ¹H : (400 MHz, D2O) δ ppm 7.75 (d, 2 H), 7.7 (d, 2 H), 7.6 (d, 2 H), 7.5 (m, 2 H), 7.45 (m, 1 H), 4.45/4.35 (2d, 2 H), 3.75-3.1 (m, 4 H), 2.95 (t, 2 H), 2.2-1.85 (m, 3 H), 1.8 (m, 1 H), 1.7-1.05 (m, 6 H)
ESI/FIA/HR et MS/MS : EI-HR : [M+H]+ = 431.2093 (431.2094)
Analyse Elémentaire : C=64.46(64.17);H=7.17(7.26);N=6.46(6.51)

### Intermédiaire 194 : 3-{4-[Bis(tert-butoxycarbonyl)amino]butyl}-4-éthoxy-4-oxo-1-[(2-phénylphényl)méthyl]-1,4-azaphosphépane-3-carboxylate de tert-butyle

L'intermédiaire 194 est obtenu selon la procédure H précédemment décrite en remplaçant le bromure de benzyle par du bromure de 2-phénylbenzyle.
RMN ¹H : (400 MHz, dmso-d6) δ ppm 7.88 (d, 1 H), 7.48-7.2 (m, 5 H), 7.35 (m, 2 H), 7.15 (d, 1 H), 4.05 (m, 2 H), 3.9/3.7 (2*d, 2 H), 3.42 (t, 2 H), 3.2/2.65 (dd, 2 H), 2.33 (m, 2 H), 2-1.5 (m, 6 H), 1.42 (s, 18 H), 1.4 (m, 2 H), 1.4 (s, 9 H), 1.25 (t, 3 H), 1.15/1 (2*m, 2 H)

### EXEMPLE 260 : Acide 3-(4-aminobutyl)-4-hydroxy-4-oxo-1-[(2-phénylphényl)méthyl]-1,4-azaphosphépane-3-carboxylique

L'exemple 260 est obtenu à partir de l'intermédiaire 194 selon la procédure D précédemment décrite.
RMN ¹H : (400 MHz, D2O) δ ppm 7.7-7.3 (m, 9 H), 4.45 (d, 1 H), 4.4 (d, 1 H), 3.25-3 (m, 4 H), 2.95 (q, 2 H), 1.95-1.5 (m, 5 H), 1.65 (m, 2 H), 1.35 (m, 1 H), 1.3-1.05 (m, 2 H)
ESI/FIA/HR et MS/MS : [M+H]+ = 431.2094 (431.2094)
Analyse Elémentaire : C=63.81(64.17);H=7.03(7.26);N=6.45(6.51)

### Intermédiaire 67 : 5-{4-[Bis(tert-butoxycarbonyl)amino]butyl}-4-éthoxy-4-oxo-1-[(2-phénylphényl)méthyl]-1,4-azaphosphépane-5-carboxylate de tert-butyle

L'intermédiaire 67 est obtenu selon la procédure H précédemment décrite en remplaçant le bromure de benzyle par du bromure de 2-phénylbenzyle.

### EXEMPLE 261 : Acide 5-(4-aminobutyl)-4-hydroxy-4-oxo-1-[(2-phénylphényl)méthyl]-1,4-azaphosphépane-5-carboxylique

L'exemple 261 est obtenu à partir de l'intermédiaire 67 selon la procédure D précédemment décrite.
RMN ¹H : (300/500 MHz, D2O) δ ppm 7.63/7.61 (m, 1 H), 7.54-7.45 (m, 4 H), 7.44 (m, 1 H), 7.38 (m, 1 H), 7.33/7.31 (m, 2 H), 4.38 (m, 2 H), 3.48-2.77 (m, 4 H), 2.94 (m, 2 H), 2.05/1.42 (m, 2 H), 2.03/1.43 (m, 2 H), 2.01/1.66 (m, 2 H), 1.6 (m, 2 H), 1.33/1.14 (m, 2 H)
RMN ¹³C : (300/500 MHz, D2O) δ ppm 178.5, 143.3, 130.4, 129.4, 129.3, 129.2, 128.7, 127.7, 57.8, 55.1/51.6, 38.8, 31.7, 28.9, 26.3, 25.9, 20.5
RMN ³¹P : (300/500 MHz, D2O) δ ppm 36.5
ESI/FIA/HR et MS/MS : [M+H]+ = 431.2095 (431.2094)
Analyse Elémentaire : C=63.81(64.17);H=7.14(7.26);N=6.45(6.51)

### Préparation de l'exemple 262 :

### Intermédiaire 195

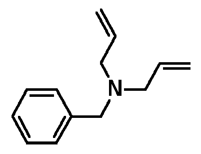

A une solution dégazée à l'Argon pendant 30 min de benzylamine (3 g, 28 mmol) et de palladium acétate (0.31 g, 1.4 mmol) dans du THF (64 mL) sont additionnés successivement l'alcool allylique (15.27 mL, 224 mmol, 8 eq), le triéthylborane (6.4 mL, 6.39 mmol, 0.23 eq) et le tributylphosphore (1.17 mL, 5.6 mmol, 0.2 eq). Le milieu réactionnel est dégazé pendant 15 min avec de l'Argon et chauffé à 70°C pendant 20h. Le milieu est concentré sous pression réduite. Le résidu obtenu est purifié par flash chromatographie sur gel de silice en utilisant un gradient de DCM/AcOEt (de 100% à 95:5) comme éluant. L'intermédiaire 195 (3.45 g, 18.4 mmol) est obtenu sous forme d'une huile avec un rendement de 66 %.
RMN ¹H : (400 MHz, CDCl₃) δ ppm 7.3 (m, 5 H), 5.9 (m, 2 H), 5.15 (m, 4 H), 3.6 (s, 2 H), 3.1 (s, 4 H)

### Intermédiaire 196 :

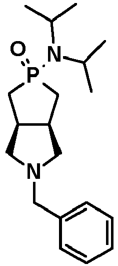

A une suspension de chlorure d'aluminium (3.27 g, 24.6 mmol, 2 eq) dans du DCM (41.5 mL) à -20 °C et sous atmosphère d'Argon, est additionnée une solution de diisopropylamino-phosphordichloride (4.96 g, 24.6 mmol, 2 eq) dans du DCM (41.5 mL). Le mélange est agité 1h à température ambiante puis refroidi à -20°C et l'intermédiaire 195 (2.3 g, 12.3 mmol) en solution dans 10 mL de DCM est alors ajouté. Le milieu réactionnel est agité pendant 16h à température ambiante puis chauffé 1h au reflux. Une solution (25 mL, 1:1) d'EDTA (0.2M dans H₂O) et NaHCO₃ (10% dans H₂O) est alors additionnée à 0°C et le mélange est agité 16h à température ambiante. Le milieu réactionnel est ensuite additionné sur 100 mL de DCM refroidi par un bain glace et basifié avec une solution saturée en Na₂CO₃. La phase organique est décantée et lavée par H₂O (2 x 50 mL), séchée sur MgSO₄ et concentrée sous vide. Le résidu obtenu est purifié par flash chromatographie sur colonne de silice en utilisant comme éluant un gradient AcOEt/THF (de 100% à 95:5). L'intermédiaire 196 (2.3 g, 6.8 mmol) est obtenu sous forme d'une huile avec un rendement de 54%.
RMN ¹H : (400 MHz, dmso-d6) δ ppm 7.3 (m, 5 H), 3.52 (2s, 2 H), 3.33 (m, 2 H), 2.78/2.6 (m, 2 H), 2.62 (m, 2 H), 2.3 (m, 2 H), 1.99/1.72 (m, 2 H), 1.4 (m, 2 H), 1.17 (2d, 12 H) RMN ³¹P : (400 MHz, DMSO-d6) δ ppm 69.2/66.9

### Intermédiaire 197 :

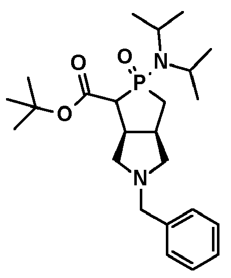

A une solution de l'intermédiaire 196 (2.3 g, 6.87 mmol) dans du THF (16.5 mL) à -70°C et sous une atmosphère d'Argon, est additionnée une solution de LDA 2N dans du THF (4.81 mL, 9.6 mmol, 1.4 eq). Après 15 min d'agitation à -70°C, une solution de (Boc)₂O (2.1 g, 9.6 mmol, 1.4 eq) dans du THF (5 mL) est ajoutée goutte à goutte et l'agitation est maintenue pendant 90 min à -70°C. 1.4 eq de LDA 2N dans du THF (4.81 mL, 9.6 mmol, 1.4 eq) sont alors rajoutés. A la fin de l'addition, le milieu réactionnel est maintenu à -70°C pendant 90 min. Une solution saturée de NH₄Cl (30 mL) ainsi que de l'AcOEt (60 mL) sont alors ajoutés et le milieu réactionnel est remonté lentement à température ambiante. Le mélange est extrait avec de l'AcOEt (2 x 100 mL). Les phases organiques sont rassemblées, séchées puis concentrées sous pression réduite. Le produit obtenu est purifié par flash chromatographie sur gel de silice en utilisant comme éluant un gradient AcOEt/THF (50:50 à 20:80). L'intermédiaire 197 (0.808 g, 1.86 mmol), mélange de diastéréoisomères, est obtenu sous forme d'une huile jaune avec un rendement de 27%.
RMN ¹H : (400 MHz, dmso-d6) δ ppm 7.25 (m, 5 H), 3.55 (m, 2 H), 3.25 (m, 2 H), 2.91 (m, 1 H), 2.72 (m, 2 H), 2.6/2.3 (m, 4 H), 2.15/1.65 (m, 2 H), 1.39 (s, 9 H), 1.15 (m, 12 H) RMN ³¹P : (400 MHz, DMSO-d6) δ ppm 68.58

### Intermédiaire 198 : (3aR*, 4S*, 6aS*)-2-Benzyl-4-{4-[bis(tert-butoxycarbonyl)amino]butyl}-5-[di(propan-2-yl)amino]-5-oxo-octahydrophospholo[3,4-c]pyrrole-4-carboxylate de tert-butyle

A une suspension de NaH à 60% (0.12 g, 2 mmol, 1.1 eq) dans du DMSO (6 mL) est additionnée sous Argon une solution de l'intermédiaire 204 (0.71 g, 2 mmol, 1.1 eq) dans du DMSO (1.5 mL). L'intermédiaire 197 (0.8 g, 1.84 mmol) en solution dans du DMSO (2 mL) est alors additionné et le mélange est agité pendant 15h à température ambiante. Le milieu réactionnel est alors hydrolysé à 0°C avec une solution aqueuse de NH₄Cl (10 mL) et extrait avec DCM (50 mL). La phase organique est lavée avec H₂O (2 x 10 mL), séchée sur MgSO₄ et concentrée sous vide. Le résidu obtenu est purifié par flash chromatographie sur gel de silice en utilisant comme éluant un gradient AcOEt/THF (de 100% à 80:20). L'intermédiaire 198 (0.232 g, 0.33 mmol), mélange de diastéréoisomères, est obtenu sous forme d'une huile avec un rendement de 18%.
RMN ¹H : (400 MHz, dmso-d6) δ ppm 7.3 (m, 4 H), 7.22 (m, 1 H), 3.58/3.43 (d)+(d, 1+1 H), 3.42 (m, 2 H), 3.29 (m, 1 H), 3.1/2.09 (m)+(m, 1+1 H), 2.87 (m, 1 H), 2.82/2.59 (m)+(m, 1+1 H), 2.81 (m, 1 H), 2.05/1.46 (m)+(m, 1+1 H), 1.92/1.65 (m)+(m, 1 + 1 H), 1.45 (m, 2 H), 1.42 (2*(s, 27 H), 1.26/1.09 (m)+(m, 1+1 H), 1.17 (d, 12 H)
RMN ¹³C : (400 MHz, DMSO-d6) δ ppm 128.1, 126.6, 60.8, 59.1, 55.7, 45.5, 45.4, 44.1, 34.3, 29.1, 27.7, 27.3, 26.6, 22.9, 22.3

### EXEMPLE 262 : Acide (3aR*,4S*,6aS*)-4-(4-aminobutyl)-2-benzyl-5-hydroxy-5-oxo-octahydrophospholo[3,4-c]pyrrole-4-carboxylique, trifluoroacétate

L'intermédiaire 198 (0.232 g, 0.328 mmol) et l'acide chlorhydrique 6N (5 mL, 30 mmol) sont portés au reflux pendant 7h. Le milieu réactionnel est concentré sous pression réduite puis lyophilisé. Le résidu est purifié par chromatographie sur gel de silice RP18 en phase inverse en utilisant comme éluant un gradient H₂O/MeCN/TFA. L'exemple 262 (0.040 g, 0.109 mmol) est obtenu sous forme d'un sel de TFA après lyophilisation avec un rendement de 33%.
RMN ¹H : (500 MHz, D₂O+NaOD) δ ppm 7.4-7.25 (m, 5 H), 3.59 (m, 2 H), 3.18/2.07 (m)+(m, 1+1 H), 2.9 (m, 1 H), 2.84/2.37 (m)+(m, 1+1 H), 2.7 (m, 1 H), 2.48 (m, 2 H), 1.85/1.31 (m)+(m, 1+1 H), 1.78/1.22 (m)+(m, 1 + 1 H), 1.31 (m, 2 H), 1.24/1.07 (m)+(m, 1+1 H)
RMN ¹³C : (500 MHz, D₂O+NaOD) δ ppm 127-129, 60.9, 59.2, 55.5, 44.1, 40.1, 33.5, 32.2, 29.3,28,23
ESI/FIA/HR et MS/MS : [M+H]+ = 367.1789 (367.1786)

### Préparation de l'exemple 263

### Intermédiaire 199 :

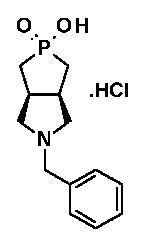

L'intermédiaire 196 (6.15 g, 18.4 mmol) et l'acide chlorhydrique 6N (12.2 mL, 73.2 mmol) sont portés au reflux pendant 6h. Le milieu réactionnel est concentré sous vide, repris par de l'éthanol (50 mL) et concentré sous pression réduite. L'intermédiaire 199 (7.57 g, 26.3 mmol) est utilisé sans autre purification.
RMN ¹H : (400 MHz, dmso-d6) δ ppm 11.2 (si, 1 H), 7.6 (m, 2 H), 7.4 (m, 3 H), 4.3 (d, 2 H), 2.95;2.8 (m, 2*1H H), 3.6;3.3 (m, 2 H), 3.2;2.95 (m, 2 H), 1.85;1.5 (m, 4 H)

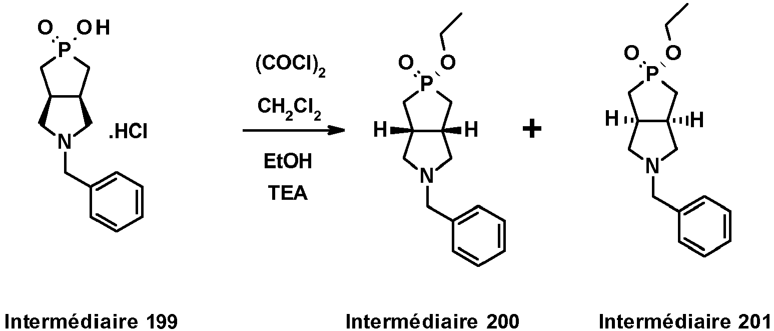

### Intermédiaires 200 et 201 :

A une solution de l'intermédiaire 199 (5.29 g, 18.4 mmol) en solution dans DCM (170 mL) à 0°C et sous atmosphère d'Argon est additionnée goutte à goutte une solution de chlorure d'oxalyle (3.16 mL, 36.8 mmol, 2 eq). Le milieu réactionnel est agité 4 h à température ambiante, évaporé sous vide puis séché sous pression réduite. Le résidu est repris dans du DCM anhydre (150 mL) et de la DMAP (0.0225 g, 0.18 mmol) est alors additionnée. Le mélange est refroidi à -70°C. La TEA (3.1 mL, 22 mmol, 1.2eq) et de l'EtOH (1.3 mL, 22 mmol, 1.2 eq) sont ajoutés successivement. Le mélange est agité 2h à température ambiante, coulé sur une solution aqueuse de NH₄Cl puis basifié avec une solution aqueuse de NaHCO₃. La solution est extraite avec du DCM (150 mL). La phase organique est lavée par H₂O (2 x 50 mL), séchée sur Na₂SO₄ puis concentrée sous vide. Le produit obtenu est purifié par flash chromatographie sur gel de silice en utilisant un gradient DCM/EtOH (de 95:5 à 85:15) comme éluant. Les intermédiaires 200 (0.291 g, 1.04 mmol) et 201 (2.95 g, 10.56 mmol) sont obtenus avec un rendement respectif de 5% et de 57%.

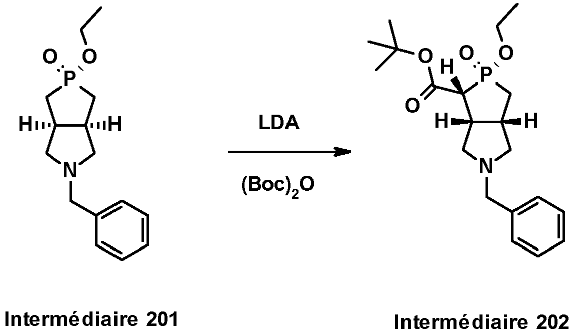

### Intermédiaire 202 :

A une solution de l'intermédiaire 201 (2.95 g, 10.5 mmol) dans du THF (31 mL) à -70°C et sous Argon, est additionnée une solution de LDA 2M dans le THF (14.7 mmol, 7.39 mL, 1.4 eq). Après 15 min à -70°C, une solution de Boc₂O (4.16 g, 14.7 mmol, 1.4 eq) dans 10 mL de THF est alors additionnée goutte à goutte. L'agitation est maintenue pendant 90 min et 1.4 eq de LDA 2M dans le THF (14.7 mmol, 7.39 mL) sont alors additionnés goutte à goutte. A la fin de l'addition, le milieu réactionnel est maintenu à -70°C pendant 90 min. Une solution saturée de NH₄Cl (30 mL) ainsi que de l'AcOEt (60 mL) sont ajoutés et le milieu réactionnel est remonté lentement à température ambiante. Le produit est alors extrait avec AcOEt (2 x 150 mL). Les phases organiques sont rassemblées, lavées avec une solution saturée en NaCl (2 x 150 mL), séchées sur MgSO₄ et concentrées sous pression réduite. Le résidu obtenu est purifié par flash chromatographie sur gel de silice en utilisant un gradient AcOEt/THF (de 100% à 70:30) comme éluant. L'intermédiaire 202 (2.52 g, 6.64 mmol) est obtenu sous forme d'une huile jaunâtre avec un rendement de 63 %.

### Intermédiaire 203 : (3aR*,4R*,6aS*)-2-Benzyl-4-{4-[bis(tert-butoxycarbonyl)amino]-butyl}-5-éthoxy-5-oxo-octahydrophospholo[3,4-c]pyrrole-4-carboxylate de tert-butyle

Dans un ballon tricol de 250 mL sont introduits successivement sous une atmosphère d'argon du DMSO (5 mL) et du NaH à 60% (0.425 g, 10.6 mol, 1.6 eq). Le ballon est maintenu à température ambiante à l'aide d'un bain d'eau. Une solution de l'intermédiaire 204 (2.57 g, 7.3 mol, 1.1 eq) dans du DMSO (7.2 mL) est alors ajoutée goutte à goutte sur une période de 5 minutes. Une solution de l'intermédiaire 202 (2.52 g, 6.64 mmol) dans du DMSO (7.2 mL) est alors additionnée goutte à goutte en maintenant la température en dessous de 20°C. Après 7h, le mélange réactionnel est refroidi à l'aide d'un bain d'eau glacée et hydrolysé par ajout de 5 mL d'une solution saturée de NH₄Cl. Le mélange est ensuite extrait avec du DCM (3 x 50 mL). Les phases organiques sont alors rassemblées, lavées avec une solution saturée de NaCl (2 x 50 mL), et séchées sur MgSO₄ avant d'être concentrées sous pression réduite. Le résidu ainsi obtenu, est purifié par chromatographie sur gel de silice en utilisant un mélange AcOEt/THF (de 100% à 80:20) comme éluant. L'intermédiaire 203 (1.86 g, 2.86 mmol) est obtenu avec un rendement de 43%.
RMN ¹H : (500 MHz, CDCl₃) δ ppm 7.29 (m, 4 H), 7.23 (m, 1 H), 4.15 (m, 2 H), 3.66/3.58 (d)+(d, 1+1 H), 3.58 (m, 2 H), 3.06/2.49 (m)+(m, 1+1 H), 2.99/2.23 (m)+(m, 1+1 H), 2.73 (m, 1 H), 2.69 (m, 1 H), 2.07/1.63 (m)+(m, 1+1 H), 2.02/1.78 (m)+(m, 1+1 H), 1.65-1.5 (m, 2 H), 1.45/1.19 (s)+(s, 18+9 H), 1.4 (m, 2 H), 1.31 (t, 3 H)
RMN ¹³C : (500 MHz, CDCl₃) δ ppm 128.1, 126.6, 61.2, 60.4, 59.4, 56.7, 45.8, 45.1, 33.8, 32.9, 29.2, 27.9, 26.9, 22.1, 16.5
RMN ³¹P : (500 MHz, CDCl₃) δ ppm 74.9

### EXEMPLE 263 : Acide (3aR*,4R*,6aS*)-4-(4-aminobutyl)-2-benzyl-5-hydroxy-5-oxo-octahydrophospholo[3,4-c]pyrrole-4-carboxylique

L'intermédiaire 203 (1.85 g, 2.84 mmol) et le bromure de triméthylsilane (4.5 mL, 34.11 mmol, 12 eq) en solution dans DCM (20 mL) sont agités la nuit à température ambiante puis concentrés sous vide. Le résidu est repris dans du MeOH (20 mL), agité 20min puis concentré sous vide. Le produit est repris dans du DCM (20 mL) et le TFA (4.22 mL, 56.8 mmol, 20 eq) est additionné. Le mélange est agité la nuit à température ambiante. Le milieu réactionnel est alors concentré sous pression réduite et purifié par chromatographie en phase inverse en utilisant un gradient H₂O/CH₃CN comme éluant. L'exemple 263 (0.29 g, 0.79 mmol) est obtenu sous forme d'un lyophilisat avec un rendement de 28%.
RMN ¹H : (500 MHz, D₂O+NaOD) δ ppm 7.4-7.25 (m, 5 H), 3.57 (m, 2 H), 3.08/2.38 (m, 1+1 H), 3.04/2.17 (m)+(m, 1+1 H), 2.52 (t, 2 H), 2.45 (m, 1 H), 2.42 (m, 1 H), 1.73/1.45 (m)+(m, 1+1 H), 1.67 (m, 2 H), 1.35 (m, 2 H), 1.28 (m, 2 H)
RMN ¹³C : (500 MHz, D₂O+NaOD) δ ppm 127-129, 60.5, 59.1, 57.4, 46.1, 40, 33.9, 33.2, 32.4, 28.3, 22.9
C=58.27(59.01);H=7.24(7.43);N=7.58(7.65)
ESI/FIA/HR et MS/MS : [M+H]⁺ = 367.1775 (367.1786)

### ETUDE PHARMACOLOGIQUE

### EXEMPLE 264 : Inhibition de TAFIa (Test Hippuryl-Arg)

Le TAFI humain (4nM) est incubé avec la thrombine humaine (10nM) et la thrombomoduline humaine (5nM) en présence de calcium 10mM. Après 20 minutes d'incubation, la réaction d'activation est stoppée par ajout de PPACK (1µM final), inhibiteur irréversible de la thrombine.
Les réactions se déroulent en tampon Hepes (25mM Hepes ,137mM NaCl, 3.5mM KCl) + 0.1% albumine bovine, pH 7.4 à 28°C et sous agitation.
Le composé à tester est ajouté à la solution de TAFIa (2nM) et incubé pendant 45 minutes en présence de Hippuryl-Arginine (5mM). La réaction est stoppée par ajout d'acide chlorhydrique (1M) neutralisé ensuite avec de l'hydroxyde de sodium (1M) puis le milieu est tamponné par de l'hydroxyphosphate disodique (1M pH 7.4). Le produit de la réaction - l'acide hippurique - est révélé par ajout de chlorure de cyanuryle (6%). Le mélange réactionnel est agité (vortex) puis centrifugé. Le surnageant est transféré dans une microplaque 96 puits, l'absorbance est mesurée au spectrophotométre à 405nm (Spectramax plus, Molecular Devices).

La valeur de DO d'un puits contenant les réactifs sans le TAFI est soustraite de toutes les valeurs de DO mesurées. Le pourcentage d'inhibition du TAFIa à une concentration donnée du composé à tester est déterminé à l'aide de la formule suivante : $% inhibition = 100 - \left[\left(DO composé \times 100\right) / DO véhicule\right]$

Les composés sont évalués à 10nM et 20nM dans les conditions expérimentales décrites ci-dessus et les résultats exprimés en pourcentage d'inhibition par rapport à un contrôle contenant le véhicule en absence de composé.

| **Exemple** | **% inhibition à 10 nM** | **% inhibition à 20 nM** |
|---|---|---|
| 48 | 45 (+/- 6) | 54 (+/- 9) |
| 49 | 60 (+/- 3) | 73 (+/- 3) |
| 50 | 53 (+/- 5) | 66 (+/- 6) |
| 104 | 37 (+/- 13) | 68 (+/- 3) |
| 105 | 44 (+/- 10) | 66 (+/- 10) |
| 108 | 25 (+/- 1) | 29 (+/- 6) |
| 109 | 58 (+/- 10) | 71 (+/- 9) |
| 113 | 68 (+/- 14) | 90 (+/- 9) |
| 114 | 42 (+/- 11) | 51 (+/- 1) |
| 164 | 66 (+/- 18) | 87 (+/- 10) |
| 165 | 76 (+/- 8) | 91 (+/- 4) |
| 166 | 66 (+/- 11) | 93 (+/- 14) |
| 167 | 61 (+/-3) | 67 (+/-1) |
| 174 | 43 (+/-12) | 51 (+/-2) |
| 175 | 55 (+/- 2) | 77 (+/- 1) |
| 179 | 74 | 83 (+/- 3) |
| 180 | 54 (+/- 8) | 73 (+/- 9) |
| 182 | 81 (+/-1) | 95 (+/- 7) |
| 183 | 73 (+/- 8) | 88 (+/- 14) |
| 184 | 67 (+/- 6) | 95 (+/- 1) |
| 185 | 69 (+/- 1) | 85 (+/- 0) |
| 187 | 74 (+/- 14) | 97 (+/- 2) |
| 188 | 64 (+/- 1) | 86 (+/- 14) |
| 189 | 71 (+/- 8) | 78 (+/- 7) |
| 190 | 88 (+/- 12) | 95 (+/- 6) |
| 191 | 66 (+/- 4) | 83 (+/- 4) |
| 192 | 77 (+/- 11) | 91 (+/- 6) |
| 194 | 64 (+/- 2) | 80 (+/-0) |
| 195 | 74 (+/- 4) | 87 (+/- 3) |
| 196 | 77 (+/- 1) | 91 (+/- 1) |
| 197 | 60 (+/- 1) | 78 (+/- 1) |
| 198 | 72 (+/- 6) | 91 (+/- 5) |
| 199 | 54 (+/- 6) | 73 (+/- 2) |
| 200 | 28 (+/- 11) | 46 (+/- 8) |
| 201 | 69 (+/- 5) | 84 (+/- 3) |
| 202 | 74 (+/- 3) | 87 (+/- 2) |
| 203 | 75 (+/- 4) | 89 (+/- 1) |
| 204 | 70 (+/- 7) | 86 (+/- 2) |
| 206 | 67 (+/- 7) | 80 (+/- 5) |
| 207 | 71 (+/- 13) | 85 (+/- 10) |
| 208 | 35 (+/- 2) | 46 (+/- 7) |
| 209 | 76 (+/- 4) | 87 (+/- 2) |
| 210 | 71 | 79 (+/- 9) |
| 211 | 85 (+/- 2) | 95 (+/- 0) |
| 212 | 62 (+/- 6) | 86 (+/- 2) |
| 213 | 83 (+/- 4) | 92 (+/- 1) |
| 214 | 79 (+/- 4) | 91 (+/- 1) |
| 215 | 82 (+/- 4) | 91 (+/-2) |
| 216 | 71 (+/-4) | 85 (+/- 4) |
| 217 | 66 (+/- 10) | 83 (+/- 5) |
| 218 | 67 (+/- 6) | 83 (+/- 6) |
| 219 | 92 (+/- 9) | 98 (+/- 3) |
| 220 | 83 (+/- 3) | 97 (+/- 1) |
| 221 | 75 (+/- 2) | 88 (+/- 3) |
| 222 | 73 (+/- 5) | 85 (+/- 4) |
| 223 | 71 (+/-2) | 83 (+/- 4) |
| 224 | 86 (+/- 3) | 93 (+/- 2) |
| 225 | 76 (+/- 2) | 85 (+/- 3) |
| 226 | 71 (+/-3) | 88 (+/- 3) |
| 227 | 84 (+/- 7) | 91 (+/- 13) |
| 228 | 63 (+/- 4) | 82 (+/- 1) |
| 229 | 81 | 89 |
| 232 | 80 (+/- 10) | 89 (+/- 19) |
| 234 | 68 (+/- 8) | 79 (+/- 1) |
| 235 | 60 (+/- 3) | 77 (+/- 5) |
| 236 | 81 (+/- 4) | 91 (+/- 6) |
| 237 | 77 (+/- 5) | 84 (+/- 4) |
| 238 | 70 (+/- 7) | 87 (+/- 0) |
| 239 | 85 (+/- 3) | 96 (+/-4) |
| 240 | 66 (+/- 1) | 80 (+/- 5) |
| 241 | 77 (+/- 11) | 91 (+/-9) |
| 242 | 75 (+/- 2) | 88 (+/- 1) |
| 244 | 49 (+/- 1) | 68 (+/- 6) |
| 245 | 38 (+/- 2) | 52 (+/- 5) |
| 246 | 72 (+/- 4) | 86 (+/- 1) |
| 247 | 85 (+/- 16) | 89 (+/- 11) |
| 248 | 87 (+/- 14) | 95 (+/- 6) |
| 249 | 88 (+/- 6) | 94 (+/- 4) |
| 250 | 88 (+/- 1) | 97 (+/- 1) |
| 251 | 70 (+/- 14) | 90 (+/- 11) |
| 252 | 76 (+/- 7) | 90 (+/- 2) |

### EXEMPLE 265 : Composition pharmaceutique -comprimé

Formule de préparation pour 1000 comprimés dosés à 10 mg :

| | |
|---|---|
| Composé de l'un des Exemples 1 à 263 | 10 g |
| Hydroxypropylcellulose | 2 g |
| Amidon de blé | 10 g |
| Lactose | 100 g |
| Stéarate de magnésium | 3 g |
| Talc | 3 g |

### EXEMPLE 266 : Composition pharmaceutique -comprimé en association avec la warfarine

Formule de préparation pour 1000 comprimés dosés à 10 mg :

| | |
|---|---|
| Composé de l'un des Exemples 1 à 263 | 10 g |
| Warfarine | 2 g |
| Hydroxypropylcellulose | 2 g |
| Amidon de blé | 10 g |
| Lactose | 100 g |
| Stéarate de magnésium | 3 g |
| Talc | 3 g |

### EXEMPLE 267 : Composition pharmaceutique -comprimé en association avec l'aspirine

Formule de préparation pour 1000 comprimés dosés à 10 mg :

| | |
|---|---|
| Composé de l'un des Exemples 1 à 263 | 10 g |
| Aspirine | 100 g |
| Hydroxypropylcellulose | 2 g |
| Amidon de blé | 10 g |
| Lactose | 100 g |
| Stéarate de magnésium | 3 g |
| Talc | 3 g |

### EXEMPLE 268 : Solution injectable

Formule de préparation pour 10 ml de solution :

| | |
|---|---|
| Composé de l'un des Exemples 1 à 263 | 200 mg |
| Préparation injectable de NaCl 0.9% | 10 ml |

## Revendications

1. Composé de formule (I) : 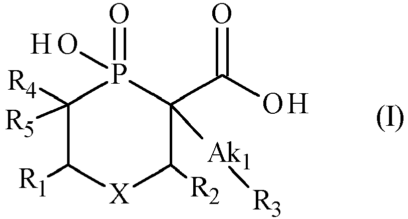 dans laquelle :
Ak₁ représente une chaîne alkyle en C₁-C₆,
X représente -(CH₂)ₘ-, -CH(R)-, -N(R)-, -CH₂-N(R)-, -N(R)-CH₂- ou -CH₂-N(R)-CH₂-,
m représente 0 ou un entier de 1 à 4,
R représente un atome d'hydrogène ou un groupe choisi parmi alkyle C₁-C₆, -Ak₂-Ar₁,-Ak₂-Ar₁-Ar₂ et -Ak₂-Ar₁-O-Ar₂, -Ak₂-cycloalkyle ou -Ak₂-OH,
Ak₂ représente une chaîne alkyle linéaire ou ramifiée en C₁-C₆,
Ar₁ et Ar₂, identiques ou différents, représentent chacun un groupement aryle ou hétéroaryle,
R₁ et R₂ représentent chacun un atome d'hydrogène lorsque X représente -(CH₂)ₘ-,-CH(R)-, -N(R)-, -CH₂-N(R)- ou -N(R)-CH₂-,
ou bien forment ensemble une liaison lorsque X représente -CH₂-N(R)-CH₂-,
R₃ représente NH₂, Cy-NH₂, Cy-Ak₃-NH₂ ou pipéridin-4-yle,
Cy représente un groupement choisi parmi cycloalkyle, aryle et hétéroaryle,
Ak₃ représente une chaîne alkyle en C₁-C₃,
R₄ et R₅, identiques ou différents, représentent chacun un atome d'hydrogène ou un atome de fluor,
ses isomères optiques, ainsi que ses sels d'addition à un acide pharmaceutiquement acceptable.

2. Composé de formule (I) selon la revendication 1, dans laquelle R₁, R₂, R₄ et R₅ représentent chacun un atome d'hydrogène, R₃ représente NH₂, X représente -N(R)-,-CH₂-N(R)-, -N(R)-CH₂- ou -CH₂-N(R)-CH₂-, et R représente un groupe choisi parmi-Ak₂-Ar₁, -Ak₂-Ar₁-Ar₂ et -Ak₂-Ar₁-O-Ar₂, où Ak₂, Ar₁ et Ar₂ sont tels que définis dans la revendication 1.

3. Composé de formule (la), cas particulier des composés de formule (I) selon la revendication 1 : 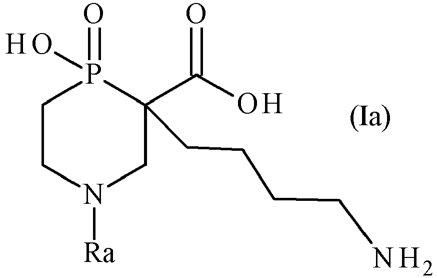 dans laquelle Ra représente un groupe choisi parmi -CH₂-Ar₁ et -CH₂-Ar₁-Ar₂, où Ar₁ et Ar₂ sont tels que définis dans la revendication 1.

4. Composé de formule (I) selon la revendication 1, qui est l'acide (36)-3-(4-aminobutyl)-1-[[2-(3,4-diméthoxyphényl)-4-fluorophényl]méthyl]-4-hydroxy-4-oxo-1,4-azaphosphinane-3-carboxylique, ainsi que ses isomères optiques et ses sels d'addition à un acide pharmaceutiquement acceptable.

5. Composé de formule (I) selon la revendication 1, qui est l'acide (3S)-3-(4-aminobutyl)-1-[[4-fluoro-2-(4-méthylphényl)phényl]méthyl]-4-hydroxy-4-oxo-1,4-azaphosphinane-3-carboxylique, ainsi que ses isomères optiques et ses sels d'addition à un acide pharmaceutiquement acceptable.

6. Composé de formule (I) selon la revendication 1, qui est l'acide (36)-3-(4-aminobutyl)-1-[[4-fluoro-2-(4-méthoxyphényl)phényl]méthyl]-4-hydroxy-4-oxo-1,4-azaphosphinane-3-carboxylique, ainsi que ses isomères optiques et ses sels d'addition à un acide pharmaceutiquement acceptable.

7. Composé de formule (I) selon la revendication 1, qui est l'acide (3*S*)-3-(4-aminobutyl)-1-[[4-fluoro-2-(4-fluorophényl)phényl]méthyl]-4-hydroxy-4-oxo-1,4-azaphosphinane-3-carboxylique, ainsi que ses isomères optiques et ses sels d'addition à un acide pharmaceutiquement acceptable.

8. Composé de formule (I) selon la revendication 1, qui est l'acide (3*S*)-3-(4-aminobutyl)-4-hydroxy-1-[(4-hydroxy-2-phénylphényl)méthyl]-4-oxo-1,4-azaphosphinane-3-carboxylique, ainsi que ses isomères optiques et ses sels d'addition à un acide pharmaceutiquement acceptable.

9. Composé de formule (I) selon la revendication 1, qui est l'acide (3*S*)-3-(4-aminobutyl)-4-hydroxy-1-[2-(6-méthoxypyridin-3-yl)benzyl]-4-oxo-1,4-azaphosphinane-3-carboxylique, ainsi que ses isomères optiques et ses sels d'addition à un acide pharmaceutiquement acceptable.

10. Composé de formule (I) selon la revendication 1, qui est l'acide (3*S*)-3-(4-aminobutyl)-1-[[2-(4-chlorophényl)-4-fluorophényl]méthyl]-4-hydroxy-4-oxo-1,4-azaphosphinane-3-carboxylique, ainsi que ses isomères optiques et ses sels d'addition à un acide pharmaceutiquement acceptable.

11. Composé de formule (I) selon la revendication 1, qui est l'acide (3*S*)-3-(4-aminobutyl)-1-[4-fluoro-2-(1-méthyl-1*H*-imidazol-5-yl)benzyl]-4-hydroxy-4-oxo-1,4-azaphosphinane-3-carboxylique, ainsi que ses isomères optiques et ses sels d'addition à un acide pharmaceutiquement acceptable.

12. Composé de formule (I) selon la revendication 1, qui est l'acide (3*S*)-3-(4-aminobutyl)-1-[2-(1,2-diméthyl-1*H*-imidazol-5-yl)-4-fluorobenzyl]-4-hydroxy-4-oxo-1,4-azaphosphinane-3-carboxylique, ainsi que ses isomères optiques et ses sels d'addition à un acide pharmaceutiquement acceptable.

13. Composé de formule (I) selon la revendication 1, qui est l'acide (3*S*)-3-(4-aminobutyl)-4-hydroxy-1-[[4-hydroxy-2-(4-méthylphényl)phényl]méthyl]-4-oxo-1,4-azaphosphinane-3-carboxylique, ainsi que ses isomères optiques et ses sels d'addition à un acide pharmaceutiquement acceptable.

14. Composé de formule (I) selon la revendication 1, qui est l'acide (3*S*)-3-(4-aminobutyl)-4-hydroxy-1-[4-hydroxy-2-(imidazo[1,2-*a*]pyridin-3-yl)benzyl]-4-oxo-1,4-azaphosphinane-3-carboxylique, ainsi que ses isomères optiques et ses sels d'addition à un acide pharmaceutiquement acceptable.

15. Composition pharmaceutique contenant un composé de formule (I) selon l'une quelconque des revendications 1 à 14, en combinaison avec un ou plusieurs excipients ou véhicules inertes, non toxiques, pharmaceutiquement acceptables.

16. Composition pharmaceutique selon la revendication 15, **caractérisée en ce qu'**elle contient en outre un fibrinolytique, un anticoagulant ou un antiplaquettaire.

17. Composé selon l'une quelconque des revendications 1 à 14 destiné à être utilisé en tant qu'inhibiteur de TAFIa.

18. Composé selon l'une quelconque des revendications 1 à 14 destiné à être utilisé dans le traitement, la prévention ou la prévention secondaire des accidents vasculaires cérébraux, de l'infarctus du myocarde, de l'angine de poitrine, de l'artérite des membres inférieurs, des thromboses, notamment les thromboses veineuses, de l'embolie pulmonaire, des anévrismes aortiques ou des démences.

19. Composé selon l'une quelconque des revendications 1 à 14 destiné à être utilisé selon la revendication 17 ou 18, en association avec un fibrinolytique, un anticoagulant ou un antiplaquettaire.

20. Composé selon l'une quelconque des revendications 1 à 14 destiné à être utilisé selon la revendication 19, en association avec un fibrinolytique injectable choisi parmi l'altéplase et la tenectéplase.

## Patentansprüche

1. Verbindung der Formel (I): 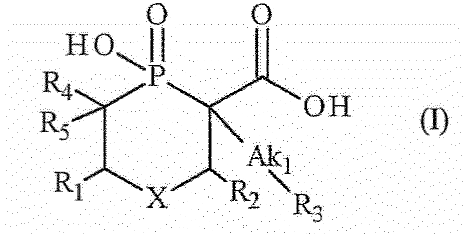 in der:
Ak₁ eine C₁-C₆-Alkylkette bedeutet,
X -(CH₂)ₘ-, -CH(R)-, -N(R)-, -CH₂-N(R)-, -N(R)-CH₂- oder -CH₂-N(R)-CH₂-bedeutet,
m 0 oder eine ganze Zahl mit einem Wert von 1 bis 4 bedeutet,
R ein Wasserstoffatom oder eine Gruppe ausgewählt aus C₁-C₆-Alkyl, -Ak₂-Ar₁, -Ak₂-Ar₁-Ar₂ und -Ak₂-Ar₁-O-Ar₂, -Ak₂-Cycloalkyl oder -Ak₂-OH bedeutet,
Ak₂ eine geradkettige oder verzweigte C₁-C₆-Alkylkette bedeutet,
Ar₁ und Ar₂, die gleichartig oder verschieden sind, jeweils eine Aryl- oder Heteroarylgruppe bedeuten,
R₁ und R₂ jeweils ein Wasserstoffatom darstellen, wenn X -(CH₂)ₘ-, -CH(R)-, -N(R)-, -CH₂-N(R)- oder -N(R)-CH₂- bedeutet,
oder gemeinsam eine Bindung bilden, wenn X -CH₂-N(R)-CH₂- bedeutet,
R₃ NH₂, Cy-NH₂, Cy-Ak₃-NH₂ oder Piperidin-4-yl bedetuet,
Cy eine Gruppe ausgewählt aus Cycloalkyl, Aryl und Heteroaryl bedeutet,
Ak₃ eine C₁-C₃-Alkylkette bedeutet,
R₄ und R₅, die gleichartig oder verschieden sind, jeweils ein Wasserstoffatom oder ein Fluoratom bedeuten,
ihre optischen Isomeren sowie ihre Additionssalze mit einer pharmazeutisch annehmbaren Säure.

2. Verbindung der Formel (I) nach Anspruch 1, worin R₁, R₂, R₄ und R₅ jeweils ein Wasserstoffatom bedeuten, R₃ NH₂ bedeutet, X -N(R)-, -CH₂-N(R)-, -N(R)-CH₂- oder -CH₂N(R)-CH₂- bedeutet und R eine Gruppe bedeutet ausgewählt aus -Ak₂-Ar₁, -Ak₂-Ar₁-Ar₂ und -Ak₂-Ar₁-O-Ar₂, worin Ak₂, Ar₁ und Ar₂ die in Anspruch 1 angegebenen Bedeutungen besitzen.

3. Verbindung der Formel (Ia), Sonderfall der Verbindungen der Formel (I) nach Anspruch 1: 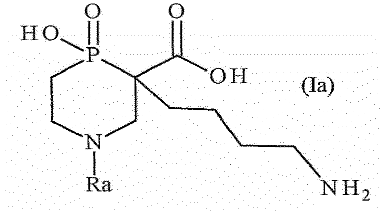 in der Ra eine Gruppe ausgewählt aus -CH₂-Ar₁ und -CH₂-Ar₁-Ar₂ bedeutet, worin Ar₁ und Ar₂ die in Anspruch 1 angegebenen Bedeutungen besitzen.

4. Verbindung der Formel (I) nach Anspruch 1, nämlich (3*S*)-3-(4-Aminobutyl)-1-[[2-(3,4-dimethoxyphenyl)-4-fluorphenyl]-methyl]-4-hydroxy-4-oxo-1,4-azaphosphinan-3-carbonsäure sowie ihre optischen Isomeren und ihre Additionssalze mit einer pharmazeutisch annehmbaren Säure.

5. Verbindung der Formel (I) nach Anspruch 1, nämlich (3*S*)-3-(4-Aminobutyl)-1-[[4-fluor-2-(4-methylphenyl)-phenyl]-methyl]-4-hydroxy-4-oxo-1,4-azaphosphinan-3-carbonsäure sowie ihre optischen Isomeren und ihre Additionssalze mit einer pharmazeutisch annehmbaren Säure.

6. Verbindung der Formel (I) nach Anspruch 1, nämlich (3*S*)-3-(4-Aminobutyl)-1-[[4-fluor-2-(4-methoxyphenyl)-phenyl]-methyl]-4-hydroxy-4-oxo-1,4-azaphosphinan-3-carbonsäure sowie ihre optischen Isomeren und ihre Additionssalze mit einer pharmazeutisch annehmbaren Säure.

7. Verbindung der Formel (I) nach Anspruch 1, nämlich (3*S*)-3-(4-Aminobutyl)-1-[[4-fluor-2-(4-fluorphenyl)-phenyl]-methyl]-4-hydroxy-4-oxo-1,4-azaphosphinan-3-carbonsäure sowie ihre optischen Isomeren und ihre Additionssalze mit einer pharmazeutisch annehmbaren Säure.

8. Verbindung der Formel (I) nach Anspruch 1, nämlich (3*S*)-3-(4-Aminobutyl)-4-hydroxy-1-[(4-hydroxy-2-phenylphenyl)-methyl]-4-oxo-1,4-azaphosphinan-3-carbonsäure sowie ihre optischen Isomeren und ihre Additionssalze mit einer pharmazeutisch annehmbaren Säure.

9. Verbindung der Formel (I) nach Anspruch 1, nämlich (3*S*)-3-(4-Aminobutyl)-4-hydroxy-1-[2-(6-methoxypyridin-3-yl)-benzyl]-4-oxo-1,4-azaphosphinan-3-carbonsäure sowie ihre optischen Isomeren und ihre Additionssalze mit einer pharmazeutisch annehmbaren Säure.

10. Verbindung der Formel (I) nach Anspruch 1, nämlich (3*S*)-3-(4-Aminobutyl)-1-[[2-(4-chlorphenyl)-4-fluorphenyl]-methyl]-4-hydroxy-4-oxo-1,4-azaphosphinan-3-carbonsäure sowie ihre optischen Isomeren und ihre Additionssalze mit einer pharmazeutisch annehmbaren Säure.

11. Verbindung der Formel (I) nach Anspruch 1, nämlich (3*S*)-3-(4-Aminobutyl)-1-[4-fluor-2-(1-methyl-1*H*-imidazol-5-yl)-benzyl]-4-hydroxy-4-oxo-1,4-azaphosphinan-3-carbonsäure sowie ihre optischen Isomeren und ihre Additionssalze mit einer pharmazeutisch annehmbaren Säure.

12. Verbindung der Formel (I) nach Anspruch 1, nämlich (3*S*)-3-(4-Aminobutyl)-1-[2-(1,2-dimethyl-1*H*-imidazol-5-yl)-4-fluorbenzyl]-4-hydroxy-4-oxo-1,4-azaphosphinan-3-carbonsäure sowie ihre optischen Isomeren und ihre Additionssalze mit einer pharmazeutisch annehmbaren Säure.

13. Verbindung der Formel (I) nach Anspruch 1, nämlich (3*S*)-3-(4-Aminobutyl)-4-hydroxy-1-[[4-hydroxy-2-(4-methylphenyl)-phenyl]-methyl]-4-oxo-1,4-azaphosphinan-3-carbonsäure sowie ihre optischen Isomeren und ihre Additionssalze mit einer pharmazeutisch annehmbaren Säure.

14. Verbindung der Formel (I) nach Anspruch 1, nämlich (3*S*)-3-(4-Aminobutyl)-4-hydroxy-1-[4-hydroxy-2-(imidazo[1,2-*a*]pyridin-3-yl)-benzyl]-4-oxo-1,4-azaphosphinan-3-carbonsäure sowie ihre optischen Isomeren und ihre Additionssalze mit einer pharmazeutisch annehmbaren Säure.

15. Pharmazeutische Zubereitung enthaltend eine Verbindung der Formel (I) nach einem der Ansprüche 1 bi 14 in Kombination mit einem oder mehreren inerten, nichttoxischen und pharmazeutisch annehmbaren Hilfsstoffen oder Trägermaterialien.

16. Pharmazeutische Zubereitung nach Anspruch 15, **dadurch gekennzeichnet, dass** sie zusätzlich ein Fibrinolytikum, ein Antikoagulans oder Antiplättchenmittel enthält.

17. Verbindung nach einem der Ansprüche 1 bis 14 zur Verwendung als TAFIa-Inhibitor.

18. Verbindung nach einem der Ansprüche 1 bis 14 zur Verwendung bei der Behandlung, der Vorbeugung oder sekundären Vorbeugung von Schlaganfällen, Myokardinfarkt, Angina pectoris, Artheritis der unteren Gliedmaßen, Thrombosen, insbesondere venösen Thrombosen, Lungenemobilie, Aoartenaneurysmen oder Demenzen.

19. Verbindung nach einem der Ansprüche 1 bis 14 zur Verwendung gemäß Anspruch 17 oder 18 in Kombination mit einem Fibrinolytikum, einem Antikoagulans oder einem Antiplättchenmittel.

20. Verbindung nach einem der Ansprüche 1 bis 14 zur Verwendung gemäß Anspruch 19 in Kombination mit einem injizierbaren Fibrinolyticum ausgewählt aus Alteplase und Tenecteplase.

## Claims

1. Compound of formula (I): 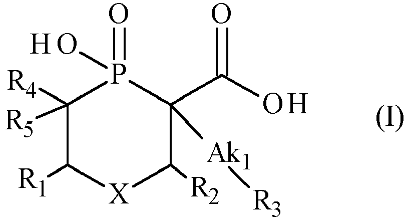 wherein:
Ak₁ represents a C₁-C₆-alkyl chain,
X represents -(CH₂)ₘ-, -CH(R)-, -N(R)-, -CH₂-N(R)-, -N(R)-CH₂- or -CH₂-N(R)-CH₂-,
m represents 0 or an integer from 1 to 4,
R represents a hydrogen atom or a group selected from C₁-C₆-alkyl, -Ak₂-Ar₁, -Ak₂-Ar₁-Ar₂ and -Ak₂-Ar₁-O-Ar₂, -Ak₂-cycloalkyl or -Ak₂-OH,
Ak₂ represents a linear or branched C₁-C₆-alkyl chain,
Ar₁ and Ar₂, which may be identical or different, each represent an aryl or heteroaryl group,
R₁ and R₂ each represent a hydrogen atom when X represents -(CH₂)ₘ-, -CH(R)-, -N(R)-, -CH₂-N(R)- or -N(R)-CH₂-,
or together form a bond when X represents -CH₂-N(R)-CH₂-,
R₃ represents NH₂, Cy-NH₂, Cy-Ak₃-NH₂ or piperidin-4-yl,
Cy represents a group selected from cycloalkyl, aryl and heteroaryl,
Ak₃ represents a C₁-C₃-alkyl chain,
R₄ and R₅, which may be identical or different, each represent a hydrogen atom or a fluorine atom,
its optical isomers, and addition salts thereof with a pharmaceutically acceptable acid.

2. Compound of formula (I) according to claim 1, wherein R₁, R₂, R₄ and R₅ each represent a hydrogen atom, R₃ represents NH₂, X represents -N(R)-, -CH₂-N(R)-, -N(R)-CH₂- or -CH₂-N(R)-CH₂-, and R represents a group selected from -Ak₂-Ar₁, -Ak₂-Ar₁-Ar₂ and -Ak₂-Ar₁-O-Ar₂, wherein Ak₂, Ar₁ and Ar₂ are as defined in claim 1.

3. Compound of formula (Ia), a particular case of the compounds of formula (I) according to claim 1: 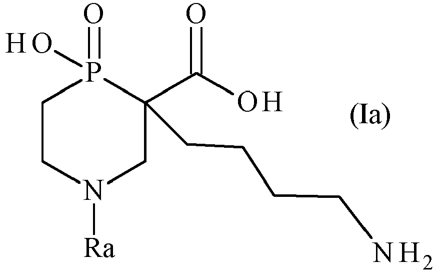 wherein Ra represents a group selected from -CH₂-Ar₁ and -CH₂-Ar₁-Ar₂, wherein Ar₁ and Ar₂ are as defined in claim 1.

4. Compound of formula (I) according to claim 1, which is (3*S*)-3-(4-aminobutyl)-1-[[2-(3,4-dimethoxyphenyl)-4-fluorophenyl]methyl]-4-hydroxy-4-oxo-1,4-azaphosphinane-3-carboxylic acid, and its optical isomers, and addition salts thereof with a pharmaceutically acceptable acid.

5. Compound of formula (I) according to claim 1, which is (3*S*)-3-(4-aminobutyl)-1-[[4-fluoro-2-(4-methylphenyl)phenyl]methyl]-4-hydroxy-4-oxo-1,4-azaphosphinane-3-carboxylic acid, and its optical isomers, and addition salts thereof with a pharmaceutically acceptable acid.

6. Compound of formula (I) according to claim 1, which is (3*S*)-3-(4-aminobutyl)-1-[[4-fluoro-2-(4-methoxyphenyl)phenyl]methyl]-4-hydroxy-4-oxo-1,4-azaphosphinane-3-carboxylic acid, and its optical isomers, and addition salts thereof with a pharmaceutically acceptable acid.

7. Compound of formula (I) according to claim 1, which is (3*S*)-3-(4-aminobutyl)-1-[[4-fluoro-2-(4-fluorophenyl)phenyl]methyl]-4-hydroxy-4-oxo-1,4-azaphosphinane-3-carboxylic acid, and its optical isomers, and addition salts thereof with a pharmaceutically acceptable acid.

8. Compound of formula (I) according to claim 1, which is (3*S*)-3-(4-aminobutyl)-4-hydroxy-1-[(4-hydroxy-2-phenylphenyl)methyl]-4-oxo-1,4-azaphosphinane-3-carboxylic acid, and its optical isomers, and addition salts thereof with a pharmaceutically acceptable acid.

9. Compound of formula (I) according to claim 1, which is (3*S*)-3-(4-aminobutyl)-4-hydroxy-1-[2-(6-methoxypyridin-3-yl)benzyl]-4-oxo-1,4-azaphosphinane-3-carboxylic acid, and its optical isomers, and addition salts thereof with a pharmaceutically acceptable acid.

10. Compound of formula (I) according to claim 1, which is (3*S*)-3-(4-aminobutyl)-1-[[2-(4-chlorophenyl)-4-fluorophenyl]methyl]-4-hydroxy-4-oxo-1,4-azaphosphinane-3-carboxylic acid, and its optical isomers, and addition salts thereof with a pharmaceutically acceptable acid.

11. Compound of formula (I) according to claim 1, which is (3*S*)-3-(4-aminobutyl)-1-[4-fluoro-2-(1-methyl-1*H*-imidazol-5-yl)benzyl]-4-hydroxy-4-oxo-1,4-azaphosphinane-3-carboxylic acid, and its optical isomers, and addition salts thereof with a pharmaceutically acceptable acid.

12. Compound of formula (I) according to claim 1, which is (3*S*)-3-(4-aminobutyl)-1-[2-(1,2-dimethyl-1*H-*dazol-5-yl)-4-fluorobenzyl]-4-hydroxy-4-oxo-1,4-azaphosphinane-3-carboxylic acid, and its optical isomers, and addition salts thereof with a pharmaceutically acceptable acid.

13. Compound of formula (I) according to claim 1, which is (3*S*)-3-(4-aminobutyl)-4-hydroxy-1-[[4-hydroxy-2-(4-methylphenyl)phenyl]methyl]-4-oxo-1,4-azaphosphinane-3-carboxylic acid, and its optical isomers, and addition salts thereof with a pharmaceutically acceptable acid.

14. Compound of formula (I) according to claim 1, which is (3*S*)-3-(4-aminobutyl)-4-hydroxy-1-[4-hydroxy-2-(imidazo[1,2-*a*]pyridin-3-yl)benzyl]-4-oxo-1,4-azaphosphinane-3-carboxylic acid, and its optical isomers, and addition salts thereof with a pharmaceutically acceptable acid.

15. Pharmaceutical composition comprising a compound of formula (I) according to any one of claims 1 to 14, in combination with one or more inert, non-toxic, pharmaceutically acceptable excipients or carriers.

16. Pharmaceutical composition according to claim 15, **characterised in that** it further comprises a fibrinolytic, an anticoagulant or an anti-platelet agent.

17. Compound according to any one of claims 1 to 14 for use as a TAFIa inhibitor.

18. Compound according to any one of claims 1 to 14 for use in the treatment, prevention or secondary prevention of cerebrovascular accidents, myocardial infarction, angina pectoris, arteritis of the lower limbs, thromboses, especially venous thromboses, pulmonary embolism, aortic aneurysms or dementias.

19. Compound according to any one of claims 1 to 14 for use according to claim 17 or 18, in association with a fibrinolytic, an anticoagulant or an anti-platelet agent.

20. Compound according to any one of claims 1 to 14 for use according to claim 19, in association with an injectable fibrinolytic selected from alteplase and tenecteplase.
